# EUROPEAN PATENT APPLICATION

(11) **EP 4 112 730 A1**
(43) Date of publication of application: **04.01.2023**
(21) Application number: 21761538.4
(22) Date of filing: 01.03.2021
(51) Int. Cl.: C12N 15/62, A61K 35/15, A61K 38/19, A61P 35/00, A61P 37/06, A61P 37/08, C07K 14/52, C07K 14/74, C07K 19/00, C12N 5/0783, C12N 5/10, C12N 15/12, C12N 15/19, C12N 15/63

(54) **IMMUNOREGULATORY METHOD, NUCLEIC ACID COMPOSITION FOR IMMUNOREGULATION AND USE THEREOF**

(30) Priority: 28.02.2020 JP 2020033331
(71) Applicant: National University Corporation Kanazawa University, Ishikawa 920-1192 (JP); Nissan Chemical Corporation, Tokyo 103-6119 (JP)
(72) Inventor: HANAYAMA Rikinari, Kanazawa-shi, Ishikawa 920-1192 (JP); YAMANO Tomoyoshi, Kanazawa-shi, Ishikawa 920-1192 (JP); MATOBA Kazutaka, Shiraoka-shi, Saitama 349-0294 (JP)
(74) Representative: dompatent von Kreisler Selting Werner - Partnerschaft von Patent- und Rechtsanwälten mbB
(86) International application number: PCT/JP2021/007779
(87) International publication number: WO 2021/172596

(57) **Abstract**

To provide a polynucleotide that makes it possible to produce extracellular vesicles capable of satisfactorily activating, etc., antigen-specific T cells. Provided is a polynucleotide including at least one sequence selected from the group consisting of (a) a sequence encoding a fusion protein (A) including an antigen-presenting MHC molecule and capable of presenting the antigen-presenting MHC molecule extramembranously of an extracellular vesicle; (b) a sequence encoding a fusion protein (B) including at least one T cell stimulating cytokine or a subunit thereof and capable of presenting the T cell stimulating cytokine extramembranously of an extracellular vesicle; (c) a sequence encoding a fusion protein (C) including a T cell co-stimulatory molecule and capable of presenting the T cell co-stimulatory molecule extramembranously of an extracellular vesicle; (d) a sequence encoding a fusion protein (D) including an antigen-presenting MHC molecule and at least one T cell stimulating cytokine or a subunit thereof and capable of presenting the antigen and the T cell stimulating cytokine extramembranously of an extracellular vesicle; (e) a sequence encoding a fusion protein (E) including an antigen-presenting MHC molecule, at least one T cell stimulating cytokine or a subunit thereof, and a T cell co-stimulatory molecule and capable of presenting the antigen, the T cell stimulating cytokine, and the T cell co-stimulatory molecule extramembranously of an extracellular vesicle.

## Description

### Technical Field

The present invention relates to an immunoregulatory method, a nucleic acid composition for immunoregulation, and a use thereof.

### Background Art

It is known that antigen-specific T cells (for example, cytotoxic T cells, helper T cells, and the like) play a central role in an immune reaction such as elimination of cancer cells and the like by living bodies or regulation of responses to auto-antigens, allergic substances, and the like. The antigen-specific T cells recognize a binding complex of MHC molecules on cell surfaces of antigen-presenting cells such as dendritic cells or macrophages, and antigens derived from cancer, allergic substances, and the like, at a T cell receptor, and activate, proliferate, and differentiate. The activated antigen-specific T cells specifically injure cancer cells and the like presenting antigens, and regulate responses to auto-antigens, allergic substances, and the like. Therefore, it is considered that activation, proliferation, and differentiation of the antigen-specific T cells are particularly important in the immune reaction.

As a method for activating the antigen-specific T cells, not only a method for expressing a chimeric antigen receptor in T cells that has already been put into practical use, but also other methods have been developed. For example, Patent Literature 1 discloses that nanoparticles containing MHC molecules and T-cell costimulatory molecules on surfaces thereof proliferate antigen-specific T cells. In addition, Non Patent Literature 1 discloses that exosomes in which IL-12 is expressed on membranes via PTGFRN proliferate tumor antigen-specific CD8-positive T cells.

### Citation List

### Patent Literature

Patent Literature 1: JP 2016-520518 A

### Non Patent Literatures

Non Patent Literature 1: Katherine Kirwin, et al., "Exosome Surface Display of IL-12 Results in Tumor-Retained Pharmacology with Superior Potency and Limited Systemic Exposure Compared to Recombinant IL-12", November 6, 2019, 34th Annual Meeting of the Society for Immuno-therapy of Cancer
Non Patent Literature 2: Journal of Extracellular Vesicles (2018); 7:1535750

### Summary of Invention

### Technical Problem

As a novel method capable of activating antigen-specific T cells, the present inventors tried a method using extracellular vesicles containing MHC molecules and T-cell costimulatory molecules in membranes. However, when an attempt was made to active antigen-specific T cells using the extracellular vesicles, it was found for the first time that antigen-specific T cells could not be satisfactorily activated.

Therefore, an object of the present invention is to provide a novel immunoregulatory method, a nucleic acid composition for immunoregulation, and a use thereof.

### Solution to Problem

In view of the above problems, as a result of conducting intensive studies, the present inventors have surprisingly found that antigen-specific T cells can be satisfactorily activated using polynucleotides capable of producing extracellular vesicles containing MHC molecules and T-cell stimulatory cytokines in membranes, thereby completing the present invention.

Therefore, the present invention includes the followings.

[0] An extracellular vesicle presenting an antigen-presenting MHC molecule and a T-cell stimulatory cytokine outside membrane thereof.
[1] An antigen-presenting extracellular vesicle, the membrane of which contains:
   (A) a protein which comprises an antigen-presenting MHC molecule and is capable of presenting the antigen outside the membrane; and
   (B) a protein which comprises a first T-cell stimulatory cytokine or a subunit thereof and is capable of presenting the first T-cell stimulatory cytokine outside the membrane.
[2] The antigen-presenting extracellular vesicle according to [1], wherein the membrane of the antigen-presenting extracellular vesicle contains:
   (A) a fusion protein or a protein complex which comprises an antigen-presenting MHC molecule, and a membrane protein capable of being expressed in membrane of an extracellular vesicle or a transmembrane domain thereof or a protein capable of binding to membrane of an extracellular vesicle or a domain thereof, and is capable of presenting the antigen outside the membrane; and
   (B) a fusion protein which comprises a first T-cell stimulatory cytokine or a subunit thereof, and a membrane protein capable of being expressed in membrane of an extracellular vesicle or a transmembrane domain thereof or a protein capable of binding to membrane of an extracellular vesicle or a domain thereof, and is capable of presenting the first T-cell stimulatory cytokine outside the membrane.
[3] The antigen-presenting extracellular vesicle according to [1] or [2], wherein the membrane of the antigen-presenting extracellular vesicle contains:
   (A) a fusion protein or a protein complex which comprises an antigen-presenting MHC molecule and a tetraspanin or a transmembrane domain thereof or MFG-E8 or a domain thereof, and is capable of presenting the antigen outside the membrane; and
   (B) a fusion protein which comprises a first T-cell stimulatory cytokine or a subunit thereof and a partial sequence of a tetraspanin, and is capable of presenting the first T-cell stimulatory cytokine outside the membrane, in which the partial sequence of the tetraspanin contains at least two transmembrane domains, and the first T-cell stimulatory cytokine is disposed between the two transmembrane domains, or
   (B) a fusion protein which comprises a first T-cell stimulatory cytokine or a subunit thereof and MFG-E8 or a domain thereof, and is capable of presenting the first T-cell stimulatory cytokine outside the membrane.
[4] The antigen-presenting extracellular vesicle according to any one of [1] to [3], wherein the membrane of the antigen-presenting extracellular vesicle contains:
   (A) a fusion protein capable of presenting an antigen peptide outside the membrane, wherein the fusion protein comprises an amino acid sequence consisting of, from an N-terminal side thereof,
      (A-1) an MHC molecule-restricted antigen peptide,
      (A-2) a spacer sequence which may be optionally present,
      (A-3) a single chain MHC molecule,
      (A-4) a spacer sequence which may be optionally present, and
      (A-5) a tetraspanin, or
   (A) a protein complex capable of presenting an antigen peptide outside the membrane, wherein the protein complex contains:
      a fusion protein comprising an amino acid sequence consisting of, from an N-terminal side thereof,
      (A-1) an MHC molecule-restricted antigen peptide,
      (A-2) a spacer sequence which may be optionally present,
      (A-3) an MHC class Iα chain, β₂ microglobulin, an MHC class IIα chain, or an MHC class IIβ chain,
      (A-4) a spacer sequence which may be optionally present, and
      (A-5) a tetraspanin; and
      (A-6) a protein comprising an amino acid sequence of β₂ microglobulin, an MHC class Iα chain, an MHC class IIβ chain, or an MHC class IIα chain; and
   (B) a fusion protein comprising an amino acid sequence consisting of, from an N-terminal side thereof,
      (B-1) a partial sequence of a tetraspanin containing, from an N-terminal side thereof, a transmembrane domain 1, a small extracellular loop, a transmembrane domain 2, a small intracellular loop, and a transmembrane domain 3,
      (B-2) a spacer sequence which may be optionally present,
      (B-3) a first T-cell stimulatory cytokine,
      (B-4) a spacer sequence which may be optionally present, and
      (B-5) a partial sequence of a tetraspanin containing a transmembrane domain 4,
      the fusion protein being capable of presenting the first T-cell stimulatory cytokine outside the membrane, or
   (B) a fusion protein comprising an amino acid sequence consisting of, from an N-terminal side thereof,
      (B-3) a first T-cell stimulatory cytokine,
      (B-4) a spacer sequence which may be optionally present, and
      (B-5) MFG-E8,
      the fusion protein being capable of presenting the first T-cell stimulatory cytokine outside the membrane.
[5] The antigen-presenting extracellular vesicle according to [4], wherein the membrane of the antigen-presenting extracellular vesicle contains:
   (A) a fusion protein capable of presenting an antigen peptide outside the membrane, in which the fusion protein comprises an amino acid sequence consisting of, from an N-terminal side thereof,
      (A-1) an MHC class I molecule-restricted antigen peptide,
      (A-2) a spacer sequence which may be optionally present,
      (A-3) a single chain MHC class I molecule,
      (A-4) a spacer sequence which may be optionally present, and
      (A-5) a tetraspanin.
[6] The antigen-presenting extracellular vesicle according to [4], wherein the membrane of the antigen-presenting extracellular vesicle contains:
   (A) a protein complex capable of presenting an antigen peptide outside the membrane, wherein the protein complex contains:
      a fusion protein comprising an amino acid sequence consisting of, from an N-terminal side thereof,
      (A-1) an MHC class II molecule-restricted antigen peptide,
      (A-2) a spacer sequence which may be optionally present,
      (A-3) an MHC class IIβ chain,
      (A-4) a spacer sequence which may be optionally present, and
      (A-5) a tetraspanin; and
      (A-6) a protein comprising an amino acid sequence of an MHC class IIα chain.
[7] The antigen-presenting extracellular vesicle according to any one of [1] to [6], wherein the first T-cell stimulatory cytokine is IL-2, IL-4, IL-6, IL-12, a subunit of IL-12, or TGF-β.
[8] The antigen-presenting extracellular vesicle according to any one of [1] to [7], wherein the membrane of the antigen-presenting extracellular vesicle contains:
   (C) a protein which comprises a T-cell costimulatory molecule and is capable of allowing the T-cell costimulatory molecule to interact with T cells.
[9] The antigen-presenting extracellular vesicle according to any one of [1] to [8], wherein the membrane of the antigen-presenting extracellular vesicle contains:
   (C) a fusion protein which comprises a T-cell costimulatory molecule, and a membrane protein capable of being expressed in membrane of an extracellular vesicle or a transmembrane domain thereof or a protein capable of binding to membrane of an extracellular vesicle or a domain thereof, and is capable of allowing the T-cell costimulatory molecule to interact with T cells.
[10] The antigen-presenting extracellular vesicle according to any one of [1] to [9], wherein the membrane of the antigen-presenting extracellular vesicle contains:
   (C) a fusion protein which comprises a T-cell costimulatory molecule, and a tetraspanin or a transmembrane domain thereof or MFG-E8 or a domain thereof, and is capable of allowing the T-cell costimulatory molecule to interact with T cells.
[11] The antigen-presenting extracellular vesicle according to any one of [1] to [10], wherein the membrane of the antigen-presenting extracellular vesicle contains:
   (C) a fusion protein comprising an amino acid sequence consisting of, from an N-terminal side thereof,
   (C-1) a T-cell costimulatory molecule,
   (C-2) a spacer sequence which may be present, and
   (C-3) a tetraspanin,
   the fusion protein being capable of allowing the T-cell costimulatory molecule to interact with T cells.
[12] The antigen-presenting extracellular vesicle according to any one of [1] to [11], wherein the extracellular vesicle is an exosome.
[13] Polynucleotides encoding any one of:
   (i) the fusion protein or protein complex of (A) defined in any one of [2] to [6];
   (ii) the fusion protein of (B) defined in any one of [2] to [4]; and
   (iii) the fusion protein of (C) defined in any one of [9] to [11].
[14] A vector comprising at least one polynucleotide selected from the polynucleotides according to [13].
[15] A cell transformed with a single vector or a combination of two or more vectors, the vector comprising:
   (i) a polynucleotide encoding the fusion protein or protein complex of (A) defined in any one of [2] to [6];
   (ii) a polynucleotide encoding the fusion protein of (B) defined in any one of [2] to [4]; and optionally,
   (iii) a polynucleotide encoding the fusion protein of (C) defined in any one of [9] to [11].
[16] A culture supernatant obtained by culturing the cell according to [15].
[17] An antigen-presenting extracellular vesicle obtained from the culture supernatant according to [16].
[18] A method for preparing the antigen-presenting extracellular vesicle according to any one of [1] to [12], the method comprising a step of collecting a culture supernatant obtained by culturing the cell according to [15].
[19] A pharmaceutical composition comprising the antigen-presenting extracellular vesicle according to any one of [1] to [12] and [17] or the culture supernatant according to [16].
[20] A pharmaceutical composition for treating or preventing an infectious disease, comprising the antigen-presenting extracellular vesicle according to any one of [1] to [12] and [17] or the culture supernatant according to [16].
[21] A pharmaceutical composition for treating or preventing cancer, comprising the antigen-presenting extracellular vesicle according to any one of [5] and [7] to [12].
[22] A pharmaceutical composition for treating or preventing an autoimmune disease, comprising the antigen-presenting extracellular vesicle according to any one of [6] to [12].
[23] A pharmaceutical composition for treating or preventing an allergic disease, comprising the antigen-presenting extracellular vesicle according to any one of [6] to [12].
[24] A method for activating and/or proliferating T cells against a specific antigen, comprising contacting the antigen-presenting extracellular vesicle according to any one of [1] to [12] and [17] with T cells in vitro or ex vivo.
[25] The antigen-presenting extracellular vesicle according to any one of [1] to [11], wherein the protein or protein complex defined in (A) and the protein or protein complex defined in (B) are fused to each other.
[26] The antigen-presenting extracellular vesicle according to any one of [8] to [11], wherein the protein or protein complex defined in (A) and the protein or protein complex defined in (C) are fused to each other.
[27] The antigen-presenting extracellular vesicle according to any one of [8] to [11], wherein the protein or the protein complex defined in (B) and the protein or protein complex defined in (C) are fused to each other.
[28] The antigen-presenting extracellular vesicle according to any one of [8] to [11], wherein the protein or the protein complex defined in (A), the protein or protein complex defined in (B), and the protein or protein complex defined in (C) are fused to each other.
[29] The antigen-presenting extracellular vesicle according to any one of [25] to [27], wherein the extracellular vesicle is an exosome.
[30] The pharmaceutical composition according to [21], further containing an immune checkpoint inhibitor.
[31] The pharmaceutical composition according to [30], wherein the immune checkpoint inhibitor is present on the membrane of the antigen-presenting extracellular vesicle.
[32] The pharmaceutical composition according to [30] or [31], wherein the immune checkpoint inhibitor is selected from the group consisting of an anti-PD-1 antibody or an active fragment thereof, an anti-CTLA-4 antibody or an active fragment thereof, and a PD-L1 antibody or an active fragment thereof.

[1A] An antigen-presenting extracellular vesicle, the membrane of which contains,
   (D) a fusion protein which comprises an antigen-presenting MHC molecule and at least one T-cell stimulatory cytokine or subunit thereof, and is capable of presenting the antigen and the T-cell stimulatory cytokine outside the membrane.
[2A] The antigen-presenting extracellular vesicle according to [1A], in wherein the fusion protein comprises the antigen-presenting MHC molecule, the at least one T-cell stimulatory cytokine or subunit thereof, and a membrane protein capable of being localized to membrane of an extracellular vesicle or a transmembrane domain thereof or a protein capable of binding to membrane of an extracellular vesicle or a membrane-binding domain thereof.
[3A] The antigen-presenting extracellular vesicle according to [2A], wherein the membrane protein capable of being localized on membrane of an extracellular vesicle or the protein capable of binding to membrane of an extracellular vesicle is a tetraspanin or MFG-E8.
[4A] The antigen-presenting extracellular vesicle according to [3A], wherein the fusion protein comprises an amino acid sequence encoding, from an N-terminal side thereof,
(D-1) an MHC molecule-restricted antigen peptide,
(D-2) a spacer sequence which may be present,
(D-3) a single chain MHC molecule,
(D-4) a spacer sequence which may be present, and
(D-5) a fusion peptide comprising a tetraspanin or a transmembrane domain thereof or MFG-E8 or a transmembrane domain thereof, and the at least one T-cell stimulatory cytokine or subunit thereof, in this order.
[5A] The antigen-presenting extracellular vesicle according to [3A], wherein the fusion protein contains an amino acid sequence encoding, from an N-terminal side thereof,
(D-1) a fusion peptide comprising a tetraspanin or a transmembrane domain thereof or MFG-E8 or a transmembrane domain thereof, and the at least one T-cell stimulatory cytokine or subunit thereof,
(D-2) a spacer sequence which may be optionally present,
(D-3) a single chain MHC molecule,
(D-4) a spacer sequence which may be optionally present, and
(D-5) an MHC molecule-restricted antigen peptide, in this order.
[6A] The antigen-presenting extracellular vesicle according to [4A] or [5A], in which the fusion peptide comprises an amino acid sequence encoding, from an N-terminal side thereof,
   (1) a partial sequence of a tetraspanin containing a transmembrane domain 1, a small extracellular loop, a transmembrane domain 2, a small intracellular loop, and a transmembrane domain 3,
   (2) a spacer sequence which may be optionally present,
   (3) the at least one T-cell stimulatory cytokine or subunit thereof,
   (4) a spacer sequence which may be optionally present, and
   (5) a partial sequence of a tetraspanin containing a transmembrane domain 4, in this order.
[7A] The antigen-presenting extracellular vesicle according to [4A] or [5A], wherein the fusion peptide comprises an amino acid sequence encoding, from an N-terminal side thereof,
   (1) the at least one T-cell stimulatory cytokine or subunit thereof,
   (2) a spacer sequence which may be optionally present, and
   (3) MFG-E8, in this order.
[8A] The antigen-presenting extracellular vesicle according to [4A] or [5A], wherein the MHC molecule-restricted antigen peptide is an MHC class I molecule-restricted antigen peptide, and the single chain MHC molecule comprises an extracellular domain of an MHC class Iα chain.
[9A] The antigen-presenting extracellular vesicle according to [4A] or [5A], wherein the MHC molecule-restricted antigen peptide is an MHC class II molecule-restricted antigen peptide, and the single chain MHC molecule comprises an extracellular domain of an MHC class IIα chain and/or an extracellular domain of an MHC class IIβ chain.
[10A] The antigen-presenting extracellular vesicle according to any one of [1A] to [9A], wherein the membrane of the antigen-presenting extracellular vesicle further contains (C) a protein which comprises at least one T-cell costimulatory molecule and is capable of allowing the T-cell costimulatory molecule to interact with T cells.
[11A] The antigen-presenting extracellular vesicle according to [10A], wherein the protein capable of interacting with T cells comprises the at least one T-cell costimulatory molecule, and a membrane protein capable of being expressed in membrane of an extracellular vesicle or a transmembrane domain thereof or a protein capable of binding to membrane of an extracellular vesicle or a domain thereof.
[12A] The antigen-presenting extracellular vesicle according to [11A], wherein the protein capable of interacting with T cells comprises the at least one T-cell costimulatory molecule, and a tetraspanin or a transmembrane domain thereof or MFG-E8 or a domain thereof.
[13A] The antigen-presenting extracellular vesicle according to [12A], wherein the protein capable of interacting with T cells comprises
   (C) an amino acid sequence encoding, from an N-terminal side thereof,
   (C-1) the at least one T-cell costimulatory molecule,
   (C-2) a spacer sequence which may be optionally present, and
   (C-3) a tetraspanin, in this order.
[14A] The antigen-presenting extracellular vesicle according to any one of [10A] to [13A], wherein the fusion protein (D) is fused to the protein (C) capable of interacting with T cells.
[15A] The antigen-presenting extracellular vesicle according to any one of [1A] to [14A], wherein the extracellular vesicle is an exosome.
[0014] [1B] A pharmaceutical composition comprising the antigen-presenting extracellular vesicle according to any one of [1A] to [15A] and a pharmacologically acceptable carrier.
[1C] A pharmaceutical composition for treating or preventing cancer comprising the antigen-presenting extracellular vesicle according to any one of [1A] to [15A], wherein the antigen peptide preferably includes a cancer antigen peptide.
[2C] A pharmaceutical composition for treating or preventing an autoimmune disease comprising the antigen-presenting extracellular vesicle according to any one of [1A] to [15A], wherein the antigen peptide preferably includes an auto-antigen peptide.
[3C] A pharmaceutical composition for treating or preventing an allergic disease comprising the antigen-presenting extracellular vesicle according to any one of [1A] to [15A], wherein the antigen peptide preferably includes an allergen.
[4C] The pharmaceutical composition according to [1C], further containing an immune checkpoint inhibitor.
[5C] The pharmaceutical composition according to [4C], wherein the immune checkpoint inhibitor is present on the membrane of the antigen-presenting extracellular vesicle.
[6C] The pharmaceutical composition according to [4C] or [5C], wherein the immune checkpoint inhibitor is selected from the group consisting of an anti-PD-1 antibody or an active fragment thereof, an anti-CTLA-4 antibody or an active fragment thereof, and a PD-L1 antibody or an active fragment thereof.
[7C] A pharmaceutical composition for treating or preventing an infectious disease comprising the antigen-presenting extracellular vesicle according to any one of [1A] to [15A] and a pharmacologically acceptable carrier, wherein the antigen peptide is preferably derived from an infectious pathogen that causes an infectious disease.
[1D] The antigen-presenting extracellular vesicle according to any one of [1A] to [15A] for use in treating or preventing cancer, wherein the antigen peptide preferably includes a cancer antigen peptide.
[2D] The antigen-presenting extracellular vesicle according to any one of [1A] to [15A] for use in treating or preventing an autoimmune disease, wherein the antigen peptide preferably includes an auto-antigen peptide.
[3D] The antigen-presenting extracellular vesicle according to any one of [1A] to [15A] for use in treating or preventing an allergic disease, wherein the antigen peptide preferably includes an allergen.
[4D] The antigen-presenting extracellular vesicle foe use according to [1D] used together with an immune checkpoint inhibitor.
[5D] The antigen-presenting extracellular vesicle for use according to [4D], wherein the immune checkpoint inhibitor is present on the membrane of the antigen-presenting extracellular vesicle.
[6D] The antigen-presenting extracellular vesicle for use according to [4D] or [5D], wherein the immune checkpoint inhibitor is selected from the group consisting of an anti-PD-1 antibody or an active fragment thereof, an anti-CTLA-4 antibody or an active fragment thereof, and a PD-L1 antibody or an active fragment thereof.
[7D] The antigen-presenting extracellular vesicle according to any one of [1A] to [15A] for use in treating or preventing an infectious disease, wherein the antigen peptide is preferably derived from an infectious pathogen that causes an infectious disease.
[1E] Use of the antigen-presenting extracellular vesicle according to any one of [1A] to [15A] in the manufacture of a medicament for treating or preventing cancer, wherein the antigen peptide preferably includes a cancer antigen peptide.
[2E] Use of the antigen-presenting extracellular vesicle according to any one of [1A] to [15A] in the manufacture of a medicament for treating or preventing an autoimmune disease, wherein the antigen peptide preferably includes an auto-antigen peptide.
[3E] Use of the antigen-presenting extracellular vesicle according to any one of [1A] to [15A] in the manufacture of a medicament for treating or preventing an allergic disease, wherein the antigen peptide preferably includes an allergen.
[4E] The use according to [1E], wherein the pharmaceutical is used together with an immune checkpoint inhibitor.
[5E] The use according to [4E], wherein the immune checkpoint inhibitor is present on the membrane of the antigen-presenting extracellular vesicle.
[6E] The use according to [4E] or [5E], wherein the immune checkpoint inhibitor is selected from the group consisting of an anti-PD-1 antibody or an active fragment thereof, an anti-CTLA-4 antibody or an active fragment thereof, and a PD-L1 antibody or an active fragment thereof.
[7E] Use of the antigen-presenting extracellular vesicle according to any one of [1A] to [15A] the manufacture of a medicament for treating or preventing an infectious disease, wherein the antigen peptide is preferably derived from an infectious pathogen that causes an infectious disease.
[1F] A method for treating or preventing cancer in a subject, the method comprising:
   administering an effective amount of the antigen-presenting extracellular vesicle according to any one of [1A] to [15A] to the subject to activate and/or proliferate T cells that recognize a caner antigen in the subject and to allow the activated and/or proliferated T cells to attack cancer cells; wherein the activated and/or proliferated T cells are preferably CD8-positive cytotoxic T cells, and the antigen peptide preferably includes a cancer antigen peptide.
[2F] A method for treating or preventing an autoimmune disease in a subject, the method comprising administering an effective amount of the antigen-presenting extracellular vesicle according to any one of [1A] to [15A] to the subject to activate and/or proliferate T cells that recognize an auto-antigen in the subject and to desensitize an immune response to the auto-antigen in the subject, wherein the activated and/or proliferated T cells are preferably CD4-positive regulatory T cells (Treg), and the antigen peptide preferably includes an auto-antigen peptide.
[3F] A method for treating or preventing an allergic disease in a subject, wherein a method for treating or preventing an autoimmune disease comprises administering an effective amount of the antigen-presenting extracellular vesicle according to any one of [1A] to [15A] to the subject to activate and/or proliferate T cells that recognize an allergen in the subject and to desensitize an immune response to the auto-antigen in the subject, wherein the activated and/or proliferated T cells are preferably CD4-positive regulatory T cells (Treg), and the antigen peptide preferably includes an auto-antigen peptide.
[4F] The method according to [1F], wherein the antigen-presenting extracellular vesicle is administered together with an immune checkpoint inhibitor.
[5F] The method according to [4F], wherein the immune checkpoint inhibitor is present on the membrane of the antigen-presenting extracellular vesicle.
[6F] The method according to [4F] or [5F], wherein the immune checkpoint inhibitor is selected from the group consisting of an anti-PD-1 antibody or an active fragment thereof, an anti-CTLA-4 antibody or an active fragment thereof, and a PD-L1 antibody or an active fragment thereof.
[7F] A method for treating or preventing an infectious disease in a subject, the method comprising:
   administering an effective amount of the antigen-presenting extracellular vesicle according to any one of [1A] to [15A] to the subject to,
      1) secrete inflammatory cytokines and to activate innate immunity of the subject, and/or
      2) provide acquired immunity to an infectious pathogen that causes the infectious disease to the subject,
   so that, in the subject's body, the infectious pathogen that causes the infectious disease is eliminated and/or a proliferation of the infectious pathogen is suppressed.
[1G] A method for activating and/or proliferating T cells against a specific antigen, the method comprising contacting the antigen-presenting extracellular vesicle according to any one of [1A] to [15A] with T cells in vitro or ex vivo.
[1H] A polynucleotide encoding:
   (i) the fusion protein or protein complex (D) defined in any one of [1A] to [9A];
   (ii) the protein (C) capable of interacting with T cells defined in any one of [10A] to [13A]; or
   (iii) the fusion protein of the fusion protein (D) and the protein (C) capable of interacting with T cells defined in [14A].
[2H] A vector comprising the nucleic acid according to [1H].
[1I] A polynucleotide comprising at least one sequence selected from the group consisting of:
   (a) a sequence encoding a fusion protein (A) which comprises an antigen-presenting MHC molecule and is capable of presenting the antigen-presenting MHC molecule outside membrane of an extracellular vesicle;
   (b) a sequence encoding a fusion protein (B) which comprises at least one T-cell stimulatory cytokine or subunit thereof and is capable of presenting the T-cell stimulatory cytokine outside membrane of an extracellular vesicle;
   (c) a sequence encoding a fusion protein (C) which comprises a T-cell costimulatory molecule and is capable of presenting the T-cell costimulatory molecule outside membrane of an extracellular vesicle;
   (d) a sequence encoding a fusion protein (D) which comprises an antigen-presenting MHC molecule and at least one T-cell stimulatory cytokine or subunit thereof and is capable of presenting the antigen and the T-cell stimulatory cytokine outside membrane of an extracellular vesicle; and
   (e) a sequence encoding a fusion protein (E) which comprises an antigen-presenting MHC molecule, at least one T-cell stimulatory cytokine or subunit thereof, and a T-cell costimulatory molecule, and is capable of presenting the antigen, the T-cell stimulatory cytokine, and the T-cell costimulatory molecule outside membrane of an extracellular vesicle.
[21] The polynucleotide according to [1I], wherein the fusion protein defined as (A) contains an antigen-presenting MHC molecule, and a membrane protein capable of being expressed in membrane of an extracellular vesicle or a transmembrane domain thereof or a protein capable of binding to membrane of an extracellular vesicle or a domain thereof.
[31] The polynucleotide according to [1I], wherein the fusion protein defined as (B) comprises at least one T-cell stimulatory cytokine or subunit thereof, and a membrane protein capable of being expressed in membrane of an extracellular vesicle or a transmembrane domain thereof or a protein capable of binding to membrane of an extracellular vesicle or a domain thereof.
[41] The polynucleotide according to [1I], wherein the fusion protein defined as (A) comprises an antigen-presenting MHC molecule, and a tetraspanin or a transmembrane domain thereof or MFG-E8 or a domain thereof.
[51] The polynucleotide according to [1I], wherein the fusion protein defined as (B) comprises at least one T-cell stimulatory cytokine or subunit thereof and a partial sequence of a tetraspanin, the partial sequence of the tetraspanin contains at least two transmembrane domains, and the at least one T-cell stimulatory cytokine is disposed between the two transmembrane domains.
[61] The polynucleotide according to [1I], wherein the fusion protein defined as (B) comprises at least one T-cell stimulatory cytokine or subunit thereof and MFG-E8 or a domain thereof.
[71] The polynucleotide according to [1I], wherein the fusion protein defined as (A) comprises an amino acid sequence consisting of, from an N-terminal side thereof,
(A-1) an MHC molecule-restricted antigen peptide,
(A-2) a spacer sequence which may be optionally present,
(A-3) a single chain MHC molecule,
(A-4) a spacer sequence which may be optionally present, and
(A-5) a tetraspanin.
[81] The polynucleotide according to [1I], wherein the fusion protein defined as (A) comprises an amino acid sequence consisting of, from an N-terminal side thereof,
(A-1) an MHC molecule-restricted antigen peptide,
(A-2) a spacer sequence which may be optionally present,
(A-3) an MHC class Iα chain, β₂ microglobulin, an MHC class IIα chain, or an MHC class IIβ chain,
(A-4) a spacer sequence which may be optionally present, and
(A-5) a tetraspanin.
[91] The polynucleotide according to [81], wherein an amino acid sequence of β₂ microglobulin, an MHC class Iα chain, an MHC class IIβ chain, or an MHC class IIα chain is further comprised in the fusion protein.
[10I] The polynucleotide according to [1I], wherein the fusion protein defined as (B) comprises an amino acid sequence consisting of, from an N-terminal side thereof,
(B-1) a partial sequence of a tetraspanin containing, from an N-terminal side thereof, a transmembrane domain 1, a small extracellular loop, a transmembrane domain 2, a small intracellular loop, and a transmembrane domain 3,
(B-2) a spacer sequence which may be optionally present,
(B-3) a first T-cell stimulatory cytokine,
(B-4) a spacer sequence which may be optionally present, and
(B-5) a partial sequence of a tetraspanin containing a transmembrane domain 4.
[11I] The polynucleotide according to [1I], wherein the fusion protein defined as (B) comprises an amino acid sequence consisting of, from an N-terminal side thereof,
(B-3) a first T-cell stimulatory cytokine,
(B-4) a spacer sequence which may be optionally present, and
(B-5) MFG-E8.
[12I] The polynucleotide according to [1I], wherein the fusion protein defined as (A) comprises an amino acid sequence consisting of, from an N-terminal side thereof,
(A-1) an MHC class I molecule-restricted antigen peptide,
(A-2) a spacer sequence which may be optionally present,
(A-3) a single chain MHC class I molecule,
(A-4) a spacer sequence which may be optionally present, and
(A-5) a tetraspanin.
[13I] The polynucleotide according to [1I], wherein the fusion protein defined as (A) comprises an amino acid sequence consisting of, from an N-terminal side thereof,
(A-1) an MHC class II molecule-restricted antigen peptide,
(A-2) a spacer sequence which may be optionally present,
(A-3) an MHC class IIβ chain,
(A-4) a spacer sequence which may be optionally present, and
(A-5) a tetraspanin.
[14I] The polynucleotide according to [131], wherein (A-6) an amino acid sequence of an MHC class IIα chain is further comprised.
[15I] The polynucleotide according to [1I], wherein the T-cell stimulatory cytokine is IL-2, IL-4, IL-6, IL-12, a subunit of IL-12, or TGF-β.
[16I] The polynucleotide according to [1I], wherein the fusion protein defined as (C) comprises a T-cell costimulatory molecule, and a membrane protein capable of being expressed in membrane of an extracellular vesicle or a transmembrane domain thereof or a protein capable of binding to membrane of an extracellular vesicle or a domain thereof.
[17I] The polynucleotide according to [1I], wherein the fusion protein defined as (C) comprises a T-cell costimulatory molecule, and a tetraspanin or a transmembrane domain thereof or MFG-E8 or a domain thereof.
[18I] The polynucleotide according to [1I], wherein the fusion protein defined as (C) comprises an amino acid sequence consisting of, from an N-terminal side thereof,
(C-1) a T-cell costimulatory molecule,
(C-2) a spacer sequence which may be optionally present, and
(C-3) a tetraspanin.
[19I] The polynucleotide according to [1I], wherein the fusion protein defined as (D) comprises the antigen-presenting MHC molecule, the at least one T-cell stimulatory cytokine or subunit thereof, and a membrane protein capable of being localized to membrane of an extracellular vesicle or a transmembrane domain thereof or a protein capable of binding to membrane of an extracellular vesicle or a membrane-binding domain thereof.
[20I] The polynucleotide according to [191], wherein the membrane protein capable of being localized onto membrane of an extracellular vesicle or the protein capable of binding to membrane of an extracellular vesicle is a tetraspanin or MFG-E8.
[21I] The polynucleotide according to [1I], wherein the fusion protein defined as (D) comprises an amino acid sequence encoding, from an N-terminal side thereof,
(D-1) an MHC molecule-restricted antigen peptide,
(D-2) a spacer sequence which may be optionally present,
(D-3) a single chain MHC molecule,
(D-4) a spacer sequence which may be optionally present, and
(D-5) a fusion peptide comprising a tetraspanin or a transmembrane domain thereof or MFG-E8 or a transmembrane domain thereof, and the at least one T-cell stimulatory cytokine or subunit thereof, in this order.
[22I] The polynucleotide according to [1I], wherein the fusion protein defined as (D) contains an amino acid sequence encoding, from an N-terminal side thereof,
(D-1) a fusion peptide comprising a tetraspanin or a transmembrane domain thereof or MFG-E8 or a transmembrane domain thereof, and the at least one T-cell stimulatory cytokine or subunit thereof,
(D-2) a spacer sequence which may be optionally present,
(D-3) a single chain MHC molecule,
(D-4) a spacer sequence which may be optionally present, and
(D-5) an MHC molecule-restricted antigen peptide, in this order.
[23I] The polynucleotide according to [211] or [221], wherein the fusion peptide comprising a tetraspanin or a transmembrane domain thereof or MFG-E8 or a transmembrane domain thereof, and the at least one T-cell stimulatory cytokine or subunit thereof contains an amino acid sequence encoding, from an N-terminal side thereof,
   (1) a partial sequence of a tetraspanin containing a transmembrane domain 1, a small extracellular loop, a transmembrane domain 2, a small intracellular loop, and a transmembrane domain 3,
   (2) a spacer sequence which may be optionally present,
   (3) the at least one T-cell stimulatory cytokine or subunit thereof,
   (4) a spacer sequence which may be optionally present, and
   (5) a partial sequence of a tetraspanin containing a transmembrane domain 4, in this order.
[24I] The polynucleotide according to [211] or [221], wherein the fusion peptide comprising a tetraspanin or a transmembrane domain thereof or MFG-E8 or a transmembrane domain thereof, and the at least one T-cell stimulatory cytokine or subunit thereof comprises an amino acid sequence encoding, from an N-terminal side thereof,
   (1) the at least one T-cell stimulatory cytokine or subunit thereof,
   (2) a spacer sequence which may be optionally present, and
   (3) MFG-E8, in this order.
[25I] The polynucleotide according to [211] or [221], wherein the MHC molecule-restricted antigen peptide is an MHC class I molecule-restricted antigen peptide, and the single chain MHC molecule comprises an extracellular domain of an MHC class Iα chain.
[26I] The polynucleotide according to [211] or [221], wherein the MHC molecule-restricted antigen peptide is an MHC class II molecule-restricted antigen peptide, and the single chain MHC molecule comprises an extracellular domain of an MHC class IIα chain and/or an extracellular domain of an MHC class IIβ chain.
[1J] The polynucleotide according to [1I], comprising the sequence defined as (a) and the sequence defined as (b).
[2J] The polynucleotide according to [1J], further comprising the sequence defined as (c).
[3J] The polynucleotide according to [1I], comprising the sequence defined as (d).
[4J] The polynucleotide according to [3J], further comprising the sequence defined as (c).
[5J] The polynucleotide according to [1I], comprising the sequence defined as (e).
[6J] A vector comprising the polynucleotide according to any one of [1I] to [5J].
[0024] [1K] A pharmaceutical composition comprising the polynucleotide according to any one of [1I] to [5J] or the vector according to [6J], and a pharmacologically acceptable carrier.
[1L] A pharmaceutical composition for treating or preventing cancer, comprising the polynucleotide according to any one of [1I] to [5J] or the vector according to [6J].
[2L] A pharmaceutical composition for treating or preventing an autoimmune disease, comprising the polynucleotide according to any one of [1I] to [5J] or the vector according to [6J], wherein the antigen peptide preferably includes an auto-antigen peptide.
[3L] A pharmaceutical composition for treating or preventing an allergic disease, comprising the polynucleotide according to any one of [1I] to [5J] or the vector according to [6J], wherein the antigen peptide preferably includes an allergen.
[4L] A pharmaceutical composition for treating or preventing an infectious disease, comprising the polynucleotide according to any one of [1I] to [5J] or the vector according to [6J], and a pharmacologically acceptable carrier, wherein the antigen peptide is preferably derived from an infectious pathogen that causes an infectious disease.
[1M] The polynucleotide according to any one of [1I] to [5J] or the vector according to [6J], for use in treating and preventing cancer, wherein the antigen peptide preferably includes a cancer antigen peptide.
[2M] The polynucleotide according to any one of [1I] to [5J] or the vector according to [6J], for use in treating and preventing an autoimmune disease, wherein the antigen peptide preferably includes an auto-antigen peptide.
[3M] The polynucleotide according to any one of [1I] to [5J] or the vector according to [6J], for use in treating and preventing an allergic disease, wherein the antigen peptide preferably includes an allergen.
[4M] The polynucleotide according to any one of [1I] to [5J] or the vector according to [6J], for use in treating and preventing an infectious disease, wherein the antigen peptide is preferably derived from an infectious pathogen that causes an infectious disease.
[1N] Use of the polynucleotide according to any one of [1I] to [5J] or the vector according to [6J] in the manufacture of a medicament for treating or preventing cancer, wherein the antigen peptide preferably includes a cancer antigen peptide.
[2N] Use of the polynucleotide according to any one of [1I] to [5J] or the vector according to [6J] in the manufacture of a medicament for treating or preventing an autoimmune disease, wherein the antigen peptide preferably includes an auto-antigen peptide.
[3N] Use of the polynucleotide according to any one of [1I] to [5J] or the vector according to [6J] in the manufacture of a medicament for treating or preventing an allergic disease, wherein the antigen peptide preferably includes an allergen.
[4N] Use of the polynucleotide according to any one of [1I] to [5J] or the vector according to [6J] in the manufacture of a medicament for treating or preventing an infectious disease, wherein the antigen peptide is preferably derived from an infectious pathogen that causes an infectious disease.
[1O] A method for treating or preventing cancer in a subject, the method comprising:
   administering an effective amount of the polynucleotide according to any one of [1I] to [5J] or the vector according to [6J] to the subject to activate and/or proliferate T cells that recognize a cancer antigen in the subject and to attack cancer cells in the activated and/or proliferated T cells, wherein the activated and/or proliferated T cells are preferably CD8-positive cytotoxic T cells, and the antigen peptide preferably includes a cancer antigen peptide.
[2O] A method for treating or preventing an autoimmune disease in a subject, the method comprising administering an effective amount of the polynucleotide according to any one of [1I] to [5J] or the vector according to [6J] to the subject to activate and/or proliferate T cells that recognize an auto-antigen in the subject and to desensitize an immune response to the auto-antigen in the subject, wherein the activated and/or proliferated T cells are preferably CD4-positive regulatory T cells (Treg), and the antigen peptide preferably includes an auto-antigen peptide.
[3O] A method for treating or preventing an allergic disease in a subject, the method comprising administering an effective amount of the polynucleotide according to any one of [1I] to [5J] or the vector according to [6J] to the subject to activate and/or proliferate T cells that recognize an allergen in the subject and to desensitize an immune response to the auto-antigen in the subject, wherein the activated and/or proliferated T cells are preferably CD4-positive regulatory T cells (Treg), and the antigen peptide preferably includes an allergen.
[4O] A method for treating or preventing an infectious disease in a subject, the method comprising:
   administering an effective amount of the polynucleotide according to any one of [1I] to [5J] or the vector according to [6J] to the subject to,
      1) secrete inflammatory cytokines and to activate innate immunity of the subject, and/or
      2) provide acquired immunity to an infectious pathogen that causes the infectious disease to the subject,
   so that, in the subject's body, the infectious pathogen that causes the infectious disease is eliminated and/or a proliferation of the infectious pathogen is suppressed.
[1P] A method for activating and/or proliferating T cells against a specific antigen, the method comprising:
   introducing the polynucleotide according to any one of [1I] to [5J] or the vector according to [6J] to cells in vitro or ex vivo to produce antigen-presenting cells and/or antigen-presenting extracellular vesicles; and
   contacting the produced antigen-presenting cells and/or antigen-presenting extracellular vesicles with T cells in vitro or ex vivo.

### Advantageous Effects of Invention

According to the present invention, it is possible to satisfactorily activate antigen-specific T cells by using a cell (i.e., antigen-presenting cell) containing an MHC molecule and a T-cell stimulatory cytokine in membrane thereof and a polynucleotide for producing an extracellular vesicle (i.e., antigen-presenting extracellular vesicle).

### Brief Description of Drawings

Fig. 1A illustrates a model diagram of an antigen peptide-single chain MHC class I molecule (sc-Trimer)-CD81 fusion protein.
Fig. 1B illustrates an amino acid sequence of the antigen peptide-single chain MHC class I molecule (sc-Trimer)-CD81 fusion protein.
Fig. 1C illustrates a model diagram of a CD80-CD9 fusion protein.
Fig. 1D illustrates an amino acid sequence of the CD80-CD9 fusion protein.
Fig. 1E illustrates a model diagram of a CD63-IL-2 fusion protein.
Fig. 1F illustrates an amino acid sequence of the CD63-IL-2 fusion protein.
Fig. 1G illustrates a model diagram of an antigen peptide-MHC class IIβ chain (sc-Dimer)-CD81 fusion protein.
Fig. 1H illustrates an amino acid sequence of the antigen peptide-MHC class IIβ chain (sc-Dimer)-CD81 fusion protein.
Fig. 1I illustrates an amino acid sequence of an MHC class IIα chain.
Fig. 1J illustrates a model diagram of a TGF-β-MFG-E8 fusion protein.
Fig. 1K illustrates an amino acid sequence of the TGF-β-MFG-E8 fusion protein.
Fig. 1L illustrates a model diagram of a CD81-IL-4 fusion protein.
Fig. 1M illustrates an amino acid sequence of the CD81-IL-4 fusion protein.
Fig. IN illustrates a nucleic acid sequence of a sc-Trimer-CD81-IL-2 fusion protein.
Fig. 1O illustrates a nucleic acid sequence of the CD81-IL-4 fusion protein.
Fig. 2A illustrates a model diagram of an antigen-presenting extracellular vesicle of Example 1.
Fig. 2B illustrates a model diagram of an antigen-presenting extracellular vesicle of Example 2.
Fig. 2C illustrates a model diagram of an antigen-presenting extracellular vesicle of Example 3.
Fig. 2D illustrates a model diagram of an antigen-presenting extracellular vesicle of Example 4.
Fig. 2E illustrates a model diagram of an antigen-presenting extracellular vesicle of Example 5.
Fig. 2F illustrates a model diagram of an antigen-presenting extracellular vesicle of Example 6.
Fig. 2G illustrates a model diagram of an antigen-presenting extracellular vesicle of Example 7.
Fig. 2H illustrates a model diagram of an antigen-presenting extracellular vesicle of Example 8.
Fig. 21 illustrates a model diagram of an antigen-presenting extracellular vesicle of Example 9.

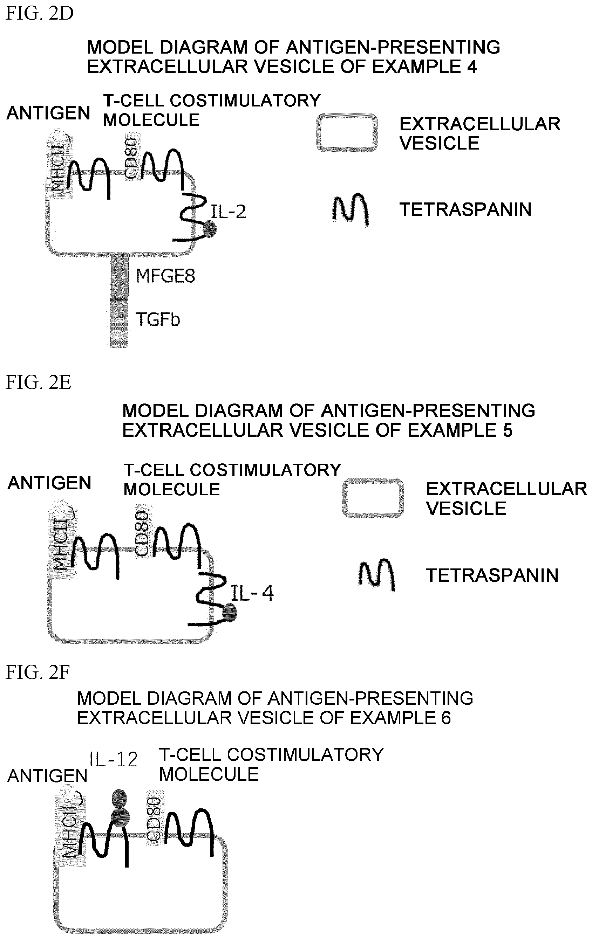

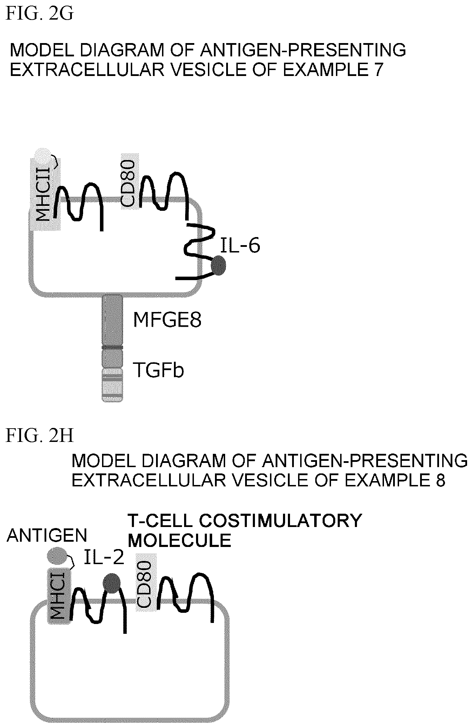

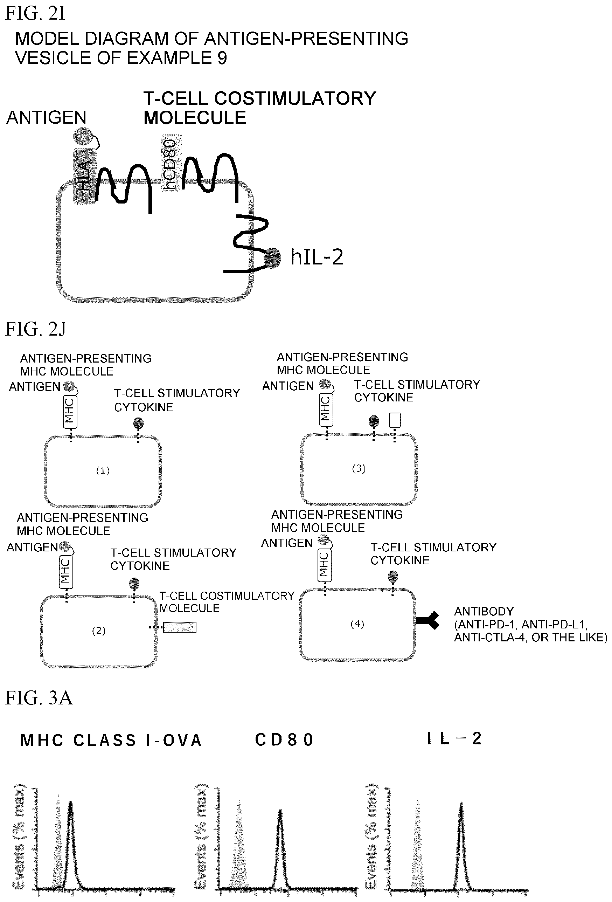

Fig. 2J illustrates a model diagram of antigen-presenting extracellular vesicles of other embodiments.
Fig. 3A illustrates results obtained by analyzing fusion proteins contained in the membrane of the antigen-presenting extracellular vesicle of Example 2 by flow cytometry in Test Example 1-1.

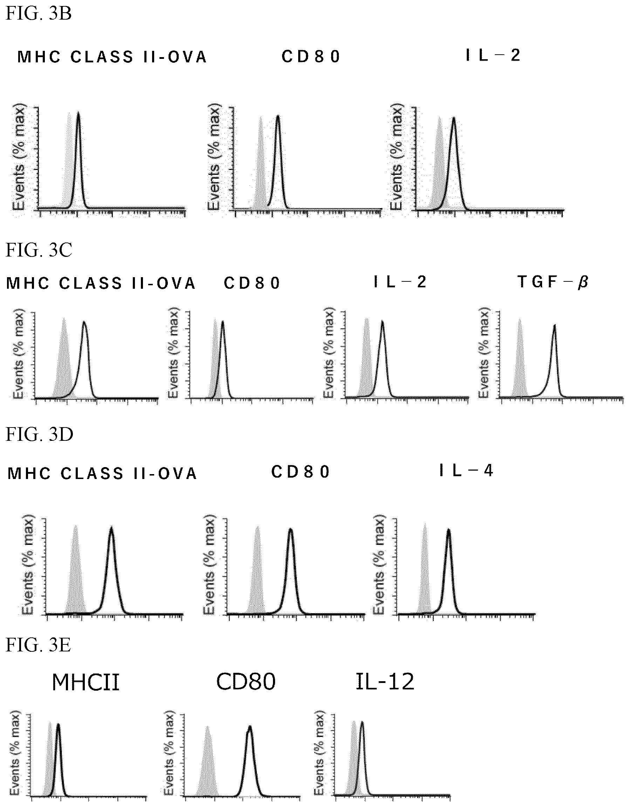

Fig. 3B illustrates results obtained by analyzing fusion proteins contained in the membrane of the antigen-presenting extracellular vesicle of Example 3 by flow cytometry in Test Example 1-2.
Fig. 3C illustrates results obtained by analyzing fusion proteins contained in the membrane of the antigen-presenting extracellular vesicle of Example 4 by flow cytometry in Test Example 1-3.
Fig. 3D illustrates results obtained by analyzing fusion proteins contained in the membrane of the antigen-presenting extracellular vesicle of Example 5 by flow cytometry in Test Example 1-4.
Fig. 3E illustrates results obtained by analyzing fusion proteins contained in the membrane of the antigen-presenting extracellular vesicle of Example 6 by flow cytometry in Test Example 1-5.

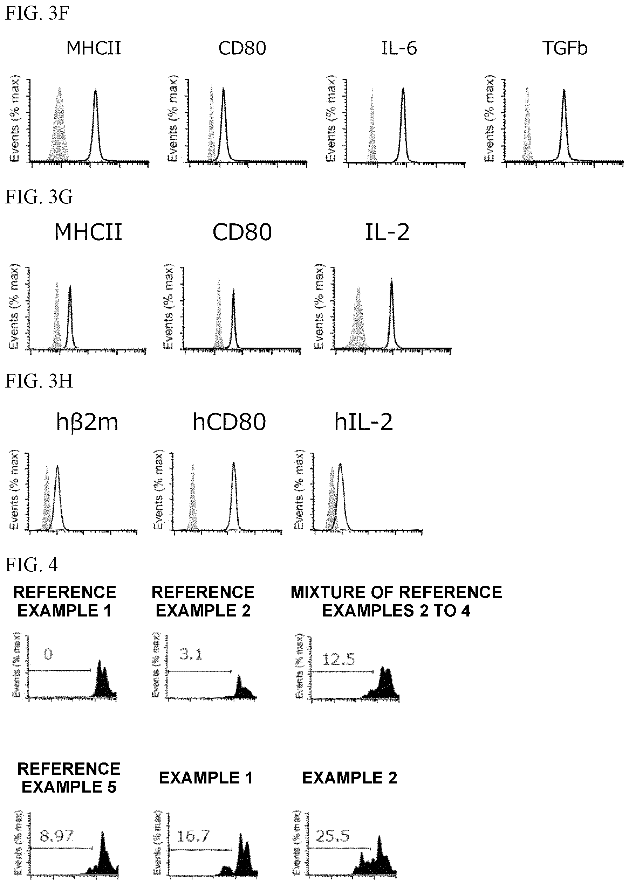

Fig. 3F illustrates results obtained by analyzing fusion proteins contained in the membrane of the antigen-presenting extracellular vesicle of Example 7 by flow cytometry in Test Example 1-6.
Fig. 3G illustrates results obtained by analyzing fusion proteins contained in the membrane of the antigen-presenting extracellular vesicle of Example 8 by flow cytometry in Test Example 1-7.
Fig. 3H illustrates results obtained by analyzing fusion proteins contained in the membrane of the antigen-presenting extracellular vesicle of Example 9 by flow cytometry in Test Example 1-8.
Fig. 4 illustrates results obtained by evaluating whether the antigen-presenting extracellular vesicles of Examples 1 and 2 activate antigen-specific CD8-positive T cells (OT-1 T cells) in vitro in Test Example 2.

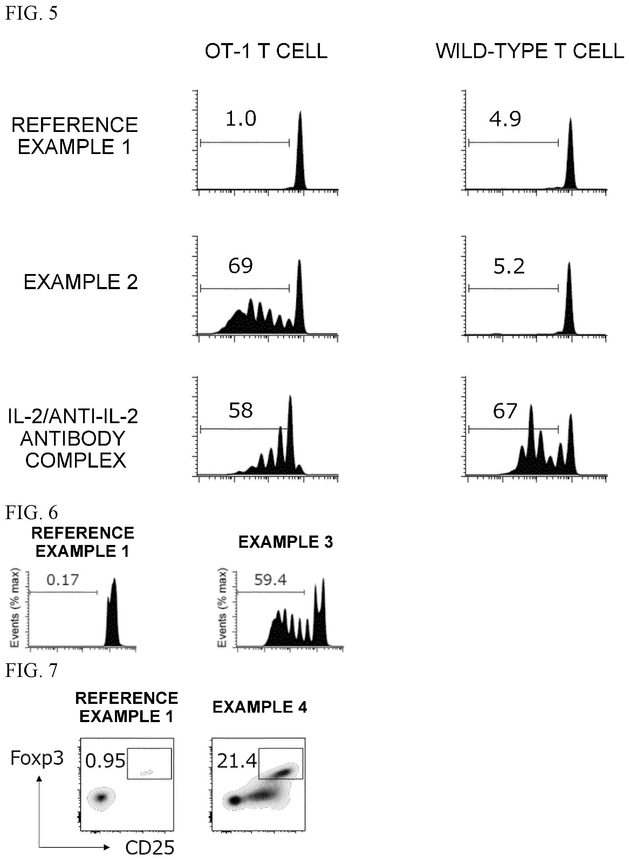

Fig. 5 illustrates results obtained by evaluating whether the antigen-presenting extracellular vesicle of Example 2 activates antigen-specific CD8-positive T cells (OT-1) in vivo in Test Example 3.
Fig. 6 illustrates results obtained by evaluating whether the antigen-presenting extracellular vesicle of Example 3 activates antigen-specific CD4-positive T cells in vitro in Test Example 4.
Fig. 7 illustrates results obtained by evaluating whether the antigen-presenting extracellular vesicle of Example 4 induces differentiation of antigen-specific CD4-positive T cells (OT-2 T cells) into regulatory T cells in vitro in Test Example 5.

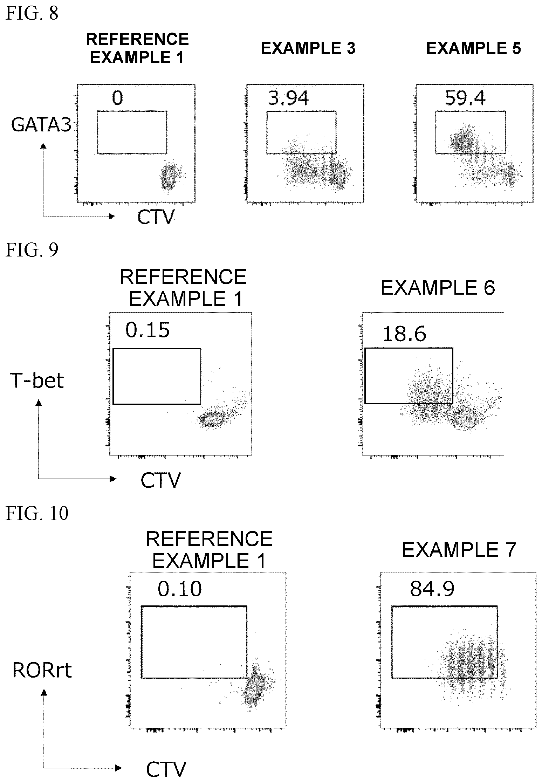

Fig. 8 illustrates results obtained by evaluating whether the antigen-presenting extracellular vesicles of Examples 3 and 5 induce differentiation of antigen-specific CD4-positive T cells (OT-2 T cells) into Th2T cells in vitro in Test Example 6.
Fig. 9 illustrates results obtained by evaluating whether the antigen-presenting extracellular vesicle of Example 6 induces differentiation of antigen-specific CD4-positive T cells into Th1 cells in vitro in Test Example 7.
Fig. 10 illustrates results obtained by evaluating whether the antigen-presenting extracellular vesicle of Example 7 induces differentiation of antigen-specific CD4-positive T cells into Th17 cells in vitro in Test Example 8.

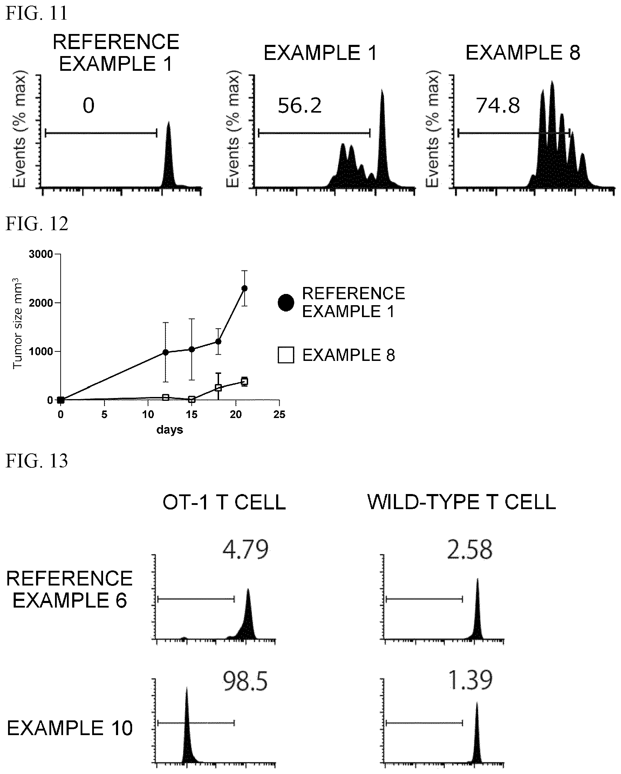

Fig. 11 illustrates that antigen-specific CD8-positive T cells are remarkably proliferated by the antigen-presenting extracellular vesicles of Examples 1 and 8 in Test Example 9.
Fig. 12 illustrates that B16 melanoma cells are remarkably suppressed by the antigen-presenting extracellular vesicle of Example 8 in Test Example 10.
Fig. 13 illustrates results obtained by evaluating whether mRNA of Example 10 activates antigen-specific CD8-positive T cells (OT-1) in vivo in Test Example 11.

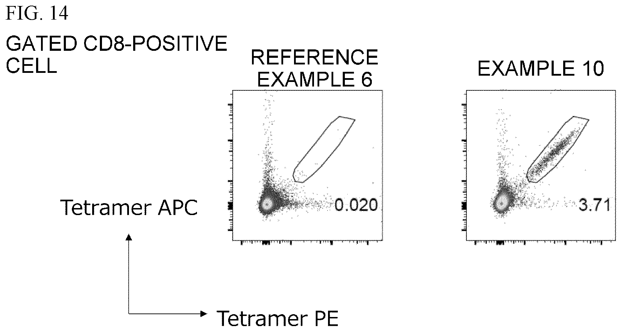

Fig. 14 illustrates results obtained by evaluating whether mRNA of Example 10 activates intrinsic antigen-specific CD8-positive T cells in vivo in Test Example 12.

### Description of Embodiments

### Definitions

### Extracellular vesicle

The "extracellular vesicle" used in the present specification is not particularly limited as long as it is a vesicle secreted from cells, and examples thereof include exosomes, microvesicles (MV), and apoptotic bodies.

The "exosome" used in the present specification means a vesicle of about 20 to about 500 nm (preferably about 20 to about 200 nm, more preferably about 25 to about 150 nm, and still more preferably about 30 to about 100 nm), the vesicle being derived from an endocytosis pathway. Examples of constituent components of the exosome include a protein and a nucleic acid (mRNA, miRNA, or non-coated RNA). The exosome has a function of controlling intercellular communication. Examples of a maker molecule of the exosome include Alix, Tsg101, a tetraspanin, a flotillin, and phosphatidylserine.

The "microvesicle" used in the present specification means a vesicle of about 50 to about 1,000 nm, the vesicle being derived from a cytoplasmic membrane. Examples of constituent components of the microvesicle include a protein and a nucleic acid (mRNA, miRNA, non-coated RNA, or the like). The microvesicle has a function of controlling intercellular communication and the like. Examples of a marker molecule of the microvesicle include integrin, selectin, CD40, and CD 154.

The "apoptotic body" used in the present specification means a vesicle of about 500 to about 2,000 nm, the vesicle being derived from a cytoplasmic membrane. Examples of constituent components of the apoptotic body include a fragmented nucleus and a cell organelle. The apoptotic body has a function of inducing phagocytosis and the like. Examples of a maker molecule of the apoptotic body include Annexin V and phosphatidylserine.

The "antigen-presenting extracellular vesicle" used in the present specification means an extracellular vesicle presenting an antigen outside membrane thereof.

### Major Histocompatibility Gene Complex Molecule

The "major histocompatibility complex (hereinafter, also referred to as "MHC") molecule" used in the present specification is not particularly limited as long as it has an antigen-binding gap and can bind to an antigen to be presented to a T cell, a T cell precursor, or the like. Examples of the MHC molecule include an MHC class I molecule and an MHC class II molecule. The MHC molecule may be derived from any animal species. Examples thereof include a human leukocyte antigen (HLA) in a human and an H2 system in a mouse.

HLA corresponding to the MHC class I molecule may be classified into subtypes such as HLA-A, HLA-B, HLA-Cw, HLA-F, and HLA-G. Polymorphism (allele) is known for these subtypes. Examples of polymorphism of HLA-A include HLA-A1, HLA-A0201, and HLA-A24, examples of polymorphism of HLA-B include HLA-B7, HLA-B40, and HLA-B4403, and examples of polymorphism of HLA-Cw include HLA-Cw0301, HLA-Cw0401, and HLA-Cw0602.

HLA corresponding to the MHC class II molecule may be classified into subtypes such as HLA-DR, HLA-DQ, and HLA-DP.

The MHC molecule described in the present specification is not limited as long as the function thereof can be exhibited, and an amino acid sequence identity of a wild-type amino acid sequence (for example, in a case of an MHC class I molecule: for example, an MHC class Iα chain of SEQ ID NO: 9 or the like, β₂ microglobulin of SEQ ID NO: 7 or the like, a single chain MHC class I molecule of SEQ ID NO: 65 or the like, and the like; and in a case of an MHC class II molecule: for example, an MHC class IIα chain of SEQ ID NO: 71 or the like, an MHC class IIβ chain of SEQ ID NO: 37 or the like, a single chain MHC class II molecule, and the like) may be 80% or more, preferably 90% or more, more preferably 95% or more, still more preferably 98% or more, and further still more preferably 99% or more. Alternatively, the MHC molecule described in the present specification may be obtained by deletion, insertion, and/or substitution of one or a plurality of amino acids with respect to the wild-type amino acid sequence as long as it can exhibit the function thereof.

The "antigen-presenting MHC molecule" used in the present specification is not particularly limited as long as it is an MHC molecule presenting an antigen, and examples thereof include an antigen-presenting MHC class I molecule and an antigen-presenting MHC class II molecule. Examples of the "antigen-presenting MHC class I molecule" include a complex of an antigen, an MHC class Iα chain or an extracellular domain thereof, and β₂ microglobulin; a complex of an antigen and a single chain MHC class I molecule; a fusion protein in which an antigen and a single chain MHC class I molecule are bound; and a complex an antigen, and a fusion protein of an extracellular domain of an MHC class Iα chain and another protein or a domain thereof or a fragment thereof (for example, a fusion protein of an extracellular domain of an MHC class Iα chain and an Fc portion of an antibody, a fusion protein of an extracellular domain of an MHC class Iα chain and a transmembrane domain of another membrane protein, and the like) Examples of the "antigen-presenting MHC class II molecule" include a complex of an antigen, an MHC class IIα chain or an extracellular domain thereof, and an MHC class IIβ chain or an extracellular domain thereof; a complex of an antigen and a single chain MHC class II molecule; a complex of a fusion protein in which an antigen and an MHC class IIβ chain are bound and an MHC class IIα chain; and a complex of a fusion protein of an antigen, an extracellular domain of an MHC class IIα chain and another protein or a domain thereof or a fragment thereof (for example, a fusion protein of an extracellular domain of an MHC class IIα chain and an Fc portion of an antibody; a fusion protein of an extracellular domain of an MHC class IIα chain and a transmembrane domain of another membrane protein; and the like), and a fusion protein of an extracellular domain of an MHC class IIβ chain and another protein or a domain thereof or a fragment thereof (for example, a fusion protein of an extracellular domain of an MHC class IIβ chain and an Fc portion of an antibody; a fusion protein of an amino acid sequence containing an extracellular domain of an MHC class IIβ chain and a transmembrane domain of another membrane protein; and the like).

The "single chain MHC molecule", the "single chain MHC class I molecule", or the "single chain MHC class II molecule" used in the present specification means a fusion protein in which an α chain of an MHC molecule (or an MHC class I molecule or an MHC class II molecule) or an extracellular domain thereof, and a β chain or an extracellular domain thereof or β₂ microglobulin are linked by a spacer sequence, if necessary. Examples of the "single chain MHC class I molecule" include a fusion protein in which an MHC class Iα chain and β₂ microglobulin are linked by a spacer sequence, if necessary. Examples of the "single chain MHC class II molecule" include a fusion protein in which an MHC class IIα chain and an MHC class IIβ chain are linked by a spacer sequence, if necessary.

The "protein (or a fusion protein, a protein complex, or the like) which comprises an antigen-presenting MHC molecule and is capable of presenting the antigen (or an antigen peptide) outside membrane" used in the present specification means a protein comprising at least an antigen-presenting MHC molecule and presenting an antigen (or an antigen peptide) outside membrane, in which the protein is capable of presenting an antigen to T cells and the like (a fusion protein, a protein complex, or the like). The "protein (or a fusion protein, a protein complex, or the like) comprising an antigen-presenting MHC molecule and presenting an antigen (or an antigen peptide) outside the membrane" may be expressed in the form of a fusion protein, a protein complex, or the like using a plasmid or the like so that the protein is expressed in membrane of an extracellular vesicle. Alternatively, in a case where a soluble antigen-presenting MHC molecule (although not limited thereto, a fusion protein comprising an MHC class Iα chain and an immunoglobulin heavy chain described in Patent Literature 1; a soluble MHC class I molecule described in JP 2007-161719 A, or the like) is used, the "protein (or a fusion protein, a protein complex, or the like) which comprises an antigen-presenting MHC molecule and is capable of presenting an antigen (or an antigen peptide) outside the membrane" may be a protein in which a soluble antigen-presenting MHC molecule and an extracellular vesicle are bound to membrane of the extracellular vesicle by a lipid linker, a peptide linker, or the like, if necessary (for example, the method described in JP 2018-104341 A or the like may be referred to). Alternatively, the protein may be a mixture of a protein in which a desired tag (for example, a His tag, a FLAG tag, a PNE tag (SEQ ID NO: 79: NYHLENEVARLKKL), or the like) is added to the N-terminus or C-terminus of a soluble antigen-presenting MHC molecule (the tag may be expressed as a fusion protein together with other constituent elements, for example, may be bound to an additionally prepared soluble antigen-presenting MHC molecule by a linker or the like, if necessary), and an extracellular vesicle containing a protein comprising an antibody against the tag or an antigen-binding fragment thereof (for example, scFv, Fab, or a nanobody) (for example, an antibody itself against the tag or an antigen-binding fragment thereof (for example, scFv, Fab, or a nanobody) bound to the membrane of the extracellular vesicle by a linker or the like, if necessary; a fusion protein in which a nanobody for the tag is bound to the N-terminus or C-terminus of a membrane protein capable of being expressed in membrane of an extracellular vesicle or a transmembrane domain thereof) in membrane under desired conditions (for example, the method using a PNE tag and an antibody against the tag described in Raj D, et al., Gut., 2019 Jun; 68(6): 1052-1064, and the like, may be referred to).

### Antigen

The "antigen" used in the present specification is not particularly limited as long as it can have antigenicity, and includes not only peptide antigens but also non-peptide antigens (for example, constituent elements of a bacterial membrane such as mycolic acid and lipoarabinomannan) such as phospholipids and complex carbohydrates.

The "antigen peptide" used in the present specification is not particularly limited as long as it is a peptide that can be an antigen, and may be naturally derived, synthetically derived, or commercially available. Examples of the antigen peptide include, but are not limited to, tumor-associated antigen peptides such as WT-1, an α-fetal protein, MAGE-1, MAGE-3, placental alkaline phosphatase Sialyl-Lewis X, CA-125, CA-19, TAG-72, epithelial glycoprotein 2, 5T4, an α-fetal protein receptor, M2A, tyrosinase, Ras, p53, Her-2/neu, EGF-R, an estrogen receptor, a progesterone receptor, myc, BCR-ABL, HPV-type 16, melanotransferrin, MUC1, CD10, CD19, CD20, CD37, CD45R, an IL-2 receptor α chain, a T cell receptor, prostatic acid phosphatase, GP100, MelanA/Mart-1, gp75/brown, BAGE, S-100, itokeratin, CYFRA21-1, and Ep-CAM; self-antigen peptides such as insulin, glutamic acid decarboxylase, ICA512/IA-2 protein tyrosine phosphatase, ICA12, ICA69, preproinsulin, HSP60, carboxypeptidase H, periferin, GM1-2, vitronectin, β-crystallin, carreticulin, serotransferase, keratin, pyruvate carboxylase, C1, billin 2, nucleosome, ribonucleoprotein, myelin oligodendrocyte glycoprotein, myelin-associated glycoprotein, myelin/oligodendrocyte basic protein, oligodendrocyte-specific protein, myelin basic protein, and proteolipid protein; antigen peptides derived from infectious pathogens such as protozoa (for example, plasmodium, leishmania, and trypanosoma), bacteria (for example, gram-positive cocci, gram-positive rods, gram-negative bacteria, and anaerobic bacteria), fungi (for example, *Aspergillus, Blastomycosis*, *Candida, Coccidioidomycosis, Cryptococcus, Histoplasma, Paracoccidioidomycosis,* and *Sporoslix*)*,* viruses (for example, adenovirus, simple herpesvirus, papillomavirus, respiratory synthiavirus, poxvirus, HIV, influenza virus, and coronavirus such as SARS-CoV or SARS-CoV2), intracellular parasites (for example, Chlamydiaceae, Mycoplasmataceae, Acholeplasma, and Rickettsiaceae), and helminths (for example, nematodes, trematodes, and tapeworms); and other antigen peptides such as prion.

The antigen peptide may comprise an allergen that causes allergic symptoms. Examples of the allergen include exogenous peptides such as peptides derived from house dust, mites, animals (for example, companion animals such as cats and dogs), and pollens (for example, Japanese cedar or Japanese cypress), in addition to the peptides derived from protozoa, bacteria, fungi, intracellular parasites, and helminths. More specifically, proteins contained in Japanese cedar such as Cryj 1 are exemplified. Alternatively, the allergen that causes allergic symptoms may be derived from food. Examples of the allergen that causes allergic symptoms for food include peptides derived from chicken egg, cow milk, wheat, buckwheat, crab, shrimp, and peanut.

The "MHC molecule-restricted antigen peptide" used in the present specification means an antigen peptide capable of binding to an MHC molecule in vitro, in vivo, and/or ex vivo. The number of amino acid residues of the "MHC molecule-restricted antigen peptide" is usually about 7 to about 30. Examples of the "MHC molecule-restricted antigen peptide" include an MHC class I molecule-restricted antigen peptide and an MHC class II molecule-restricted antigen peptide.

The "MHC class I molecule-restricted antigen peptide" used in the present specification means an antigen peptide capable of binding to an MHC class I molecule in vitro, in vivo, and/or ex vivo. When the MHC class I molecule-restricted antigen peptide is presented outside membrane of the extracellular vesicle, for example, the antigen peptide is recognized by precursor T cells or the like, and cytotoxic T cells or the like can be induced. The number of amino acid residues of the "MHC class I molecule-restricted antigen peptide" is usually about 7 to about 30, preferably about 7 to about 25, more preferably about 7 to about 20, still more preferably about 7 to about 15, and further still more about 7 to about 12.

The "MHC class II molecule-restricted antigen peptide" used in the present specification means an antigen peptide capable of binding to an MHC class II molecule in vitro, in vivo, and/or ex vivo. When the MHC class II molecule-restricted antigen peptide is presented outside membrane of the extracellular vesicle, for example, the antigen peptide is recognized by precursor T cells or the like, and α-T cells or the like can be induced. The number of amino acid residues of the "MHC class II molecule-restricted antigen peptide" is usually about 7 to about 30, preferably about 10 to about 25, and more preferably about 12 to about 24.

The "MHC molecule-restricted antigen peptide", the "MHC class I molecule-restricted antigen peptide", or the "MHC class II molecule-restricted antigen peptide" is not particularly limited as long as it is an antigen peptide capable of binding to an MHC molecule, an MHC class I molecule, or an MHC class II molecule.

### T-Cell Stimulatory Cytokine

The "T-cell stimulatory cytokine" used in the present specification is not particularly limited as long as it is a cytokine capable of stimulating (for example, activating, suppressing, or the like) T cells via a receptor or the like expressed on the membrane of the T cell. Examples of the T-cell stimulatory cytokine include, are not limited to, IL-2, IL-4, IL-6, IL-12, TGF-β, IFN-α, and IFN-γ. Among them, a T-cell stimulatory cytokine capable of forming a multimer of homo or hetero subunits (for example, IL-12, TGF-β, or the like) may be a T-cell stimulatory cytokine comprising a continuous amino acid sequence linked by a peptide linker or the like, if necessary, as long as it is functional (that is, as long as it can have a desired pharmacological activity).

The T-cell stimulatory cytokines described in the present specification may be derived from any animal species. Examples of the T-cell stimulatory cytokine include T-cell stimulatory cytokines derived from animals such as mammals, for example, rodents such as a mouse and a rat; lagomorph such as a rabbit; ungulates such as a pig, a cow, a goat, a horse, and a sheep; carnivora such as a dog and a cat; and primates such as a human, a monkey, a rhesus monkey, a crab-eating macaque, a marmoset, an orangutan, and a chimpanzee. The T-cell stimulatory cytokine described in the present specification is preferably derived from rodents or primates, and more preferably derived from a mouse or a human.

The T-cell stimulatory cytokine described in the present specification may have an amino acid sequence identity of 80% or more, preferably 90% or more, more preferably 95% or more, still more preferably 98% or more, and further still more preferably 99% or more with respect to a wild-type amino acid sequence thereof (for example, in the case of IL-2, for example, SEQ ID NO: 25 or the like; and in the case of IL-4, for example, SEQ ID NO: 53 or the like), as long as it can exhibit the function thereof. Alternatively, the T-cell stimulatory cytokine described in the present specification may be obtained by deletion, insertion, and/or substitution of one or a plurality of amino acids with respect to the wild-type amino acid sequence as long as it can exhibit the function thereof.

The "protein which comprises a (first or second) T-cell stimulatory cytokine and is capable of presenting the (first or second) T-cell stimulatory cytokine outside membrane" used in the present specification means a protein which comprises at least a T-cell stimulatory cytokine and is capable of presenting the T-cell stimulatory cytokine outside membrane of an extracellular vesicle. The "protein which comprises a (first or second) T-cell stimulatory cytokine and is capable of presenting the (first or second) T-cell stimulatory cytokine outside membrane" may be expressed by using a plasmid or the like as a fusion protein having a fragment comprising a T-cell stimulatory cytokine and a membrane protein or a transmembrane domain thereof so that the protein is expressed in the membrane of the extracellular vesicle. Alternatively, in a case where a soluble T-cell stimulatory cytokine (examples thereof include, but are not limited to, a T-cell stimulatory cytokine itself; a fusion protein of a T-cell stimulatory cytokine and an Fc portion of an antibody; and a complex of a T-cell stimulatory cytokine and an antibody that recognizes the T-cell stimulatory cytokine or an antigen-binding fragment thereof (for example, scFv, Fab, or a nanobody)) is used, the "protein which comprises a (first or second) T-cell stimulatory cytokine and is capable of presenting the (first or second) T-cell stimulatory cytokine outside membrane" may be a protein in which a soluble T-cell stimulatory cytokine and an extracellular vesicle are bound to membrane of an extracellular vesicle by a lipid linker, a peptide linker, or the like, if necessary (for example, the method described in JP 2018-104341 A or the like may be referred to). Alternatively, the protein may be a mixture of a protein in which a desired tag (for example, a His tag, a FLAG tag, or a PNE tag) is added to the N-terminus or C-terminus of a soluble T-cell stimulatory cytokine (the tag may be expressed as a fusion protein together with other constituent elements, for example, may be bound to an additionally prepared soluble T-cell stimulatory cytokine by a linker or the like, if necessary), and an extracellular vesicle containing a protein comprising an antibody against the tag or an antigen-binding fragment thereof (for example, scFv, Fab, or a nanobody) (for example, an antibody itself against the tag or an antigen-binding fragment thereof (for example, scFv, Fab, or a nanobody) bound to the membrane of the extracellular vesicle by a linker or the like, if necessary; a fusion protein in which a nanobody for the tag is bound to the N-terminus or C-terminus of a membrane protein capable of being expressed in membrane of an extracellular vesicle or a transmembrane domain thereof) in membrane under desired conditions (for example, the method using a PNE tag and an antibody against the tag described in Raj D, et al., Gut., 2019 Jun; 68(6): 1052-1064, and the like, may be referred to). Note that in a case of a T-cell stimulatory cytokine formed by multimers of subunits, when one of the subunits is a protein that can be presented outside membrane of an extracellular vesicle, the remaining subunits do not need to be in a form that can be presented outside the membrane. When one of the subunits is a protein capable of being presented outside membrane of an extracellular vesicle, a functional T-cell stimulatory cytokine can be constructed outside the membrane of the extracellular vesicle by adding or co-expressing other subunits.

### T-Cell Costimulatory Molecule

The "T-cell costimulatory molecule" used in the present specification means a molecule that can contribute to activation of T cells by interacting with a molecule present on membrane of a T cell such as CD28 or CD134. Examples of the T-cell costimulatory molecule include, but are not limited to, molecules such as CD80 and CD86, or extracellular domains thereof or functional fragments thereof; antibodies such as an anti-CD28 antibody and an anti-CD 134 antibody or antigen-binding fragments thereof (for example, scFv, Fab, or a nanobody); and a fusion protein (or a complex or an aggregate) of them with a transmembrane domain of another protein or an Fc portion of an antibody.

The T-cell costimulatory molecule described in the present specification may be derived from any animal species. Examples of the T-cell costimulatory molecule include T-cell costimulatory molecules derived from animals such as mammals, for example, rodents such as a mouse, a rat, a hamster, and a guinea pig; lagomorph such as a rabbit; ungulates such as a pig, a cow, a goat, a horse, and a sheep; carnivora such as a dog and a cat; and primates such as a human, a monkey, a rhesus monkey, a crab-eating macaque, a marmoset, an orangutan, and a chimpanzee. The T-cell costimulatory molecule described in the present specification is preferably derived from rodents or primates, and more preferably derived from a mouse or a human.

The T-cell costimulatory molecule described in the present specification may have an amino acid sequence identity of 80% or more, preferably 90% or more, more preferably 95% or more, still more preferably 98% or more, and further still more preferably 99% or more with respect to a wild-type amino acid sequence thereof (for example, in the case of CD80, for example, SEQ ID NO: 67 or the like), as long as it can exhibit the function described above. Alternatively, the T-cell costimulatory molecule described in the present specification may be obtained by deletion, insertion, and/or substitution of one or a plurality of amino acids with respect to the wild-type amino acid sequence as long as it can exhibit the function thereof.

The "protein which comprises a T-cell costimulatory molecule and is capable of allowing the T-cell costimulatory molecule to interact with T cells" used in the present specification means a protein which comprises at least a T-cell costimulatory molecule and is capable of interacting with a molecule present in membrane of the T cell. That is, it means that the at least a portion capable of interacting with T cells present in the T-cell costimulatory molecule is located outside the membrane of the extracellular vesicle. The "protein which comprises a T-cell costimulatory molecule and is capable of allowing the T-cell costimulatory molecule to interact with T cells" may be expressed by using a plasmid or the like so that it is expressed in the membrane of the extracellular vesicle. Alternatively, in a case where a soluble T-cell costimulatory molecule (examples thereof include, but are not limited to, a fusion protein of an extracellular domain of CD80 and an Fc portion of an antibody; and an anti-CD28 antibody or an antigen-binding fragment thereof (for example, scFv, Fab, or a nanobody)) is used, the "protein which comprises a T-cell costimulatory molecule and is capable of allowing the T-cell costimulatory molecule to interact with T cells" may be a protein in which a soluble T-cell costimulatory molecule and an extracellular vesicle are bound to membrane of the extracellular vesicle by a lipid linker, a spacer sequence, or the like, if necessary (for example, the method described in JP 2018-104341 A or the like may be referred to). Alternatively, the protein may be a mixture of a protein in which a desired tag (for example, a His tag, a FLAG tag, or a PNE tag) is added to the N-terminus or C-terminus of a soluble T-cell costimulatory molecule (the tag may be expressed as a fusion protein together with other constituent elements, for example, may be bound to an additionally prepared soluble T-cell costimulatory molecule by a linker or the like, if necessary), and an extracellular vesicle containing a protein comprising an antibody against the tag or an antigen-binding fragment thereof (for example, scFv, Fab, or a nanobody) (for example, an antibody itself against the tag or an antigen-binding fragment thereof (for example, scFv, Fab, or a nanobody) bound to the membrane of the extracellular vesicle by a linker or the like, if necessary; a fusion protein in which a nanobody for the tag is bound to the N-terminus or C-terminus of a membrane protein capable of being expressed in membrane of an extracellular vesicle or a transmembrane domain thereof) in membrane under desired conditions (for example, the method using a PNE tag and an antibody against the tag described in Raj D, et al., Gut., 2019 Jun; 68(6): 1052-1064, and the like, may be referred to).

As the "membrane protein capable of being expressed in membrane of an extracellular vesicle or the transmembrane domain thereof' used in the present specification, any membrane protein or a transmembrane domain thereof can be selected as long as it can be expressed in the membrane of the extracellular vesicle. The "membrane protein capable of being expressed in membrane of an extracellular vesicle or a transmembrane domain thereof' is preferably a membrane protein known to be capable of being expressed in an extracellular vesicle (for example, exosome or the like) (for example, a tetraspanin, CD58, ICAM-1, PTGFRN (for example, see Non Patent Literature 1, WO 2019/183578 A, and the like), and the like), or a transmembrane domain thereof.

As the "protein capable of binding to membrane of an extracellular vesicle or the domain thereof' used in the present specification, any protein or a domain thereof can be selected as long as it can be bound in the membrane of the extracellular vesicle. The "protein capable of binding to membrane of an extracellular vesicle or the domain thereof' is preferably a protein known to be capable of binding to membrane of an extracellular vesicle (for example, exosome or the like) (for example, MFG-E8 or a domain thereof (for example, a C1 or C2 domain of MFG-E8 described in Alain Delcayre, et al., Blood Cells, Molecules, and Diseases 35 (2005) 158-168)).

The "membrane protein capable of being expressed in membrane of an extracellular vesicle or the transmembrane domain thereof' or the "protein capable of binding to membrane of an extracellular vesicle or the domain thereof' described in the present specification may be derived from any animal species. Examples of the T-cell stimulatory cytokine include T-cell stimulatory cytokines derived from animals such as mammals, for example, rodents such as a mouse and a rat; lagomorph such as a rabbit, ungulates such as a pig, a cow, a goat, a horse, and a sheep; carnivora such as a dog and a cat; and primates such as a human, a monkey, a rhesus monkey, a crab-eating macaque, a marmoset, an orangutan, and a chimpanzee. The "membrane protein capable of being expressed in membrane of an extracellular vesicle or the transmembrane domain thereof' or the "protein capable of binding to membrane of an extracellular vesicle or the domain thereof' described in the present specification is preferably derived from rodents or primates, and is more preferably derived from a mouse or a human.

According to Non Patent Literature 2, markers of mammalian extracellular vesicles are classified as follows.

Examples of a membrane protein or a GPI anchor protein that can be used as a marker protein of an extracellular vesicle include:
1) tissue non-specific
   tetraspanins (CD63, CD9, CD81, and CD82), other multiple transmembrane type membrane proteins (CD47 and hetero trimer G proteins (guanine nucleotide-binding proteins (GNA)),
   MHC class I (HLA-A/B/C,H2-K/D/Q),
   integrin (ITGA/ITGB), a transferrin receptor (TFR2);
   LAMP1/2;
   heparan sulfate proteoglycans ((including syndecan (SDC));
   extracellular matrix metalloprotease inducer (EMMPRIN) (also referred to as BSG or CD147);
   ADAM10;
   CD73 that is a GPI anchored 5' nucleotidase (NT5E),
   CD55 and CD59 that are GPI anchored complement binding proteins; and sonic hedgehog protein (SHH); and
2) cell/tissue specific
   several tetraspanins: TSPAN8 (epithelial specific), CD37, and CD53 (leukocyte-specific);
   PECAM1 (endothelial specific);
   ERBB2 (breast cancer specific);
   EPCAM (epithelial specific);
   CD90 (THY1) (mesenchymal stem cell-specific);
   CD45 (PTPRC) (immune cell-specific), CD41 (ITGA2B), or CD42a (GP9) (platelet-specific);
   glycophorin A (GYPA) (erythroid specific);
   CD14 (monocyte specific), MHC class II (HLA-DR/DP/DQ,H2-A);
   CD3 (T cell specific);
   acetylcholinesterase/AChE-S (neuronal cell-specific), AChE-E (erythroid specific); and
   amyloid βA4/APP (neuronal cell-specific).

Therefore, although not limited thereto, a protein that is a marker of an extracellular vesicle may be used as the "membrane protein capable of being expressing in membrane of an extracellular vesicle" or the "the protein capable of binding to membrane of an extracellular vesicle" in the present invention.

The "tetraspanin" used in the present specification means a protein belonging to a tetraspanin family (for example, but are not limited to, CD9, CD53, CD63, CD81, CD82, CD151, and the like). The tetraspanin usually contains, from an N-terminal side thereof, a transmembrane domain 1 (hereinafter, referred to as "TM1"), a small extracellular loop (hereinafter, referred to as "SEL"), a transmembrane domain 2 (hereinafter, referred to as "TM2"), a small intracellular loop (hereinafter, referred to as "SIL"), a transmembrane domain 3 (hereinafter, referred to as "TM3"), a large extracellular loop (hereinafter, referred to as "LEL"), and a transmembrane domain 4 (hereinafter, referred to as "TM4"), and thus is a quadruple transmembrane type, and both the N-terminus and the C-terminus are present on the cytoplasmic side. For example, in a case where the tetraspanin is mouse CD63 (amino acid sequences: 1 to 238, SEQ ID NO: 27), the tetraspanin may typically contain TM1, SEL, TM2, SIL, and TM3 in the amino acid sequence from about 1 to about 110, LEL in the amino acid sequence from about 111 to about 200, and TM4 in the amino acid sequence from about 201 to about 238.

Each domain (for example, TM1, SEL, SIL, LTL, or the like) in the "tetraspanin" described in the present specification may be derived from the same tetraspanin, or may be derived from different tetraspanins in whole or in part.

The tetraspanin described in the present specification may have an amino acid sequence identity of 80% or more, preferably 90% or more, more preferably 95% or more, still more preferably 98% or more, and further still more preferably 99% or more with respect to a wild-type amino acid sequence thereof (for example, in the case of CD9 with a full length, for example, SEQ ID NO: 21 or the like; in the case of CD63 with a full length, for example, SEQ ID NO: 27 or the like; and in the case of CD81 with a full length, for example, SEQ ID NO: 15 or the like), as long as it can be expressed in the membrane of the extracellular vesicle. Alternatively, the tetraspanin described in the present specification may be obtained by deletion, insertion, and/or substitution of one or a plurality of amino acids with respect to the wild-type amino acid sequence as long as it can be expressed in the membrane of the extracellular vesicle.

A partial sequence of the tetraspanin (for example, each domain; a partial sequence containing TM1, SEL, TM2, SIL, and TM3 (for example, in the case of CD63, SEQ ID NO: 57 or the like; and in the case of CD81, SEQ ID NO: 61 or the like); a partial sequence containing TM4 (for example, in the case of CD63, SEQ ID NO: 59 or the like; and in the case of CD81, SEQ ID NO: 63 or the like)) described in the present specification may have an amino acid sequence identity of 80% or more, preferably 90% or more, more preferably 95% or more, still more preferably 98% or more, and further still more preferably 99% or more with respect to a wild-type amino acid sequence thereof. Alternatively, the partial sequence of the tetraspanin described in the present specification may be obtained by deletion, insertion, and/or substitution of one or a plurality of amino acids with respect to the wild-type amino acid sequence.

MFG-E8 described in the present specification may have an amino acid sequence identity of 80% or more, preferably 90% or more, more preferably 95% or more, still more preferably 98% or more, and further still more preferably 99% or more with respect to a wild-type amino acid sequence thereof (for example, SEQ ID NO: 49 or the like), as long as it can bind to the membrane of the extracellular vesicle. Alternatively, MFG-E8 described in the present specification may be obtained by deletion, insertion, and/or substitution of one or a plurality of amino acids with respect to the wild-type amino acid sequence as long as it can bind to the membrane of the extracellular vesicle.

CD58, PTGFRN, or the like described in the present specification may have an amino acid sequence identity of 80% or more, preferably 90% or more, more preferably 95% or more, still more preferably 98% or more, and further still more preferably 99% or more with respect to a wild-type amino acid sequence thereof, as long as it can be expressed in the membrane of the extracellular vesicle or can bind to the membrane of the extracellular vesicle. Alternatively, CD58, PTGFRN, or the like described in the present specification may be obtained by deletion, insertion, and/or substitution of one or a plurality of amino acids with respect to the wild-type amino acid sequence as long as it can be expressed in the membrane of the extracellular vesicle or can bind to the membrane of the extracellular vesicle.

### Spacer Sequence

The "spacer sequence" used in the present specification means any sequence having at least one amino acid residue that is present between two or more proteins or partial sequences or domains thereof. The spacer sequence can be used, for example, when two or more proteins or partial sequences or domains thereof are linked. The spacer sequence contains a peptide linker. A length of the amino acid residue of the spacer sequence is usually 1 to about 50, preferably about 2 to about 28, and more preferably about 4 to about 25. Examples of the spacer sequence include, but are not limited to, (GGGXS)ₙGₘ (wherein, X is independently A or G each time it appears, n is 1 to 8, and n, and m is 0 to 3) (for example, SEQ ID NO: 5, 11, 29, 39, or the like); (GGGS)nGm (wherein, n is 1 to 10, and m is 0 to 3); and TₐS_{b}(GGX)ₙGₘ (wherein, X is independently S or T each time it appears, n is 1 to 8, m is 0 to 3, a is 0 or 1, and b is 0 or 1) (for example, SEQ ID NO: 77 or the like).

### Antigen-Presenting Extracellular Vesicles Described in Present Specification

In an embodiment of the present invention, there is provided an extracellular vesicle presenting an antigen-presenting MHC molecule and a T-cell stimulatory cytokine outside membrane (the model is illustrated in (1) of Fig. 2J).

Such an extracellular vesicle may present an antigen-presenting MHC molecule and a T-cell stimulatory cytokine outside membrane thereof by containing proteins specified in the following (A) and (B), or may present an antigen-presenting MHC molecule and a T-cell stimulatory cytokine outside membrane thereof by containing a protein specified in (D).

Alternatively, an antigen-presenting MHC molecule and a T-cell stimulatory cytokine may be attached to membrane surface of an isolated extracellular vesicle later. An attachment method is not particularly limited, an antigen-presenting MHC molecule and a T-cell stimulatory cytokine may be attached to membrane surface by binding each phospholipid to an antigen-presenting MHC molecule and a T-cell stimulatory cytokine and incorporating a new lipid moiety into membrane of an extracellular vesicle. Phosphatidylserine is present on the surface of the extracellular vesicle. Therefore, each protein obtained by fusing an antigen-presenting MHC molecule or a T-cell stimulatory cytokine desired to be presented to MFG-E8 binding to phosphatidylserine is synthesized and purified, and the fusion protein and an extracellular vesicle are mixed, such that an extracellular vesicle presenting an antigen-presenting MHC molecule and a T-cell stimulatory cytokine on membrane surface can be prepared. In addition, an antigen-presenting MHC molecule to which a PNEtag is attached and a T-cell stimulatory cytokine may be added later to an extracellular vesicle pre-expressing a peptide neoepitope (PNE) nanobody to be presented on membrane surface of the extracellular vesicle. A biotinylated antigen-presenting MHC molecule and a T-cell stimulatory cytokine may be added to the extracellular vesicle expressing streptavidin to be presented on the membrane surface of the extracellular vesicle.

In an embodiment of the present invention, the extracellular vesicle may present a plurality of kinds (2, 3, 4, and 5 kinds) of antigen-presenting MHC molecules and a plurality of kinds (2, 3, 4, and 5 kinds) of T-cell stimulatory cytokines (in order to identify each T-cell stimulatory cytokine, hereinafter, it may be referred to as a first T-cell stimulatory cytokine, a second T-cell stimulatory cytokine, third or higher T-cell stimulatory cytokines, and the like) outside the membrane. Alternatively, the extracellular vesicle may be an extracellular vesicle presenting one kind of an antigen-presenting MHC molecule and a plurality of kinds of cell stimulatory cytokines outside membrane (a model of an extracellular vesicle presenting one kind of an antigen-presenting MHC molecule and two kinds of T-cell stimulatory cytokines outside membrane is illustrated in (3) of Fig. 2J).

In an embodiment of the present invention, there is provided an antigen-presenting extracellular vesicle the membrane of which contains:
(A) a protein which contains an antigen-presenting MHC molecule and is capable of presenting the antigen outside membrane; and
(B) a protein which contains a first T-cell stimulatory cytokine and is capable of presenting the first T-cell stimulatory cytokine outside membrane.

### Constitutional Requirement (A)

The "protein which comprises an antigen-presenting MHC molecule and is capable of presenting the antigen outside membrane" of the (A) above may comprise another protein or a domain thereof, or the like in addition to the antigen-presenting MHC molecule as long as it is a protein capable of presenting an antigen outside membrane of an extracellular vesicle.

In an embodiment of the present invention, the (A) above is a fusion protein or a protein complex which comprises an antigen-presenting MHC molecule, and a membrane protein capable of being expressed in membrane of an extracellular vesicle or a transmembrane domain thereof or a protein capable of binding to membrane of an extracellular vesicle or a domain thereof, and is capable of presenting the antigen outside membrane.

In an embodiment of the present invention, the (A) above is a fusion protein or a protein complex which comprises an antigen-presenting MHC molecule, and a tetraspanin or a transmembrane domain thereof or MFG-E8 or a domain thereof, and is capable of presenting the antigen outside membrane.

In an embodiment of the present invention, the (A) above is
(A) a fusion protein capable of presenting an antigen peptide outside membrane, in which the fusion protein comprises an amino acid sequence consisting of, from an N-terminal side thereof,
   (A-1) an MHC molecule-restricted antigen peptide,
   (A-2) a spacer sequence which may be present,
   (A-3) a single chain MHC molecule,
   (A-4) a spacer sequence which may be present, and
   (A-5) a tetraspanin, or
(A) a protein complex capable of presenting an antigen peptide outside membrane, in which the protein complex contains,
a fusion protein comprising an amino acid sequence consisting of, from an N-terminal side thereof,
   (A-1) an MHC molecule-restricted antigen peptide,
   (A-2) a spacer sequence which may be present,
   (A-3) an MHC class Iα chain, β₂ microglobulin, an MHC class IIα chain, or an MHC class IIβ chain,
   (A-4) a spacer sequence which may be present, and
   (A-5) a tetraspanin;
   and
(A-6) a protein comprising an amino acid sequence of β₂ microglobulin, an MHC class Iα chain, an MHC class IIβ chain, or an MHC class IIα chain.

In an embodiment of the present invention, the (A) above is
(A) a fusion protein capable of presenting an antigen peptide outside membrane, in which the fusion protein comprises an amino acid sequence consisting of, from an N-terminal side thereof,
   (A-1) an MHC class I molecule-restricted antigen peptide,
   (A-2) a spacer sequence which may be present,
   (A-3) a single chain MHC class I molecule,
   (A-4) a spacer sequence which may be present, and
   (A-5) a tetraspanin.

In addition, in an embodiment of the present invention, the (A) above is
(A) a fusion protein capable of presenting an antigen peptide outside membrane, in which the fusion protein contains an amino acid sequence consisting of, from an N-terminal side thereof,
   (A-1) an MHC class II molecule-restricted antigen peptide,
   (A-2) a spacer sequence which may be present,
   (A-3) a single chain MHC class II molecule,
   (A-4) a spacer sequence which may be present, and
   (A-5) a tetraspanin.

In an embodiment of the present invention, the (A) above is
(A) a protein complex capable of presenting an antigen peptide outside membrane, in which the protein complex contains:
   a fusion protein comprising an amino acid sequence consisting of, from an N-terminal side thereof,
   (A-1) an MHC class I molecule-restricted antigen peptide,
   (A-2) a spacer sequence which may be present,
   (A-3) β₂ microglobulin,
   (A-4) a spacer sequence which may be present, and
   (A-5) a tetraspanin;
   and
   (A-6) a protein comprising an amino acid sequence of an MHC class Iα chain.

In addition, in an embodiment of the present invention, the (A) above is
(A) a protein complex capable of presenting an antigen peptide outside membrane, in which the protein complex contains:
   a fusion protein comprising an amino acid sequence consisting of, from an N-terminal side thereof,
   (A-1) an MHC class I molecule-restricted antigen peptide,
   (A-2) a spacer sequence which may be present,
   (A-3) an MHC class Iα chain,
   (A-4) a spacer sequence which may be present, and
   (A-5) a tetraspanin;
   and
   (A-6) a protein comprising an amino acid sequence of β₂ microglobulin.

In addition, in an embodiment of the present invention, the (A) above is
(A) a protein complex capable of presenting an antigen peptide outside membrane, in which the protein complex contains:
   a fusion protein comprising an amino acid sequence consisting of, from an N-terminal side thereof,
   (A-1) an MHC class II molecule-restricted antigen peptide,
   (A-2) a spacer sequence which may be present,
   (A-3) an MHC class IIβ chain,
   (A-4) a spacer sequence which may be present, and
   (A-5) a tetraspanin; and
   (A-6) a protein comprising an amino acid sequence of an MHC class IIα chain.

In addition, in an embodiment of the present invention, the (A) above is
(A) a protein complex capable of presenting an antigen peptide outside membrane, in which the protein complex contains:
   a fusion protein comprising an amino acid sequence consisting of, from an N-terminal side thereof,
   (A-1) an MHC class II molecule-restricted antigen peptide,
   (A-2) a spacer sequence which may be present,
   (A-3) an MHC class IIα chain,
   (A-4) a spacer sequence which may be present, and
   (A-5) a tetraspanin;
   and
   (A-6) a protein comprising an amino acid sequence of an MHC class IIβ chain.

In an embodiment of the present invention, in a case where the (A-3) above is a "single chain MHC class I molecule", the "single chain MHC class I molecule" consists of, from an N-terminal side thereof, β₂ microglobulin (for example, SEQ ID NO: 7 or the like, or a sequence having an amino acid sequence identity thereto of 80% or more, preferably 90% or more, more preferably 95% or more, still more preferably 98% or more, and further still more preferably 99% or more), a spacer sequence which may be present (when present, for example, SEQ ID NOS: 5, 11, 29, 39, 77, and the like), and an MHC class Iα chain (for example, SEQ ID NO: 9 or the like, or a sequence having an amino acid sequence identity thereto of 80% or more, preferably 90% or more, more preferably 95% or more, still more preferably 98% or more, and further still more preferably 99% or more). In an embodiment of the present invention, in a case where the (A-3) above is a "single chain MHC class I molecule", the "single chain MHC class I molecule" contains SEQ ID NO: 65 or a sequence having an amino acid sequence identity thereto of 80% or more, preferably 90% or more, more preferably 95% or more, still more preferably 98% or more, and further still more preferably 99% or more.

In an embodiment of the present invention, in a case where the (A-3) above is a "single chain MHC class II molecule", the "single chain MHC class II molecule" consists of, from an N-terminal side thereof, an MHC class IIβ chain, a spacer sequence which may be present, and an MHC class IIα chain.

In an embodiment of the present invention, in a case where the (A-3) and/or (A-6) above is "β₂ microglobulin", the "β₂ microglobulin" comprises SEQ ID NO: 7 or a sequence having an amino acid sequence identity thereto of 80% or more, preferably 90% or more, more preferably 95% or more, still more preferably 98% or more, and further still more preferably 99% or more.

In an embodiment of the present invention, in a case where the (A-3) and/or (A-6) above is an "MHC class Iα chain", the "MHC class Iα chain" comprises SEQ ID NO: 9 or a sequence having an amino acid sequence identity thereto of 80% or more, preferably 90% or more, more preferably 95% or more, still more preferably 98% or more, and further still more preferably 99% or more.

In an embodiment of the present invention, in a case where the (A-3) and/or (A-6) above is an "MHC class IIβ chain", the "MHC class IIβ chain" comprises SEQ ID NO: 37 or a sequence having an amino acid sequence identity thereto of 80% or more, preferably 90% or more, more preferably 95% or more, still more preferably 98% or more, and further still more preferably 99% or more.

In an embodiment of the present invention, in a case where the (A-3) and/or (A-6) above is an "MHC class IIα chain", the "MHC class IIα chain" comprises SEQ ID NO: 71 or a sequence having an amino acid sequence identity thereto of 80% or more, preferably 90% or more, more preferably 95% or more, still more preferably 98% or more, and further still more preferably 99% or more.

The "spacer sequence which may be present" of (A-2) and (A-4) in each of the embodiments may be independently selected when present. When (A-2) is present, for example, the spacer sequence may be, for example, a spacer sequence of SEQ ID NO: 5, 11, 29, 39, 77, or the like. When (A-4) is present, for example, the spacer sequence may be, for example, a spacer sequence of SEQ ID NO: 5, 11, 29, 39, 77, or the like.

In an embodiment of the present invention, the tetraspanin of (A-5) in each of the embodiments is selected from the group consisting of CD9, CD63, and CD81. In an embodiment of the present invention, the tetraspanin of (A-5) in each of the embodiments is CD81 (preferably, SEQ ID NO: 15 or the like or a sequence having an amino acid sequence identity thereto of 80% or more, preferably 90% or more, more preferably 95% or more, still more preferably 98% or more, and further still more preferably 99% or more).

In an embodiment of the present invention, the (A) above is
(A) a fusion protein capable of presenting an antigen peptide outside membrane, in which an amino acid sequence consists of, from an N-terminal side thereof,
   (A-1) an MHC class I molecule-restricted antigen peptide,
   (A-2) a spacer sequence of SEQ ID NO: 5,
   (A-3) a single chain MHC class I molecule of SEQ ID NO: 65 (or a sequence having an amino acid sequence identity thereto of 80% or more, preferably 90% or more, more preferably 95% or more, still more preferably 98% or more, and further still more preferably 99% or more), and
   (A-5) a tetraspanin of SEQ ID NO: 15 (or a sequence having an amino acid sequence identity thereto of 80% or more, preferably 90% or more, more preferably 95% or more, still more preferably 98% or more, and further still more preferably 99% or more).

In an embodiment of the present invention, the (A) above is
(A) a protein complex capable of presenting an antigen peptide outside membrane, in which the protein complex contains:
   a fusion protein of which an amino acid sequence consists of, from an N-terminal side thereof,
   (A-1) an MHC class II molecule-restricted antigen peptide,
   (A-2) a spacer sequence of SEQ ID NO: 39,
   (A-3) an MHC class IIβ chain of SEQ ID NO: 37 (or a sequence having an amino acid sequence identity thereto of 80% or more, preferably 90% or more, more preferably 95% or more, still more preferably 98% or more, and further still more preferably 99% or more), and
   (A-5) a tetraspanin of SEQ ID NO: 15 (or a sequence having an amino acid sequence identity thereto of 80% or more, preferably 90% or more, more preferably 95% or more, still more preferably 98% or more, and further still more preferably 99% or more);
      and
      (A-6) an MHC class IIα chain of SEQ ID NO: 71 (or a sequence having an amino acid sequence identity thereto of 80% or more, preferably 90% or more, more preferably 95% or more, still more preferably 98% or more, and further still more preferably 99% or more).

### Constitutional Requirement (B)

The "protein which comprises a first T-cell stimulatory cytokine and is capable of presenting the first T-cell stimulatory cytokine outside membrane" of the (B) above may comprise another protein or a domain thereof, or the like in addition to the first T-cell stimulatory cytokine as long as it is a protein capable of presenting the first T-cell stimulatory cytokine outside membrane.

In an embodiment of the present invention, the (B) above is a fusion protein which comprises a first T-cell stimulatory cytokine, and a membrane protein capable of being expressed in membrane of an extracellular vesicle or a transmembrane domain thereof or a protein capable of binding to membrane of an extracellular vesicle or a domain thereof, and is capable of presenting the first T-cell stimulatory cytokine outside membrane.

In an embodiment of the present invention, the (B) above is
(B) a fusion protein which comprises a first T-cell stimulatory cytokine and a partial sequence of a tetraspanin and is capable of presenting the first T-cell stimulatory cytokine outside membrane, in which the partial sequence of the tetraspanin contains at least two transmembrane domains and the first T-cell stimulatory cytokine is disposed between the two transmembrane domains, or
(B) a fusion protein which comprises a first T-cell stimulatory cytokine and MFG-E8 or a domain thereof and is capable of presenting the first T-cell stimulatory cytokine.

Examples of the expression "the partial sequence of the tetraspanin contains at least two transmembrane domains and the first T-cell stimulatory cytokine is disposed between the two transmembrane domains" used in the present specification include a case where the partial sequence of the tetraspanin contains at least TM1 and TM2 of the tetraspanin, and the first T-cell stimulatory cytokine is disposed between TM1 and TM2, and a case where the partial sequence of the tetraspanin contains at least TM3 and TM4 of the tetraspanin, and the first T-cell stimulatory cytokine is disposed between TM3 and TM4.

In an embodiment of the present invention, the (B) above is
(B) a fusion protein comprising an amino acid sequence consisting of, from an N-terminal side thereof,
   (B-1) a partial sequence of a tetraspanin containing, from an N-terminal side thereof, a transmembrane domain 1, a small extracellular loop, a transmembrane domain 2, a small intracellular loop, and a transmembrane domain 3,
   (B-2) a spacer sequence which may be present,
   (B-3) a first T-cell stimulatory cytokine,
   (B-4) a spacer sequence which may be present, and
   (B-5) a partial sequence of a tetraspanin containing a transmembrane domain 4,
   the fusion protein being capable of presenting the first T-cell stimulatory cytokine outside membrane, or
(B) a fusion protein comprising an amino acid sequence consisting of, from an N-terminal side thereof,
   (B-3) a first T-cell stimulatory cytokine,
   (B-4) a spacer sequence which may be present, and
   (B-5) MFG-E8,
   the fusion protein being capable of presenting the first T-cell stimulatory cytokine outside membrane.

As disclosed in WO 2016/139354 A, it has been reported that the tetraspanin can be expressed in membranes even when a large extracellular loop (LEL) thereof is entirely or partially replaced by a different amino acid sequence. Therefore, the first T-cell stimulatory cytokine of (B-3) may be inserted in place of the LEL of the tetraspanin or may be inserted at any site in the LEL of the tetraspanin or a partial sequence thereof, by a spacer sequence which may be present.

The "partial sequence of the tetraspanin containing a transmembrane domain 1, a small extracellular loop, a transmembrane domain 2, a small intracellular loop, and a transmembrane domain 3" of (B-1) usually does not contain a transmembrane domain 4 of the tetraspanin. The "partial sequence of the tetraspanin containing a transmembrane domain 1, a small extracellular loop, a transmembrane domain 2, a small intracellular loop, and a transmembrane domain 3" of (B-1) may contain a large extracellular loop or a partial sequence thereof. In (B-1), the transmembrane domain 1, the small extracellular loop, the transmembrane domain 2, the small intracellular loop, and the transmembrane domain 3 may be sequences derived from different tetraspanins, respectively, or all the domains may be sequences derived from the same tetraspanin. Preferably, in (B-1), all the transmembrane domain 1, the small extracellular loop, the transmembrane domain 2, the small intracellular loop, and the transmembrane domain 3 may be sequences derived from the same tetraspanin.

In an embodiment of the present invention, in (B-1), all the partial sequences of the tetraspanin containing a transmembrane domain 1, a small extracellular loop, a transmembrane domain 2, a small intracellular loop, and a transmembrane domain 3 are partial sequences derived from CD9, CD63, or CD81. In an embodiment of the present invention, all the partial sequences of the tetraspanin containing a transmembrane domain 1, a small extracellular loop, a transmembrane domain 2, a small intracellular loop, and a transmembrane domain 3 of (B-1) are preferably partial sequences derived from CD63 or CD81 (preferably, SEQ ID NO: 57, SEQ ID NO: 61, or the like, or a sequence having an amino acid sequence identity thereto of 80% or more, preferably 90% or more, more preferably 95% or more, still more preferably 98% or more, and further still more preferably 99% or more).

The "partial sequence of the tetraspanin containing a transmembrane domain 4" of (B-5) usually does not contain a transmembrane domain 1, a small extracellular loop, a transmembrane domain 2, a small intracellular loop, and a transmembrane domain 3 of the tetraspanin. The "partial sequence of the tetraspanin containing a transmembrane domain 4" of (B-5) may contain a large extracellular loop or a partial sequence thereof. The transmembrane domain 4 in (B-5) may be a sequence derived from a tetraspanin different from that in (B-1), or may be a sequence derived from the same tetraspanin as that in (B-1). Preferably, the transmembrane domain 4 in (B-5) is a sequence derived from the same tetraspanin as that in (B-1). In an embodiment of the present invention, in (B-5), the partial sequence of the tetraspanin containing a transmembrane domain 4 is a partial sequence derived from CD9, CD63, or CD81. In an embodiment of the present invention, the partial sequence of the tetraspanin containing a transmembrane domain 4 of (B-5) is a partial sequence derived from CD63 or CD81 (preferably, SEQ ID NO: 59, SEQ ID NO: 63, or the like, or a sequence having an amino acid sequence identity thereto of 80% or more, preferably 90% or more, more preferably 95% or more, still more preferably 98% or more, and further still more preferably 99% or more).

In an embodiment of the present invention, the "partial sequence of the tetraspanin containing a transmembrane domain 1, a small extracellular loop, a transmembrane domain 2, a small intracellular loop, and a transmembrane domain 3" of (B-1) is a partial sequence derived from CD63 (preferably, SEQ ID NO: 57 or the like or a sequence having an amino acid sequence identity thereto of 80% or more, preferably 90% or more, more preferably 95% or more, still more preferably 98% or more, and further still more preferably 99% or more), and the "partial sequence of the tetraspanin containing a transmembrane domain 4" of (B-5) is a partial sequence derived from CD63 (preferably, SEQ ID NO: 59 or the like or a sequence having an amino acid sequence identity thereto of 80% or more, preferably 90% or more, more preferably 95% or more, still more preferably 98% or more, and further still more preferably 99% or more). In an embodiment of the present invention, the "partial sequence of the tetraspanin containing a transmembrane domain 1, a small extracellular loop, a transmembrane domain 2, a small intracellular loop, and a transmembrane domain 3" of (B-1) is a partial sequence derived from CD81 (preferably, SEQ ID NO: 61 or the like or a sequence having an amino acid sequence identity thereto of 80% or more, preferably 90% or more, more preferably 95% or more, still more preferably 98% or more, and further still more preferably 99% or more), and the "partial sequence of the tetraspanin containing a transmembrane domain 4" of (B-5) is a partial sequence derived from CD81 (preferably, SEQ ID NO: 63 or the like or a sequence having an amino acid sequence identity thereto of 80% or more, preferably 90% or more, more preferably 95% or more, still more preferably 98% or more, and further still more preferably 99% or more).

The fusion protein of the (B) above is a fusion protein comprising a partial sequence of a tetraspanin, and in a case where one or more of the (A) above and (C) present in some cases described below contain a fusion protein comprising an amino acid sequence of a tetraspanin, the fusion protein of the (B) above may be a fusion protein different from the fusion protein of the (A) above and/or (C) present in some cases described below, or may constitute a part of the fusion protein of the (A) above and/or (C) present in some cases described below. The expression that the fusion protein of the (B) above "constitutes a part of the fusion protein of the (A) above and/or (C) present in some cases described below" includes, for example, a case where the tetraspanin of (A-5) constitutes the fusion protein of (B), and/or a case where a tetraspanin of (C-3) present in some cases described below is the fusion protein of (B).

The "MFG-E8" of the (B-5) above is preferably SEQ ID NO: 49 or the like or a sequence having an amino acid sequence identity thereto of 80% or more, preferably 90% or more, more preferably 95% or more, still more preferably 98% or more, and further still more preferably 99% or more.

In an embodiment of the present invention, in (B-3) of each of the embodiments, the first T-cell stimulatory cytokine is IL-2, IL-4, IL-6, IL-12, or TGF-β. In an embodiment of the present invention, the first T-cell stimulatory cytokine in (B-3) in each of the embodiments is IL-2 (preferably, SEQ ID NO: 25 or the like or a sequence having an amino acid sequence identity thereto of 80% or more, preferably 90% or more, more preferably 95% or more, still more preferably 98% or more, and further still more preferably 99% or more), IL-4 (preferably, SEQ ID NO: 53 or the like or a sequence having an amino acid sequence identity thereto of 80% or more, preferably 90% or more, more preferably 95% or more, still more preferably 98% or more, and further still more preferably 99% or more), or TGF-β (preferably, SEQ ID NO: 73 or the like or a sequence having an amino acid sequence identity thereto of 80% or more, preferably 90% or more, more preferably 95% or more, still more preferably 98% or more, and further still more preferably 99% or more).

The "spacer sequence which may be present" in (B-2) and (B-4) in each of the embodiments may be independently selected when present. When (B-2) is present, for example, the spacer sequence may be, for example, a spacer sequence of SEQ ID NO: 5, 11, 29, 39, 77, or the like. When (B-4) is present, for example, the spacer sequence may be, for example, a spacer sequence of SEQ ID NO: 5, 11, 29, 39, 77, or the like.

In an embodiment of the present invention, the (B) above is
(B) a fusion protein of which an amino acid sequence consists of, from an N-terminal side thereof,
   (B-1) a partial sequence of a tetraspanin of SEQ ID NO: 57 (or a sequence having an amino acid sequence identity thereto of 80% or more, preferably 90% or more, more preferably 95% or more, still more preferably 98% or more, and further still more preferably 99% or more),
   (B-2) a spacer sequence of SEQ ID NO: 29,
   (B-3) a first T-cell stimulatory cytokine that is IL-2 of SEQ ID NO: 25 (or a sequence having an amino acid sequence identity thereto of 80% or more, preferably 90% or more, more preferably 95% or more, still more preferably 98% or more, and further still more preferably 99% or more),
   (B-4) a spacer sequence of SEQ ID NO: 29, and
   (B-5) a partial sequence of a tetraspanin of SEQ ID NO: 59 (or a sequence having an amino acid sequence identity thereto of 80% or more, preferably 90% or more, more preferably 95% or more, still more preferably 98% or more, and further still more preferably 99% or more),
the fusion protein being capable of presenting the first T-cell stimulatory cytokine outside membrane.

In an embodiment of the present invention, the (B) above is a fusion protein capable of presenting the first T-cell stimulatory cytokine of SEQ ID NO: 31 (or a sequence having an amino acid sequence identity thereto of 80% or more, preferably 90% or more, more preferably 95% or more, still more preferably 98% or more, and further still more preferably 99% or more), outside membrane.

In an embodiment of the present invention, the (B) above is
(B) a fusion protein of which an amino acid sequence consists of, from an N-terminal side thereof,
   (B-1) a partial sequence of a tetraspanin of SEQ ID NO: 61 (or a sequence having an amino acid sequence identity thereto of 80% or more, preferably 90% or more, more preferably 95% or more, still more preferably 98% or more, and further still more preferably 99% or more),
   (B-2) a spacer sequence of SEQ ID NO: 29,
   (B-3) a first T-cell stimulatory cytokine that is IL-4 of SEQ ID NO: 53 (or a sequence having an amino acid sequence identity thereto of 80% or more, preferably 90% or more, more preferably 95% or more, still more preferably 98% or more, and further still more preferably 99% or more),
   (B-4) a spacer sequence of SEQ ID NO: 29, and
   (B-5) a partial sequence of a tetraspanin of SEQ ID NO: 63 (or a sequence having an amino acid sequence identity thereto of 80% or more, preferably 90% or more, more preferably 95% or more, still more preferably 98% or more, and further still more preferably 99% or more),
the fusion protein being capable of presenting the first T-cell stimulatory cytokine outside membrane.

In an embodiment of the present invention, the (B) above is a fusion protein capable of presenting the first T-cell stimulatory cytokine of SEQ ID NO: 55 (or a sequence having an amino acid sequence identity thereto of 80% or more, preferably 90% or more, more preferably 95% or more, still more preferably 98% or more, and further still more preferably 99% or more), outside membrane.

In an embodiment of the present invention, the above (B) is
(B) a fusion protein of which an amino acid sequence consists of, from an N-terminal side thereof,
   (B-3) a first T-cell stimulatory cytokine that is TGF-β of SEQ ID NO: 73 (or a sequence having an amino acid sequence identity thereto of 80% or more, preferably 90% or more, more preferably 95% or more, still more preferably 98% or more, and further still more preferably 99% or more),
   (B-4) a spacer sequence of SEQ ID NO: 29, and
   (B-5) MFG-E8 of SEQ ID NO: 49 (or a sequence having an amino acid sequence identity thereto of 80% or more, preferably 90% or more, more preferably 95% or more, still more preferably 98% or more, and further still more preferably 99% or more),
the fusion protein being capable of presenting the first T-cell stimulatory cytokine outside membrane.

In an embodiment of the present invention, the (B) above is a fusion protein capable of presenting the first T-cell stimulatory cytokine of SEQ ID NO: 75 (or a sequence having an amino acid sequence identity thereto of 80% or more, preferably 90% or more, more preferably 95% or more, still more preferably 98% or more, and further still more preferably 99% or more), outside membrane.

### Second (or Higher) T-Cell Stimulatory Cytokines

The antigen-presenting extracellular vesicle described in the present specification may further contain second (or higher) T-cell stimulatory cytokines in addition to the first T-cell stimulatory cytokine. Therefore, in an embodiment of the present invention, the antigen-presenting extracellular vesicle described in the present specification may further contain a second T-cell stimulatory cytokine. In particular, in a case where the MHC molecule capable of presenting an antigen is an MHC class II molecule capable of presenting an antigen, it is preferable that the antigen-presenting extracellular vesicle described in the present specification contains a second T-cell stimulatory cytokine.

The second (or higher) T-cell stimulatory cytokines may be inserted into, for example, the (B) above (for example, the second (or higher) T-cell stimulatory cytokines may be linked to the N-terminus and/or the C-terminus of the "first T-cell stimulatory cytokine" of (B-3) by a spacer sequence or the like, if necessary). Alternatively, similar to the first T-cell stimulatory cytokine, the second (or higher) T-cell stimulatory cytokines may be contained in the membrane of the antigen-presenting extracellular vesicle described in the present specification as a protein (or a fusion protein) different from the protein (or the fusion protein) of the constitutional requirement (B) described in the present specification by having the same configuration as that of the constitutional requirement (B) described in the present specification.

In an embodiment of the present invention, the second T-cell stimulatory cytokine is IL-2, IL-4, IL-6, IL-12, or TGF-β. In an embodiment of the present invention, the second T-cell stimulatory cytokine is TGF-β (preferably, SEQ ID NO: 73 or the like or a sequence having an amino acid sequence identity thereto of 80% or more, preferably 90% or more, more preferably 95% or more, still more preferably 98% or more, and further still more preferably 99% or more).

In an embodiment of the present invention, the first T-cell stimulatory cytokine is IL-2 or IL-4 (preferably, SEQ ID NO: 25, SEQ ID NO: 53, or the like or a sequence having an amino acid sequence identity thereto of 80% or more, preferably 90% or more, more preferably 95% or more, still more preferably 98% or more, and further still more preferably 99% or more), and the second T-cell stimulatory cytokine is TGF-β (preferably, SEQ ID NO: 73 or a sequence having an amino acid sequence identity thereto of 80% or more, preferably 90% or more, more preferably 95% or more, still more preferably 98% or more, and further still more preferably 99% or more).

In an embodiment of the present invention, the antigen-presenting extracellular vesicle described in the present specification is an antigen-presenting extracellular vesicle the membrane of which contains:
(A) a fusion protein or a protein complex which contains an antigen-presenting MHC molecule, and a membrane protein capable of being expressed in membrane of an extracellular vesicle or a transmembrane domain thereof or a protein capable of binding to membrane of an extracellular vesicle or a domain thereof, and is capable of presenting the antigen outside membrane; and
(B) a fusion protein which contains a first T-cell stimulatory cytokine, and a membrane protein capable of being expressed in membrane of an extracellular vesicle or a transmembrane domain thereof or a protein capable of binding to membrane of an extracellular vesicle or a domain thereof, and is capable of presenting the first T-cell stimulatory cytokine outside membrane.

In an embodiment of the present invention, the antigen-presenting extracellular vesicle described in the present specification is an antigen-presenting extracellular vesicle the membrane of which contains:
(A) a fusion protein or a protein complex which contains an antigen-presenting MHC molecule and a tetraspanin or a transmembrane domain thereof or MFG-E8 or a domain thereof, and is capable of presenting the antigen outside membrane; and
(B) a fusion protein which contains a first T-cell stimulatory cytokine and a partial sequence of a tetraspanin, and is capable of presenting the first T-cell stimulatory cytokine outside membrane, in which the partial sequence of the tetraspanin contains at least two transmembrane domains, and the first T-cell stimulatory cytokine is disposed between the two transmembrane domains, or
(B) a fusion protein which contains a first T-cell stimulatory cytokine and MFG-E8 or a domain thereof, and is capable of presenting the first T-cell stimulatory cytokine outside membrane.

In an embodiment of the present invention, the antigen-presenting extracellular vesicle described in the present specification is an antigen-presenting extracellular vesicle the membrane of which contains:
(A) a fusion protein capable of presenting an antigen peptide outside membrane, in which the fusion protein contains an amino acid sequence consisting of, from an N-terminal side thereof,
   (A-1) an MHC molecule-restricted antigen peptide,
   (A-2) a spacer sequence which may be present,
   (A-3) a single chain MHC molecule,
   (A-4) a spacer sequence which may be present, and
   (A-5) a tetraspanin, or
(A) a protein complex capable of presenting an antigen peptide outside membrane, in which the protein complex contains,
   a fusion protein comprising an amino acid sequence consisting of, from an N-terminal side thereof,
   (A-1) an MHC molecule-restricted antigen peptide,
   (A-2) a spacer sequence which may be present,
   (A-3) an MHC class Iα chain, β₂ microglobulin, an MHC class IIα chain, or an MHC class IIβ chain,
   (A-4) a spacer sequence which may be present, and
   (A-5) a tetraspanin,
      and
(A-6) a protein comprising an amino acid sequence of β₂ microglobulin, an MHC class Iα chain, an MHC class IIβ chain, or an MHC class IIα chain; and
(B) a fusion protein comprising an amino acid sequence consisting of, from an N-terminal side thereof,
   (B-1) a partial sequence of a tetraspanin containing, from an N-terminal side thereof, a transmembrane domain 1, a small extracellular loop, a transmembrane domain 2, a small intracellular loop, and a transmembrane domain 3,
   (B-2) a spacer sequence which may be present,
   (B-3) a first T-cell stimulatory cytokine,
   (B-4) a spacer sequence which may be present, and
   (B-5) a partial sequence of a tetraspanin containing a transmembrane domain 4,
   the fusion protein being capable of presenting the first T-cell stimulatory cytokine outside membrane, or
(B) a fusion protein comprising an amino acid sequence consisting of, from an N-terminal side thereof,
   (B-3) a first T-cell stimulatory cytokine,
   (B-4) a spacer sequence which may be present, and
   (B-5) MFG-E8,
   the fusion protein being capable of presenting the first T-cell stimulatory cytokine outside membrane.

In an embodiment of the present invention, the antigen-presenting extracellular vesicle described in the present specification is an antigen-presenting extracellular vesicle the membrane of which contains:
(A) a fusion protein capable of presenting an antigen peptide outside membrane, in which the fusion protein contains an amino acid sequence consisting of, from an N-terminal side thereof,
   (A-1) an MHC class I molecule-restricted antigen peptide,
   (A-2) a spacer sequence which may be present,
   (A-3) a single chain MHC class I molecule,
   (A-4) a spacer sequence which may be present, and
   (A-5) a tetraspanin.

In an embodiment of the present invention, the antigen-presenting extracellular vesicle described in the present specification is an antigen-presenting extracellular vesicle the membrane of which contains:
(A) a protein complex capable of presenting an antigen peptide outside membrane, in which the protein complex contains:
   a fusion protein comprising an amino acid sequence consisting of, from an N-terminal side thereof,
   (A-1) an MHC class II molecule-restricted antigen peptide,
   (A-2) a spacer sequence which may be present,
   (A-3) an MHC class IIβ chain,
   (A-4) a spacer sequence which may be present, and
   (A-5) a tetraspanin;
      and
   (A-6) a protein comprising an amino acid sequence of an MHC class IIα chain.

In an embodiment of the present invention, the first T-cell stimulatory cytokine is IL-2, IL-4, IL-6, IL-12, or TGF-β, and provides the antigen-presenting extracellular vesicle described in the present specification.

In an embodiment of the present invention, the antigen-presenting extracellular vesicle is the extracellular vesicle described in the present specification that further presents a T-cell costimulatory molecule outside membrane (exemplifying a model thereof in (2) of Fig. 2J).

Such an extracellular vesicle may present a T-cell costimulatory molecule outside membrane by containing a protein specified in the following (C) in membrane thereof.

Alternatively, a T-cell costimulatory molecule may be attached to membrane surface of an isolated extracellular vesicle later. An attachment method is not particularly limited, an antigen-presenting MHC molecule and a T-cell stimulatory cytokine may be attached to membrane surface by binding each phospholipid to a T-cell costimulatory molecule and incorporating a new lipid moiety into membrane of an extracellular vesicle.

In an embodiment of the present invention, the antigen-presenting extracellular vesicle described in the present specification is an antigen-presenting extracellular vesicle the membrane of which contains:
(C) a protein which comprises a T-cell costimulatory molecule and is capable of allowing the T-cell costimulatory molecule to interact with T cells.

### Constitutional Requirement (C)

The "protein which comprises a T-cell costimulatory molecule and is capable of allowing the T-cell costimulatory molecule to interact with T cells" of the (C) above may contain another protein or a domain thereof, or the like in addition to the T-cell costimulatory molecule as long as it is a protein capable of allowing a T-cell costimulatory molecule to interact with T cells.

In an embodiment of the present invention, the (C) above is a fusion protein which comprises a T-cell costimulatory molecule, and a membrane protein capable of being expressed in membrane of an extracellular vesicle or a transmembrane domain thereof or a protein capable of binding to membrane of an extracellular vesicle or a domain thereof, and is capable of allowing the T-cell costimulatory molecule to interact with T cells.

In an embodiment of the present invention, the (C) above is a fusion protein which comprises a T-cell costimulatory molecule, and a tetraspanin or a transmembrane domain thereof or MFG-E8 or a domain thereof, and is capable of allowing the T-cell costimulatory molecule to interact with T cells.

In an embodiment of the present invention, the above (C) is
(C) a fusion protein comprising an amino acid sequence consisting of, from an N-terminal side thereof,
(C-1) a T-cell costimulatory molecule,
(C-2) a spacer sequence which may be present, and
(C-3) a tetraspanin,
the fusion protein being capable of allowing the T-cell costimulatory molecule to interact with T cells.

In an embodiment of the present invention, the T-cell costimulatory molecule of (C-1) is CD80 or CD86. In an embodiment of the present invention, the T-cell costimulatory molecule in (C-1) is CD80 (preferably, SEQ ID NO: 67 or the like or a sequence having an amino acid sequence identity thereto of 80% or more, preferably 90% or more, more preferably 95% or more, still more preferably 98% or more, and further still more preferably 99% or more).

The "spacer sequence which may be present" of (C-2) may be, for example, a spacer sequence of SEQ ID NO: 5, 11, 29, 39, 77, or the like when present.

In an embodiment of the present invention, the tetraspanin of (C-3) is selected from the group consisting of CD9, CD63, and CD81. In an embodiment of the present invention, the tetraspanin in (C-3) is CD9 (preferably, SEQ ID NO: 21 or the like or a sequence having an amino acid sequence identity thereto of 80% or more, preferably 90% or more, more preferably 95% or more, still more preferably 98% or more, and further still more preferably 99% or more).

In an embodiment of the present invention, the above (C) is
(C) a fusion protein of which an amino acid sequence consists of, from an N-terminal side thereof,
(C-1) a T-cell costimulatory molecule that is CD80 of SEQ ID NO: 67 (or a sequence having an amino acid sequence identity thereto of 80% or more, preferably 90% or more, more preferably 95% or more, still more preferably 98% or more, and further still more preferably 99% or more), and
(C-3) a tetraspanin of SEQ ID NO: 21 (or a sequence having an amino acid sequence identity thereto of 80% or more, preferably 90% or more, more preferably 95% or more, still more preferably 98% or more, and further still more preferably 99% or more),
the fusion protein being capable of allowing the T-cell costimulatory molecule to interact with T cells.

In an embodiment of the present invention, the (C) above is a fusion protein capable of allowing the T-cell costimulatory molecule of SEQ ID NO: 69 (or a sequence having an amino acid sequence identity thereto of 80% or more, preferably 90% or more, more preferably 95% or more, still more preferably 98% or more, and further still more preferably 99% or more), to interact with T cells.

In an embodiment of the present invention, the antigen-presenting extracellular vesicle described in the present specification is an antigen-presenting extracellular vesicle the membrane of which contains:
(A) a fusion protein of which an amino acid sequence consists of, from an N-terminal side thereof,
   (A-1) an MHC class I molecule-restricted antigen peptide,
   (A-2) a spacer sequence of SEQ ID NO: 5,
   (A-3) a single chain MHC class I molecule of SEQ ID NO: 65 (or a sequence having an amino acid sequence identity thereto of 80% or more, preferably 90% or more, more preferably 95% or more, still more preferably 98% or more, and further still more preferably 99% or more), and
   (A-5) a tetraspanin of SEQ ID NO: 15 (or a sequence having an amino acid sequence identity thereto of 80% or more, preferably 90% or more, more preferably 95% or more, still more preferably 98% or more, and further still more preferably 99% or more),
   the fusion protein being capable of presenting an antigen peptide outside membrane; and
(B) a fusion protein of which an amino acid sequence consists of, from an N-terminal side thereof,
   (B-1) a partial sequence of a tetraspanin of SEQ ID NO: 57 (or a sequence having an amino acid sequence identity thereto of 80% or more, preferably 90% or more, more preferably 95% or more, still more preferably 98% or more, and further still more preferably 99% or more),
   (B-2) a spacer sequence of SEQ ID NO: 29,
   (B-3) a first T-cell stimulatory cytokine that is IL-2 of SEQ ID NO: 25 (or a sequence having an amino acid sequence identity thereto of 80% or more, preferably 90% or more, more preferably 95% or more, still more preferably 98% or more, and further still more preferably 99% or more),
   (B-4) a spacer sequence of SEQ ID NO: 29, and
   (B-5) a partial sequence of a tetraspanin of SEQ ID NO: 59 (or a sequence having an amino acid sequence identity thereto of 80% or more, preferably 90% or more, more preferably 95% or more, still more preferably 98% or more, and further still more preferably 99% or more),
   the fusion protein being capable of presenting the first T-cell stimulatory cytokine outside membrane.

In an embodiment of the present invention, the antigen-presenting extracellular vesicle described in the present specification is an antigen-presenting extracellular vesicle the membrane of which contains:
(A) a fusion protein of which an amino acid sequence consists of, from an N-terminal side thereof,
   (A-1) an MHC class I molecule-restricted antigen peptide,
   (A-2) a spacer sequence of SEQ ID NO: 5,
   (A-3) a single chain MHC class I molecule of SEQ ID NO: 65 (or a sequence having an amino acid sequence identity thereto of 80% or more, preferably 90% or more, more preferably 95% or more, still more preferably 98% or more, and further still more preferably 99% or more), and
   (A-5) a tetraspanin of SEQ ID NO: 15 (or a sequence having an amino acid sequence identity thereto of 80% or more, preferably 90% or more, more preferably 95% or more, still more preferably 98% or more, and further still more preferably 99% or more),
   the fusion protein being capable of presenting an antigen peptide outside membrane; and
(B) a fusion protein capable of presenting the first T-cell stimulatory cytokine of SEQ ID NO: 31 (or a sequence having an amino acid sequence identity thereto of 80% or more, preferably 90% or more, more preferably 95% or more, still more preferably 98% or more, and further still more preferably 99% or more), outside membrane.

In an embodiment of the present invention, the antigen-presenting extracellular vesicle described in the present specification is an antigen-presenting extracellular vesicle the membrane of which contains:
(A) a fusion protein of which an amino acid sequence consists of, from an N-terminal side thereof,
   (A-1) an MHC class I molecule-restricted antigen peptide,
   (A-2) a spacer sequence of SEQ ID NO: 5,
   (A-3) a single chain MHC class I molecule of SEQ ID NO: 65 (or a sequence having an amino acid sequence identity thereto of 80% or more, preferably 90% or more, more preferably 95% or more, still more preferably 98% or more, and further still more preferably 99% or more), and
   (A-5) a tetraspanin of SEQ ID NO: 15 (or a sequence having an amino acid sequence identity thereto of 80% or more, preferably 90% or more, more preferably 95% or more, still more preferably 98% or more, and further still more preferably 99% or more),
   the fusion protein capable of presenting an antigen peptide outside membrane;
(B) a fusion protein of which an amino acid sequence consists of, from an N-terminal side thereof,
   (B-1) a partial sequence of a tetraspanin of SEQ ID NO: 57 (or a sequence having an amino acid sequence identity thereto of 80% or more, preferably 90% or more, more preferably 95% or more, still more preferably 98% or more, and further still more preferably 99% or more),
   (B-2) a spacer sequence of SEQ ID NO: 29,
   (B-3) a first T-cell stimulatory cytokine that is IL-2 of SEQ ID NO: 25 (or a sequence having an amino acid sequence identity thereto of 80% or more, preferably 90% or more, more preferably 95% or more, still more preferably 98% or more, and further still more preferably 99% or more),
   (B-4) a spacer sequence of SEQ ID NO: 29, and
   (B-5) a partial sequence of a tetraspanin of SEQ ID NO: 59 (or a sequence having an amino acid sequence identity thereto of 80% or more, preferably 90% or more, more preferably 95% or more, still more preferably 98% or more, and further still more preferably 99% or more),
   the fusion protein being capable of presenting the first T-cell stimulatory cytokine outside membrane; and
(C) a fusion protein of which an amino acid sequence consists of, from an N-terminal side thereof,
   (C-1) a T-cell costimulatory molecule that is CD80 of SEQ ID NO: 67 (or a sequence having an amino acid sequence identity thereto of 80% or more, preferably 90% or more, more preferably 95% or more, still more preferably 98% or more, and further still more preferably 99% or more), and
   (C-3) a tetraspanin of SEQ ID NO: 21 (or a sequence having an amino acid sequence identity thereto of 80% or more, preferably 90% or more, more preferably 95% or more, still more preferably 98% or more, and further still more preferably 99% or more),
   the fusion protein being capable of allowing the T-cell costimulatory molecule to interact with T cells.

In an embodiment of the present invention, the antigen-presenting extracellular vesicle described in the present specification is an antigen-presenting extracellular vesicle the membrane of which contains:
(A) a fusion protein of which an amino acid sequence consists of, from an N-terminal side thereof,
   (A-1) an MHC class I molecule-restricted antigen peptide,
   (A-2) a spacer sequence of SEQ ID NO: 5,
   (A-3) a single chain MHC class I molecule of SEQ ID NO: 65 (or a sequence having an amino acid sequence identity thereto of 80% or more, preferably 90% or more, more preferably 95% or more, still more preferably 98% or more, and further still more preferably 99% or more), and
   (A-5) a tetraspanin of SEQ ID NO: 15 (or a sequence having an amino acid sequence identity thereto of 80% or more, preferably 90% or more, more preferably 95% or more, still more preferably 98% or more, and further still more preferably 99% or more),
   the fusion protein capable of presenting an antigen peptide outside membrane;
(B) a fusion protein capable of presenting the first T-cell stimulatory cytokine of SEQ ID NO: 31 (or a sequence having an amino acid sequence identity thereto of 80% or more, preferably 90% or more, more preferably 95% or more, still more preferably 98% or more, and further still more preferably 99% or more), outside membrane; and
(C) a fusion protein capable of allowing the T-cell costimulatory molecule of SEQ ID NO: 69 (or a sequence having an amino acid sequence identity thereto of 80% or more, preferably 90% or more, more preferably 95% or more, still more preferably 98% or more, and further still more preferably 99% or more), to interact with T cells.

In an embodiment of the present invention, the antigen-presenting extracellular vesicle described in the present specification is an antigen-presenting extracellular vesicle the membrane of which contains:
(A) a protein complex capable of presenting an antigen peptide outside membrane, in which the protein complex contains:
   a fusion protein of which an amino acid sequence consists of, from an N-terminal side thereof,
   (A-1) an MHC class II molecule-restricted antigen peptide,
   (A-2) a spacer sequence of SEQ ID NO: 39,
   (A-3) an MHC class IIβ chain of SEQ ID NO: 37 (or a sequence having an amino acid sequence identity thereto of 80% or more, preferably 90% or more, more preferably 95% or more, still more preferably 98% or more, and further still more preferably 99% or more), and
   (A-5) a tetraspanin of SEQ ID NO: 15 (or a sequence having an amino acid sequence identity thereto of 80% or more, preferably 90% or more, more preferably 95% or more, still more preferably 98% or more, and further still more preferably 99% or more);
   and
   (A-6) an MHC class IIα chain of SEQ ID NO: 71 (or a sequence having an amino acid sequence identity thereto of 80% or more, preferably 90% or more, more preferably 95% or more, still more preferably 98% or more, and further still more preferably 99% or more);
(B) a fusion protein of which an amino acid sequence consists of, from an N-terminal side thereof,
   (B-1) a partial sequence of a tetraspanin of SEQ ID NO: 57 (or a sequence having an amino acid sequence identity thereto of 80% or more, preferably 90% or more, more preferably 95% or more, still more preferably 98% or more, and further still more preferably 99% or more),
   (B-2) a spacer sequence of SEQ ID NO: 29,
   (B-3) a first T-cell stimulatory cytokine that is IL-2 of SEQ ID NO: 25 (or a sequence having an amino acid sequence identity thereto of 80% or more, preferably 90% or more, more preferably 95% or more, still more preferably 98% or more, and further still more preferably 99% or more),
   (B-4) a spacer sequence of SEQ ID NO: 29, and
   (B-5) a partial sequence of a tetraspanin of SEQ ID NO: 59 (or a sequence having an amino acid sequence identity thereto of 80% or more, preferably 90% or more, more preferably 95% or more, still more preferably 98% or more, and further still more preferably 99% or more),
      the fusion protein being capable of presenting the first T-cell stimulatory cytokine outside membrane; and
(C) a fusion protein of which an amino acid sequence consists of, from an N-terminal side thereof,
   (C-1) a T-cell costimulatory molecule that is CD80 of SEQ ID NO: 67 (or a sequence having an amino acid sequence identity thereto of 80% or more, preferably 90% or more, more preferably 95% or more, still more preferably 98% or more, and further still more preferably 99% or more), and
   (C-3) a tetraspanin of SEQ ID NO: 21 (or a sequence having an amino acid sequence identity thereto of 80% or more, preferably 90% or more, more preferably 95% or more, still more preferably 98% or more, and further still more preferably 99% or more),
   the fusion protein being capable of allowing the T-cell costimulatory molecule to interact with T cells.

In an embodiment of the present invention, the antigen-presenting extracellular vesicle described in the present specification is an antigen-presenting extracellular vesicle the membrane of which contains:
(A) a protein complex capable of presenting an antigen peptide outside membrane, in which the protein complex contains:
   a fusion protein of which an amino acid sequence consists of, from an N-terminal side thereof,
   (A-1) an MHC class II molecule-restricted antigen peptide,
   (A-2) a spacer sequence of SEQ ID NO: 39,
   (A-3) an MHC class IIβ chain of SEQ ID NO: 37 (or a sequence having an amino acid sequence identity thereto of 80% or more, preferably 90% or more, more preferably 95% or more, still more preferably 98% or more, and further still more preferably 99% or more), and
   (A-5) a tetraspanin of SEQ ID NO: 15 (or a sequence having an amino acid sequence identity thereto of 80% or more, preferably 90% or more, more preferably 95% or more, still more preferably 98% or more, and further still more preferably 99% or more);
      and
      (A-6) an MHC class IIα chain of SEQ ID NO: 71 (or a sequence having an amino acid sequence identity thereto of 80% or more, preferably 90% or more, more preferably 95% or more, still more preferably 98% or more, and further still more preferably 99% or more);
(B) a fusion protein capable of presenting the first T-cell stimulatory cytokine of SEQ ID NO: 31 (or a sequence having an amino acid sequence identity thereto of 80% or more, preferably 90% or more, more preferably 95% or more, still more preferably 98% or more, and further still more preferably 99% or more), outside membrane; and
(C) a fusion protein capable of allowing the T-cell costimulatory molecule of SEQ ID NO: 69 (or a sequence having an amino acid sequence identity thereto of 80% or more, preferably 90% or more, more preferably 95% or more, still more preferably 98% or more, and further still more preferably 99% or more), to interact with T cells.

In an embodiment of the present invention, the antigen-presenting extracellular vesicle described in the present specification is an antigen-presenting extracellular vesicle the membrane of which contains:
(A) a protein complex capable of presenting an antigen peptide outside membrane, in which the protein complex contains:
   a fusion protein of which an amino acid sequence consists of, from an N-terminal side thereof,
      (A-1) an MHC class II molecule-restricted antigen peptide,
      (A-2) a spacer sequence of SEQ ID NO: 39,
      (A-3) an MHC class IIβ chain of SEQ ID NO: 37 (or a sequence having an amino acid sequence identity thereto of 80% or more, preferably 90% or more, more preferably 95% or more, still more preferably 98% or more, and further still more preferably 99% or more), and
      (A-5) a tetraspanin of SEQ ID NO: 15 (or a sequence having an amino acid sequence identity thereto of 80% or more, preferably 90% or more, more preferably 95% or more, still more preferably 98% or more, and further still more preferably 99% or more);
   and
      (A-6) an MHC class IIα chain of SEQ ID NO: 71 (or a sequence having an amino acid sequence identity thereto of 80% or more, preferably 90% or more, more preferably 95% or more, still more preferably 98% or more, and further still more preferably 99% or more);
(B) a fusion protein of which an amino acid sequence consists of, from an N-terminal side thereof,
   (B-1) a partial sequence of a tetraspanin of SEQ ID NO: 57 (or a sequence having an amino acid sequence identity thereto of 80% or more, preferably 90% or more, more preferably 95% or more, still more preferably 98% or more, and further still more preferably 99% or more),
   (B-2) a spacer sequence of SEQ ID NO: 29,
   (B-3) a first T-cell stimulatory cytokine that is IL-2 of SEQ ID NO: 25 (or a sequence having an amino acid sequence identity thereto of 80% or more, preferably 90% or more, more preferably 95% or more, still more preferably 98% or more, and further still more preferably 99% or more),
   (B-4) a spacer sequence of SEQ ID NO: 29, and
   (B-5) a partial sequence of a tetraspanin of SEQ ID NO: 59 (or a sequence having an amino acid sequence identity thereto of 80% or more, preferably 90% or more, more preferably 95% or more, still more preferably 98% or more, and further still more preferably 99% or more),
   the fusion protein being capable of presenting the first T-cell stimulatory cytokine outside membrane;
(B') a fusion protein of which an amino acid sequence consists of, from an N-terminal side thereof,
   (B-3) a second T-cell stimulatory cytokine that is TGF-β of SEQ ID NO: 73 (or a sequence having an amino acid sequence identity thereto of 80% or more, preferably 90% or more, more preferably 95% or more, still more preferably 98% or more, and further still more preferably 99% or more),
   (B-4) a spacer sequence of SEQ ID NO: 29, and
   (B-5) MFG-E8 of SEQ ID NO: 49 (or a sequence having an amino acid sequence identity thereto of 80% or more, preferably 90% or more, more preferably 95% or more, still more preferably 98% or more, and further still more preferably 99% or more),
   the fusion protein being capable of presenting the second T-cell stimulatory cytokine outside membrane; and
(C) a fusion protein of which an amino acid sequence consists of, from an N-terminal side thereof,
   (C-1) a T-cell costimulatory molecule that is CD80 of SEQ ID NO: 67 (or a sequence having an amino acid sequence identity thereto of 80% or more, preferably 90% or more, more preferably 95% or more, still more preferably 98% or more, and further still more preferably 99% or more), and
   (C-3) a tetraspanin of SEQ ID NO: 21 (or a sequence having an amino acid sequence identity thereto of 80% or more, preferably 90% or more, more preferably 95% or more, still more preferably 98% or more, and further still more preferably 99% or more),
   the fusion protein being capable of allowing the T-cell costimulatory molecule to interact with T cells.

In an embodiment of the present invention, the antigen-presenting extracellular vesicle described in the present specification is an antigen-presenting extracellular vesicle the membrane of which contains:
(A) a protein complex capable of presenting an antigen peptide outside membrane, in which the protein complex contains:
   a fusion protein of which an amino acid sequence consists of, from an N-terminal side thereof,
   (A-1) an MHC class II molecule-restricted antigen peptide,
   (A-2) a spacer sequence of SEQ ID NO: 39,
   (A-3) an MHC class IIβ chain of SEQ ID NO: 37 (or a sequence having an amino acid sequence identity thereto of 80% or more, preferably 90% or more, more preferably 95% or more, still more preferably 98% or more, and further still more preferably 99% or more),
   (A-5) a tetraspanin of SEQ ID NO: 15 (or a sequence having an amino acid sequence identity thereto of 80% or more, preferably 90% or more, more preferably 95% or more, still more preferably 98% or more, and further still more preferably 99% or more);
   and
   (A-6) an MHC class IIα chain of SEQ ID NO: 71 (or a sequence having an amino acid sequence identity thereto of 80% or more, preferably 90% or more, more preferably 95% or more, still more preferably 98% or more, and further still more preferably 99% or more);
(B) a fusion protein capable of presenting the first T-cell stimulatory cytokine of SEQ ID NO: 31 (or a sequence having an amino acid sequence identity thereto of 80% or more, preferably 90% or more, more preferably 95% or more, still more preferably 98% or more, and further still more preferably 99% or more), outside membrane;
(B') a fusion protein capable of presenting the second T-cell stimulatory cytokine of SEQ ID NO: 75 (or a sequence having an amino acid sequence identity thereto of 80% or more, preferably 90% or more, more preferably 95% or more, still more preferably 98% or more, and further still more preferably 99% or more), outside membrane; and
(C) a fusion protein capable of allowing the T-cell costimulatory molecule of SEQ ID NO: 69 (or a sequence having an amino acid sequence identity thereto of 80% or more, preferably 90% or more, more preferably 95% or more, still more preferably 98% or more, and further still more preferably 99% or more), to interact with T cells.

In an embodiment of the present invention, the antigen-presenting extracellular vesicle described in the present specification is an antigen-presenting extracellular vesicle the membrane of which contains:
(A) a protein complex capable of presenting an antigen peptide outside membrane, in which the protein complex contains:
   a fusion protein of which an amino acid sequence consists of, from an N-terminal side thereof,
      (A-1) an MHC class II molecule-restricted antigen peptide,
      (A-2) a spacer sequence of SEQ ID NO: 39,
      (A-3) an MHC class IIβ chain of SEQ ID NO: 37 (or a sequence having an amino acid sequence identity thereto of 80% or more, preferably 90% or more, more preferably 95% or more, still more preferably 98% or more, and further still more preferably 99% or more), and
      (A-5) a tetraspanin of SEQ ID NO: 15 (or a sequence having an amino acid sequence identity thereto of 80% or more, preferably 90% or more, more preferably 95% or more, still more preferably 98% or more, and further still more preferably 99% or more);
   and
      (A-6) an MHC class IIα chain of SEQ ID NO: 71 (or a sequence having an amino acid sequence identity thereto of 80% or more, preferably 90% or more, more preferably 95% or more, still more preferably 98% or more, and further still more preferably 99% or more);
(B) a fusion protein of which an amino acid sequence consists of, from an N-terminal side thereof,
   (B-1) a partial sequence of a tetraspanin of SEQ ID NO: 61 (or a sequence having an amino acid sequence identity thereto of 80% or more, preferably 90% or more, more preferably 95% or more, still more preferably 98% or more, and further still more preferably 99% or more),
   (B-2) a spacer sequence of SEQ ID NO: 29,
   (B-3) a first T-cell stimulatory cytokine that is IL-4 of SEQ ID NO: 53 (or a sequence having an amino acid sequence identity thereto of 80% or more, preferably 90% or more, more preferably 95% or more, still more preferably 98% or more, and further still more preferably 99% or more),
   (B-4) a spacer sequence of SEQ ID NO: 29, and
   (B-5) a partial sequence of a tetraspanin of SEQ ID NO: 63 (or a sequence having an amino acid sequence identity thereto of 80% or more, preferably 90% or more, more preferably 95% or more, still more preferably 98% or more, and further still more preferably 99% or more),
   the fusion protein being capable of presenting the first T-cell stimulatory cytokine outside membrane; and
(C) a fusion protein of which an amino acid sequence consists of, from an N-terminal side thereof,
   (C-1) a T-cell costimulatory molecule that is CD80 of SEQ ID NO: 67 (or a sequence having an amino acid sequence identity thereto of 80% or more, preferably 90% or more, more preferably 95% or more, still more preferably 98% or more, and further still more preferably 99% or more), and
   (C-3) a tetraspanin of SEQ ID NO: 21 (or a sequence having an amino acid sequence identity thereto of 80% or more, preferably 90% or more, more preferably 95% or more, still more preferably 98% or more, and further still more preferably 99% or more),
   the fusion protein being capable of allowing the T-cell costimulatory molecule to interact with T cells.

In an embodiment of the present invention, the antigen-presenting extracellular vesicle described in the present specification is an antigen-presenting extracellular vesicle the membrane of which contains:
(A) a protein complex capable of presenting an antigen peptide outside membrane, in which the protein complex contains:
   a fusion protein of which an amino acid sequence consists of, from an N-terminal side thereof,
   (A-1) an MHC class II molecule-restricted antigen peptide,
   (A-2) a spacer sequence of SEQ ID NO: 39,
   (A-3) an MHC class IIβ chain of SEQ ID NO: 37 (or a sequence having an amino acid sequence identity thereto of 80% or more, preferably 90% or more, more preferably 95% or more, still more preferably 98% or more, and further still more preferably 99% or more), and
   (A-5) a tetraspanin of SEQ ID NO: 15 (or a sequence having an amino acid sequence identity thereto of 80% or more, preferably 90% or more, more preferably 95% or more, still more preferably 98% or more, and further still more preferably 99% or more);
      and
      (A-6) an MHC class IIα chain of SEQ ID NO: 71 (or a sequence having an amino acid sequence identity thereto of 80% or more, preferably 90% or more, more preferably 95% or more, still more preferably 98% or more, and further still more preferably 99% or more);
(B) a fusion protein capable of presenting the first T-cell stimulatory cytokine of SEQ ID NO: 55 (or a sequence having an amino acid sequence identity thereto of 80% or more, preferably 90% or more, more preferably 95% or more, still more preferably 98% or more, and further still more preferably 99% or more), outside membrane; and
(C) a fusion protein capable of allowing the T-cell costimulatory molecule of SEQ ID NO: 69 (or a sequence having an amino acid sequence identity thereto of 80% or more, preferably 90% or more, more preferably 95% or more, still more preferably 98% or more, and further still more preferably 99% or more), to interact with T cells.

In an embodiment of the present invention, as for the (A), (B), and (C) above, (A) and (B) may be fused to form one molecule, (B) and (C) may be fused to form one molecule, and (A), (B), and (C) are fused to form one molecule. Such a fusion molecule may be translated as one protein molecule with or without a spacer sequence between (A), (B), and (C), or the proteins of (A), (B), and (C) may be fused by chemical crosslinking (for example, a disulfide bond between cysteine residues) to form one molecule.

Alternatively, the (A), (B), and (C) above may be functionally fused by sharing an element for localizing the proteins thereof in the extracellular vesicle, that is, a site of a "membrane protein capable of being expressed in membrane of an extracellular vesicle or a transmembrane domain thereof' or a "protein capable of binding to membrane of an extracellular vesicle or a domain thereof'.

For example, in an embodiment of the present invention,
the antigen-presenting extracellular vesicle may also contain (D) a fusion protein comprising:
   (1) an antigen-presenting MHC molecule;
   (2) at least one T-cell stimulatory cytokine; and
   (3) a "membrane protein capable of being expressed in membrane of an extracellular vesicle or a transmembrane domain thereof' or a "protein capable of binding to membrane of an extracellular vesicle or a domain thereof',
the fusion protein being fused in a form of sharing the site of the "membrane protein capable of being expressed in membrane of an extracellular vesicle or the transmembrane domain thereof' or the "protein capable of binding to membrane of an extracellular vesicle or the domain thereof' in (A) and (B);
a fusion protein (F) comprising:
   (1) an antigen-presenting MHC molecule;
   (2) a T-cell costimulatory molecule; and
   (3) a "membrane protein capable of being expressed in membrane of an extracellular vesicle or a transmembrane domain thereof' or a "protein capable of binding to membrane of an extracellular vesicle or a domain thereof',
the fusion protein being fused in a form of sharing the site of the "membrane protein capable of being expressed in membrane of an extracellular vesicle or the transmembrane domain thereof' or the "protein capable of binding to membrane of an extracellular vesicle or the domain thereof' in (A) and (C);
a fusion protein (G) comprising:
   (1) at least one T-cell stimulatory cytokine;
   (2) a T-cell costimulatory molecule; and
   (3) a "membrane protein capable of being expressed in membrane of an extracellular vesicle or a transmembrane domain thereof' or a "protein capable of binding to membrane of an extracellular vesicle or a domain thereof',
the fusion protein being fused in a form of sharing the site of the "membrane protein capable of being expressed in membrane of an extracellular vesicle or the transmembrane domain thereof' or the "protein capable of binding to membrane of an extracellular vesicle or the domain thereof' in (B) and (C);
or,
a fusion protein (E) comprising:
   (1) an antigen-presenting MHC molecule;
   (2) at least one T-cell stimulatory cytokine;
   (3) a T-cell costimulatory molecule; and
   (4) a "membrane protein capable of being expressed in membrane of an extracellular vesicle or a transmembrane domain thereof' or a "protein capable of binding to membrane of an extracellular vesicle or a domain thereof',
the fusion protein being fused in a form of sharing the site of the "membrane protein capable of being expressed in membrane of an extracellular vesicle or the transmembrane domain thereof' or the "protein capable of binding to membrane of an extracellular vesicle or the domain thereof' in (A) to (C).

In an embodiment of the present invention, the antigen-presenting extracellular vesicle may be an antigen-presenting extracellular vesicle containing a fusion protein (D) having the functions of the constitutional requirement (A) and the constitutional requirement (B) using the "protein which contains a first T-cell stimulatory cytokine and is capable of presenting the first T-cell stimulatory cytokine outside membrane" of the constitutional requirement (B), instead of the "membrane protein capable of being expressed in membrane of an extracellular vesicle or the transmembrane domain thereof or the protein capable of binding to membrane of an extracellular vesicle" of the constitutional requirement (A).

Such a fusion protein (D) having the functions of the constitutional requirement (A) and the constitutional requirement (B) may be
(D) a fusion protein which contains an antigen-presenting MHC molecule and at least one T-cell stimulatory cytokine and is capable of presenting the antigen and the T-cell stimulatory cytokine outside membrane.

The fusion protein may contain the antigen-presenting MHC molecule, the at least one T-cell stimulatory cytokine, and a membrane protein capable of being localized to membrane of an extracellular vesicle or a transmembrane domain thereof or a protein capable of binding to membrane of an extracellular vesicle or a membrane-binding domain thereof.

In the fusion protein (D) having the functions of the constitutional requirement (A) and the constitutional requirement (B), the membrane protein capable of being localized to membrane of an extracellular vesicle or the protein capable of binding to membrane of an extracellular vesicle may be a tetraspanin or MFG-E8.

The fusion protein may also comprise an amino acid sequence encoding, from an N-terminal side thereof,
(D-1) an MHC molecule-restricted antigen peptide,
(D-2) a spacer sequence which may be present,
(D-3) a single chain MHC molecule,
(D-4) a spacer sequence which may be present, and
(D-5) a fusion peptide comprising a tetraspanin or a transmembrane domain thereof or MFG-E8 or a transmembrane domain thereof, and the at least one T-cell stimulatory cytokine, in this order.

The fusion protein may also comprise an amino acid sequence encoding, from an N-terminal side thereof,
(D-1) a fusion peptide comprising a tetraspanin or a transmembrane domain thereof or MFG-E8 or a transmembrane domain thereof, and the at least one T-cell stimulatory cytokine,
(D-2) a spacer sequence which may be optionally present,
(D-3) a single chain MHC molecule,
(D-4) a spacer sequence which may be optionally present, and
(D-5) an MHC molecule-restricted antigen peptide, in this order.

Here, the fusion peptide may also comprise an amino acid sequence encoding, from an N-terminal side thereof,
(1) a partial sequence of a tetraspanin containing a transmembrane domain 1, a small intracellular loop, a transmembrane domain 2, a small extracellular loop, and a transmembrane domain 3,
(2) a spacer sequence which may be optionally present,
(3) the at least one T-cell stimulatory cytokine,
(4) a spacer sequence which may be optionally present, and
(5) a partial sequence of a tetraspanin containing a transmembrane domain 4, in this order.

The fusion peptide may also comprise an amino acid sequence encoding, from an N-terminal side thereof,
(1) the at least one of T-cell stimulatory cytokine,
(2) a spacer sequence which may be optionally present, and
(3) MFG-E8, in this order.

In an embodiment of the present invention, the MHC molecule-restricted antigen peptide is an MHC class I molecule-restricted antigen peptide, the single chain MHC molecule may contain an extracellular domain of an MHC class Iα chain, the MHC molecule-restricted antigen peptide is an MHC class II molecule-restricted antigen peptide, and the single chain MHC molecule may contain an extracellular domain of an MHC class IIα chain and/or an extracellular domain of an MHC class IIβ chain.

In the aspect containing the fusion protein (D) having the functions of the constitutional requirement (A) and the constitutional requirement (B):
(C) a protein which comprises at least one T-cell costimulatory molecule and is capable of allowing the T-cell costimulatory molecule to interact with T cells may be further contained in the membrane;
the protein capable of interacting with T cells may also comprises the at least one T-cell costimulatory molecule, and a membrane protein capable of being expressed in membrane of an extracellular vesicle or a transmembrane domain thereof or a protein capable of binding to membrane of an extracellular vesicle or a domain thereof; and
the protein capable of interacting with T cells may also comprises the at least one T-cell costimulatory molecule, and a tetraspanin or a transmembrane domain thereof or MFG-E8 or a domain thereof.

In an embodiment of the present invention, the extracellular vesicle is an exosome.

The antigen-presenting extracellular vesicle in the present specification may contain or be bound to a substance that may be therapeutically beneficial (for example, a low-molecular compound, a nucleic acid, or the like) inside the membrane thereof or in the membrane. Examples of a method for encapsulating the substance inside the membrane of the extracellular vesicle include, but are not limited to, a method in which the substance and the extracellular vesicle described in the present specification are mixed in a suitable solvent.

In an embodiment of the present invention, the antigen-presenting extracellular vesicle may contain any protein preparation. The protein preparation is not particularly limited, but may be a protein that can also exist in nature such as erythropoietin, a synthetic protein that does not exist in nature such as an immunoglobulin-CTLA4 fusion protein, or a monoclonal antibody or an active fragment thereof. These protein preparations are fusion proteins with a membrane protein capable of being localized to membrane of an extracellular vesicle or a transmembrane domain thereof or a protein capable of binding to membrane of an extracellular vesicle or a membrane-binding domain thereof, and may be localized on the surface of the antigen-presenting extracellular vesicle. Such an antigen-presenting extracellular vesicle can be prepared by transfecting cells that produce antigen-presenting extracellular vesicles with a vector for expressing a fusion protein.

Each fusion protein or protein complex or a protein preparation contained in the membrane of the antigen-presenting extracellular vesicle described in the present specification may comprise one or a plurality of detectable labels. For example, the fusion protein or the protein complex or the protein preparation may be labeled with a specific lipoprotein molecule, a fluorophore, a radioactive material, or an enzyme (for example, peroxidase or phosphatase), or the like by a conventional method. These labels may be linked to the N-terminus or the C-terminus of the fusion protein or the protein complex or the protein preparation, for example, as a constituent element of the fusion protein or the protein complex or the protein preparation.

### Polynucleotide

In an embodiment of the present invention, there is provided a polynucleotide encoding each fusion protein or protein complex in (A) and (B), and (C) present in some cases that are contained in the membrane of the antigen-presenting extracellular vesicle described in the present specification. In an embodiment of the present invention, there is provided a polynucleotide encoding each fusion protein or protein complex in (A) to (G) defined in the present specification.

In an embodiment of the present invention, there is provided a polynucleotide comprising at least one sequence selected from the group consisting of:
(a) a sequence encoding a fusion protein (A) which comprises an antigen-presenting MHC molecule and is capable of presenting the antigen-presenting MHC molecule outside membrane of an extracellular vesicle;
(b) a sequence encoding a fusion protein (B) which comprises at least one T-cell stimulatory cytokine or subunit thereof and is capable of presenting the T-cell stimulatory cytokine outside membrane of an extracellular vesicle;
(c) a sequence encoding a fusion protein (C) which comprises a T-cell costimulatory molecule and is capable of presenting the T-cell costimulatory molecule outside membrane of an extracellular vesicle;
(d) a sequence encoding a fusion protein (D) which comprises an antigen-presenting MHC molecule and at least one T-cell stimulatory cytokine or subunit thereof and is capable of presenting the antigen and the T-cell stimulatory cytokine outside membrane; and
(e) a sequence encoding a fusion protein (E) which comprises an antigen-presenting MHC molecule, at least one T-cell stimulatory cytokine or subunit thereof, and a T-cell costimulatory molecule, and is capable of presenting the antigen, the T-cell stimulatory cytokine, and the T-cell costimulatory molecule outside membrane.

The sequences (a) to (e) include the sequences specifically described in the present specification and a sequence having high homology (homology of preferably 90% or more, more preferably 95% or more, and still more preferably 99% or more), but are not particularly limited thereto. Paralogs (i.e., gene sequences generated by gene duplication) or orthologs (i.e., groups of genes having homologous functions that exist in different organisms) may be used as long as they have the same functions, and sequences having modified (prohibited, deleted, substituted, or the like) sequence information are also included.

The "polynucleotide" used in the present specification means a single-stranded or double-stranded DNA molecule or RNA molecule, or the like. The polynucleotide includes genomic DNA, cDNA, hnRNA, mRNA, and the like, and all naturally occurring or artificially modified derivatives thereof. The polynucleotide may be linear or cyclic.

The polynucleotide encoding each fusion protein or protein complex in (A) to (G) described above can be appropriately determined by those skilled in the art with reference to the amino acid sequence of the fusion protein or protein complex. Note that the amino acid sequence of each fusion protein or protein complex in (A) to (G) can be appropriately determined with reference to the amino acid sequence of each constituent element (for example, in the case of (A), (A-1) to (A-5), and (A-6) in some cases) in each fusion protein or protein complex. Any type of codon can be selected for use in determining a polynucleotide. For example, a polynucleotide may be determined in consideration of a frequency or the like of codons of cells to be transformed using a vector comprising the polynucleotide.

To the N-terminus of the polynucleotide encoding each fusion protein or protein complex in (A) to (G) described above, a polynucleotide encoding a signal peptide (signal sequence) may be added, if necessary.

As an amino acid sequence of the signal peptide, any amino acid sequence can be used, and for example, the amino acid sequence of the signal peptide may be determined in consideration of an amino acid sequence of a fusion protein to be expressed, and the like. Examples of the polynucleotide encoding a signal peptide include a polynucleotide (for example, SEQ ID NO: 2) encoding a signal peptide (for example, SEQ ID NO: 1) of β₂ microglobulin, a polynucleotide encoding a signal peptide of an MHC class Iα chain, a polynucleotide encoding a signal peptide of an MHC class IIα chain, and a polynucleotide (for example, SEQ ID NO: 34) encoding a signal peptide (for example, SEQ ID NO: 33) of an MHC class IIβ chain.

Information on each constituent element (for example, in the case of (A), (A-1) to (A-5), and (A-6) in some cases) of each fusion protein or protein complex in (A) to (G) described above, the amino acid sequence such as a signal peptide, and the polynucleotide encoding them may be appropriately obtained by searching, for example, a database of known literatures, NCBI (http://www.ncbi.nlm.nih.gov/guide/), and the like. In addition, for the amino acid sequence in the partial sequence of the tetraspanin (for example, the partial sequences in (C-1) and (C-5)) and the polynucleotide encoding the amino acid sequence, WO 2016/139354 A may be referred to.

In an embodiment of the present invention, there is provided a polynucleotide encoding any one of:
(A) a fusion protein capable of presenting an antigen peptide outside membrane, in which the fusion protein comprises an amino acid sequence consisting of, from an N-terminal side thereof,
   (A-1) an MHC molecule-restricted antigen peptide,
   (A-2) a spacer sequence which may be optionally present,
   (A-3) a single chain MHC molecule,
   (A-4) a spacer sequence which may be optionally present, and
   (A-5) a tetraspanin, or
(A) a fusion protein constituting a protein complex capable of presenting an antigen peptide outside membrane,
in which the fusion protein comprises an amino acid sequence consisting of, from an N-terminal side thereof,
   (A-1) an MHC molecule-restricted antigen peptide,
   (A-2) a spacer sequence which may be optionally present,
   (A-3) an MHC class Iα chain, β₂ microglobulin, an MHC class IIα chain, or an MHC class IIβ chain,
   (A-4) a spacer sequence which may be optionally present, and
   (A-5) a tetraspanin;
(B) a fusion protein comprising an amino acid sequence consisting of, from an N-terminal side thereof,
   (B-1) a partial sequence of a tetraspanin containing, from an N-terminal side thereof, a transmembrane domain 1, a small extracellular loop, a transmembrane domain 2, a small intracellular loop, and a transmembrane domain 3,
   (B-2) a spacer sequence which may be optionally present,
   (B-3) a first T-cell stimulatory cytokine,
   (B-4) a spacer sequence which may be optionally present, and
   (B-5) a partial sequence of a tetraspanin containing a transmembrane domain 4,
the fusion protein being capable of presenting the first T-cell stimulatory cytokine outside membrane, or
(B) a fusion protein comprising an amino acid sequence consisting of, from an N-terminal side thereof,
   (B-3) a first T-cell stimulatory cytokine,
   (B-4) a spacer sequence which may be present, and
   (B-5) MFG-E8,
the fusion protein being capable of presenting the first T-cell stimulatory cytokine outside membrane; and
(C) a fusion protein comprising an amino acid sequence consisting of, from an N-terminal side thereof,
   (C-1) a T-cell costimulatory molecule,
   (C-2) a spacer sequence which may be present, and
   (C-3) a tetraspanin,
the fusion protein being capable of allowing the T-cell costimulatory molecule to interact with T cells.

In an embodiment of the present invention, there provided a polynucleotide encoding any one of:
(A) a fusion protein of which an amino acid sequence consists of, from an N-terminal side thereof,
   (A-1) an MHC class I molecule-restricted antigen peptide,
   (A-2) a spacer sequence of SEQ ID NO: 5,
   (A-3) a single chain MHC class I molecule of SEQ ID NO: 65 (or a sequence having an amino acid sequence identity thereto of 80% or more, preferably 90% or more, more preferably 95% or more, still more preferably 98% or more, and further still more preferably 99% or more), and
   (A-5) a tetraspanin of SEQ ID NO: 15 (or a sequence having an amino acid sequence identity thereto of 80% or more, preferably 90% or more, more preferably 95% or more, still more preferably 98% or more, and further still more preferably 99% or more),
the fusion protein being capable of presenting an antigen peptide outside membrane, or
(A) a fusion protein
of which an amino acid sequence consists of, from an N-terminal side thereof,
   (A-1) an MHC class II molecule-restricted antigen peptide,
   (A-2) a spacer sequence of SEQ ID NO: 39,
   (A-3) an MHC class IIβ chain of SEQ ID NO: 37 (or a sequence having an amino acid sequence identity thereto of 80% or more, preferably 90% or more, more preferably 95% or more, still more preferably 98% or more, and further still more preferably 99% or more), and
   (A-5) a tetraspanin of SEQ ID NO: 15 (or a sequence having an amino acid sequence identity thereto of 80% or more, preferably 90% or more, more preferably 95% or more, still more preferably 98% or more, and further still more preferably 99% or more),
the fusion protein constituting a protein complex capable of presenting an antigen peptide outside membrane;
(B) a fusion protein of which an amino acid sequence consists of, from an N-terminal side thereof,
   (B-1) a partial sequence of a tetraspanin of SEQ ID NO: 57 (or a sequence having an amino acid sequence identity thereto of 80% or more, preferably 90% or more, more preferably 95% or more, still more preferably 98% or more, and further still more preferably 99% or more),
   (B-2) a spacer sequence of SEQ ID NO: 29,
   (B-3) a first T-cell stimulatory cytokine that is IL-2 of SEQ ID NO: 25 (or a sequence having an amino acid sequence identity thereto of 80% or more, preferably 90% or more, more preferably 95% or more, still more preferably 98% or more, and further still more preferably 99% or more),
   (B-4) a spacer sequence of SEQ ID NO: 29, and
   (B-5) a partial sequence of a tetraspanin of SEQ ID NO: 59 (or a sequence having an amino acid sequence identity thereto of 80% or more, preferably 90% or more, more preferably 95% or more, still more preferably 98% or more, and further still more preferably 99% or more),
the fusion protein being capable of presenting the first T-cell stimulatory cytokine outside membrane,
(B) a fusion protein of which an amino acid sequence consists of, from an N-terminal side thereof,
   (B-1) a partial sequence of a tetraspanin of SEQ ID NO: 61 (or a sequence having an amino acid sequence identity thereto of 80% or more, preferably 90% or more, more preferably 95% or more, still more preferably 98% or more, and further still more preferably 99% or more),
   (B-2) a spacer sequence of SEQ ID NO: 29,
   (B-3) a first T-cell stimulatory cytokine that is IL-4 of SEQ ID NO: 53 (or a sequence having an amino acid sequence identity thereto of 80% or more, preferably 90% or more, more preferably 95% or more, still more preferably 98% or more, and further still more preferably 99% or more),
   (B-4) a spacer sequence of SEQ ID NO: 29, and
   (B-5) a partial sequence of a tetraspanin of SEQ ID NO: 63 (or a sequence having an amino acid sequence identity thereto of 80% or more, preferably 90% or more, more preferably 95% or more, still more preferably 98% or more, and further still more preferably 99% or more),
the fusion protein being capable of presenting the first T-cell stimulatory cytokine outside membrane, or
(B') a fusion protein of which an amino acid sequence consists of, from an N-terminal side thereof,
   (B-3) a second T-cell stimulatory cytokine that is TGF-β of SEQ ID NO: 73 (or a sequence having an amino acid sequence identity thereto of 80% or more, preferably 90% or more, more preferably 95% or more, still more preferably 98% or more, and further still more preferably 99% or more),
   (B-4) a spacer sequence of SEQ ID NO: 29, and
   (B-5) MFG-E8 of SEQ ID NO: 49 (or a sequence having an amino acid sequence identity thereto of 80% or more, preferably 90% or more, more preferably 95% or more, still more preferably 98% or more, and further still more preferably 99% or more),
the fusion protein being capable of presenting the second (or first) T-cell stimulatory cytokine outside membrane; and
(C) a fusion protein of which an amino acid sequence consists of, from an N-terminal side thereof,
   (C-1) a T-cell costimulatory molecule that is CD80 of SEQ ID NO: 67 (or a sequence having an amino acid sequence identity thereto of 80% or more, preferably 90% or more, more preferably 95% or more, still more preferably 98% or more, and further still more preferably 99% or more), and
   (C-3) a tetraspanin of SEQ ID NO: 21 (or a sequence having an amino acid sequence identity thereto of 80% or more, preferably 90% or more, more preferably 95% or more, still more preferably 98% or more, and further still more preferably 99% or more),
the fusion protein being capable of allowing the T-cell costimulatory molecule to interact with T cells.

In an embodiment of the present invention, a polynucleotide encoding any one of:
(A) a fusion protein of which an amino acid sequence consists of, from an N-terminal side thereof,
   (A-1) an MHC class I molecule-restricted antigen peptide,
   (A-2) a spacer sequence of SEQ ID NO: 5,
   (A-3) a single chain MHC class I molecule of SEQ ID NO: 65 (or a sequence having an amino acid sequence identity thereto of 80% or more, preferably 90% or more, more preferably 95% or more, still more preferably 98% or more, and further still more preferably 99% or more), and
   (A-5) a tetraspanin of SEQ ID NO: 15 (or a sequence having an amino acid sequence identity thereto of 80% or more, preferably 90% or more, more preferably 95% or more, still more preferably 98% or more, and further still more preferably 99% or more),
the fusion protein being capable of presenting an antigen peptide outside membrane, or
(A) a fusion protein
of which an amino acid sequence consists of, from an N-terminal side thereof,
   (A-1) an MHC class II molecule-restricted antigen peptide,
   (A-2) a spacer sequence of SEQ ID NO: 39,
   (A-3) an MHC class IIβ chain of SEQ ID NO: 37 (or a sequence having an amino acid sequence identity thereto of 80% or more, preferably 90% or more, more preferably 95% or more, still more preferably 98% or more, and further still more preferably 99% or more), and
   (A-5) a tetraspanin of SEQ ID NO: 15 (or a sequence having an amino acid sequence identity thereto of 80% or more, preferably 90% or more, more preferably 95% or more, still more preferably 98% or more, and further still more preferably 99% or more),
the fusion protein constituting a protein complex capable of presenting an antigen peptide outside membrane;
(B) a fusion protein capable of presenting the first (or second) T-cell stimulatory cytokine of SEQ ID NO: 31, 75, or 55 (or a sequence having an amino acid sequence identity thereto of 80% or more, preferably 90% or more, more preferably 95% or more, still more preferably 98% or more, and further still more preferably 99% or more), outside membrane; and
(C) a fusion protein capable of allowing the T-cell costimulatory molecule of SEQ ID NO: 23 (or a sequence having an amino acid sequence identity thereto of 80% or more, preferably 90% or more, more preferably 95% or more, still more preferably 98% or more, and further still more preferably 99% or more), to interact with T cells.

In an embodiment of the present invention, there are provided polynucleotides including:
(A) a polynucleotide encoding a fusion protein capable of presenting an antigen peptide outside membrane, in which the polynucleotide comprises a sequence consisting of,
   (A-1) a polynucleotide encoding an MHC class I molecule-restricted antigen peptide,
   (A-2) a spacer sequence of SEQ ID NO: 6,
   (A-3) a single chain MHC class I molecule of SEQ ID NO: 66 (or a sequence having a sequence identity thereto of 80% or more, preferably 90% or more, more preferably 95% or more, still more preferably 98% or more, and further still more preferably 99% or more), and
   (A-5) a tetraspanin of SEQ ID NO: 16 (or a sequence having a sequence identity thereto of 80% or more, preferably 90% or more, more preferably 95% or more, still more preferably 98% or more, and further still more preferably 99% or more), or
(A) a polynucleotide encoding a fusion protein constituting a protein complex capable of presenting an antigen peptide outside membrane, in which the polynucleotide comprises a sequence consisting of,
   (A-1) a polynucleotide encoding an MHC class II molecule-restricted antigen peptide,
   (A-2) a spacer sequence of SEQ ID NO: 40,
   (A-3) an MHC class IIβ chain of SEQ ID NO: 38 (or a sequence having a sequence identity thereto of 80% or more, preferably 90% or more, more preferably 95% or more, still more preferably 98% or more, and further still more preferably 99% or more), and
   (A-5) a tetraspanin of SEQ ID NO: 16 (or a sequence having a sequence identity thereto of 80% or more, preferably 90% or more, more preferably 95% or more, still more preferably 98% or more, and further still more preferably 99% or more);
(B) a polynucleotide encoding a fusion protein capable of presenting a first T-cell stimulatory cytokine outside membrane, in which the polynucleotide comprises a sequence consisting of,
   (B-1) a partial sequence of a tetraspanin of SEQ ID NO: 58 (or a sequence having a sequence identity thereto of 80% or more, preferably 90% or more, more preferably 95% or more, still more preferably 98% or more, and further still more preferably 99% or more),
   (B-2) a spacer sequence of SEQ ID NO: 30,
   (B-3) a first T-cell stimulatory cytokine that is IL-2 of SEQ ID NO: 26 (or a sequence having a sequence identity thereto of 80% or more, preferably 90% or more, more preferably 95% or more, still more preferably 98% or more, and further still more preferably 99% or more),
   (B-4) a spacer sequence of SEQ ID NO: 30, and
   (B-5) a partial sequence of a tetraspanin of SEQ ID NO: 60 (or a sequence having a sequence identity thereto of 80% or more, preferably 90% or more, more preferably 95% or more, still more preferably 98% or more, and further still more preferably 99% or more),
(B) a polynucleotide encoding a fusion protein capable of presenting a first T-cell stimulatory cytokine outside membrane, in which the polynucleotide comprises a sequence consisting of,
   (B-1) a partial sequence of a tetraspanin of SEQ ID NO: 62 (or a sequence having a sequence identity thereto of 80% or more, preferably 90% or more, more preferably 95% or more, still more preferably 98% or more, and further still more preferably 99% or more),
   (B-2) a spacer sequence of SEQ ID NO: 30,
   (B-3) a first T-cell stimulatory cytokine that is IL-4 of SEQ ID NO: 54 (or a sequence having a sequence identity thereto of 80% or more, preferably 90% or more, more preferably 95% or more, still more preferably 98% or more, and further still more preferably 99% or more),
   (B-4) a spacer sequence of SEQ ID NO: 30, and
   (B-5) a partial sequence of a tetraspanin of SEQ ID NO: 64 (or a sequence having a sequence identity thereto of 80% or more, preferably 90% or more, more preferably 95% or more, still more preferably 98% or more, and further still more preferably 99% or more), or
(B') a polynucleotide encoding a fusion protein capable of presenting a second (or first) T-cell stimulatory cytokine outside membrane, in which the polynucleotide comprises a sequence consisting of,
   (B-3) a second (or first) T-cell stimulatory cytokine that is TGF-β of SEQ ID NO: 74 (or a sequence having a sequence identity thereto of 80% or more, preferably 90% or more, more preferably 95% or more, still more preferably 98% or more, and further still more preferably 99% or more),
   (B-4) a spacer sequence of SEQ ID NO: 30, and
   (B-5) MFG-E8 of SEQ ID NO: 50 (or a sequence having a sequence identity thereto of 80% or more, preferably 90% or more, more preferably 95% or more, still more preferably 98% or more, and further still more preferably 99% or more); and
(C) a polynucleotide encoding a fusion protein capable of allowing the T-cell costimulatory molecule to interact with T cells, in which the polynucleotide comprises a sequence consisting of,
   (C-1) a T-cell costimulatory molecule that is CD80 of SEQ ID NO: 68 (or a sequence having a sequence identity thereto of 80% or more, preferably 90% or more, more preferably 95% or more, still more preferably 98% or more, and further still more preferably 99% or more), and
   (C-3) a tetraspanin of SEQ ID NO: 22 (or a sequence having a sequence identity thereto of 80% or more, preferably 90% or more, more preferably 95% or more, still more preferably 98% or more, and further still more preferably 99% or more).

In an embodiment of the present invention, there is provided a polynucleotide encoding:
(A) a polynucleotide encoding a fusion protein capable of presenting an antigen peptide outside membrane, in which the polynucleotide comprises a sequence consisting of,
   (A-1) a polynucleotide encoding an MHC class I molecule-restricted antigen peptide,
   (A-2) a spacer sequence of SEQ ID NO: 6,
   (A-3) a single chain MHC class I molecule of SEQ ID NO: 66 (or a sequence having a sequence identity thereto of 80% or more, preferably 90% or more, more preferably 95% or more, still more preferably 98% or more, and further still more preferably 99% or more), and
   (A-5) a tetraspanin of SEQ ID NO: 16 (or a sequence having a sequence identity thereto of 80% or more, preferably 90% or more, more preferably 95% or more, still more preferably 98% or more, and further still more preferably 99% or more), or
(A) a polynucleotide encoding a fusion protein constituting a protein complex capable of presenting an antigen peptide outside membrane, in which the polynucleotide comprises a sequence consisting of,
   (A-1) a polynucleotide encoding an MHC class II molecule-restricted antigen peptide,
   (A-2) a spacer sequence of SEQ ID NO: 40,
   (A-3) an MHC class IIβ chain of SEQ ID NO: 38 (or a sequence having a sequence identity thereto of 80% or more, preferably 90% or more, more preferably 95% or more, still more preferably 98% or more, and further still more preferably 99% or more), and
   (A-5) a tetraspanin of SEQ ID NO: 16 (or a sequence having a sequence identity thereto of 80% or more, preferably 90% or more, more preferably 95% or more, still more preferably 98% or more, and further still more preferably 99% or more);
(B) a polynucleotide encoding a fusion protein capable of presenting the first (or second) T-cell stimulatory cytokine of SEQ ID NO: 32, 76, or 56 (or a sequence having a sequence identity thereto of 80% or more, preferably 90% or more, more preferably 95% or more, still more preferably 98% or more, and further still more preferably 99% or more), outside membrane; or
(C) a polynucleotide encoding a fusion protein capable of allowing the T-cell costimulatory molecule of SEQ ID NO: 24 (or a sequence having a sequence identity thereto of 80% or more, preferably 90% or more, more preferably 95% or more, still more preferably 98% or more, and further still more preferably 99% or more), to interact with T cells.

As an embodiment of the present invention,
there is provided a polynucleotide in which the fusion protein defined as (D) comprises the antigen-presenting MHC molecule, the at least one T-cell stimulatory cytokine or subunit thereof, and a membrane protein capable of being localized to membrane of an extracellular vesicle or a transmembrane domain thereof or a protein capable of binding to membrane of an extracellular vesicle or a membrane-binding domain thereof.

The membrane protein capable of being localized to membrane of an extracellular vesicle or the protein capable of binding to membrane of an extracellular vesicle may be a tetraspanin or MFG-E8.

As an embodiment of the present invention,
there is provided a polynucleotide in which the fusion protein defined as (D) comprises an amino acid sequence encoding, from an N-terminal side thereof,
(D-1) an MHC molecule-restricted antigen peptide,
(D-2) a spacer sequence which may be present,
(D-3) a single chain MHC molecule,
(D-4) a spacer sequence which may be present, and
(D-5) a fusion peptide comprising a tetraspanin or a transmembrane domain thereof or MFG-E8 or a transmembrane domain thereof, and the at least one T-cell stimulatory cytokine or subunit thereof, in this order (here, each of the constituent elements (D-1) to (D-5) has the aspect described in the present specification),
or,
a polynucleotide in which the fusion protein defined as (D) contains an amino acid sequence encoding, from an N-terminal side thereof,
(D-1) a fusion peptide comprising a tetraspanin or a transmembrane domain thereof or MFG-E8 or a transmembrane domain thereof, and the at least one T-cell stimulatory cytokine or subunit thereof,
(D-2) a spacer sequence which may be present,
(D-3) a single chain MHC molecule,
(D-4) a spacer sequence which may be present, and
(D-5) an MHC molecule-restricted antigen peptide, in this order (here, each of the constituent elements (D-1) to (D-5) has the aspect described in the present specification).

In the aspect, the fusion peptide comprising a tetraspanin or a transmembrane domain thereof or MFG-E8 or a transmembrane domain thereof, and the at least one T-cell stimulatory cytokine or subunit thereof may contain an amino acid sequence encoding, from an N-terminal side thereof,
(1) a partial sequence of a tetraspanin containing a transmembrane domain 1, a small extracellular loop, a transmembrane domain 2, a small intracellular loop, and a transmembrane domain 3,
(2) a spacer sequence which may be present,
(3) the at least one T-cell stimulatory cytokine or subunit thereof,
(4) a spacer sequence which may be present, and
(5) a partial sequence of a tetraspanin containing a transmembrane domain 4, in this order,
or,
the fusion peptide comprising a tetraspanin or a transmembrane domain thereof or MFG-E8 or a transmembrane domain thereof, and the at least one T-cell stimulatory cytokine or subunit thereof may contain an amino acid sequence encoding, from an N-terminal side thereof,
(1) the at least one T-cell stimulatory cytokine or subunit thereof,
(2) a spacer sequence which may be present, and
(3) MFG-E8, in this order.

Here, the MHC molecule-restricted antigen peptide may be an MHC class I molecule-restricted antigen peptide, the single chain MHC molecule may contain an extracellular domain of an MHC class Iα chain, the MHC molecule-restricted antigen peptide may be an MHC class II molecule-restricted antigen peptide, and the single chain MHC molecule may contain an extracellular domain of an MHC class IIα chain and/or an extracellular domain of an MHC class IIβ chain.

In an embodiment of the present invention, there is provided a polynucleotide containing the sequence defined as (a) and the sequence defined as (b).

The polynucleotide may further contain the sequence defined as (c).

In an embodiment of the present invention,
there is provided a polynucleotide containing the sequence defined as (d).

Examples of such a sequence include a nucleic acid sequence of SEQ ID NO: 136 encoding an amino acid sequence of SEQ ID NO: 135.

In the aspect, the polynucleotide may contain the sequence defined as (c).

In an embodiment of the present invention, there is provided a polynucleotide containing the sequence defined as (e).

In an embodiment of the present invention, as for the (A), (B), and (C) above, (A) and (B) may be fused to form a polynucleotide encoding fusion proteins to be one molecule, (B) and (C) may be fused to form a polynucleotide encoding fusion proteins to be one molecule, and (A), (B), and (C) may be fused to form a polynucleotide encoding fusion proteins to be one molecule. Such a polynucleotide may encode one fusion protein with or without a spacer sequence between (A), (B), and (C).

Alternatively, the polynucleotide in an embodiment of the present invention may encode a fusion protein obtained by functionally fusing the (A), (B), and (C) above by sharing an element for localizing the proteins thereof in the extracellular vesicle, that is, a site of a "membrane protein capable of being expressed in membrane of an extracellular vesicle or a transmembrane domain thereof' or a "protein capable of binding to membrane of an extracellular vesicle or a domain thereof'.

For example, in an embodiment of the present invention,
the polynucleotide may be a polynucleotide encoding (D) a fusion protein comprising:
   (1) an antigen-presenting MHC molecule;
   (2) at least one T-cell stimulatory cytokine; and
   (3) a "membrane protein capable of being expressed in membrane of an extracellular vesicle or a transmembrane domain thereof' or a "protein capable of binding to membrane of an extracellular vesicle or a domain thereof',
the fusion protein being fused in a form of sharing the site of the "membrane protein capable of being expressed in membrane of an extracellular vesicle or the transmembrane domain thereof' or the "protein capable of binding to membrane of an extracellular vesicle or the domain thereof' in (A) and (B);
a polynucleotide encoding a fusion protein (F) containing:
   (1) an antigen-presenting MHC molecule;
   (2) a T-cell costimulatory molecule; and
   (3) a "membrane protein capable of being expressed in membrane of an extracellular vesicle or a transmembrane domain thereof' or a "protein capable of binding to membrane of an extracellular vesicle or a domain thereof',
the fusion protein being fused in a form of sharing the site of the "membrane protein capable of being expressed in membrane of an extracellular vesicle or the transmembrane domain thereof' or the "protein capable of binding to membrane of an extracellular vesicle or the domain thereof' in (A) and (C);
a polynucleotide encoding a fusion protein (G) containing:
   (1) at least one T-cell stimulatory cytokine;
   (2) a T-cell costimulatory molecule; and
   (3) a "membrane protein capable of being expressed in membrane of an extracellular vesicle or a transmembrane domain thereof' or a "protein capable of binding to membrane of an extracellular vesicle or a domain thereof',
the fusion protein being fused in a form of sharing the site of the "membrane protein capable of being expressed in membrane of an extracellular vesicle or the transmembrane domain thereof' or the "protein capable of binding to membrane of an extracellular vesicle or the domain thereof' in (B) and (C);
or,
a polynucleotide encoding a fusion protein (E) containing:
   (1) an antigen-presenting MHC molecule;
   (2) at least one T-cell stimulatory cytokine;
   (2) a T-cell costimulatory molecule; and
   (4) a "membrane protein capable of being expressed in membrane of an extracellular vesicle or a transmembrane domain thereof' or a "protein capable of binding to membrane of an extracellular vesicle or a domain thereof',
the fusion protein being fused in a form of sharing the site of the "membrane protein capable of being expressed in membrane of an extracellular vesicle or the transmembrane domain thereof' or the "protein capable of binding to membrane of an extracellular vesicle or the domain thereof' in (A) to (C).

In an embodiment of the present invention, the polynucleotide may be a polynucleotide encoding a fusion protein which contains an antigen-presenting MHC molecule and at least one T-cell stimulatory cytokine and is capable of presenting the antigen and the T-cell stimulatory cytokine, the fusion protein being the fusion protein (D) having the functions of the constitutional requirement (A) and the constitutional requirement (B) using the protein of the constitutional requirement (B) which contains a first T-cell stimulatory cytokine and is capable of presenting the first T-cell stimulatory cytokine outside membrane, instead of the membrane protein capable of being expressed in membrane of an extracellular vesicle or the transmembrane domain thereof or the protein capable of binding to membrane of an extracellular vesicle of the constitutional requirement (A).

Such a fusion protein (D) having the functions of the constitutional requirement (A) and the constitutional requirement (B) may be a fusion protein which contains an antigen-presenting MHC molecule and at least one T-cell stimulatory cytokine and is capable of presenting the antigen and the T-cell stimulatory cytokine outside membrane.

The fusion protein may comprise the antigen-presenting MHC molecule, the at least one T-cell stimulatory cytokine, and a membrane protein capable of being localized to membrane of an extracellular vesicle or a transmembrane domain thereof or a protein capable of binding to membrane of an extracellular vesicle or a membrane-binding domain thereof.

In the fusion protein (D) having the functions of the constitutional requirement (A) and the constitutional requirement (B), the membrane protein capable of being localized to membrane of an extracellular vesicle or the protein capable of binding to membrane of an extracellular vesicle may be a tetraspanin or MFG-E8.

The fusion protein may also comprise an amino acid sequence encoding, from an N-terminal side thereof,
(D-1) an MHC molecule-restricted antigen peptide,
(D-2) a spacer sequence which may be optionally present,
(D-3) a single chain MHC molecule,
(D-4) a spacer sequence which may be optionally present, and
(D-5) a fusion peptide comprising a tetraspanin or a transmembrane domain thereof or MFG-E8 or a transmembrane domain thereof, and the at least one T-cell stimulatory cytokine, in this order.

The fusion protein may also contain an amino acid sequence encoding, from an N-terminal side thereof,
(D-1) a fusion peptide comprising a tetraspanin or a transmembrane domain thereof or MFG-E8 or a transmembrane domain thereof, and the at least one T-cell stimulatory cytokine,
(D-2) a spacer sequence which may be optionally present,
(D-3) a single chain MHC molecule,
(D-4) a spacer sequence which may be optionally present, and
(D-5) an MHC molecule-restricted antigen peptide, in this order.

Here, the fusion peptide may also comprise an amino acid sequence encoding, from an N-terminal side thereof,
(1) a partial sequence of a tetraspanin containing a transmembrane domain 1, a small intracellular loop, a transmembrane domain 2, a small extracellular loop, and a transmembrane domain 3,
(2) a spacer sequence which may be present,
(3) the at least one T-cell stimulatory cytokine,
(4) a spacer sequence which may be present, and
(5) a partial sequence of a tetraspanin containing a transmembrane domain 4, in this order.

The fusion peptide may also comprise an amino acid sequence encoding, from an N-terminal side thereof,
(1) the at least one of T-cell stimulatory cytokine,
(2) a spacer sequence which may be present, and
(3) MFG-E8, in this order.

In an embodiment of the present invention, the MHC molecule-restricted antigen peptide is an MHC class I molecule-restricted antigen peptide, the single chain MHC molecule may contain an extracellular domain of an MHC class Iα chain, the MHC molecule-restricted antigen peptide is an MHC class II molecule-restricted antigen peptide, and the single chain MHC molecule may contain an extracellular domain of an MHC class IIα chain and/or an extracellular domain of an MHC class IIβ chain.

In the aspect containing the fusion protein (D) having the functions of the constitutional requirement (A) and the constitutional requirement (B):
(C) a protein which contains at least one T-cell costimulatory molecule and is capable of allowing the T-cell costimulatory molecule to interact with T cells may be further contained in the membrane;
the protein capable of interacting with T cells may also contain the at least one T-cell costimulatory molecule, and a membrane protein capable of being expressed in membrane of an extracellular vesicle or a transmembrane domain thereof or a protein capable of binding to membrane of an extracellular vesicle or a domain thereof; and
the protein capable of interacting with T cells may also contain the at least one T-cell costimulatory molecule, and a tetraspanin or a transmembrane domain thereof or MFG-E8 or a domain thereof.

### Vector and Kit

In an embodiment of the present invention, there is provided a vector comprising at least one polynucleotide selected from the polynucleotides described in the present specification.

The "vector" used in the present specification means any vector (examples thereof include, but are not limited to, a plasmid vector, a cosmid vectors a phage vector such as a phage, a viral vector such as an adenovirus vector or a baculovirus vector, and an artificial chromosome vector). The vector includes an expression vector, a cloning vector, and the like. The expression vector may generally contain a desired coding sequence and an appropriate polynucleotide required for expression of an operably linked coding sequence in a host organism (for example, a plant, an insect, an animal, or the like) or in an in vitro expression system. The cloning vector may be used to manipulate and/or amplify a desired polynucleotide fragment. The cloning vector may delete functional sequences required for expression of a desired polynucleotide fragment.

In an embodiment of the invention, all the polynucleotides described in the present specification may be inserted into the same vector, or two or more polynucleotides may be inserted into different vectors, as long as they can be operably inserted. In an embodiment of the present invention, there is provided a kit containing a combination of two or more vectors containing at least one polynucleotide selected from the polynucleotides described in the present specification.

### Transformed cells

In an embodiment of the present invention, there is provided a cell transformed with a vector comprising,
(i) a polynucleotide encoding the fusion protein or the protein complex of (A) described in the present specification,
(ii) a polynucleotide encoding the fusion protein of (B) described in the present specification, and
(iii) a polynucleotide encoding the fusion protein of (C) described in the present specification.

In an embodiment of the present invention, there is provided a cell transformed with a single vector or a combination of two or more vectors, the vector comprising,
(i) a polynucleotide encoding the fusion protein or the protein complex of (A) described in the present specification,
(ii) a polynucleotide encoding the fusion protein of (B) described in the present specification, and optionally,
(iii) a polynucleotide encoding the fusion protein of (C) described in the present specification.

In the cell of an embodiment of the present invention, as for the (A), (B), and (C) above, the cell may be transformed with a vector comprising a polynucleotide encoding fusion proteins to be one molecule obtained by fusing (A) and (B), a vector comprising a polynucleotide encoding fusion proteins to be one molecule obtained by fusing (B) and (C), or a vector comprising a polynucleotide encoding fusion proteins to be one molecule obtained by fusing (A), (B), and (C). Such a polynucleotide may encode one fusion protein with or without a spacer sequence between (A), (B), and (C).

Alternatively, the polynucleotide may encode a fusion protein obtained by functionally fusing the (A), (B), and (C) above by sharing an element for localizing the proteins thereof in the extracellular vesicle, that is, a site of a "membrane protein capable of being expressed in membrane of an extracellular vesicle or a transmembrane domain thereof' or a "protein capable of binding to membrane of an extracellular vesicle or a domain thereof'.

For example, in an embodiment of the present invention,
the cell may be transformed with a vector comprising a polynucleotide encoding a fusion protein (D) comprising:
   (1) an antigen-presenting MHC molecule;
   (2) at least one T-cell stimulatory cytokine; and
   (3) a "membrane protein capable of being expressed in membrane of an extracellular vesicle or a transmembrane domain thereof' or a "protein capable of binding to membrane of an extracellular vesicle or a domain thereof',
the fusion protein being fused in a form of sharing the site of the "membrane protein capable of being expressed in membrane of an extracellular vesicle or the transmembrane domain thereof' or the "protein capable of binding to membrane of an extracellular vesicle or the domain thereof' in (A) and (B);
a vector comprising a polynucleotide encoding a fusion protein (F) comprising:
   (1) an antigen-presenting MHC molecule;
   (2) a T-cell costimulatory molecule; and
   (3) a "membrane protein capable of being expressed in membrane of an extracellular vesicle or a transmembrane domain thereof' or a "protein capable of binding to membrane of an extracellular vesicle or a domain thereof',
the fusion protein being fused in a form of sharing the site of the "membrane protein capable of being expressed in membrane of an extracellular vesicle or the transmembrane domain thereof' or the "protein capable of binding to membrane of an extracellular vesicle or the domain thereof' in (A) and (C);
a vector comprising a polynucleotide encoding a fusion protein (G) comprising:
   (1) at least one T-cell stimulatory cytokine;
   (2) a T-cell costimulatory molecule; and
   (3) a "membrane protein capable of being expressed in membrane of an extracellular vesicle or a transmembrane domain thereof' or a "protein capable of binding to membrane of an extracellular vesicle or a domain thereof',
the fusion protein being fused in a form of sharing the site of the "membrane protein capable of being expressed in membrane of an extracellular vesicle or the transmembrane domain thereof' or the "protein capable of binding to membrane of an extracellular vesicle or the domain thereof' in (B) and (C);
or,
a vector comprising a polynucleotide encoding a fusion protein (E) comprising:
   (1) an antigen-presenting MHC molecule;
   (2) at least one T-cell stimulatory cytokine;
   (3) a T-cell costimulatory molecule; and
   (4) a "membrane protein capable of being expressed in membrane of an extracellular vesicle or a transmembrane domain thereof' or a "protein capable of binding to membrane of an extracellular vesicle or a domain thereof',
the fusion protein being fused in a form of sharing the site of the "membrane protein capable of being expressed in membrane of an extracellular vesicle or the transmembrane domain thereof' or the "protein capable of binding to membrane of an extracellular vesicle or the domain thereof' in (A) to (C).

Alternatively, in an embodiment of the present invention,
there is provided a cell transformed with a vector comprising,
(iv) a polynucleotide encoding the fusion protein of (D) described in the present specification, in which the fusion protein contains an antigen-presenting MHC molecule and at least one T-cell stimulatory cytokine and is capable of presenting the antigen and the T-cell stimulatory cytokine outside membrane, the fusion protein being the fusion protein having the functions of the constitutional requirement (A) and the constitutional requirement (B) using the protein of the constitutional requirement (B) which contains a first T-cell stimulatory cytokine and is capable of presenting the first T-cell stimulatory cytokine outside membrane, instead of the membrane protein capable of being expressed in membrane of an extracellular vesicle or the transmembrane domain thereof or the protein capable of binding to membrane of an extracellular vesicle of the constitutional requirement (A).

The expression "transformed with a single vector or a combination of two or more vectors" means that, for example, the cell may be transformed with a single vector in which all the polynucleotides (i) to (iv) are inserted into the same vector, or may be transformed with a combination of two or more vectors in which two or more of the polynucleotides (i) to (iv) are inserted into different vectors.

In a case where (A) is a fusion protein, examples of "a single vector or a combination of two or more vectors" include the followings:
- a vector comprising a polynucleotide encoding the fusion protein of (A) and a polynucleotide encoding the fusion protein of (B);
- a combination of a vector comprising a polynucleotide encoding the fusion protein of (A) and a vector comprising a polynucleotide encoding the fusion protein of (B);
- a vector comprising a polynucleotide encoding the fusion protein of (A), a polynucleotide encoding the fusion protein of (B), and a polynucleotide encoding the fusion protein of (C);
- a combination of a vector comprising a polynucleotide encoding the fusion protein of (A) and a polynucleotide encoding the fusion protein of (B), and a vector comprising a polynucleotide encoding the fusion protein of (C);
- a combination of a vector comprising a polynucleotide encoding the fusion protein of (A) and a polynucleotide encoding the fusion protein of (C), and a vector comprising a polynucleotide encoding the fusion protein of (B);
- a combination of a vector comprising a polynucleotide encoding the fusion protein of (B) and a polynucleotide encoding the fusion protein of (C), and a vector comprising a polynucleotide encoding the fusion protein of (A); and
- a combination of a vector comprising a polynucleotide encoding the fusion protein of (A), a vector comprising a polynucleotide encoding the fusion protein of (B), and a vector comprising a polynucleotide encoding the fusion protein of (C).

Alternatively, in a case where (A) is a protein complex, examples of "a single vector or a combination of two or more vectors" include the followings:
- a vector comprising a polynucleotide encoding a fusion protein comprising an amino acid sequence consisting of (A-1) to (A-5), a polynucleotide encoding a protein comprising (A-6), and a polynucleotide encoding the fusion protein of (B);
- a combination of a vector comprising a polynucleotide encoding a fusion protein comprising an amino acid sequence consisting of (A-1) to (A-5) and a polynucleotide encoding a protein comprising (A-6), and a vector comprising a polynucleotide encoding the fusion protein of (B);
- a combination of a vector comprising a polynucleotide encoding a fusion protein comprising an amino acid sequence consisting of (A-1) to (A-5) and a polynucleotide encoding the fusion protein of (B), and a vector comprising a polynucleotide encoding a protein comprising (A-6);
- a combination of a vector comprising a polynucleotide encoding a fusion protein comprising an amino acid sequence consisting of (A-1) to (A-5) and a vector comprising a polynucleotide encoding a protein comprising (A-6) and a polynucleotide encoding the fusion protein of (B);
- a combination of a vector comprising a polynucleotide encoding a fusion protein comprising an amino acid sequence consisting of (A-1) to (A-5), a vector comprising a polynucleotide encoding a protein comprising (A-6), and a vector comprising a polynucleotide encoding the fusion protein of (B);
- a vector comprising a polynucleotide encoding a fusion protein comprising an amino acid sequence consisting of (A-1) to (A-5), a polynucleotide encoding a protein comprising (A-6), a polynucleotide encoding the fusion protein of (B), and a polynucleotide encoding the fusion protein of (C);
- a combination of a vector comprising a polynucleotide encoding a fusion protein comprising an amino acid sequence consisting of (A-1) to (A-5), a polynucleotide encoding a protein comprising (A-6), and a polynucleotide encoding the fusion protein of (B), and a vector comprising a polynucleotide encoding the fusion protein of (C);
- a combination of a vector comprising a polynucleotide encoding a fusion protein comprising an amino acid sequence consisting of (A-1) to (A-5), a polynucleotide encoding the fusion protein of (B), and a polynucleotide encoding the fusion protein of (C), and a vector comprising a polynucleotide encoding a protein comprising (A-6);
- a combination of a vector comprising a polynucleotide encoding a protein comprising (A-6), a polynucleotide encoding the fusion protein of (B), and a polynucleotide encoding the fusion protein of (C), and a vector comprising a polynucleotide encoding a fusion protein comprising an amino acid sequence consisting of (A-1) to (A-5);
- a combination of a vector comprising a polynucleotide encoding a fusion protein comprising an amino acid sequence consisting of (A-1) to (A-5), a polynucleotide encoding a protein comprising (A-6), and a polynucleotide encoding the fusion protein of (C), and a vector comprising a polynucleotide encoding the fusion protein of (B);
- a combination of a vector comprising a polynucleotide encoding a fusion protein comprising an amino acid sequence consisting of (A-1) to (A-5) and a polynucleotide encoding a protein comprising (A-6), and a vector comprising a polynucleotide encoding the fusion protein of (B) and a polynucleotide encoding the fusion protein of (C);
- a combination of a vector comprising a polynucleotide encoding a fusion protein comprising an amino acid sequence consisting of (A-1) to (A-5) and a polynucleotide encoding the fusion protein of (B), and a vector comprising a polynucleotide encoding a protein comprising (A-6) and a polynucleotide encoding the fusion protein of (C);
- a combination of a vector comprising a polynucleotide encoding a fusion protein comprising an amino acid sequence consisting of (A-1) to (A-5) and a polynucleotide encoding the fusion protein of (C), and a vector comprising a polynucleotide encoding a protein comprising (A-6) and a polynucleotide encoding the fusion protein of (B);
- a combination of a vector comprising a polynucleotide encoding a fusion protein comprising an amino acid sequence consisting of (A-1) to (A-5), a vector comprising a polynucleotide encoding a protein comprising (A-6), and a vector comprising a polynucleotide encoding the fusion protein of (B) and a polynucleotide encoding the fusion protein of (C);
- a combination of a vector comprising a polynucleotide encoding a fusion protein comprising an amino acid sequence consisting of (A-1) to (A-5), a vector comprising a polynucleotide encoding the fusion protein of (B), and a vector comprising a polynucleotide encoding a protein comprising (A-6) and a polynucleotide encoding the fusion protein of (C);
- a combination of a vector comprising a polynucleotide encoding a fusion protein comprising an amino acid sequence consisting of (A-1) to (A-5), a vector comprising a polynucleotide encoding the fusion protein of (C), and a vector comprising a polynucleotide encoding a protein comprising (A-6) and a polynucleotide encoding the fusion protein of (B);
- a combination of a vector comprising a polynucleotide encoding a protein comprising (A-6), a vector comprising a polynucleotide encoding the fusion protein of (B), and a vector comprising a polynucleotide encoding a fusion protein comprising an amino acid sequence consisting of (A-1) to (A-5) and a polynucleotide encoding the fusion protein of (C);
- a combination of a vector comprising a polynucleotide encoding a protein comprising (A-6), a vector comprising a polynucleotide encoding the fusion protein of (C), and a vector comprising a polynucleotide encoding a fusion protein comprising an amino acid sequence consisting of (A-1) to (A-5) and a polynucleotide encoding the fusion protein of (B);
- a combination of a vector comprising a polynucleotide encoding the fusion protein of (B), a vector comprising a polynucleotide encoding the fusion protein of (C), and a vector comprising a polynucleotide encoding a fusion protein comprising an amino acid sequence consisting of (A-1) to (A-5) and a polynucleotide encoding a protein comprising (A-6); and
- a combination of a vector comprising a polynucleotide encoding a fusion protein comprising an amino acid sequence consisting of (A-1) to (A-5), a vector comprising a polynucleotide encoding a protein comprising (A-6), a vector comprising a polynucleotide encoding the fusion protein of (B), and a vector comprising a polynucleotide encoding the fusion protein of (C).

The cell to be transformed is not particularly limited as long as the antigen-presenting extracellular vesicle described in the present specification can be obtained after the transformation, and may be a primary cultured cell or an established cell, which may be a normal cell or a lesion cell containing cancerous or tumorigenic cells. In addition, the origin of the cell to be transformed is not particularly limited, and examples thereof include cells derived from animals such as mammals, for example, rodents such as a mouse, a rat, a hamster, and a guinea pig; lagomorph such as a rabbit; ungulates such as a pig, a cow, a goat, a horse, and a sheep; carnivora such as a dog and a cat; and primates such as a human, a monkey, a rhesus monkey, a crab-eating macaque, a marmoset, an orangutan, and a chimpanzee, plant-derived cells, and insect-derived cells. The cell to be transformed is preferably an animal-derived cell. Examples of the animal-derived cells include, but are not limited to, human embryonic kidney cells (including HEK293T cells and the like), human FL cells, Chinese hamster ovary cells (CHO cells), COS-7, Vero, mouse L cells, and rat GH3.

A method for transforming the cell is not particularly limited as long as it is a method capable of introducing a target polynucleotide into a cell. For example, the method for transforming the cell may be an electroporation method, a microinjection method, a calcium phosphate method, a cationic lipid method, a method using a liposome, a method using a non-liposomal material such as polyethyleneimine, a viral infection method, or the like.

The transformed cell may be a transformed cell transiently expressing the fusion protein or protein complex of (A), (B), (C), (D), (E), (F), and/or (G), or a transformed cell (stable cell strain) stably expressing the fusion protein or protein complex of (A), (B), (C), (D), (E), (F), and/or (G).

The culture conditions of the cell to be transformed are not particularly limited. For example, when the transformed cell is an animal-derived cell, for example, a medium generally used for cell culture or the like (for example, an RPMI1640 medium, an Eagle's MEM medium, a Dulbecco's modified Eagle medium (DMEM medium), a Ham F12 medium, or any combination thereof), a medium obtained by adding other components such as fetal bovine serum, antibiotics, and amino acids, or the like may be used, and the cell may be cultured (for example, under being left or shaking), for example, in the presence of about 1 to about 10% (preferably about 2 to about 5%) of CO₂ at about 30 to about 40°C (preferably about 37°C) for a predetermined time (for example, about 0.5 hours to about 240 hours (preferably about 5 to about 120 hours, and more preferably about 12 to about 72 hours)).

A culture supernatant obtained by culturing the transformed cell may comprise the antigen-presenting extracellular vesicles described in the present specification. Therefore, when the transformed cell is cultured to obtain the antigen-presenting extracellular vesicles described in the present specification, a medium (for example, a Dulbecco's modified Eagle medium or the like containing about 1 to about 5% fetal bovine serum from which exosomes are removed) from which extracellular vesicles such as exosomes are removed may be used, if necessary.

### Culture Supernatant

In an embodiment of the present invention, a culture supernatant obtained by culturing the transformed cell described in the present specification is provided.

The antigen-presenting extracellular vesicles contained in the culture supernatant described in the present specification can be further collected, for example, by purifying (for example, centrifugation, chromatography, and the like), concentrating, and isolating the culture supernatant.

In an embodiment of the present invention, antigen-presenting extracellular vesicles obtained from the culture supernatant described in the present specification are provided.

### Method for Preparing Antigen-Presenting Extracellular Vesicles Described in Present Specification

The antigen-presenting extracellular vesicles described in the present specification may be obtained by, for example, means such as genetic recombination techniques known to those skilled in the art (for example, by the method described below or by the method described in Examples), but the present invention is not limited to.

A polynucleotide encoding the proteins of (A) and (B) described above (or (D) instead of (A) and (B)), and if necessary, (C), respectively, is obtained by normal genetic recombination techniques, and can be operably inserted into the same or different vectors. In a case where two or more polynucleotides encoding the proteins of (A) and (B) (or (D) instead of (A) and (B)), and if necessary, (C), respectively, are inserted into the same vector, each of the polynucleotides may be operably linked to the same or different promoters. The obtained single or two or more vectors can be transformed into cells simultaneously or sequentially to obtain transformed cells (may be transformed cells that transiently express these fusion proteins, or may be transformed cells (stable strains) that stably express these fusion proteins). The obtained transformed cells are cultured under desired conditions to obtain a culture supernatant, and the obtained culture supernatant is purified (for example, purification using centrifugation, antibodies (for example, antibodies recognizing a protein or the like contained in membrane of an extracellular vesicle), chromatography, flow cytometry, or the like), concentrated (for example, ultrafiltration or the like), and dried, such that the antigen-presenting extracellular vesicles described in the present specification can be obtained.

Alternatively, in a case where soluble proteins are used as the proteins of (A) and (B) (or (D) instead of (A) and (B)) described above, and if necessary, (C), for example, the antigen-presenting extracellular vesicles described in the present specification may be obtained by the following method.

As soluble proteins, the (A) and (B) (or (D) instead of (A) and (B)) described above, and if necessary, (C) obtained by normal genetic recombination techniques are used, or commercially available products thereof may be used. Next, extracellular vesicles are obtained from desired cells, for example, by a known method, the method described in the present specification, or a method similar thereto. Next, the obtained extracellular vesicles and one or more the soluble proteins described above are reacted in a desired solvent under desired conditions (for example, the method described in JP 2018-104341 A and the like may be referred to). The antigen-presenting extracellular vesicles described in the present specification can be obtained by carrying out this operation under appropriately changed conditions until the soluble proteins of (A) and (B) (or (D) instead of (A) and (B)), and if necessary, (C), are contained in the membrane of the extracellular vesicle.

Alternatively, in a case where soluble proteins are used as the proteins of (A) and (B) (or (D) instead of (A) and (B)) described above, and if necessary, (C), for example, the antigen-presenting extracellular vesicles described in the present specification may be obtained by the following method.

As the soluble proteins of (A) and (B) (or (D) instead of (A) and (B)), and if necessary, (C), proteins containing a desired tag added to the N-terminus or C-terminus thereof (examples thereof include a His tag, a FLAG tag, and a PNE tag of SEQ ID NO: 79, and all the tags may be the same tag or different types of tags) are obtained by normal genetic recombination techniques. Next, extracellular vesicles are obtained from the desired cells, for example, by known methods, the methods described in the present specification, or methods similar thereto, and antibodies against these tags or antigen-binding fragments thereof (for example, scFv, Fab, or a nanobody, such as an anti-PNE tag nanobody of SEQ ID NO: 83) and the like are bound to the extracellular vesicles via a peptide linker or the like, if necessary; alternatively, polynucleotides (for example, SEQ ID NO: 88, 90, and the like) are obtained by normal genetic recombination techniques, the polynucleotides encoding a fusion protein (for example, a fusion protein of SEQ ID NO: 89 of an anti-PNE nanobody (SEQ ID NO: 83), CD8a (SEQ ID NO: 85), and CD81 (SEQ ID NO: 15)) to which an antibody or an antigen-binding fragment thereof (for example, scFv, Fab, or a nanobody) at the N-terminus or C-terminus of a membrane protein capable of being expressed in membrane of an extracellular vesicle or a transmembrane domain thereof, or the like is bonded, transformed cells (the fusion protein may be a transformed cell that is transiently expressed or a transformed cell (stable strain) that is stably expressed) are obtained by transforming cells using the polynucleotides operably inserted into a vector, the obtained transformed cell are cultured or the like, and extracellular vesicles are recovered by the method described above and the like. The antigen-presenting extracellular vesicles described in the present specification may be obtained by mixing the soluble proteins (A) and (B), and if necessary, (C) to which a tag is added, and extracellular vesicles containing, in membranes thereof, proteins containing antibodies against to the tag or antigen-binding fragments thereof (for example, scFv, Fab, and a nanobody) under predetermined conditions.

Alternatively, the antigen-presenting extracellular vesicles described in the specification may be obtained from the transformed cells obtained by performing transformation using a combination of polynucleotides encoding the fusion proteins of (A) to (G).

Alternatively, the antigen-presenting extracellular vesicles described in the present specification may be obtained by a combination of two or more of the methods described above.

The antigen-presenting extracellular vesicles described in the present specification may recognize that the proteins of (A) and (B) (or (D) instead of (A) and (B)), and if necessary, (C) are contained in the membrane by, for example, methods such as flow cytometry, ELISA, and Western blotting.

In an embodiment of the present invention, there is provided a method for preparing the antigen-presenting extracellular vesicles described in the present specification, the method comprising collecting a culture supernatant obtained by culturing the transformed cells described in the present specification.

In an embodiment of the present invention, there is provided a method for preparing the antigen-presenting extracellular vesicles described in the present specification, the method comprising:
simultaneously or sequentially (preferably simultaneously) transforming cells with a single vector or a combination of two or more vectors, the vector comprising,
   (i) a polynucleotide encoding the fusion protein or the protein complex of (A) described in the present specification,
   (ii) a polynucleotide encoding the fusion protein of (B) described in the present specification, and optionally,
   (iii) a polynucleotide encoding the fusion protein of (C) described in the present specification; and
collecting a culture supernatant obtained by culturing the obtained transformed cells.

Alternatively, in an embodiment of the present invention, there is provided a method for preparing the antigen-presenting extracellular vesicles described in the present specification, the method comprising:
simultaneously or sequentially (preferably simultaneously) transforming cells with a single vector or a combination of two or more vectors, the vector comprising,
(iv) a polynucleotide encoding the fusion protein of (D) described in the present specification, in which the fusion protein comprises an antigen-presenting MHC molecule and at least one T-cell stimulatory cytokine and is capable of the antigen and the T-cell stimulatory cytokine outside membrane, and optionally,
(iii) a polynucleotide encoding the fusion protein of (C) described in the present specification; and
collecting a culture supernatant obtained by culturing the obtained transformed cells.

Alternatively, in an embodiment of the present invention, there is provided a method for preparing the antigen-presenting extracellular vesicles described in the present specification, the method comprising:
transforming cells with a vector comprising,
(v) a polynucleotide encoding the fusion protein of (E) described in the present specification, in which the fusion protein contains an antigen-presenting MHC molecule, at least one T-cell stimulatory cytokine, and a T-cell costimulatory molecule, and is capable of the antigen and the T-cell stimulatory cytokine outside membrane; and
collecting a culture supernatant obtained by culturing the obtained transformed cells.

In an embodiment of the present invention, antigen-presenting extracellular vesicles obtained from the culture supernatant described in the present specification are provided.

In an embodiment of the present invention, there is provided an antigen-presenting extracellular vesicle obtained by a method comprising:
simultaneously or sequentially (preferably simultaneously) transforming cells with a single vector or a combination of two or more vectors, the vector comprising,
   (i) a polynucleotide encoding the fusion protein or the protein complex of (A) described in the present specification,
   (ii) a polynucleotide encoding the fusion protein of (B) described in the present specification, and optionally,
   (iii) a polynucleotide encoding the fusion protein of (C) described in the present specification; and
collecting a culture supernatant obtained by culturing the obtained transformed cells.

Alternatively, in an embodiment of the present invention, there is provided an antigen-presenting extracellular vesicle obtained by a method comprising:
simultaneously or sequentially (preferably simultaneously) transforming cells with a single vector or a combination of two or more vectors, the vector comprising,
(iv) a polynucleotide encoding the fusion protein of (D) described in the present specification, in which the fusion protein comprises an antigen-presenting MHC molecule and at least one T-cell stimulatory cytokine and is capable of the antigen and the T-cell stimulatory cytokine outside membrane, and optionally,
(iii) a polynucleotide encoding the fusion protein of (C) described in the present specification; and
collecting a culture supernatant obtained by culturing the obtained transformed cells.

Alternatively, in an embodiment of the present invention, there is provided an antigen-presenting extracellular vesicle obtained by a method comprising:
transforming cells with,
(v) a polynucleotide encoding the fusion protein of (E) described in the present specification, in which the fusion protein comprises an antigen-presenting MHC molecule, at least one T-cell stimulatory cytokine, and a T-cell costimulatory molecule, and is capable of the antigen and the T-cell stimulatory cytokine outside membrane; and
collecting a culture supernatant obtained by culturing the obtained transformed cells.

### Composition and Use

In an embodiment of the present invention, there is provided a composition (for example, a pharmaceutical composition) containing the antigen-presenting extracellular vesicle described in the present specification, a polynucleotide and/or a vector comprising the same, and/or a transformed cell and/or a culture supernatant thereof. In an embodiment of the present invention, there is provided a pharmaceutical composition comprising the antigen-presenting extracellular vesicle described in the present specification or the culture supernatant described in the present specification.

Examples of the composition (for example, the pharmaceutical composition) described in the present specification comprise, but are not limited to, additives such as an excipient, a lubricant, a binder, a disintegrant, a pH regulator, a solvent, a solubilizing aid, a suspending agent, an isotonicifier, a buffer, an analgesic, a preservative, an antioxidant, a colorant, a sweetener, and a surfactant. Those skilled in the art can appropriately select the types of these additives, the amount of these additives used, and the like depending on the purpose. In a case where the pharmaceutical composition is used, these additives are preferably pharmacologically acceptable carriers. Furthermore, in a case where the composition described in the present specification contains a polynucleotide, it is preferable to contain carriers suitable for a drug delivery (DD) of nucleic acids, although not required, and examples of these carriers include lipid nanoparticles (LNP) and polymers (for example, PEI).

The composition (for example, the pharmaceutical composition) described in the present specification can be formulated into, for example, a tablet, a coated tablet, an orally disintegrating tablet, a chewable agent, a pill, granules, fine granules, a powder, a hard capsule, a soft capsule, a solution (examples thereof include a syrup, an injection, and a lotion), a suspension, an emulsion, a jelly, a patch, an ointment, a cream, an inhalant, a suppository, and the like by a method known per se together with the additives described above. The composition may be an oral agent or a parenteral agent. The formulated composition may further contain other beneficial components (for example, other therapeutically beneficial components) depending on the purpose thereof.

The composition according to an embodiment of the present invention can enhance acquired immunity (cellular immunity and/or humoral immunity) to a specific antigen as shown in test examples, and can be used as a pharmaceutical composition for treating or preventing an infectious disease caused by an infectious pathogen when a peptide derived from an infectious pathogen (pathogenic bacteria, viruses, or the like) is used as an antigen.

In addition, as shown in the test examples, the composition according to an embodiment of the present invention can eliminate infectious pathogens by allowing induction of inflammatory cytokines and activating innate immunity (including mobilizing and activating neutrophils, monocytes, macrophages, and the like to phagocytize pathogenic bacteria), and can be used as a pharmaceutical composition for treating or preventing an infectious disease caused by infectious pathogens.

The antigen-presenting extracellular vesicle (preferably the antigen-presenting extracellular vesicle containing an MHC class I-restricted antigen peptide and an MHC class I molecule in the membrane), the polynucleotide and/or the vector comprising the same, and/or the transformed cell and/or the culture supernatant thereof described in the present specification, or the composition comprising them (for example, the pharmaceutical composition) may be useful for treating or preventing cancer.

Therefore, in an embodiment of the present invention, there are provided, for treating or preventing cancer, the antigen-presenting extracellular vesicle, the polynucleotide and/or the vector comprising the polynucleotide, and/or the transformed cell and/or the culture supernatant thereof described in the present specification, or the composition (for example, a pharmaceutical composition) containing them. As shown in the test examples, the antigen-presenting extracellular vesicles and the like according to an embodiment of the present invention can proliferate and activate antigen-specific cytotoxic T cells to be used, and when a tumor-associated antigen peptide is used as an antigen to be used, the proliferated and activated cytotoxic T cells recognize and attack cancer cells, such that the cancer cells can be killed.

In another embodiment of the present invention, there is provided a use of the antigen-presenting extracellular vesicle, the polynucleotide and/or the vector comprising the polynucleotide, and/or the transformed cell and/or the culture supernatant thereof described in the present specification, or the composition (for example, a pharmaceutical composition) comprising them, in the manufacture of a medicament for treating or preventing cancer.

In still another embodiment of the present invention, there is provided a method for treating or preventing cancer, the method including administering an effective amount of the antigen-presenting extracellular vesicle, the polynucleotide and/or the vector comprising the polynucleotide, and/or the transformed cell and/or the culture supernatant thereof described in the present specification, or the composition comprising them to a subject in need thereof.

The cancer includes any solid cancer or blood cancer, and examples thereof include, but are not limited to, small cell lung cancer, non-small cell lung cancer, breast cancer, esophageal cancer, stomach cancer, small intestine cancer, large intestine cancer, colon cancer, rectal cancer, pancreatic cancer, prostate cancer, bone marrow cancer, kidney cancer (including kidney cell cancer), parathyroid cancer, adrenal cancer, ureteral cancer, liver cancer, bile duct cancer, cervical cancer, ovarian cancer (for example, the tissue type thereof is serous gland cancer, mucous gland cancer, clear cell adenocarcinoma cancer, and the like), testicular cancer, bladder cancer, external pudendal cancer, penis cancer, thyroid cancer, head and neck cancer, craniopharyngeal cancer, pharyngeal cancer, tongue cancer, skin cancer, Merkel cell cancer, melanoma (malignant melanoma and the like), epithelial cancer, squamous cell carcinoma, basal cell cancer, childhood cancer, unknown primary cancer, fibrosarcoma, mucosal sarcoma, liposarcoma, chondrosarcoma, osteogenic sarcoma, spinal cord tumor, angiosarcoma, lymphangiosarcoma, lymphangiosarcoma, Kaposi's sarcoma, leiomyosarcoma, rhabdomyosarcoma, synovial tumor, mesothelioma, ewing tumor, seminoma, Wilms tumor, brain tumor, glioma, glioblastoma, astrocytoma, myeloblastoma, meningioma, neuroblastoma, medulloblastoma, retinoblastoma, spinal tumor, malignant lymphoma (for example, non-Hodgkin's lymphoma, Hodgkin's lymphoma, and the like), chronic or acute lymphocytic leukemia, and adult T-cell leukemia.

In an embodiment of the present invention, immune checkpoint inhibitors can be used in combination to treat or prevent cancer. The immune checkpoint inhibitors may be administered simultaneously or sequentially to a patient, or may be contained in the pharmaceutical according to the present invention.

Examples of the immune checkpoint inhibitor include, but are not limited to, a PD-1 inhibitor (for example, an anti-PD-1 antibody such as nivolumab or pembrolizumab), a CTLA-4 inhibitor (for example, an anti-CTLA-4 antibody such as ipilimumab), and a PD-L1 inhibitor (for example, an anti-PD-Ll antibody such as durvalumab, atezolizumab, or avelumab). In a case where the immune checkpoint inhibitor is an antibody or an active fragment thereof, the antibody or the active fragment thereof may be bound to a membrane protein capable of being localized onto membrane of an extracellular vesicle or a transmembrane domain thereof or a protein capable of binding to membrane of an extracellular vesicle or a membrane-binding domain thereof to be present on the membrane of the extracellular vesicle according to the present invention.

A combination of these immune checkpoint inhibitors enhances cytotoxicity against cancer cells.

The antigen-presenting extracellular vesicle (preferably the antigen-presenting extracellular vesicle containing an MHC class II-restricted antigen peptide and an MHC class II molecule in the membrane), the polynucleotide and/or the vector comprising the same, and/or the transformed cell and/or the culture supernatant thereof described in the present specification, or the composition comprising them may be useful for treating or preventing an autoimmune disease. As exemplified in the test examples, the antigen-presenting extracellular vesicles according to an embodiment of the present invention can proliferate and activate antigen-specific regulatory T cells (Treg) to be used, and when an auto-antigen peptide is used as an antigen to be used, the proliferated and activated Treg induces tolerance to the auto-antigen, such that the autoimmune disease can be treated or prevented.

Therefore, in an embodiment of the present invention, there are provided, for treating or preventing an autoimmune disease, the antigen-presenting extracellular vesicle, the polynucleotide and/or the vector comprising the polynucleotide, and/or the transformed cell and/or the culture supernatant thereof described in the present specification, or the composition (for example, a pharmaceutical composition) comprising them.

In another embodiment of the present invention, there is provided a use of the antigen-presenting extracellular vesicle, the polynucleotide and/or the vector comprising the polynucleotide, and/or the transformed cell and/or the culture supernatant thereof described in the present specification, or the composition (for example, a pharmaceutical composition) containing them, for producing a pharmaceutical for treating or preventing an autoimmune disease.

In still another embodiment of the present invention, there is provided a method for treating or preventing an autoimmune disease, the method including administering an effective amount of the antigen-presenting extracellular vesicle, the polynucleotide and/or the vector comprising the polynucleotide, and/or the transformed cell and/or the culture supernatant thereof described in the present specification, or the composition comprising them to a subject who requires them.

Examples of the autoimmune disease include, but are not limited to, asthma, psoriasis, systemic erythematosus, Guillain-Barre syndrome, Sjogren's syndrome, multiple sclerosis, myasthenia gravis, malignant anemia, Basedow's disease, Hashimoto thyroiditis, type I diabetes, Crohn's disease, inflammatory bowel disease, and rheumatoid arthritis.

The antigen-presenting extracellular vesicle (preferably the antigen-presenting extracellular vesicle containing an MHC class II-restricted antigen peptide and an MHC class II molecule in the membrane), the polynucleotide and/or the vector comprising the same, and/or the transformed cell and/or the culture supernatant thereof described in the present specification, or the composition comprising them (for example, the pharmaceutical composition) may be useful for treating or preventing an allergic disease. As shown in the test examples, the antigen-presenting extracellular vesicles according to an embodiment of the present invention can proliferate and activate antigen-specific regulatory T cells (Treg) to be used, and when an allergen is used as an antigen to be used, the proliferated and activated Treg induces tolerance to the allergen, such that the allergic disease can be treated or prevented.

Therefore, in an embodiment of the present invention, there are provided, for treating or preventing an allergic disease, the antigen-presenting extracellular vesicle, the polynucleotide and/or the vector comprising the polynucleotide, and/or the transformed cell and/or the culture supernatant thereof described in the present specification, or the composition (for example, a pharmaceutical composition) containing them.

In another embodiment of the present invention, there is provided a use of the antigen-presenting extracellular vesicle, the polynucleotide and/or the vector comprising the polynucleotide, and/or the transformed cell and/or the culture supernatant thereof described in the present specification, or the composition (for example, a pharmaceutical composition) containing them, for producing a pharmaceutical for treating or preventing an allergic disease.

In still another embodiment of the present invention, there is provided a method for treating or preventing an allergic disease, the method including administering an effective amount of the antigen-presenting extracellular vesicle, the polynucleotide and/or the vector comprising the polynucleotide, and/or the transformed cell and/or the culture supernatant thereof described in the present specification, or the composition comprising them to a subject who requires them.

Examples of the allergic disease include, but are not limited to, allergic rhinitis, atopic dermatitis, allergic asthma, allergic conjunctivitis, allergic gastro-enteritis, food allergies, drug allergies, and urticaria.

Examples of the subject to be treated or prevented from the various diseases described above include, but are not limited to, animals such as mammals, for example, rodents such as a mouse, a rat, a hamster, and a guinea pig; lagomorph such as a rabbit; ungulates such as a pig, a cow, a goat, a horse, and a sheep; carnivora such as a dog and a cat; and primates such as a human, a monkey, a rhesus monkey, a crab-eating macaque, a marmoset, an orangutan, and a chimpanzee; and plants. The subject is preferably an animal, more preferably a rodent or a primate, and sill more preferably a mouse or a human.

A dosage of a formulation obtained by formulating the antigen-presenting extracellular vesicle, the polynucleotide and/or the vector comprising the polynucleotide, and/or the transformed cell and/or the culture supernatant thereof described in the present specification, or the composition comprising them can be appropriately determined in consideration of a gender, an age, a weight, a health status, a degree of medical condition, or a diet of a subject to be administered, an administration time, an administration method, a combination with other drugs, and other factors.

### Method for Activating, Proliferating, and/or Differentiating T cells against Specific Antigen

The antigen-presenting extracellular vesicles described in the present specification can activate, proliferate, and differentiate T cells against a specific antigen by contacting with the T cells (although not limited thereto, for example, T cells or T cell populations obtained from peripheral blood, spleen, and the like) in vitro, ex vivo, and/or in vivo.

In an embodiment of the present invention, there is provided a method for activating, proliferating, and/or differentiating T cells against a specific antigen, the method comprising bringing the antigen-presenting extracellular vesicles described in the present specification into contact with T cells in vitro or ex vivo.

In an embodiment of the present invention, there are provided T cells obtained by the method described above.

The T cells obtained by the method described above may be administered to a subject in order to treat and/or prevent a disease (for example, cancer, an autoimmune disease, an allergic disease, or the like).

### Examples

Hereinafter, the present invention will be described in more detail with reference to examples, and these examples do not limit the scope of the present invention at all.

### Preparation 1 of Plasmid

A vector for expressing, on membrane of an extracellular vesicle, an MHC class I molecule capable of presenting an antigen outside membrane was prepared using a pCAG-puro vector.

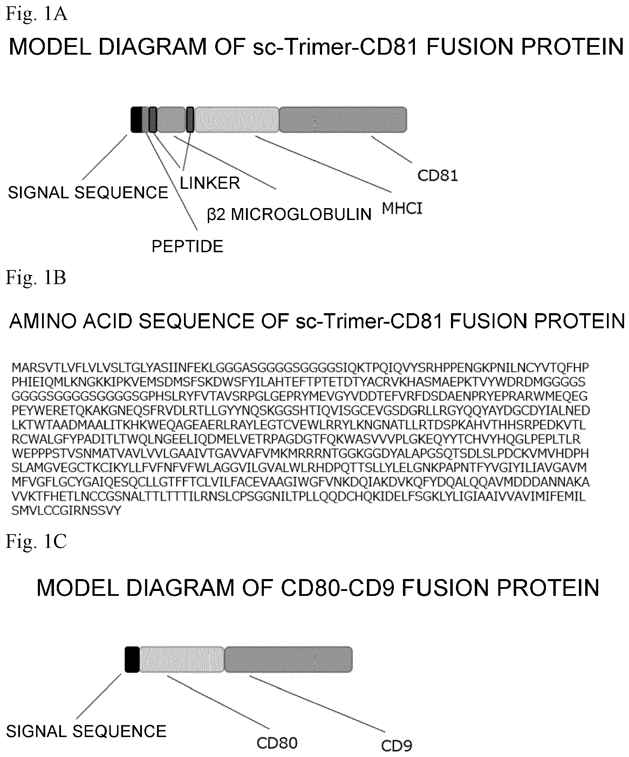

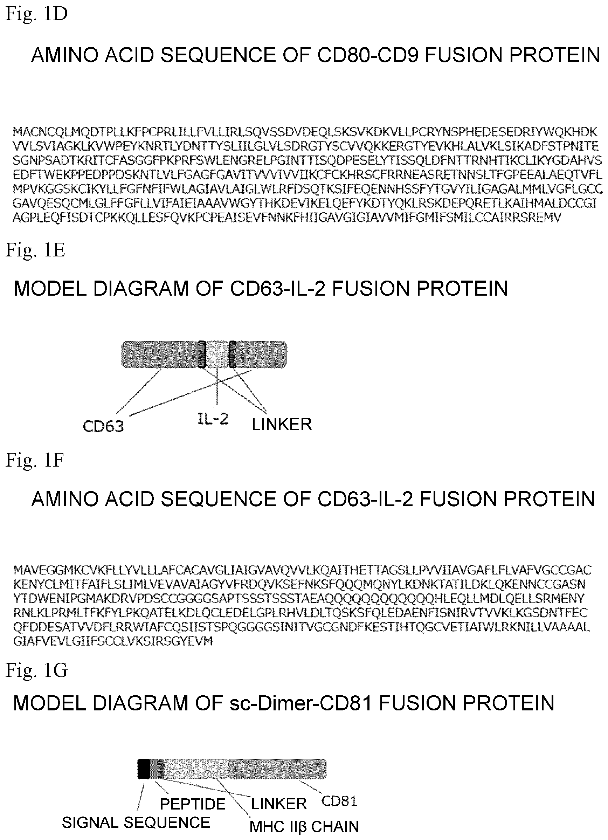

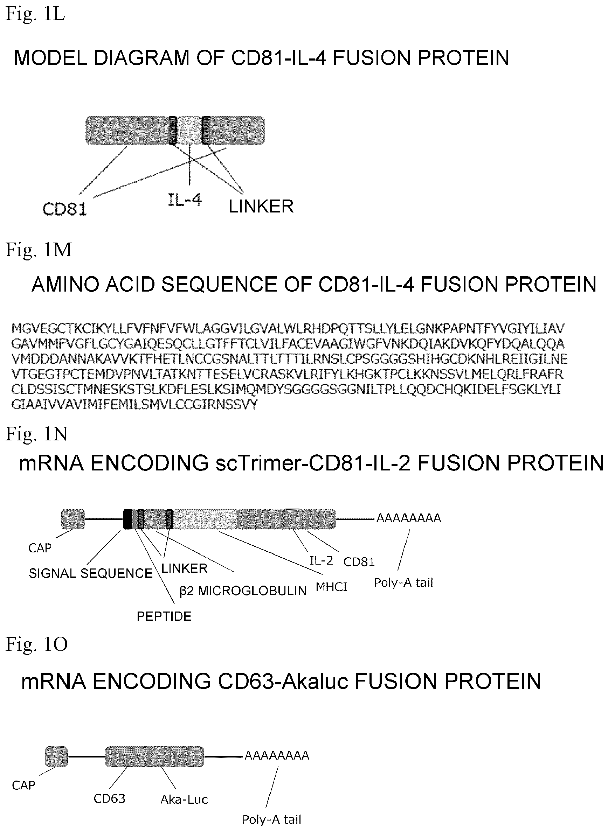

With established cloning techniques, a single chain trimer (sc-Trimer) consisting of a polynucleotide (SEQ ID NO: 2) encoding a signal peptide (amino acids 1 to 20; SEQ ID NO: 1) of β₂ microglobulin, a polynucleotide (SEQ ID NO: 4) encoding an OVA peptide (SEQ ID NO: 3) as a model antigen peptide, a peptide linker (amino acid sequence: SEQ ID NO: 5, polynucleotide: SEQ ID NO: 6), a polynucleotide (SEQ ID NO: 8) encoding a full-length sequence (amino acids 21 to 119; SEQ ID NO: 7) of β₂ microglobulin from which a signal peptide was removed, a polynucleotide (SEQ ID NO: 12) encoding a peptide linker (SEQ ID NO: 11), and a polynucleotide (SEQ ID NO: 10) encoding a full-length sequence (amino acids 22 to 369; SEQ ID NO: 9) of an MHC class Iα chain from which a signal peptide was removed was prepared (amino acid sequence: SEQ ID NO: 13; polynucleotide: SEQ ID NO: 14). Next, a polynucleotide (SEQ ID NO: 18; corresponding amino acid sequence: SEQ ID NO: 17) in which a sc-Trimer was linked to a polynucleotide (SEQ ID NO: 16) encoding a full-length sequence (amino acids 1 to 236; SEQ ID NO: 15) of CD81 as a tetraspanin was inserted into the pCAG-puro vector (Figs. 1A and 1B: hereinafter, sc-Trimer-CD81).

With the same method, in order to express CD80 as one of T-cell costimulatory molecules on membrane of an extracellular vesicle, a polynucleotide (SEQ ID NO: 24; corresponding amino acid sequence: SEQ ID NO: 23) in which a polynucleotide (SEQ ID NO: 20) encoding a full-length sequence (amino acids 1 to 306; SEQ ID NO: 19) of CD80 was linked to a polynucleotide (SEQ ID NO: 22) encoding a full-length sequence (amino acids 1 to 306; SEQ ID NO: 21) of CD9 as a tetraspanin was inserted into a pCAG-puro or pMX vector (Figs. 1C and ID: hereinafter, CD80-CD9).

With the same method, in order to express IL-2 as one of T-cell stimulatory cytokines on membrane of an extracellular vesicle, a polynucleotide (SEQ ID NO: 26) encoding a full-length sequence (amino acids 21 to 169; SEQ ID NO: 25) from which a single peptide of IL-2 was removed was inserted between the amino acids 170C and 1711 in a large extracellular loop of a mouse CD63 (amino acids 1 to 238; SEQ ID NO: 27; polynucleotide: SEQ ID NO: 28) as a tetraspanin (that is, a sequence of IL-2 was inserted between a polynucleotide (SEQ ID NO: 58) encoding a partial sequence of CD63 of SEQ ID NO: 57 and a polynucleotide (SEQ ID NO: 60) encoding a partial sequence of CD63 of SEQ ID NO: 59). Note that polynucleotides (SEQ ID NO: 30) encoding a peptide linker (amino acid sequence GGGGS: SEQ ID NO: 29) were added to the N-terminus and the C-terminus of IL-2, respectively. The polynucleotide (SEQ ID NO: 32; corresponding amino acid sequence: SEQ ID NO: 31) was inserted into the pCAG-puro vector (Figs. IE and IF: hereinafter, CD63-IL-2).

### Preparation 2 of Plasmid:

A vector for expressing, on membrane of an extracellular vesicle, an MHC class II molecule capable of presenting an antigen outside membrane was prepared using a pCAG-puro vector.

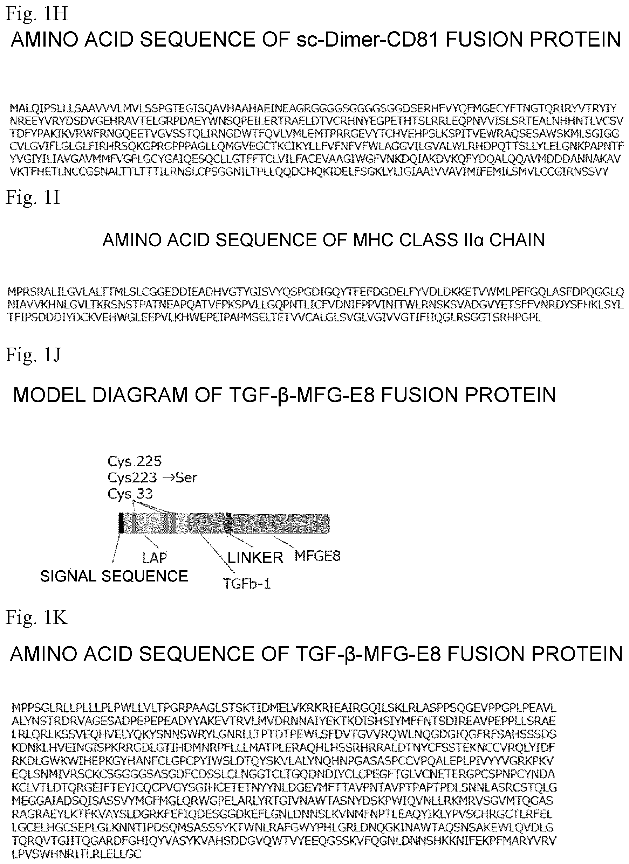

With established cloning techniques, a single chain dimer (sc-Dimer) in which a polynucleotide (SEQ ID NO: 34) encoding a signal peptide (amino acids 1 to 27; SEQ ID NO: 33) of an MHC class IIβ chain, a polynucleotide (SEQ ID NO: 36) encoding an OVA peptide (SEQ ID NO: 35) as a model antigen peptide, and a polynucleotide (SEQ ID NO: 38) encoding a full-length sequence (amino acids 28 to 265; SEQ ID NO: 37) of an MHC class IIβ chain from which a signal peptide was removed were linked by a polynucleotide (SEQ ID NO: 40) encoding a peptide linker (SEQ ID NO: 39) was prepared (amino acid sequence: SEQ ID NO: 41; polynucleotide: SEQ ID NO: 42). Next, a polynucleotide (SEQ ID NO: 44; corresponding amino acid sequence: SEQ ID NO: 43) in which a sc-Dimer was linked to a polynucleotide (SEQ ID NO: 16) encoding a full-length sequence (amino acids 1 to 236; SEQ ID NO: 15) of CD81 as a tetraspanin was inserted into the pCAG-puro vector (Figs. 1G and 1H: hereinafter, sc-Dimer-CD81).

A polynucleotide (SEQ ID NO: 46) encoding a full-length sequence (amino acids 1 to 256; SEQ ID NO: 45) of an MHC class IIα chain as a constituent element of an MHC class II molecule was inserted into another pCAG-puro vector (Fig. 1I: hereinafter, an MHC class IIα chain).

With the same method, in order to express TGF-β1 as one of T-cell stimulatory cytokines on membrane of an extracellular vesicle, a polynucleotide (SEQ ID NO: 48) encoding a full-length sequence (amino acids 1 to 390; SEQ ID NO: 47) of TGF-β1 in which three 33^{rd}, 223^{rd}, and 225^{th} C's of a LAP domain were changed to S's and a polynucleotide (SEQ ID NO: 50) encoding a full-length sequence (amino acids 23 to 463; SEQ ID NO: 49) from which a signal peptide of MFG-E8 in which 89^{th} D was changed to E was removed were linked by a polynucleotide (SEQ ID NO: 30) encoding a peptide linker (SEQ ID NO: 29). The polynucleotide (SEQ ID NO: 52; corresponding amino acid sequence: SEQ ID NO: 51) was inserted into the pCAG-puro vector (Figs. 1J and 1K: hereinafter, TGF-β-MFG-E8).

With the same method, in order to express IL-4 as one of T-cell stimulatory cytokines on membrane of an extracellular vesicle, a polynucleotide (SEQ ID NO: 54) encoding a full-length sequence (amino acids 21 to 140; SEQ ID NO: 53) from which a single peptide of IL-4 was removed was inserted between the amino acids 177S and 178G in a large extracellular loop of a mouse CD81 (amino acids 1 to 236; SEQ ID NO: 15; polynucleotide: SEQ ID NO: 16) as a tetraspanin (that is, a sequence of IL-4 was inserted between a polynucleotide (SEQ ID NO: 62) encoding a partial sequence of CD81 of SEQ ID NO: 61 and a polynucleotide (SEQ ID NO: 64) encoding a partial sequence of CD81 of SEQ ID NO: 63). Note that polynucleotides (SEQ ID NO: 30) encoding a peptide linker (amino acid sequence GGGGS; SEQ ID NO: 29) were added to the N-terminus and the C-terminus of IL-4, respectively. The polynucleotide (SEQ ID NO: 56; corresponding amino acid sequence: SEQ ID NO: 55) was inserted into the pCAG-puro vector (Figs. 1L and 1M: hereinafter, CD81-IL-4).

### Preparation 3 of Plasmid:

### sc-Dimer-CD81-IL-12p40

At the sc-Dimer, a polynucleotide (SEQ ID NO: 92) encoding a protein (SEQ ID NO: 91) obtained by fusing CD81 to IL-12p40 as a subunit of IL-12 as a T-cell stimulatory cytokine was inserted into a pCAG-puro vector, thereby preparing a vector expressing a fusion protein.

### IL-12p35

A polynucleotide (SEQ ID NO: 98) encoding IL-12p35 (SEQ ID NO: 97) as one subunit of IL-12 was inserted into a pCAG-puro or pMX vector to prepare a vector expressing IL-12p35.

### CD81-IL-6

In order to express IL-6 as one of T-cell stimulatory cytokines on membrane of an extracellular vesicle, a polynucleotide (SEQ ID NO: 100) encoding a full-length sequence (SEQ ID NO: 99) from which a signal peptide of IL-6 was removed was introduced into a polynucleotide encoding an extracellular loop of CD81 as a tetraspanin, and a polynucleotide (SEQ ID NO: 102) encoding a CD81-IL-6 fusion protein (SEQ ID NO: 101) was inserted into a pCAG-puro or pMX vector, thereby preparing a vector expressing a fusion protein.

### hCD80-hCD9

In order to express human CD80 as one of T-cell costimulatory molecules on membrane of an extracellular vesicle, a polynucleotide (SEQ ID NO: 108) encoding a fusion protein (SEQ ID NO: 107) of human CD80 and human CD9 as a tetraspanin was inserted into a pCAG-puro or pMX vector, thereby preparing a vector expressing a fusion protein.

### sc-Trimer-CD81-IL-2

In order to express IL-2 as one of T-cell stimulatory cytokines on membrane of an extracellular vesicle, similar to the CD81-IL-4, a polynucleotide encoding a fusion peptide of CD81-IL2 was prepared, a sequence of the polynucleotide was linked to a nucleotide encoding a sc-Trimer-, and a polynucleotide (SEQ ID NO: 136) encoding sc-Trimer-CD81-IL-2 (SEQ ID NO: 135) was prepared and inserted into a pCAG-puro or pMX vector, thereby preparing a vector expressing a fusion protein.

### hsc-Trimer-hCD81

Using the sc-Trimer-CD81 as a human gene sequence (using HLA-A2402 as a sequence of MHC-I), a polynucleotide (SEQ ID NO: 132) encoding hsc-Trimer-hCD81 (SEQ ID NO: 131) was prepared and inserted into a pCAG-puro or pMX vector to prepare a vector expressing a fusion protein.

### SARS-CoV2sc-Trimer-hCD81

Using a SARS-CoV-2 peptide (amino acid sequence: SEQ ID NO: 141; polynucleotide: SEQ ID NO: 142) as an antigen and HLA-A0201 as an MHC molecule, a polynucleotide (SEQ ID NO: 148) encoding an antigen-presenting MHC molecule (SARS-CoV2sc-Trimer; amino acid sequence: SEQ ID NO: 147) was prepared and was further linked to a polynucleotide encoding hCD81, thereby preparing a polynucleotide (SEQ ID NO: 150) encoding SARS-CoV2sc-Trimer-hCD81 (SEQ ID NO: 149). The prepared polynucleotide was inserted into a pCAG-puro or pMX vector to prepare a vector expressing a fusion protein.

### hCD63-hIL-2

The CD63-IL-2 was prepared using a human gene sequence. A polynucleotide (SEQ ID NO: 116) encoding hCD63-hIL-2 (SEQ ID NO: 115) was prepared and inserted into a pCAG-puro or pMX vector to prepare a vector expressing a fusion protein.

### CD63-Akaluc

As a negative control, CD63 and Akaluc luciferase were fused to prepare a polynucleotide (SEQ ID NO: 140) for localizing an AlkaLuc fusion protein (SEQ ID NO: 139) to an extracellular vesicle, and the polynucleotide was inserted into a pCAG-puro or pMX vector, thereby preparing a vector expressing a fusion protein.

The respective sequences used in examples are shown in Tables 1 to 13. Note that the underline portion in each sequence indicates a signal peptide.

**[Table 1-1]**

| | Sequence | SEQ ID NO: |
|---|---|---|
| Signal peptide of *β*₂ microglobulin | MARSVTLVFLVLVSLTGLYA | 1 |
| | ATGGCTCGCTCGGTGACCCTGGTCTTTCTGGTGCTTGTCTCACTGACCGGCCTGTAT GCT | 2 |
| OVA peptide 1 (for MHC class I molecule) | SIINFEKL | 3 |
| | TCCATTATAAATTTTGAAAAGTTG | 1 |
| Peptide linker 1 | GGGASGGGGSGGGGS | 5 |
| | GGCGGAGGTGCCTCTGGCGGTGGGGGCAGCGGTGGAGGGGGCAGT | 6 |
| *β*₂ Microglobulin (from which signal peptide is removed) | | 7 |
| | ATCCAGAAAACCCCTCAAATTCAAGTATACTCACGCCACCCACCGGAGAATGGGAAG CCGAACATACTGAACTGCTACGTAACACAGTTCCACCCGCCTCACATTGAAATCCAA ATGCTGAAGAACGGGAAAAAAATTCCTAAAGTAGAGATGTCAGATATGTCCTTCAGC AAGGACTGGTCTTTCTATATCCTGGCTCACACTGAATTCACCCCCACTGAGACTGAT ACATACGCCTGCAGAGTTAAGCATGCCAGTATGGCCGAGCCCAAGACCGTCTACTGG GATCGAGACATG | 8 |
| MHC class I α chain (from which signal peptide is removed) | GPHSLRYFVTAVSRPGLGEPRYMEVGYVDDTEFVRFDSDAENPRYEPRARWMEQEGP EYWERETQKAKGNEQSFRVDLRTLLGYYNQSKGGSHTIQVISGCEVGSDGRLLRGYQ QYAYDGCDYIALNEDLKTWTAADMAALITKHKWEQAGEAERLRAYLEGTCVEWLRRY LKNGNATLLRTDSPKAHVTHHSRPEDKVTLRCWALGFYPADITLTWQLNGEELIQDM ELVETRPAGDGTFQKWASVVVPLGKEQYYTCHVYHQGLPEPLTLRWEPPPSTVSNMA TVAVLVVLGAAIVTGAVVAFVMKMRRRNTGGKGGDYALAPGSQTSDLSLPDCKVMVH DPHSLA | 9 |
| | GGCCCACACTCGCTGAGGTATTTCGTCACCGCCGTGTCCCGGCCCGGCCTCGGGGAG CCCCGGTACATGGAAGTCGGCTACGTGGACGACACGGAGTTCGTGCGCTTCGACAGC GACGCGGAGAATCCGAGATATGAGCCGCGGGCGCGGTGGATGGAGCAGGAGGGGCCC GAGTATTGGGAGCGGGAGACACAGAAAGCCAAGGGCAATGAGCAGAGTTTCCGAGTG GACCTGAGGACCCTGCTCGGCTACTACAACCAGAGCAAGGGCGGCTCTCACACTATT CAGGTGATCTCTGGCTGTGAAGTGGGGTCCGACGGGCGACTCCTCCGCGGGTACCAG CAGTACGCCTACGACGGCTGCGATTACATCGCCCTGAACGAAGACCTGAAAACGTGG ACGGCGGCGGACATGGCGGCGCTGATCACCAAACACAAGTGGGAGCAGGCTGGTGAA | 10 |

**[Table 1-2]**

| | | |
|---|---|---|
| | GCAGAGAGACTCAGGGCCTACCTGGAGGGCACGTGCGTGGAGTGGCTCCGCAGATAC CTGAAGAACGGGAACGCGACGCTGCTGCGCACAGATTCCCCAAAGGCCCATGTGACC CATCACAGCAGACCTGAAGATAAAGTCACCCTGAGGTGCTGGGCCCTGGGCTTCTAC CCTGCTGACATCACCCTGACCTGGCAGTTGAATGGGGAGGAGCTGATCCAGGACATG GAGCTTGTGGAGACCAGGCCTGCAGGGGATGGAACCTTCCAGAAGTGGGCATCTGTG GTGGTGCCTCTTGGGAAGGAGCAGTATTACACATGCCATGTGTACCATCAGGGGCTG CCTGAGCCCCTCACCCTGAGATGGGAGCCTCCTCCATCCACTGTCTCCAACATGGCG ACCGTTGCTGTTCTGGTTGTCCTTGGAGCTGCAATAGTCACTGGAGCTGTGGTGGCT TTTGTGATGAAGATGAGAAGGAGAAACACAGGTGGAAAAGGAGGGGACTATGCTCTG GCTCCAGGCTCCCAGACCTCTGATCTGTCTCTCCCAGATTGTAAAGTGATGGTTCAT GACCCTCATTCTCTAGCG | |
| Peptide linker 2 | GGGGSGGGGSGGGGSGGGGS | 11 |
| | GGGGGGGGAGGCTCCGGTGGAGGGGGGTCTGGAGGGGGGGGGTCTGGTGGAGGCGGA AGT | 12 |
| Single chain MHC class I molecule (*β*₂ microglobulin (from which signal peptide is removed) + peptide linker 2 + MHC class I α chain (from which signal peptide is removed)) | IQKTPQIQVYSRHPPENGKPNILNCYVTQFHPPHIEIQMLKNGKKIPKVEMSDMSFS KDWSFYILAHTEFTPTETDTYACRVKHASMAEPKTVYWDRDMGGGGSGGGGSGGGGS GGGGSGPHSLRYFVTAVSRPGLGEPRYMEVGYVDDTEFVRFDSDAENPRYEPRARWM EQEGPEYWERETQKAKGNEQSFRVDLRTLLGYYNQSKGGSHTIQVISGCEVGSDGRL LRGYQQYAYDGCDYIALNEDLKTWTAADMAALITKHKWEQAGEAERLRAYLEGTCVE WLRRYLKNGNATLLRTDSPKAHVTHHSRPEDKVTLRCWALGFYPADITLTWQLNGEE LIQDMELVETRPAGDGTFQKWASVVVPLGKEQYYTCHVYHQGLPEPLTLRWEPPPST VSNMATVAVLVVLGAAIVTGAVVAFVMKMRRRNTGGKGGDYALAPGSQTSDLSLPDC KVMVHDPHSLA | 65 |
| | ATCCAGAAAACCCCTCAAATTCAAGTATACTCACGCCACCCACCGGAGAATGGGAAG CCGAACATACTGAACTGCTACGTAACACAGTTCCACCCGCCTCACATTGAAATCCAA ATGCTGAAGAACGGGAAAAAAATTCCTAAAGTAGAGATGTCAGATATGTCCTTCAGC AAGGACTGGTCTTTCTATATCCTGGCTCACACTGAATTCACCCCCACTGAGACTGAT ACATACGCCTGCAGAGTTAAGCATGCCAGTATGGCCGAGCCCAAGACCGTCTACTGG GATCGAGACATGGGGGGGGGAGGCTCCGGTGGAGGGGGGTCTGGAGGGGGGGGGTCT GGTGGAGGCGGAAGTGGCCCACACTCGCTGAGGTATTTCGTCACCGCCGTGTCCCGG CCCGGCCTCGGGGAGCCCCGGTACATGGAAGTCGGCTACGTGGACGACACGGAGTTC GTGCGCTTCGACAGCGACGCGGAGAATCCGAGATATGAGCCGCGGGCGCGGTGGATG GAGCAGGAGGGGCCCGAGTATTGGGAGCGGGAGACACAGAAAGCCAAGGGCAATGAG | 66 |

**[Table 1-3]**

| | | |
|---|---|---|
| | CAGAGTTTCCGAGTGGACCTGAGGACCCTGCTCGGCTACTACAACCAGAGCAAGGGC GGCTCTCACACTATTCAGGTGATCTCTGGCTGTGAAGTGGGGTCCGACGGGCGACTC CTCCGCGGGTACCAGCAGTACGCCTACGACGGCTGCGATTACATCGCCCTGAACGAA GACCTGAAAACGTGGACGGCGGCGGACATGGCGGCGCTGATCACCAAACACAAGTGG GAGCAGGCTGGTGAAGCAGAGAGACTCAGGGCCTACCTGGAGGGCACGTGCGTGGAG TGGCTCCGCAGATACCTGAAGAACGGGAACGCGACGCTGCTGCGCACAGATTCCCCA AAGGCCCATGTGACCCATCACAGCAGACCTGAAGATAAAGTCACCCTGAGGTGCTGG GCCCTGGGCTTCTACCCTGCTGACATCACCCTGACCTGGCAGTTGAATGGGGAGGAG CTGATCCAGGACATGGAGCTTGTGGAGACCAGGCCTGCAGGGGATGGAACCTTCCAG AAGTGGGCATCTGTGGTGGTGCCTCTTGGGAAGGAGCAGTATTACACATGCCATGTG TACCATCAGGGGCTGCCTGAGCCCCTCACCCTGAGATGGGAGCCTCCTCCATCCACT GTCTCCAACATGGCGACCGTTGCTGTTCTGGTTGTCCTTGGAGCTGCAATAGTCACT GGAGCTGTGGTGGCTTTTGTGATGAAGATGAGAAGGAGAAACACAGGTGGAAAAGGA GGGGACTATGCTCTGGCTCCAGGCTCCCAGACCTCTGATCTGTCTCTCCCAGATTGT AAAGTGATGGTTCATGACCCTCATTCTCTAGCG | |
| sc-Tri mer (OVA peptide 1 + peptide linker 1 + single chain MHC class I molecule) | MARSVTLVFLVLVSLTGLYASIINFEKLGGGASGGGGSGGGGSIQKTPQIQVYSRHP PENGKPNILNCYVTQFHPPHIEIQMLKNGKKIPKVEMSDMSFSKDWSFYILAHTEFT PTETDTYACRVKHASMAEPKTVYWDRDMGGGGSGGGGSGGGGSGGGGSGPHSLRYFV TAVSRPGLGEPRYMEVGYVDDTEFVRFDSDAENPRYEPRARWMEQEGPEYWERETQK AKGNEQSFRVDLRTLLGYYNQSKGGSHTIQVISGCEVGSDGRLLRGYQQYAYDGCDY IALNEDLKTWTAADMAALITKHKWEQAGEAERLRAYLEGTCVEWLRRYLKNGNATLL RTDSPKAHVTHHSRPEDKVTLRCWALGFYPADITLTWQLNGEELIQDMELVETRPAG DGTFQKWASVVVPLGKEQYYTCHVYHQGLPEPLTLRWEPPPSTVSNMATVAVLVVLG AAIVTGAVVAFVMKMRRRNTGGKGGDYALAPGSQTSDLSLPDCKVMVHDPHSLA | 13 |
| | ATGGCTCGCTCGGTGACCCTGGTCTTTCTGGTGCTTGTCTCACTGACCGGCCTGTAT GCTTCCATTATAAATTTTGAAAAGTTGGGCGGAGGTGCCTCTGGCGGTGGGGGCAGC GGTGGAGGGGGCAGTATCCAGAAAACCCCTCAAATTCAAGTATACTCACGCCACCCA CCGGAGAATGGGAAGCCGAACATACTGAACTGCTACGTAACACAGTTCCACCCGCCT CACATTGAAATCCAAATGCTGAAGAACGGGAAAAAAATTCCTAAAGTAGAGATGTCA GATATGTCCTTCAGCAAGGACTGGTCTTTCTATATCCTGGCTCACACTGAATTCACC CCCACTGAGACTGATACATACGCCTGCAGAGTTAAGCATGCCAGTATGGCCGAGCCC AAGACCGTCTACTGGGATCGAGACATGGGGGGGGGAGGCTCCGGTGGAGGGGGGTCT GGAGGGGGGGGGTCTGGTGGAGGCGGAAGTGGCCCACACTCGCTGAGGTATTTCGTC | 14 |

**[Table 1-4]**

| | | |
|---|---|---|
| | ACCGCCGTGTCCCGGCCCGGCCTCGGGGAGCCCCGGTACATGGAAGTCGGCTACGTG GACGACACGGAGTTCGTGCGCTTCGACAGCGACGCGGAGAATCCGAGATATGAGCCG CGGGCGCGGTGGATGGAGCAGGAGGGGCCCGAGTATTGGGAGCGGGAGACACAGAAA GCCAAGGGCAATGAGCAGAGTTTCCGAGTGGACCTGAGGACCCTGCTCGGCTACTAC AACCAGAGCAAGGGCGGCTCTCACACTATTCAGGTGATCTCTGGCTGTGAAGTGGGG TCCGACGGGCGACTCCTCCGCGGGTACCAGCAGTACGCCTACGACGGCTGCGATTAC ATCGCCCTGAACGAAGACCTGAAAACGTGGACGGCGGCGGACATGGCGGCGCTGATC ACCAAACACAAGTGGGAGCAGGCTGGTGAAGCAGAGAGACTCAGGGCCTACCTGGAG GGCACGTGCGTGGAGTGGCTCCGCAGATACCTGAAGAACGGGAACGCGACGCTGCTG CGCACAGATTCCCCAAAGGCCCATGTGACCCATCACAGCAGACCTGAAGATAAAGTC ACCCTGAGGTGCTGGGCCCTGGGCTTCTACCCTGCTGACATCACCCTGACCTGGCAG TTGAATGGGGAGGAGCTGATCCAGGACATGGAGCTTGTGGAGACCAGGCCTGCAGGG GATGGAACCTTCCAGAAGTGGGCATCTGTGGTGGTGCCTCTTGGGAAGGAGCAGTAT TACACATGCCATGTGTACCATCAGGGGCTGCCTGAGCCCCTCACCCTGAGATGGGAG CCTCCTCCATCCACTGTCTCCAACATGGCGACCGTTGCTGTTCTGGTTGTCCTTGGA GCTGCAATAGTCACTGGAGCTGTGGTGGCTTTTGTGATGAAGATGAGAAGGAGAAAC ACAGGTGGAAAAGGAGGGGACTATGCTCTGGCTCCAGGCTCCCAGACCTCTGATCTG TCTCTCCCAGATTGTAAAGTGATGGTTCATGACCCTCATTCTCTAGCG | |
| CD81 | MGVEGCTKCIKYLLFVFNFVFWLAGGVILGVALWLRHDPQTTSLLYLELGNKPAPNT FYVGIYILIAVGAVMMFVGFLGCYGAIQESQCLLGTFFTCLVILFACEVAAGIWGFV NKDQIAKDVKQFYDQALQQAVMDDDANNAKAVVKTFHETLNCCGSNALTTLTTTILR NSLCPSGGNILTPLLQQDCHQKIDELFSGKLYLIGIAAIVVAVIMIFEMILSMVLCC GIRNSSVY | 15 |
| | ATGGGGGTGGAGGGCTGCACCAAATGCATCAAATACCTGCTCTTCGTCTTCAATTTC GTCTTCTGGCTGGCTGGAGGCGTGATCCTAGGTGTAGCTCTGTGGTTGCGTCATGAT CCACAGACCACCAGCCTGCTGTACCTGGAACTGGGAAACAAACCGGCACCCAACACC TTCTACGTGGGCATCTACATTCTCATTGCTGTGGGAGCTGTGATGATGTTTGTAGGC TTCCTGGGGTGCTATGGGGCCATCCAGGAGTCCCAGTGTCTGCTGGGGACGTTCTTC ACCTGCCTTGTGATCCTGTTTGCCTGTGAGGTGGCTGCAGGCATCTGGGGCTTCGTA AACAAAGACCAGATCGCCAAGGATGTGAAGCAGTTCTATGACCAGGCCCTTCAGCAA GCTGTGATGGATGATGATGCCAACAATGCCAAGGCTGTGGTGAAGACTTTCCATGAG ACGCTCAACTGTTGTGGCTCCAACGCACTGACCACACTGACTACCACCATACTGAGG AACAGCCTGTGTCCCTCAGGCGGCAACATACTCACCCCCTTACTGCAGCAAGATTGT | 16 |

**[Table 1-5]**

| | | |
|---|---|---|
| | CATCAGAAAATCGATGAGCTCTTCTCTGGGAAGCTGTACCTCATTGGAATTGCAGCC ATTGTGGTAGCTGTCATTATGATCTTTGAGATGATTCTGAGCATGGTGCTGTGCTGT GGCATCCGGAACAGCTCCGTGTACTGA | |
| sc-Tri mer-CD 81 (sc-Tr imer-C D81) | MARSVTLVFLVLVSLTGLYASIINFEKLGGGASGGGGSGGGGSIQKTPQIQVYSRHP PENGKPNILNCYVTQFHPPHIEIQMLKNGKKIPKVEMSDMSFSKDWSFYILAHTEFT PTETDTYACRVKHASMAEPKTVYWDRDMGGGGSGGGGSGGGGSGGGGSGPHSLRYFV TAVSRPGLGEPRYMEVGYVDDTEFVRFDSDAENPRYEPRARWMEQEGPEYWERETQK AKGNEQSFRVDLRTLLGYYNQSKGGSHTIQVISGCEVGSDGRLLRGYQQYAYDGCDY IALNEDLKTWTAADMAALITKHKWEQAGEAERLRAYLEGTCVEWLRRYLKNGNATLL RTDSPKAHVTHHSRPEDKVTLRCWALGFYPADITLTWQLNGEELIQDMELVETRPAG DGTFQKWASVVVPLGKEQYYTCHVYHQGLPEPLTLRWEPPPSTVSNMATVAVLVVLG AAIVTGAVVAFVMKMRRRNTGGKGGDYALAPGSQTSDLSLPDCKVMVHDPHSLAMGV EGCTKCIKYLLFVFNFVFWLAGGVILGVALWLRHDPQTTSLLYLELGNKPAPNTFYV GIYILIAVGAVMMFVGFLGCYGAIQESQCLLGTFFTCLVILFACEVAAGIWGFVNKD QIAKDVKQFYDQALQQAVMDDDANNAKAVVKTFHETLNCCGSNALTTLTTTILRNSL CPSGGNILTPLLQQDCHQKIDELFSGKLYLIGIAAIVVAVIMIFEMILSMVLCCGIR NSSVY | 17 |
| | ATGGCTCGCTCGGTGACCCTGGTCTTTCTGGTGCTTGTCTCACTGACCGGCCTGTAT GCTTCCATTATAAATTTTGAAAAGTTGGGCGGAGGTGCCTCTGGCGGTGGGGGCAGC GGTGGAGGGGGCAGTATCCAGAAAACCCCTCAAATTCAAGTATACTCACGCCACCCA CCGGAGAATGGGAAGCCGAACATACTGAACTGCTACGTAACACAGTTCCACCCGCCT CACATTGAAATCCAAATGCTGAAGAACGGGAAAAAAATTCCTAAAGTAGAGATGTCA GATATGTCCTTCAGCAAGGACTGGTCTTTCTATATCCTGGCTCACACTGAATTCACC CCCACTGAGACTGATACATACGCCTGCAGAGTTAAGCATGCCAGTATGGCCGAGCCC AAGACCGTCTACTGGGATCGAGACATGGGGGGGGGAGGCTCCGGTGGAGGGGGGTCT GGAGGGGGGGGGTCTGGTGGAGGCGGAAGTGGCCCACACTCGCTGAGGTATTTCGTC ACCGCCGTGTCCCGGCCCGGCCTCGGGGAGCCCCGGTACATGGAAGTCGGCTACGTG GACGACACGGAGTTCGTGCGCTTCGACAGCGACGCGGAGAATCCGAGATATGAGCCG CGGGCGCGGTGGATGGAGCAGGAGGGGCCCGAGTATTGGGAGCGGGAGACACAGAAA GCCAAGGGCAATGAGCAGAGTTTCCGAGTGGACCTGAGGACCCTGCTCGGCTACTAC AACCAGAGCAAGGGCGGCTCTCACACTATTCAGGTGATCTCTGGCTGTGAAGTGGGG TCCGACGGGCGACTCCTCCGCGGGTACCAGCAGTACGCCTACGACGGCTGCGATTAC ATCGCCCTGAACGAAGACCTGAAAACGTGGACGGCGGCGGACATGGCGGCGCTGATC | 18 |

**[Table 1-6]**

| | | |
|---|---|---|
| | ACCAAACACAAGTGGGAGCAGGCTGGTGAAGCAGAGAGACTCAGGGCCTACCTGGAG GGCACGTGCGTGGAGTGGCTCCGCAGATACCTGAAGAACGGGAACGCGACGCTGCTG CGCACAGATTCCCCAAAGGCCCATGTGACCCATCACAGCAGACCTGAAGATAAAGTC ACCCTGAGGTGCTGGGCCCTGGGCTTCTACCCTGCTGACATCACCCTGACCTGGCAG TTGAATGGGGAGGAGCTGATCCAGGACATGGAGCTTGTGGAGACCAGGCCTGCAGGG GATGGAACCTTCCAGAAGTGGGCATCTGTGGTGGTGCCTCTTGGGAAGGAGCAGTAT TACACATGCCATGTGTACCATCAGGGGCTGCCTGAGCCCCTCACCCTGAGATGGGAG CCTCCTCCATCCACTGTCTCCAACATGGCGACCGTTGCTGTTCTGGTTGTCCTTGGA GCTGCAATAGTCACTGGAGCTGTGGTGGCTTTTGTGATGAAGATGAGAAGGAGAAAC ACAGGTGGAAAAGGAGGGGACTATGCTCTGGCTCCAGGCTCCCAGACCTCTGATCTG TCTCTCCCAGATTGTAAAGTGATGGTTCATGACCCTCATTCTCTAGCGATGGGGGTG GAGGGCTGCACCAAATGCATCAAATACCTGCTCTTCGTCTTCAATTTCGTCTTCTGG CTGGCTGGAGGCGTGATCCTAGGTGTAGCTCTGTGGTTGCGTCATGATCCACAGACC ACCAGCCTGCTGTACCTGGAACTGGGAAACAAACCGGCACCCAACACCTTCTACGTG GGCATCTACATTCTCATTGCTGTGGGAGCTGTGATGATGTTTGTAGGCTTCCTGGGG TGCTATGGGGCCATCCAGGAGTCCCAGTGTCTGCTGGGGACGTTCTTCACCTGCCTT GTGATCCTGTTTGCCTGTGAGGTGGCTGCAGGCATCTGGGGCTTCGTAAACAAAGAC CAGATCGCCAAGGATGTGAAGCAGTTCTATGACCAGGCCCTTCAGCAAGCTGTGATG GATGATGATGCCAACAATGCCAAGGCTGTGGTGAAGACTTTCCATGAGACGCTCAAC TGTTGTGGCTCCAACGCACTGACCACACTGACTACCACCATACTGAGGAACAGCCTG TGTCCCTCAGGCGGCAACATACTCACCCCCTTACTGCAGCAAGATTGTCATCAGAAA ATCGATGAGCTCTTCTCTGGGAAGCTGTACCTCATTGGAATTGCAGCCATTGTGGTA GCTGTCATTATGATCTTTGAGATGATTCTGAGCATGGTGCTGTGCTGTGGCATCCGG AACAGCTCCGTGTACTGA | |

**[Table 2-1]**

| | Sequence | SEQ ID NO: |
|---|---|---|
| C D 8 0 | MACNCQLMQDTPLLKFPCPRLILLFVLLIRLSQVSSDVDEQLSKSVKDKVLLPCRYN SPHEDESEDRIYWQKHDKVVLSVIAGKLKVWPEYKNRTLYDNTTYSLIILGLVLSDR GTYSCVVQKKERGTYEVKHLALVKLSIKADFSTPNITESGNPSADTKRITCFASGGF PKPRFSWLENGRELPGINTTISQDPESELYTISSQLDFNTTRNHTIKCLIKYGDAHV SEDFTWEKPPEDPPDSKNTLVLFGAGFGAVITVVVIVVIIKCFCKHRSCFRRNEASR ETNNSLTFGPEEALAEQTVFL | 19 |
| | ATGGCTTGCAATTGTCAGTTGATGCAGGATACACCACTCCTCAAGTTTCCATGTCCA AGGCTCATTCTTCTCTTTGTGCTGCTGATTCGTCTTTCACAAGTGTCTTCAGATGTT GATGAACAACTGTCCAAGTCAGTGAAAGATAAGGTATTGCTGCCTTGCCGTTACAAC TCTCCTCATGAAGATGAGTCTGAAGACCGAATCTACTGGCAAAAACATGACAAAGTG GTGCTGTCTGTCATTGCTGGGAAACTAAAAGTGTGGCCCGAGTATAAGAACCGGACT TTATATGACAACACTACCTACTCTCTTATCATCCTGGGCCTGGTCCTTTCAGACCGG GGCACATACAGCTGTGTCGTTCAAAAGAAGGAAAGAGGAACGTATGAAGTTAAACAC TTGGCTTTAGTAAAGTTGTCCATCAAAGCTGACTTCTCTACCCCCAACATAACTGAG TCTGGAAACCCATCTGCAGACACTAAAAGGATTACCTGCTTTGCTTCCGGGGGTTTC CCAAAGCCTCGCTTCTCTTGGTTGGAAAATGGAAGAGAATTACCTGGCATCAATACG ACAATTTCCCAGGATCCTGAATCTGAATTGTACACCATTAGTAGCCAACTAGATTTC AATACGACTCGCAACCACACCATTAAGTGTCTCATTAAATATGGAGATGCTCACGTG TCAGAGGACTTCACCTGGGAAAAACCCCCAGAAGACCCTCCTGATAGCAAGAACACA CTTGTGCTCTTTGGGGCAGGATTCGGCGCAGTAATAACAGTCGTCGTCATCGTTGTC ATCATCAAATGCTTCTGTAAGCACAGAAGCTGTTTCAGAAGAAATGAGGCAAGCAGA GAAACAAACAACAGCCTTACCTTCGGGCCTGAAGAAGCATTAGCTGAACAGACCGTC TTCCTTTAG | 20 |
| CD9 | MPVKGGSKCIKYLLFGFNFIFWLAGIAVLAIGLWLRFDSQTKSIFEQENNHSSFYTG VYILIGAGALMMLVGFLGCCGAVQESQCMLGLFFGFLLVIFAIEIAAAVWGYTHKDE VIKELQEFYKDTYQKLRSKDEPQRETLKAIHMALDCCGIAGPLEQFISDTCPKKQLL ESFQVKPCPEAISEVFNNKFHIIGAVGIGIAVVMIFGMIFSMILCCAIRRSREMV | 21 |
| | ATGCCGGTCAAAGGAGGTAGCAAGTGCATCAAATACCTGCTCTTCGGATTTAACTTC ATCTTCTGGCTCGCTGGCATTGCAGTGCTTGCTATTGGACTATGGCTCCGATTCGAC TCTCAGACCAAGAGCATCTTCGAGCAAGAGAATAACCATTCCAGTTTCTACACAGGA | 22 |

**[Table 2-2]**

| | | |
|---|---|---|
| | GTGTACATTCTGATTGGAGCCGGGGCCCTCATGATGCTGGTTGGTTTCCTGGGCTGC TGTGGAGCTGTACAAGAGTCCCAGTGCATGCTGGGATTGTTCTTCGGGTTCCTCTTG GTGATATTCGCCATTGAGATAGCCGCCGCCGTCTGGGGCTATACCCACAAGGATGAG GTGATTAAAGAACTCCAGGAGTTTTACAAGGACACCTACCAAAAGTTACGGAGCAAG GATGAACCCCAGCGGGAAACACTCAAAGCCATCCATATGGCGTTGGACTGCTGTGGC ATAGCTGGTCCTTTGGAGCAGTTTATCTCGGACACCTGCCCCAAGAAACAGCTTTTG GAAAGTTTCCAGGTTAAGCCCTGCCCTGAAGCCATCAGTGAGGTCTTCAACAACAAG TTCCACATCATTGGAGCAGTGGGTATCGGCATCGCCGTGGTGATGATCTTCGGCATG ATCTTCAGCATGATCCTGTGCTGCGCCATCCGCAGGAGCCGAGAAATGGTCTAG | |
| CD80-C D 9 | MACNCQLMQDTPLLKFPCPRLILLFVLLIRLSQVSSDVDEQLSKSVKDKVLLPCRYN SPHEDESEDRIYWQKHDKVVLSVIAGKLKVWPEYKNRTLYDNTTYSLIILGLVLSDR GTYSCVVQKKERGTYEVKHLALVKLSIKADFSTPNITESGNPSADTKRITCFASGGF PKPRFSWLENGRELPGINTTISQDPESELYTISSQLDFNTTRNHTIKCLIKYGDAHV SEDFTWEKPPEDPPDSKNTLVLFGAGFGAVITVVVIVVIIKCFCKHRSCFRRNEASR ETNNSLTFGPEEALAEQTVFLMPVKGGSKCIKYLLFGFNFIFWLAGIAVLAIGLWLR FDSQTKSIFEQENNHSSFYTGVYILIGAGALMMLVGFLGCCGAVQESQCMLGLFFGF LLVIFAIEIAAAVWGYTHKDEVIKELQEFYKDTYQKLRSKDEPQRETLKAIHMALDC CGIAGPLEQFISDTCPKKQLLESFQVKPCPEAISEVFNNKFHIIGAVGIGIAVVMIF GMIFSMILCCAIRRSREMV | 23 |
| | ATGGCTTGCAATTGTCAGTTGATGCAGGATACACCACTCCTCAAGTTTCCATGTCCA AGGCTCATTCTTCTCTTTGTGCTGCTGATTCGTCTTTCACAAGTGTCTTCAGATGTT GATGAACAACTGTCCAAGTCAGTGAAAGATAAGGTATTGCTGCCTTGCCGTTACAAC TCTCCTCATGAAGATGAGTCTGAAGACCGAATCTACTGGCAAAAACATGACAAAGTG GTGCTGTCTGTCATTGCTGGGAAACTAAAAGTGTGGCCCGAGTATAAGAACCGGACT TTATATGACAACACTACCTACTCTCTTATCATCCTGGGCCTGGTCCTTTCAGACCGG GGCACATACAGCTGTGTCGTTCAAAAGAAGGAAAGAGGAACGTATGAAGTTAAACAC TTGGCTTTAGTAAAGTTGTCCATCAAAGCTGACTTCTCTACCCCCAACATAACTGAG TCTGGAAACCCATCTGCAGACACTAAAAGGATTACCTGCTTTGCTTCCGGGGGTTTC CCAAAGCCTCGCTTCTCTTGGTTGGAAAATGGAAGAGAATTACCTGGCATCAATACG ACAATTTCCCAGGATCCTGAATCTGAATTGTACACCATTAGTAGCCAACTAGATTTC AATACGACTCGCAACCACACCATTAAGTGTCTCATTAAATATGGAGATGCTCACGTG TCAGAGGACTTCACCTGGGAAAAACCCCCAGAAGACCCTCCTGATAGCAAGAACACA CTTGTGCTCTTTGGGGCAGGATTCGGCGCAGTAATAACAGTCGTCGTCATCGTTGTC | 24 |

**[Table 2-3]**

| | | |
|---|---|---|
| | ATCATCAAATGCTTCTGTAAGCACAGAAGCTGTTTCAGAAGAAATGAGGCAAGCAGA GAAACAAACAACAGCCTTACCTTCGGGCCTGAAGAAGCATTAGCTGAACAGACCGTC TTCCTTATGCCGGTCAAAGGAGGTAGCAAGTGCATCAAATACCTGCTCTTCGGATTT AACTTCATCTTCTGGCTCGCTGGCATTGCAGTGCTTGCTATTGGACTATGGCTCCGA TTCGACTCTCAGACCAAGAGCATCTTCGAGCAAGAGAATAACCATTCCAGTTTCTAC ACAGGAGTGTACATTCTGATTGGAGCCGGGGCCCTCATGATGCTGGTTGGTTTCCTG GGCTGCTGTGGAGCTGTACAAGAGTCCCAGTGCATGCTGGGATTGTTCTTCGGGTTC CTCTTGGTGATATTCGCCATTGAGATAGCCGCCGCCGTCTGGGGCTATACCCACAAG GATGAGGTGATTAAAGAACTCCAGGAGTTTTACAAGGACACCTACCAAAAGTTACGG AGCAAGGATGAACCCCAGCGGGAAACACTCAAAGCCATCCATATGGCGTTGGACTGC TGTGGCATAGCTGGTCCTTTGGAGCAGTTTATCTCGGACACCTGCCCCAAGAAACAG CTTTTGGAAAGTTTCCAGGTTAAGCCCTGCCCTGAAGCCATCAGTGAGGTCTTCAAC AACAAGTTCCACATCATTGGAGCAGTGGGTATCGGCATCGCCGTGGTGATGATCTTC GGCATGATCTTCAGCATGATCCTGTGCTGCGCCATCCGCAGGAGCCGAGAAATGGTC TAG | |

**[Table 3-1]**

| | Sequence | SEQ ID NO: |
|---|---|---|
| I L - 2 (from which signal peptide is removed) | APTSSSTSSSTAEAQQQQQQQQQQQHLEQLLMDLQELLSRMENYRNLKLPRMLTFK FYLPKQATELKDLQCLEDELGPLRHVLDLTQSKSFQLEDAENFISNIRVTVVKLKGS DNTFECQFDDESATVVDFLRRWIAFCQSIISTSPQ | 25 |
| | GCACCCACTTCAAGCTCCACTTCAAGCTCTACAGCGGAAGCACAGCAGCAGCAGCAG CAGCAGCAGCAGCAGCAGCAGCACCTGGAGCAGCTGTTGATGGACCTACAGGAGCTC CTGAGCAGGATGGAGAATTACAGGAACCTGAAACTCCCCAGGATGCTCACCTTCAAA TTTTACTTGCCCAAGCAGGCCACAGAATTGAAAGATCTTCAGTGCCTAGAAGATGAA CTTGGACCTCTGCGGCATGTTCTGGATTTGACTCAAAGCAAAAGCTTTCAATTGGAA GATGCTGAGAATTTCATCAGCAATATCAGAGTAACTGTTGTAAAACTAAAGGGCTCT GACAACACATTTGAGTGCCAATTCGATGATGAGTCAGCAACTGTGGTGGACTTTCTG AGGAGATGGATAGCCTTCTGTCAAAGCATCATCTCAACAAGCCCTCAA | 26 |
| CD63 | MAVEGGMKCVKFLLYVLLLAFCACAVGLIAIGVAVQVVLKQAITHETTAGSLLPVVI IAVGAFLFLVAFVGCCGACKENYCLMITFAIFLSLIMLVEVAVAIAGYVFRDQVKSE FNKSFQQQMQNYLKDNKTATILDKLQKENNCCGASNYTDWENIPGMAKDRVPDSCCI NITVGCGNDFKESTIHTQGCVETIAIWLRKNILLVAAAALGIAFVEVLGIIFSCCLV KSIRSGYEVM | 27 |
| | ATGGCGGTGGAAGGAGGAATGAAGTGTGTCAAGTTTTTGCTCTACGTTCTCCTGCTG GCCTTCTGCGCCTGTGCAGTGGGATTGATCGCCATTGGTGTAGCGGTTCAGGTTGTC TTGAAGCAGGCCATTACCCATGAGACTACTGCTGGCTCGCTGTTGCCTGTGGTCATC ATTGCAGTGGGTGCCTTCCTCTTCCTGGTGGCCTTTGTGGGCTGCTGTGGGGCCTGC AAGGAGAACTACTGTCTCATGATTACATTTGCCATCTTCCTGTCTCTTATCATGCTT GTGGAGGTGGCTGTGGCCATTGCTGGCTATGTGTTTAGAGACCAGGTGAAGTCAGAG TTTAATAAAAGCTTCCAGCAGCAGATGCAGAATTACCTTAAAGACAACAAAACAGCC ACTATTTTGGACAAATTGCAGAAAGAAAATAACTGCTGTGGAGCTTCTAACTACACA GACTGGGAAAACATCCCCGGCATGGCCAAGGACAGAGTCCCCGATTCTTGCTGCATC AACATAACTGTGGGCTGTGGGAATGATTTCAAGGAATCCACTATCCATACCCAGGGC TGCGTGGAGACTATAGCAATATGGCTAAGGAAGAACATACTGCTGGTGGCTGCAGCG GCCCTGGGCATTGCTTTTGTGGAGGTCTTGGGAATTATCTTCTCCTGCTGTCTGGTG AAGAGTATTCGAAGTGGCTATGAAGTAATGTAG | 28 |
| CD63 | MAVEGGMKCVKFLLYVLLLAFCACAVGLIAIGVAVQVVLKQAITHETTAGSLLPVVI | 57 |

**[Table 3-2]**

| | | |
|---|---|---|
| (amino acids 1 to 170) | IAVGAFLFLVAFVGCCGACKENYCLMITFAIFLSLIMLVEVAVAIAGYVFRDQVKSE FNKSFQQQMQNYLKDNKTATILDKLQKENNCCGASNYTDWENIPGMAKDRVPDSCC | |
| | ATGGCGGTGGAAGGAGGAATGAAGTGTGTCAAGTTTTTGCTCTACGTTCTCCTGCTG GCCTTCTGCGCCTGTGCAGTGGGATTGATCGCCATTGGTGTAGCGGTTCAGGTTGTC TTGAAGCAGGCCATTACCCATGAGACTACTGCTGGCTCGCTGTTGCCTGTGGTCATC ATTGCAGTGGGTGCCTTCCTCTTCCTGGTGGCCTTTGTGGGCTGCTGTGGGGCCTGC AAGGAGAACTACTGTCTCATGATTACATTTGCCATCTTCCTGTCTCTTATCATGCTT GTGGAGGTGGCTGTGGCCATTGCTGGCTATGTGTTTAGAGACCAGGTGAAGTCAGAG TTTAATAAAAGCTTCCAGCAGCAGATGCAGAATTACCTTAAAGACAACAAAACAGCC ACTATTTTGGACAAATTGCAGAAAGAAAATAACTGCTGTGGAGCTTCTAACTACACA GACTGGGAAAACATCCCCGGCATGGCCAAGGACAGAGTCCCCGATTCTTGCTGC | 58 |
| CD63 (amino acids 171 to 238) | INITVGCGNDFKESTIHTQGCVETIAIWLRKNILLVAAAALGIAFVEVLGIIFSCCL VKSIRSGYEVM | 59 |
| | ATCAACATAACTGTGGGCTGTGGGAATGATTTCAAGGAATCCACTATCCATACCCAG GGCTGCGTGGAGACTATAGCAATATGGCTAAGGAAGAACATACTGCTGGTGGCTGCA GCGGCCCTGGGCATTGCTTTTGTGGAGGTCTTGGGAATTATCTTCTCCTGCTGTCTG GTGAAGAGTATTCGAAGTGGCTATGAAGTAATGTAG | 60 |
| Peptide linker 3 | GGGGS | 29 |
| | GGAGGAGGAGGAAGC | 30 |
| C D 6 3 - I L - 2 | MAVEGGMKCVKFLLYVLLLAFCACAVGLIAIGVAVQVVLKQAITHETTAGSLLPVVI IAVGAFLFLVAFVGCCGACKENYCLMITFAIFLSLIMLVEVAVAIAGYVFRDQVKSE FNKSFQQQMQNYLKDNKTATILDKLQKENNCCGASNYTDWENIPGMAKDRVPDSCCG GGGSAPTSSSTSSSTAEAQQQQQQQQQQQHLEQLLMDLQELLSRMENYRNLKLPRM LTFKFYLPKQATELKDLQCLEDELGPLRHVLDLTQSKSFQLEDAENFISNIRVTVVK LKGSDNTFECQFDDESATVVDFLRRWIAFCQSIISTSPQGGGGSINITVGCGNDFKE STIHTQGCVETIAIWLRKNILLVAAAALGIAFVEVLGIIFSCCLVKSIRSGYEVM | 31 |
| | ATGGCGGTGGAAGGAGGAATGAAGTGTGTCAAGTTTTTGCTCTACGTTCTCCTGCTG GCCTTCTGCGCCTGTGCAGTGGGATTGATCGCCATTGGTGTAGCGGTTCAGGTTGTC TTGAAGCAGGCCATTACCCATGAGACTACTGCTGGCTCGCTGTTGCCTGTGGTCATC ATTGCAGTGGGTGCCTTCCTCTTCCTGGTGGCCTTTGTGGGCTGCTGTGGGGCCTGC AAGGAGAACTACTGTCTCATGATTACATTTGCCATCTTCCTGTCTCTTATCATGCTT GTGGAGGTGGCTGTGGCCATTGCTGGCTATGTGTTTAGAGACCAGGTGAAGTCAGAG TTTAATAAAAGCTTCCAGCAGCAGATGCAGAATTACCTTAAAGACAACAAAACAGCC | 32 |

**[Table 3-3]**

| | | |
|---|---|---|
| | ACTATTTTGGACAAATTGCAGAAAGAAAATAACTGCTGTGGAGCTTCTAACTACACA GACTGGGAAAACATCCCCGGCATGGCCAAGGACAGAGTCCCCGATTCTTGCTGCGGA GGAGGAGGAAGCGCACCCACTTCAAGCTCCACTTCAAGCTCTACAGCGGAAGCACAG CAGCAGCAGCAGCAGCAGCAGCAGCAGCAGCAGCACCTGGAGCAGCTGTTGATGGAC CTACAGGAGCTCCTGAGCAGGATGGAGAATTACAGGAACCTGAAACTCCCCAGGATG CTCACCTTCAAATTTTACTTGCCCAAGCAGGCCACAGAATTGAAAGATCTTCAGTGC CTAGAAGATGAACTTGGACCTCTGCGGCATGTTCTGGATTTGACTCAAAGCAAAAGC TTTCAATTGGAAGATGCTGAGAATTTCATCAGCAATATCAGAGTAACTGTTGTAAAA CTAAAGGGCTCTGACAACACATTTGAGTGCCAATTCGATGATGAGTCAGCAACTGTG GTGGACTTTCTGAGGAGATGGATAGCCTTCTGTCAAAGCATCATCTCAACAAGCCCT CAAGGAGGAGGAGGAAGCATCAACATAACTGTGGGCTGTGGGAATGATTTCAAGGAA TCCACTATCCATACCCAGGGCTGCGTGGAGACTATAGCAATATGGCTAAGGAAGAAC ATACTGCTGGTGGCTGCAGCGGCCCTGGGCATTGCTTTTGTGGAGGTCTTGGGAATT ATCTTCTCCTGCTGTCTGGTGAAGAGTATTCGAAGTGGCTATGAAGTAATGTAG | |

**[Table 4-1]**

| | Sequence | SEQ ID NO: |
|---|---|---|
| Signal peptide of MHC class II*β* chain | MALQIPSLLLSAAVVVLMVLSSPGTEG | 33 |
| | ATGGCTCTGCAGATCCCCAGCCTCCTCCTCTCGGCTGCTGTGGTGGTGCTGATGGTG CTGAGCAGCCCAGGGACTGAGGGC | 34 |
| OVA peptide 2 (for MHC class II molecule) | ISQAVHAAHAEINEAGR | 35 |
| | ATATCTCAAGCTGTCCATGCAGCACATGCAGAAATCAATGAAGCAGGCAGA | 36 |
| MHC class II*β* chain (from which signal peptide is removed) | GDSERHFVYQFMGECYFTNGTQRIRYVTRYIYNREEYVRYDSDVGEHRAVTELGRPD AEYWNSQPEILERTRAELDTVCRHNYEGPETHTSLRRLEQPNVVISLSRTEALNHHN TLVCSVTDFYPAKIKVRWFRNGQEETVGVSSTQLIRNGDWTFQVLVMLEMTPRRGEV YTCHVEHPSLKSPITVEWRAQSESAWSKMLSGIGGCVLGVIFLGLGLFIRHRSQKGP RGPPPAGLLQ | 37 |
| | GGAGACTCCGAAAGGCATTTCGTGTACCAGTTCATGGGCGAGTGCTACTTCACCAAC GGGACGCAGCGCATACGATATGTGACCAGATACATCTACAACCGGGAGGAGTACGTG CGCTACGACAGCGACGTGGGCGAGCACCGCGCGGTGACCGAGCTGGGGCGGCCAGAC GCCGAGTACTGGAACAGCCAGCCGGAGATCCTGGAGCGAACGCGGGCCGAGCTGGAC ACGGTGTGCAGACACAACTACGAGGGGCCGGAGACCCACACCTCCCTGCGGCGGCTT GAACAGCCCAATGTCGTCATCTCCCTGTCCAGGACAGAGGCCCTCAACCACCACAAC ACTCTGGTCTGCTCAGTGACAGATTTCTACCCAGCCAAGATCAAAGTGCGCTGGTTC CGGAATGGCCAGGAGGAGACGGTGGGGGTCTCATCCACACAGCTTATTAGGAATGGG GACTGGACCTTCCAGGTCCTGGTCATGCTGGAGATGACCCCTCGGCGGGGAGAGGTC TACACCTGTCACGTGGAGCATCCCAGCCTGAAGAGCCCCATCACTGTGGAGTGGAGG GCACAGTCTGAGTCTGCCTGGAGCAAGATGTTGAGCGGCATCGGGGGCTGCGTGCTT GGGGTGATCTTCCTCGGGCTTGGCCTTTTCATCCGTCACAGGAGTCAGAAAGGACCT CGAGGCCCTCCTCCAGCAGGGCTCCTGCAG | 38 |
| Peptide linker 1 | GGGGSGGGGSG | 39 |
| | GGAGGAGGAGGAAGCGGAGGAGGAGGAAGCGGA | 40 |
| sc-Dim er (OVA peptide 2 + peptide linker 4 + MHC class II*β* chain | MALQIPSLLLSAAVVVLMVLSSPGTEGISQAVHAAHAEINEAGRGGGGSGGGGSGGD SERHFVYQFMGECYFTNGTQRIRYVTRYIYNREEYVRYDSDVGEHRAVTELGRPDAE YWNSQPEILERTRAELDTVCRHNYEGPETHTSLRRLEQPNVVISLSRTEALNHHNTL VCSVTDFYPAKIKVRWFRNGQEETVGVSSTQLIRNGDWTFQVLVMLEMTPRRGEVYT | 41 |

**[Table 4-2]**

| | | |
|---|---|---|
| (from which signal peptide is removed)) | CHVEHPSLKSPITVEWRAQSESAWSKMLSGIGGCVLGVIFLGLGLFIRHRSQKGPRG PPPAGLLQ | |
| | ATGGCTCTGCAGATCCCCAGCCTCCTCCTCTCGGCTGCTGTGGTGGTGCTGATGGTG CTGAGCAGCCCAGGGACTGAGGGCATATCTCAAGCTGTCCATGCAGCACATGCAGAA ATCAATGAAGCAGGCAGAGGAGGAGGAGGAAGCGGAGGAGGAGGAAGCGGAGGAGAC TCCGAAAGGCATTTCGTGTACCAGTTCATGGGCGAGTGCTACTTCACCAACGGGACG CAGCGCATACGATATGTGACCAGATACATCTACAACCGGGAGGAGTACGTGCGCTAC GACAGCGACGTGGGCGAGCACCGCGCGGTGACCGAGCTGGGGCGGCCAGACGCCGAG TACTGGAACAGCCAGCCGGAGATCCTGGAGCGAACGCGGGCCGAGCTGGACACGGTG TGCAGACACAACTACGAGGGGCCGGAGACCCACACCTCCCTGCGGCGGCTTGAACAG CCCAATGTCGTCATCTCCCTGTCCAGGACAGAGGCCCTCAACCACCACAACACTCTG GTCTGCTCAGTGACAGATTTCTACCCAGCCAAGATCAAAGTGCGCTGGTTCCGGAAT GGCCAGGAGGAGACGGTGGGGGTCTCATCCACACAGCTTATTAGGAATGGGGACTGG ACCTTCCAGGTCCTGGTCATGCTGGAGATGACCCCTCGGCGGGGAGAGGTCTACACC TGTCACGTGGAGCATCCCAGCCTGAAGAGCCCCATCACTGTGGAGTGGAGGGCACAG TCTGAGTCTGCCTGGAGCAAGATGTTGAGCGGCATCGGGGGCTGCGTGCTTGGGGTG ATCTTCCTCGGGCTTGGCCTTTTCATCCGTCACAGGAGTCAGAAAGGACCTCGAGGC CCTCCTCCAGCAGGGCTCCTGCAG | 42 |
| sc-Dim er-CD8 1 | MALQIPSLLLSAAVVVLMVLSSPGTEGISQAVHAAHAEINEAGRGGGGSGGGGSGGD SERHFVYQFMGECYFTNGTQRIRYVTRYIYNREEYVRYDSDVGEHRAVTELGRPDAE YWNSQPEILERTRAELDTVCRHNYEGPETHTSLRRLEQPNVVISLSRTEALNHHNTL VCSVTDFYPAKIKVRWFRNGQEETVGVSSTQLIRNGDWTFQVLVMLEMTPRRGEVYT CHVEHPSLKSPITVEWRAQSESAWSKMLSGIGGCVLGVIFLGLGLFIRHRSQKGPRG PPPAGLLQMGVEGCTKCIKYLLFVFNFVFWLAGGVILGVALWLRHDPQTTSLLYLEL GNKPAPNTFYVGIYILIAVGAVMMFVGFLGCYGAIQESQCLLGTFFTCLVILFACEV AAGIWGFVNKDQIAKDVKQFYDQALQQAVMDDDANNAKAVVKTFHETLNCCGSNALT TLTTTILRNSLCPSGGNILTPLLQQDCHQKIDELFSGKLYLIGIAAIVVAVIMIFEM ILSMVLCCGIRNSSVY | 43 |
| | ATGGCTCTGCAGATCCCCAGCCTCCTCCTCTCGGCTGCTGTGGTGGTGCTGATGGTG CTGAGCAGCCCAGGGACTGAGGGCATATCTCAAGCTGTCCATGCAGCACATGCAGAA ATCAATGAAGCAGGCAGAGGAGGAGGAGGAAGCGGAGGAGGAGGAAGCGGAGGAGAC TCCGAAAGGCATTTCGTGTACCAGTTCATGGGCGAGTGCTACTTCACCAACGGGACG CAGCGCATACGATATGTGACCAGATACATCTACAACCGGGAGGAGTACGTGCGCTAC | 44 |

**[Table 4-3]**

| | | |
|---|---|---|
| | GACAGCGACGTGGGCGAGCACCGCGCGGTGACCGAGCTGGGGCGGCCAGACGCCGAG TACTGGAACAGCCAGCCGGAGATCCTGGAGCGAACGCGGGCCGAGCTGGACACGGTG TGCAGACACAACTACGAGGGGCCGGAGACCCACACCTCCCTGCGGCGGCTTGAACAG CCCAATGTCGTCATCTCCCTGTCCAGGACAGAGGCCCTCAACCACCACAACACTCTG GTCTGCTCAGTGACAGATTTCTACCCAGCCAAGATCAAAGTGCGCTGGTTCCGGAAT GGCCAGGAGGAGACGGTGGGGGTCTCATCCACACAGCTTATTAGGAATGGGGACTGG ACCTTCCAGGTCCTGGTCATGCTGGAGATGACCCCTCGGCGGGGAGAGGTCTACACC TGTCACGTGGAGCATCCCAGCCTGAAGAGCCCCATCACTGTGGAGTGGAGGGCACAG TCTGAGTCTGCCTGGAGCAAGATGTTGAGCGGCATCGGGGGCTGCGTGCTTGGGGTG ATCTTCCTCGGGCTTGGCCTTTTCATCCGTCACAGGAGTCAGAAAGGACCTCGAGGC CCTCCTCCAGCAGGGCTCCTGCAGATGGGGGTGGAGGGCTGCACCAAATGCATCAAA TACCTGCTCTTCGTCTTCAATTTCGTCTTCTGGCTGGCTGGAGGCGTGATCCTAGGT GTAGCTCTGTGGTTGCGTCATGATCCACAGACCACCAGCCTGCTGTACCTGGAACTG GGAAACAAACCGGCACCCAACACCTTCTACGTGGGCATCTACATTCTCATTGCTGTG GGAGCTGTGATGATGTTTGTAGGCTTCCTGGGGTGCTATGGGGCCATCCAGGAGTCC CAGTGTCTGCTGGGGACGTTCTTCACCTGCCTTGTGATCCTGTTTGCCTGTGAGGTG GCTGCAGGCATCTGGGGCTTCGTAAACAAAGACCAGATCGCCAAGGATGTGAAGCAG TTCTATGACCAGGCCCTTCAGCAAGCTGTGATGGATGATGATGCCAACAATGCCAAG GCTGTGGTGAAGACTTTCCATGAGACGCTCAACTGTTGTGGCTCCAACGCACTGACC ACACTGACTACCACCATACTGAGGAACAGCCTGTGTCCCTCAGGCGGCAACATACTC ACCCCCTTACTGCAGCAAGATTGTCATCAGAAAATCGATGAGCTCTTCTCTGGGAAG CTGTACCTCATTGGAATTGCAGCCATTGTGGTAGCTGTCATTATGATCTTTGAGATG ATTCTGAGCATGGTGCTGTGCTGTGGCATCCGGAACAGCTCCGTGTACTGA | |
| MHC class IIα chain | MPRSRALILGVLALTTMLSLCGGEDDIEADHVGTYGISVYQSPGDIGQYTFEFDGDE LFYVDLDKKETVWMLPEFGQLASFDPQGGLQNIAVVKHNLGVLTKRSNSTPATNEAP QATVFPKSPVLLGQPNTLICFVDNIFPPVINITWLRNSKSVADGVYETSFFVNRDYS FHKLSYLTFIPSDDDIYDCKVEHWGLEEPVLKHWEPEIPAPMSELTETVVCALGLSV GLVGIVVGTIFIIQGLRSGGTSRHPGPL | 45 |
| | ATGCCGCGCAGCAGAGCTCTGATTCTGGGGGTCCTCGCCCTGACCACCATGCTCAGC CTCTGTGGAGGTGAAGACGACATTGAGGCCGACCACGTAGGCACCTATGGTATAAGT GTATATCAGTCTCCTGGAGACATTGGCCAGTACACATTTGAATTTGATGGTGATGAG TTGTTCTATGTGGACTTGGATAAGAAGGAGACTGTCTGGATGCTTCCTGAGTTTGGC CAATTGGCAAGCTTTGACCCCCAAGGTGGACTGCAAAACATAGCTGTAGTAAAACAC | 46 |

**[Table 4-4]**

| | | |
|---|---|---|
| | AACTTGGGAGTCTTGACTAAGAGGTCAAATTCCACCCCAGCTACCAATGAGGCTCCT CAAGCGACTGTGTTCCCCAAGTCCCCTGTGCTGCTGGGTCAGCCCAACACCCTCATC TGCTTTGTGGACAACATCTTCCCTCCTGTGATCAACATCACATGGCTCAGAAATAGC AAGTCAGTCGCAGACGGTGTTTATGAGACCAGCTTCTTCGTCAACCGTGACTATTCC TTCCACAAGCTGTCTTATCTCACCTTCATCCCTTCTGACGATGACATTTATGACTGC AAGGTGGAACACTGGGGCCTGGAGGAGCCGGTTCTGAAACACTGGGAACCTGAGATT CCAGCCCCCATGTCAGAGCTGACAGAGACTGTGGTCTGTGCCCTGGGGTTGTCTGTG GGCCTTGTGGGCATCGTGGTGGGCACCATCTTCATCATTCAAGGCCTGCGATCAGGT GGCACCTCCAGACACCCAGGGCCTTTATGA | |

**[Table 5-1]**

| | Sequence | SEQ ID NO: |
|---|---|---|
| TGF-*β*1 | MPPSGLRLLPLLLPLPWLLVLTPGRPAAGLSTSKTIDMELVKRKRIEAIRGQILSKL RLASPPSQGEVPPGPLPEAVLALYNSTRDRVAGESADPEPEPEADYYAKEVTRVLMV DRNNAIYEKTKDISHSIYMFFNTSDIREAVPEPPLLSRAELRLQRLKSSVEQHVELY QKYSNNSWRYLGNRLLTPTDTPEWLSFDVTGVVRQWLNQGDGIQGFRFSAHSSSDSK DNKLHVEINGISPKRRGDLGTIHDMNRPFLLLMATPLERAQHLHSSRHRRALDTNYC FSSTEKNCCVRQLYIDFRKDLGWKWIHEPKGYHANFCLGPCPYIWSLDTQYSKVLAL YNQHNPGASASPCCVPQALEPLPIVYYVGRKPKVEQLSNMIVRSCKCS | 47 |
| | ATGCCGCCCTCGGGGCTGCGGCTACTGCCGCTTCTGCTCCCACTCCCGTGGCTTCTA GTGCTGACGCCCGGGAGGCCAGCCGCGGGACTCTCCACCTCTAAGACCATCGACATG GAGCTGGTGAAACGGAAGCGCATCGAAGCCATCCGTGGCCAGATCCTGTCCAAACTA AGGCTCGCCAGTCCCCCAAGCCAGGGGGAGGTACCGCCCGGCCCGCTGCCCGAGGCG GTGCTCGCTTTGTACAACAGCACCCGCGACCGGGTGGCAGGCGAGAGCGCCGACCCA GAGCCGGAGCCCGAAGCGGACTACTATGCTAAAGAGGTCACCCGCGTGCTAATGGTG GACCGCAACAACGCCATCTATGAGAAAACCAAAGACATCTCACACAGTATATATATG TTCTTCAATACGTCAGACATTCGGGAAGCAGTGCCCGAACCCCCATTGCTGTCCCGT GCAGAGCTGCGCTTGCAGAGATTAAAATCAAGTGTGGAGCAACATGTGGAACTCTAC CAGAAATATAGCAACAATTCCTGGCGTTACCTTGGTAACCGGCTGCTGACCCCCACT GATACGCCTGAGTGGCTGTCTTTTGACGTCACTGGAGTTGTACGGCAGTGGCTGAAC CAAGGAGACGGAATACAGGGCTTTCGATTCAGCGCTCACTCTTCTTCTGACAGCAAA GATAACAAACTCCACGTGGAAATCAACGGGATCAGCCCCAAACGTCGGGGCGACCTG GGCACCATCCATGACATGAACCGGCCCTTCCTGCTCCTCATGGCCACCCCCCTGGAA AGGGCCCAGCACCTGCACAGCTCACGGCACCGGAGAGCCCTGGATACCAACTATTGC TTCAGCTCCACAGAGAAGAACTGCTGTGTGCGGCAGCTGTACATTGACTTTAGGAAG GACCTGGGTTGGAAGTGGATCCACGAGCCCAAGGGCTACCATGCCAACTTCTGTCTG GGACCCTGCCCCTATATTTGGAGCCTGGACACACAGTACAGCAAGGTCCTTGCCCTC TACAACCAACACAACCCGGGCGCTTCGGCGTCACCGTGCTGCGTGCCGCAGGCTTTG GAGCCACTGCCCATCGTCTACTACGTGGGTCGCAAGCCCAAGGTGGAGCAGTTGTCC AACATGATTGTGCGCTCCTGCAAGTGCAGCTGA | 48 |
| MFG-E8 (from which signal peptide | ASGDFCDSSLCLNGGTCLTGQDNDIYCLCPEGFTGLVCNETERGPCSPNPCYNDAKC LVTLDTQRGEIFTEYICQCPVGYSGIHCETETNYYNLDGEYMFTTAVPNTAVPTPAP | 49 |

**[Table 5-2]**

| | | |
|---|---|---|
| is removed) | TPDLSNNLASRCSTQLGMEGGAIADSQISASSVYMGFMGLQRWGPELARLYRTGIVN AWTASNYDSKPWIQVNLLRKMRVSGVMTQGASRAGRAEYLKTFKVAYSLDGRKFEFI QDESGGDKEFLGNLDNNSLKVNMFNPTLEAQYIKLYPVSCHRGCTLRFELLGCELHG CSEPLGLKNNTIPDSQMSASSSYKTWNLRAFGWYPHLGRLDNQGKINAWTAQSNSAK EWLQVDLGTQRQVTGIITQGARDFGHIQYVASYKVAHSDDGVQWTVYEEQGSSKVFQ GNLDNNSHKKNIFEKPFMARYVRVLPVSWHNRITLRLELLGC | |
| | GCGTCTGGTGACTTCTGTGACTCCAGCCTGTGCCTGAACGGTGGCACCTGCTTGACG GGCCAAGACAATGACATCTACTGCCTCTGCCCTGAAGGCTTCACAGGCCTTGTGTGC AATGAGACTGAGAGAGGACCATGCTCCCCAAACCCTTGCTACAATGATGCCAAATGT CTGGTGACTTTGGACACACAGCGTGGGGAAATCTTCACCGAATACATCTGCCAGTGC CCTGTGGGCTACTCGGGCATCCACTGTGAAACCGAGACCAACTACTACAACCTGGAT GGAGAATACATGTTCACCACAGCCGTCCCCAATACTGCCGTCCCCACCCCGGCCCCC ACCCCCGATCTTTCCAACAACCTAGCCTCCCGTTGTTCTACACAGCTGGGCATGGAA GGGGGCGCCATTGCTGATTCACAGATTTCCGCCTCGTCTGTGTATATGGGTTTCATG GGCTTGCAGCGCTGGGGCCCGGAGCTGGCTCGTCTGTACCGCACAGGGATCGTCAAT GCCTGGACAGCCAGCAACTATGATAGCAAGCCCTGGATCCAGGTGAACCTTCTGCGG AAGATGCGGGTATCAGGTGTGATGACGCAGGGTGCCAGCCGTGCCGGGAGGGCGGAG TACCTGAAGACCTTCAAGGTGGCTTACAGCCTCGACGGACGCAAGTTTGAGTTCATC CAGGATGAAAGCGGTGGAGACAAGGAGTTTTTGGGTAACCTGGACAACAACAGCCTG AAGGTTAACATGTTCAACCCGACTCTGGAGGCACAGTACATAAAGCTGTACCCTGTT TCGTGCCACCGCGGCTGCACCCTCCGCTTCGAGCTCCTGGGCTGTGAGTTGCACGGA TGTTCTGAGCCCCTGGGCCTGAAGAATAACACAATTCCTGACAGCCAGATGTCAGCC TCCAGCAGCTACAAGACATGGAACCTGCGTGCTTTTGGCTGGTACCCCCACTTGGGA AGGCTGGATAATCAGGGCAAGATCAATGCCTGGACGGCTCAGAGCAACAGTGCCAAG GAATGGCTGCAGGTTGACCTGGGCACTCAGAGGCAAGTGACAGGAATCATCACCCAG GGGGCCCGTGACTTTGGCCACATCCAGTATGTGGCGTCCTACAAGGTAGCCCACAGT GATGATGGTGTGCAGTGGACTGTATATGAGGAGCAAGGAAGCAGCAAGGTCTTCCAG GGCAACTTGGACAACAACTCCCACAAGAAGAACATCTTCGAGAAACCCTTCATGGCT CGCTACGTGCGTGTCCTTCCAGTGTCCTGGCATAACCGCATCACCCTGCGCCTGGAG CTGCTGGGCTGTTAA | 50 |
| TGF-*β*1 -MFG-E 8 (TGF- *β*1+ | MPPSGLRLLPLLLPLPWLLVLTPGRPAAGLSTSKTIDMELVKRKRIEAIRGQILSKL RLASPPSQGEVPPGPLPEAVLALYNSTRDRVAGESADPEPEPEADYYAKEVTRVLMV DRNNAIYEKTKDISHSIYMFFNTSDIREAVPEPPLLSRAELRLQRLKSSVEQHVELY | 51 |

**[Table 5-3]**

| | | |
|---|---|---|
| peptide linker 3 + MFG-E8 (from which signal peptide is removed)) | QKYSNNSWRYLGNRLLTPTDTPEWLSFDVTGVVRQWLNQGDGIQGFRFSAHSSSDSK DNKLHVEINGISPKRRGDLGTIHDMNRPFLLLMATPLERAQHLHSSRHRRALDTNYC FSSTEKNCCVRQLYIDFRKDLGWKWIHEPKGYHANFCLGPCPYIWSLDTQYSKVLAL YNQHNPGASASPCCVPQALEPLPIVYYVGRKPKVEQLSNMIVRSCKCSGGGGSASGD FCDSSLCLNGGTCLTGQDNDIYCLCPEGFTGLVCNETERGPCSPNPCYNDAKCLVTL DTQRGEIFTEYICQCPVGYSGIHCETETNYYNLDGEYMFTTAVPNTAVPTPAPTPDL SNNLASRCSTQLGMEGGAIADSQISASSVYMGFMGLQRWGPELARLYRTGIVNAWTA SNYDSKPWIQVNLLRKMRVSGVMTQGASRAGRAEYLKTFKVAYSLDGRKFEFIQDES GGDKEFLGNLDNNSLKVNMFNPTLEAQYIKLYPVSCHRGCTLRFELLGCELHGCSEP LGLKNNTIPDSQMSASSSYKTWNLRAFGWYPHLGRLDNQGKINAWTAQSNSAKEWLQ VDLGTQRQVTGIITQGARDFGHIQYVASYKVAHSDDGVQWTVYEEQGSSKVFQGNLD NNSHKKNIFEKPFMARYVRVLPVSWHNRITLRLELLGC | |
| | ATGCCGCCCTCGGGGCTGCGGCTACTGCCGCTTCTGCTCCCACTCCCGTGGCTTCTA GTGCTGACGCCCGGGAGGCCAGCCGCGGGACTCTCCACCTCTAAGACCATCGACATG GAGCTGGTGAAACGGAAGCGCATCGAAGCCATCCGTGGCCAGATCCTGTCCAAACTA AGGCTCGCCAGTCCCCCAAGCCAGGGGGAGGTACCGCCCGGCCCGCTGCCCGAGGCG GTGCTCGCTTTGTACAACAGCACCCGCGACCGGGTGGCAGGCGAGAGCGCCGACCCA GAGCCGGAGCCCGAAGCGGACTACTATGCTAAAGAGGTCACCCGCGTGCTAATGGTG GACCGCAACAACGCCATCTATGAGAAAACCAAAGACATCTCACACAGTATATATATG TTCTTCAATACGTCAGACATTCGGGAAGCAGTGCCCGAACCCCCATTGCTGTCCCGT GCAGAGCTGCGCTTGCAGAGATTAAAATCAAGTGTGGAGCAACATGTGGAACTCTAC CAGAAATATAGCAACAATTCCTGGCGTTACCTTGGTAACCGGCTGCTGACCCCCACT GATACGCCTGAGTGGCTGTCTTTTGACGTCACTGGAGTTGTACGGCAGTGGCTGAAC CAAGGAGACGGAATACAGGGCTTTCGATTCAGCGCTCACTCTTCTTCTGACAGCAAA GATAACAAACTCCACGTGGAAATCAACGGGATCAGCCCCAAACGTCGGGGCGACCTG GGCACCATCCATGACATGAACCGGCCCTTCCTGCTCCTCATGGCCACCCCCCTGGAA AGGGCCCAGCACCTGCACAGCTCACGGCACCGGAGAGCCCTGGATACCAACTATTGC TTCAGCTCCACAGAGAAGAACTGCTGTGTGCGGCAGCTGTACATTGACTTTAGGAAG GACCTGGGTTGGAAGTGGATCCACGAGCCCAAGGGCTACCATGCCAACTTCTGTCTG GGACCCTGCCCCTATATTTGGAGCCTGGACACACAGTACAGCAAGGTCCTTGCCCTC TACAACCAACACAACCCGGGCGCTTCGGCGTCACCGTGCTGCGTGCCGCAGGCTTTG GAGCCACTGCCCATCGTCTACTACGTGGGTCGCAAGCCCAAGGTGGAGCAGTTGTCC AACATGATTGTGCGCTCCTGCAAGTGCAGCGGAGGAGGAGGAAGCGCGTCTGGTGAC | 52 |

**[Table 5-4]**

| | | |
|---|---|---|
| | TTCTGTGACTCCAGCCTGTGCCTGAACGGTGGCACCTGCTTGACGGGCCAAGACAAT GACATCTACTGCCTCTGCCCTGAAGGCTTCACAGGCCTTGTGTGCAATGAGACTGAG AGAGGACCATGCTCCCCAAACCCTTGCTACAATGATGCCAAATGTCTGGTGACTTTG GACACACAGCGTGGGGAAATCTTCACCGAATACATCTGCCAGTGCCCTGTGGGCTAC TCGGGCATCCACTGTGAAACCGAGACCAACTACTACAACCTGGATGGAGAATACATG TTCACCACAGCCGTCCCCAATACTGCCGTCCCCACCCCGGCCCCCACCCCCGATCTT TCCAACAACCTAGCCTCCCGTTGTTCTACACAGCTGGGCATGGAAGGGGGCGCCATT GCTGATTCACAGATTTCCGCCTCGTCTGTGTATATGGGTTTCATGGGCTTGCAGCGC TGGGGCCCGGAGCTGGCTCGTCTGTACCGCACAGGGATCGTCAATGCCTGGACAGCC AGCAACTATGATAGCAAGCCCTGGATCCAGGTGAACCTTCTGCGGAAGATGCGGGTA TCAGGTGTGATGACGCAGGGTGCCAGCCGTGCCGGGAGGGCGGAGTACCTGAAGACC TTCAAGGTGGCTTACAGCCTCGACGGACGCAAGTTTGAGTTCATCCAGGATGAAAGC GGTGGAGACAAGGAGTTTTTGGGTAACCTGGACAACAACAGCCTGAAGGTTAACATG TTCAACCCGACTCTGGAGGCACAGTACATAAAGCTGTACCCTGTTTCGTGCCACCGC GGCTGCACCCTCCGCTTCGAGCTCCTGGGCTGTGAGTTGCACGGATGTTCTGAGCCC CTGGGCCTGAAGAATAACACAATTCCTGACAGCCAGATGTCAGCCTCCAGCAGCTAC AAGACATGGAACCTGCGTGCTTTTGGCTGGTACCCCCACTTGGGAAGGCTGGATAAT CAGGGCAAGATCAATGCCTGGACGGCTCAGAGCAACAGTGCCAAGGAATGGCTGCAG GTTGACCTGGGCACTCAGAGGCAAGTGACAGGAATCATCACCCAGGGGGCCCGTGAC TTTGGCCACATCCAGTATGTGGCGTCCTACAAGGTAGCCCACAGTGATGATGGTGTG CAGTGGACTGTATATGAGGAGCAAGGAAGCAGCAAGGTCTTCCAGGGCAACTTGGAC AACAACTCCCACAAGAAGAACATCTTCGAGAAACCCTTCATGGCTCGCTACGTGCGT GTCCTTCCAGTGTCCTGGCATAACCGCATCACCCTGCGCCTGGAGCTGCTGGGCTGT TAA | |

**[Table 6-1]**

| | Sequence | SEQ ID NO: |
|---|---|---|
| I L - 4 (from which signal peptide is removed) | HIHGCDKNHLREIIGILNEVTGEGTPCTEMDVPNVLTATKNTTESELVCRASKVLRI FYLKHGKTPCLKKNSSVLMELQRLFRAFRCLDSSISCTMNESKSTSLKDFLESLKSI MQMDYS | 53 |
| | CATATCCACGGATGCGACAAAAATCACTTGAGAGAGATCATCGGCATTTTGAACGAG GTCACAGGAGAAGGGACGCCATGCACGGAGATGGATGTGCCAAACGTCCTCACAGCA ACGAAGAACACCACAGAGAGTGAGCTCGTCTGTAGGGCTTCCAAGGTGCTTCGCATA TTTTATTTAAAACATGGGAAAACTCCATGCTTGAAGAAGAACTCTAGTGTTCTCATG GAGCTGCAGAGACTCTTTCGGGCTTTTCGATGCCTGGATTCATCGATAAGCTGCACC ATGAATGAGTCCAAGTCCACATCACTGAAAGACTTCCTGGAAAGCCTAAAGAGCATC ATGCAAATGGATTACTCGTAG | 54 |
| C D 8 1 (amino acids 1 to 177) | MGVEGCTKCIKYLLFVFNFVFWLAGGVILGVALWLRHDPQTTSLLYLELGNKPAPNT FYVGIYILIAVGAVMMFVGFLGCYGAIQESQCLLGTFFTCLVILFACEVAAGIWGFV NKDQIAKDVKQFYDQALQQAVMDDDANNAKAVVKTFHETLNCCGSNALTTLTTTILR NSLCPS | 61 |
| | ATGGGGGTGGAGGGCTGCACCAAATGCATCAAATACCTGCTCTTCGTCTTCAATTTC GTCTTCTGGCTGGCTGGAGGCGTGATCCTAGGTGTAGCTCTGTGGTTGCGTCATGAT CCACAGACCACCAGCCTGCTGTACCTGGAACTGGGAAACAAACCGGCACCCAACACC TTCTACGTGGGCATCTACATTCTCATTGCTGTGGGAGCTGTGATGATGTTTGTAGGC TTCCTGGGGTGCTATGGGGCCATCCAGGAGTCCCAGTGTCTGCTGGGGACGTTCTTC ACCTGCCTTGTGATCCTGTTTGCCTGTGAGGTGGCTGCAGGCATCTGGGGCTTCGTA AACAAAGACCAGATCGCCAAGGATGTGAAGCAGTTCTATGACCAGGCCCTTCAGCAA GCTGTGATGGATGATGATGCCAACAATGCCAAGGCTGTGGTGAAGACTTTCCATGAG ACGCTCAACTGTTGTGGCTCCAACGCACTGACCACACTGACTACCACCATACTGAGG AACAGCCTGTGTCCCTCA | 62 |
| C D 8 1 (amino acids 178 to 236) | GGNILTPLLQQDCHQKIDELFSGKLYLIGIAAIVVAVIMIFEMILSMVLCCGIRNSS VY | 63 |
| | GGCGGCAACATACTCACCCCCTTACTGCAGCAAGATTGTCATCAGAAAATCGATGAG CTCTTCTCTGGGAAGCTGTACCTCATTGGAATTGCAGCCATTGTGGTAGCTGTCATT ATGATCTTTGAGATGATTCTGAGCATGGTGCTGTGCTGTGGCATCCGGAACAGCTCC GTGTACTGA | 64 |

**[Table 6-2]**

| | | |
|---|---|---|
| CD81-I L-4 | MGVEGCTKCIKYLLFVFNFVFWLAGGVILGVALWLRHDPQTTSLLYLELGNKPAPNT FYVGIYILIAVGAVMMFVGFLGCYGAIQESQCLLGTFFTCLVILFACEVAAGIWGFV NKDQIAKDVKQFYDQALQQAVMDDDANNAKAVVKTFHETLNCCGSNALTTLTTTILR NSLCPSGGGGSHIHGCDKNHLREIIGILNEVTGEGTPCTEMDVPNVLTATKNTTESE LVCRASKVLRIFYLKHGKTPCLKKNSSVLMELQRLFRAFRCLDSSISCTMNESKSTS LKDFLESLKSIMQMDYSGGGGSGGNILTPLLQQDCHQKIDELFSGKLYLIGIAAIVV AVIMIFEMILSMVLCCGIRNSSVY | 55 |
| | ATGGGGGTGGAGGGCTGCACCAAATGCATCAAATACCTGCTCTTCGTCTTCAATTTC GTCTTCTGGCTGGCTGGAGGCGTGATCCTAGGTGTAGCTCTGTGGTTGCGTCATGAT CCACAGACCACCAGCCTGCTGTACCTGGAACTGGGAAACAAACCGGCACCCAACACC TTCTACGTGGGCATCTACATTCTCATTGCTGTGGGAGCTGTGATGATGTTTGTAGGC TTCCTGGGGTGCTATGGGGCCATCCAGGAGTCCCAGTGTCTGCTGGGGACGTTCTTC ACCTGCCTTGTGATCCTGTTTGCCTGTGAGGTGGCTGCAGGCATCTGGGGCTTCGTA AACAAAGACCAGATCGCCAAGGATGTGAAGCAGTTCTATGACCAGGCCCTTCAGCAA GCTGTGATGGATGATGATGCCAACAATGCCAAGGCTGTGGTGAAGACTTTCCATGAG ACGCTCAACTGTTGTGGCTCCAACGCACTGACCACACTGACTACCACCATACTGAGG AACAGCCTGTGTCCCTCAGGAGGAGGAGGAAGCCATATCCACGGATGCGACAAAAAT CACTTGAGAGAGATCATCGGCATTTTGAACGAGGTCACAGGAGAAGGGACGCCATGC ACGGAGATGGATGTGCCAAACGTCCTCACAGCAACGAAGAACACCACAGAGAGTGAG CTCGTCTGTAGGGCTTCCAAGGTGCTTCGCATATTTTATTTAAAACATGGGAAAACT CCATGCTTGAAGAAGAACTCTAGTGTTCTCATGGAGCTGCAGAGACTCTTTCGGGCT TTTCGATGCCTGGATTCATCGATAAGCTGCACCATGAATGAGTCCAAGTCCACATCA CTGAAAGACTTCCTGGAAAGCCTAAAGAGCATCATGCAAATGGATTACTCGGGAGGA GGAGGAAGCGGCGGCAACATACTCACCCCCTTACTGCAGCAAGATTGTCATCAGAAA ATCGATGAGCTCTTCTCTGGGAAGCTGTACCTCATTGGAATTGCAGCCATTGTGGTA GCTGTCATTATGATCTTTGAGATGATTCTGAGCATGGTGCTGTGCTGTGGCATCCGG AACAGCTCCGTGTACTGA | 56 |

**[Table 7-1]**

| | Sequence | SEQ ID NO: |
|---|---|---|
| Signal peptide of MHC class II*β* chain | MALQIPSLLLSAAVVVLMVLSSPGTEG | 33 |
| | ATGGCTCTGCAGATCCCCAGCCTCCTCCTCTCGGCTGCTGTGGTGGTGCTGATGGTGCTGAGC AGCCCAGGGACTGAGGGC | 34 |
| OVA peptide 2 (for MHC class II molecule) | ISQAVHAAHAEINEAGR | 35 |
| | ATATCTCAAGCTGTCCATGCAGCACATGCAGAAATCAATGAAGCAGGCAGA | |
| MHC class II*β* chain (from which signal peptide is removed) | GDSERHFVYQFMGECYFTNGTQRIRYVTRYIYNREEYVRYDSDVGEHRAVTELGRPDAEYWNS QPEILERTRAELDTVCRHNYEGPETHTSLRRLEQPNVVISLSRTEALNHHNTLVCSVTDFYPA KIKVRWFRNGQEETVGVSSTQLIRNGDWTFQVLVMLEMTPRRGEVYTCHVEHPSLKSPITVEW RAQSESAWSKMLSGIGGCVLGVIFLGLGLFIRHRSQKGPRGPPPAGLLQ | 36 |
| | GGAGACTCCGAAAGGCATTTCGTGTACCAGTTCATGGGCGAGTGCTACTTCACCAACGGGACG CAGCGCATACGATATGTGACCAGATACATCTACAACCGGGAGGAGTACGTGCGCTACGACAGC GACGTGGGCGAGCACCGCGCGGTGACCGAGCTGGGGCGGCCAGACGCCGAGTACTGGAACAGC CAGCCGGAGATCCTGGAGCGAACGCGGGCCGAGCTGGACACGGTGTGCAGACACAACTACGAG GGGCCGGAGACCCACACCTCCCTGCGGCGGCTTGAACAGCCCAATGTCGTCATCTCCCTGTCC AGGACAGAGGCCCTCAACCACCACAACACTCTGGTCTGCTCAGTGACAGATTTCTACCCAGCC AAGATCAAAGTGCGCTGGTTCCGGAATGGCCAGGAGGAGACGGTGGGGGTCTCATCCACACAG CTTATTAGGAATGGGGACTGGACCTTCCAGGTCCTGGTCATGCTGGAGATGACCCCTCGGCGG GGAGAGGTCTACACCTGTCACGTGGAGCATCCCAGCCTGAAGAGCCCCATCACTGTGGAGTGG AGGGCACAGTCTGAGTCTGCCTGGAGCAAGATGTTGAGCGGCATCGGGGGCTGCGTGCTTGGG GTGATCTTCCTCGGGCTTGGCCTTTTCATCCGTCACAGGAGTCAGAAAGGACCTCGAGGCCCT CCTCCAGCAGGGCTCCTGCAG | 37 |
| Peptide linker 1 | GGGGSGGGGSG | 39 |
| | GGAGGAGGAGGAAGCGGAGGAGGAGGAAGCGGA | 40 |
| sc-Dime r (OVA peptide 2 + peptide linker 4 + MHC class II*β* chain (from which signal peptide is removed)) | MALQIPSLLLSAAVVVLMVLSSPGTEGISQAVHAAHAEINEAGRGGGGSGGGGSGGDSERHFV YQFMGECYFTNGTQRIRYVTRYIYNREEYVRYDSDVGEHRAVTELGRPDAEYWNSQPEILERT RAELDTVCRHNYEGPETHTSLRRLEQPNVVISLSRTEALNHHNTLVCSVTDFYPAKIKVRWFR NGQEETVGVSSTQLIRNGDWTFQVLVMLEMTPRRGEVYTCHVEHPSLKSPITVEWRAQSESAW SKMLSGIGGCVLGVIFLGLGLFIRHRSQKGPRGPPPAGLLQ | 41 |
| | ATGGCTCTGCAGATCCCCAGCCTCCTCCTCTCGGCTGCTGTGGTGGTGCTGATGGTGCTGAGC AGCCCAGGGACTGAGGGCATATCTCAAGCTGTCCATGCAGCACATGCAGAAATCAATGAAGCA GGCAGAGGAGGAGGAGGAAGCGGAGGAGGAGGAAGCGGAGGAGACTCCGAAAGGCATTTCGTG TACCAGTTCATGGGCGAGTGCTACTTCACCAACGGGACGCAGCGCATACGATATGTGACCAGA | 42 |

**[Table 7-2]**

| | | |
|---|---|---|
| | TACATCTACAACCGGGAGGAGTACGTGCGCTACGACAGCGACGTGGGCGAGCACCGCGCGGTG ACCGAGCTGGGGCGGCCAGACGCCGAGTACTGGAACAGCCAGCCGGAGATCCTGGAGCGAACG CGGGCCGAGCTGGACACGGTGTGCAGACACAACTACGAGGGGCCGGAGACCCACACCTCCCTG CGGCGGCTTGAACAGCCCAATGTCGTCATCTCCCTGTCCAGGACAGAGGCCCTCAACCACCAC AACACTCTGGTCTGCTCAGTGACAGATTTCTACCCAGCCAAGATCAAAGTGCGCTGGTTCCGG AATGGCCAGGAGGAGACGGTGGGGGTCTCATCCACACAGCTTATTAGGAATGGGGACTGGACC TTCCAGGTCCTGGTCATGCTGGAGATGACCCCTCGGCGGGGAGAGGTCTACACCTGTCACGTG GAGCATCCCAGCCTGAAGAGCCCCATCACTGTGGAGTGGAGGGCACAGTCTGAGTCTGCCTGG AGCAAGATGTTGAGCGGCATCGGGGGCTGCGTGCTTGGGGTGATCTTCCTCGGGCTTGGCCTT TTCATCCGTCACAGGAGTCAGAAAGGACCTCGAGGCCCTCCTCCAGCAGGGCTCCTGCAG | |
| sc-Dime r-CD81- IL-12α | MALQIPSLLLSAAVVVLMVLSSPGTEGISQAVHAAHAEINEAGRGGGGSGGGGSGGDSERHFV YQFMGECYFTNGTQRIRYVTRYIYNREEYVRYDSDVGEHRAVTELGRPDAEYWNSQPEILERT RAELDTVCRHNYEGPETHTSLRRLEQPNVVISLSRTEALNHHNTLVCSVTDFYPAKIKVRWFR NGQEETVGVSSTQLIRNGDWTFQVLVMLEMTPRRGEVYTCHVEHPSLKSPITVEWRAQSESAW SKMLSGIGGCVLGVIFLGLGLFIRHRSQKGPRGPPPAGLLQMGVEGCTKCIKYLLFVFNFVFW LAGGVILGVALWLRHDPQTTSLLYLELGNKPAPNTFYVGIYILIAVGAVMMFVGFLGCYGAIQ ESQCLLGTFFTCLVILFACEVAAGIWGFVNKDQIAKDVKQFYDQALQQAVMDDDANNAKAVVK TFHETLNCCGSNALTTLTTTILRNSLCPSGGGGSRVIPVSGPARCLSQSRNLLKTTDDMVKTA REKLKHYSCTAEDIDHEDITRDQTSTLKTCLPLELHKNESCLATRETSSTTRGSCLPPQKTSL MMTLCLGSIYEDLKMYQTEFQAINAALQNHNHQQIILDKGMLVAIDELMQSLNHNGETLRQKP PVGEADPYRVKMKLCILLHAFSTRVVTINRVMGYLSSAGGGGSGGNILTPLLQQDCHQKIDEL FSGKLYLIGIAAIVVAVIMIFEMILSMVLCCGIRNSSVY | 91 |
| | ATGGCTCTGCAGATCCCCAGCCTCCTCCTCTCGGCTGCTGTGGTGGTGCTGATGGTGCTGAGC AGCCCAGGGACTGAGGGCATATCTCAAGCTGTCCATGCAGCACATGCAGAAATCAATGAAGCA GGCAGAGGAGGAGGAGGAAGCGGAGGAGGAGGAAGCGGAGGAGACTCCGAAAGGCATTTCGTG TACCAGTTCATGGGCGAGTGCTACTTCACCAACGGGACGCAGCGCATACGATATGTGACCAGA TACATCTACAACCGGGAGGAGTACGTGCGCTACGACAGCGACGTGGGCGAGCACCGCGCGGTG ACCGAGCTGGGGCGGCCAGACGCCGAGTACTGGAACAGCCAGCCGGAGATCCTGGAGCGAACG CGGGCCGAGCTGGACACGGTGTGCAGACACAACTACGAGGGGCCGGAGACCCACACCTCCCTG CGGCGGCTTGAACAGCCCAATGTCGTCATCTCCCTGTCCAGGACAGAGGCCCTCAACCACCAC AACACTCTGGTCTGCTCAGTGACAGATTTCTACCCAGCCAAGATCAAAGTGCGCTGGTTCCGG AATGGCCAGGAGGAGACGGTGGGGGTCTCATCCACACAGCTTATTAGGAATGGGGACTGGACC TTCCAGGTCCTGGTCATGCTGGAGATGACCCCTCGGCGGGGAGAGGTCTACACCTGTCACGTG GAGCATCCCAGCCTGAAGAGCCCCATCACTGTGGAGTGGAGGGCACAGTCTGAGTCTGCCTGG AGCAAGATGTTGAGCGGCATCGGGGGCTGCGTGCTTGGGGTGATCTTCCTCGGGCTTGGCCTT | 92 |

**[Table 7-3]**

| | | |
|---|---|---|
| | TTCATCCGTCACAGGAGTCAGAAAGGACCTCGAGGCCCTCCTCCAGCAGGGCTCCTGCAGATG GGGGTGGAGGGCTGCACCAAATGCATCAAATACCTGCTCTTCGTCTTCAATTTCGTCTTCTGG CTGGCTGGAGGCGTGATCCTAGGTGTAGCTCTGTGGTTGCGTCATGATCCACAGACCACCAGC CTGCTGTACCTGGAACTGGGAAACAAACCGGCACCCAACACCTTCTACGTGGGCATCTACATT CTCATTGCTGTGGGAGCTGTGATGATGTTTGTAGGCTTCCTGGGGTGCTATGGGGCCATCCAG GAGTCCCAGTGTCTGCTGGGGACGTTCTTCACCTGCCTTGTGATCCTGTTTGCCTGTGAGGTG GCTGCAGGCATCTGGGGCTTCGTAAACAAAGACCAGATCGCCAAGGATGTGAAGCAGTTCTAT GACCAGGCCCTTCAGCAAGCTGTGATGGATGATGATGCCAACAATGCCAAGGCTGTGGTGAAG ACTTTCCATGAGACGCTCAACTGTTGTGGCTCCAACGCACTGACCACACTGACTACCACCATA CTGAGGAACAGCCTGTGTCCCTCAGGAGGAGGAGGAAGCAGGGTCATTCCAGTCTCTGGACCT GCCAGGTGTCTTAGCCAGTCCCGAAACCTGCTGAAGACCACAGATGACATGGTGAAGACGGCC AGAGAAAAACTGAAACATTATTCCTGCACTGCTGAAGACATCGATCATGAAGACATCACACGG GACCAAACCAGCACATTGAAGACCTGTTTACCACTGGAACTACACAAGAACGAGAGTTGCCTG GCTACTAGAGAGACTTCTTCCACAACAAGAGGGAGCTGCCTGCCCCCACAGAAGACGTCTTTG ATGATGACCCTGTGCCTTGGTAGCATCTATGAGGACTTGAAGATGTACCAGACAGAGTTCCAG GCCATCAACGCAGCACTTCAGAATCACAACCATCAGCAGATCATTCTAGACAAGGGCATGCTG GTGGCCATCGATGAGCTGATGCAGTCTCTGAATCATAATGGCGAGACTCTGCGCCAGAAACCT CCTGTGGGAGAAGCAGACCCTTACAGAGTGAAAATGAAGCTCTGCATCCTGCTTCACGCCTTC AGCACCCGCGTCGTGACCATCAACAGGGTGATGGGCTATCTGAGCTCCGCCGGAGGAGGAGGA AGCGGCGGCAACATACTCACCCCCTTACTGCAGCAAGATTGTCATCAGAAAATCGATGAGCTC TTCTCTGGGAAGCTGTACCTCATTGGAATTGCAGCCATTGTGGTAGCTGTCATTATGATCTTT GAGATGATTCTGAGCATGGTGCTGTGCTGTGGCATCCGGAACAGCTCCGTGTACTGA | |
| MHC class IIα chain | MPRSRALILGVLALTTMLSLCGGEDDIEADHVGTYGISVYQSPGDIGQYTFEFDGDELFYVDL DKKETVWMLPEFGQLASFDPQGGLQNIAVVKHNLGVLTKRSNSTPATNEAPQATVFPKSPVLL GQPNTLICFVDNIFPPVINITWLRNSKSVADGVYETSFFVNRDYSFHKLSYLTFIPSDDDIYD CKVEHWGLEEPVLKHWEPEIPAPMSELTETVVCALGLSVGLVGIVVGTIFIIQGLRSGGTSRH PGPL | 45 |
| | ATGCCGCGCAGCAGAGCTCTGATTCTGGGGGTCCTCGCCCTGACCACCATGCTCAGCCTCTGT GGAGGTGAAGACGACATTGAGGCCGACCACGTAGGCACCTATGGTATAAGTGTATATCAGTCT CCTGGAGACATTGGCCAGTACACATTTGAATTTGATGGTGATGAGTTGTTCTATGTGGACTTG GATAAGAAGGAGACTGTCTGGATGCTTCCTGAGTTTGGCCAATTGGCAAGCTTTGACCCCCAA GGTGGACTGCAAAACATAGCTGTAGTAAAACACAACTTGGGAGTCTTGACTAAGAGGTCAAAT TCCACCCCAGCTACCAATGAGGCTCCTCAAGCGACTGTGTTCCCCAAGTCCCCTGTGCTGCTG GGTCAGCCCAACACCCTCATCTGCTTTGTGGACAACATCTTCCCTCCTGTGATCAACATCACA TGGCTCAGAAATAGCAAGTCAGTCGCAGACGGTGTTTATGAGACCAGCTTCTTCGTCAACCGT | 46 |

**[Table 7-4]**

| | | |
|---|---|---|
| | GACTATTCCTTCCACAAGCTGTCTTATCTCACCTTCATCCCTTCTGACGATGACATTTATGAC TGCAAGGTGGAACACTGGGGCCTGGAGGAGCCGGTTCTGAAACACTGGGAACCTGAGATTCCA GCCCCCATGTCAGAGCTGACAGAGACTGTGGTCTGTGCCCTGGGGTTGTCTGTGGGCCTTGTG GGCATCGTGGTGGGCACCATCTTCATCATTCAAGGCCTGCGATCAGGTGGCACCTCCAGACAC CCAGGGCCTTTATGA | |

**[Table 8-1]**

| | Sequence | SEQ ID NO: |
|---|---|---|
| IL-12α (from which signal peptide is removed) | RVIPVSGPARCLSQSRNLLKTTDDMVKTAREKLKHYSCTAEDIDHEDITRDQTSTLKTCLPLE LHKNESCLATRETSSTTRGSCLPPQKTSLMMTLCLGSIYEDLKMYQTEFQAINAALQNHNHQQ IILDKGMLVAIDELMQSLNHNGETLRQKPPVGEADPYRVKMKLCILLHAFSTRVVTINRVMGY LSSA | 93 |
| | AGGGTCATTCCAGTCTCTGGACCTGCCAGGTGTCTTAGCCAGTCCCGAAACCTGCTGAAGACC ACAGATGACATGGTGAAGACGGCCAGAGAAAAACTGAAACATTATTCCTGCACTGCTGAAGAC ATCGATCATGAAGACATCACACGGGACCAAACCAGCACATTGAAGACCTGTTTACCACTGGAA CTACACAAGAACGAGAGTTGCCTGGCTACTAGAGAGACTTCTTCCACAACAAGAGGGAGCTGC CTGCCCCCACAGAAGACGTCTTTGATGATGACCCTGTGCCTTGGTAGCATCTATGAGGACTTG AAGATGTACCAGACAGAGTTCCAGGCCATCAACGCAGCACTTCAGAATCACAACCATCAGCAG ATCATTCTAGACAAGGGCATGCTGGTGGCCATCGATGAGCTGATGCAGTCTCTGAATCATAAT GGCGAGACTCTGCGCCAGAAACCTCCTGTGGGAGAAGCAGACCCTTACAGAGTGAAAATGAAG CTCTGCATCCTGCTTCACGCCTTCAGCACCCGCGTCGTGACCATCAACAGGGTGATGGGCTAT CTGAGCTCCGCC | 94 |
| CD81 (amino acids 1 to 177) | MGVEGCTKCIKYLLFVFNFVFWLAGGVILGVALWLRHDPQTTSLLYLELGNKPAPNTFYVGIY ILIAVGAVMMFVGFLGCYGAIQESQCLLGTFFTCLVILFACEVAAGIWGFVNKDQIAKDVKQF YDQALQQAVMDDDANNAKAVVKTFHETLNCCGSNALTTLTTTILRNSLCPS | 61 |
| | ATGGGGGTGGAGGGCTGCACCAAATGCATCAAATACCTGCTCTTCGTCTTCAATTTCGTCTTC TGGCTGGCTGGAGGCGTGATCCTAGGTGTAGCTCTGTGGTTGCGTCATGATCCACAGACCACC AGCCTGCTGTACCTGGAACTGGGAAACAAACCGGCACCCAACACCTTCTACGTGGGCATCTAC ATTCTCATTGCTGTGGGAGCTGTGATGATGTTTGTAGGCTTCCTGGGGTGCTATGGGGCCATC CAGGAGTCCCAGTGTCTGCTGGGGACGTTCTTCACCTGCCTTGTGATCCTGTTTGCCTGTGAG GTGGCTGCAGGCATCTGGGGCTTCGTAAACAAAGACCAGATCGCCAAGGATGTGAAGCAGTTC TATGACCAGGCCCTTCAGCAAGCTGTGATGGATGATGATGCCAACAATGCCAAGGCTGTGGTG AAGACTTTCCATGAGACGCTCAACTGTTGTGGCTCCAACGCACTGACCACACTGACTACCACC ATACTGAGGAACAGCCTGTGTCCCTCA | 62 |
| CD81 (amino acids 178 to 236) | GGNILTPLLQQDCHQKIDELFSGKLYLIGIAAIVVAVIMIFEMILSMVLCCGIRNSSVY | 63 |
| | GGCGGCAACATACTCACCCCCTTACTGCAGCAAGATTGTCATCAGAAAATCGATGAGCTCTTC TCTGGGAAGCTGTACCTCATTGGAATTGCAGCCATTGTGGTAGCTGTCATTATGATCTTTGAG ATGATTCTGAGCATGGTGCTGTGCTGTGGCATCCGGAACAGCTCCGTGTACTGA | 64 |
| CD81-IL -12α | MGVEGCTKCIKYLLFVFNFVFWLAGGVILGVALWLRHDPQTTSLLYLELGNKPAPNTFYVGIY ILIAVGAVMMFVGFLGCYGAIQESQCLLGTFFTCLVILFACEVAAGIWGFVNKDQIAKDVKQF YDQALQQAVMDDDANNAKAVVKTFHETLNCCGSNALTTLTTTILRNSLCPSGGGGSRVIPVSG | 95 |

**[Table 8-2]**

| | | |
|---|---|---|
| | PARCLSQSRNLLKTTDDMVKTAREKLKHYSCTAEDIDHEDITRDQTSTLKTCLPLELHKNESC LATRETSSTTRGSCLPPQKTSLMMTLCLGSIYEDLKMYQTEFQAINAALQNHNHQQIILDKGM LVAIDELMQSLNHNGETLRQKPPVGEADPYRVKMKLCILLHAFSTRVVTINRVMGYLSSAGGG GSGGNILTPLLQQDCHQKIDELFSGKLYLIGIAAIVVAVIMIFEMILSMVLCCGIRNSSVY | |
| | ATGGGGGTGGAGGGCTGCACCAAATGCATCAAATACCTGCTCTTCGTCTTCAATTTCGTCTTC TGGCTGGCTGGAGGCGTGATCCTAGGTGTAGCTCTGTGGTTGCGTCATGATCCACAGACCACC AGCCTGCTGTACCTGGAACTGGGAAACAAACCGGCACCCAACACCTTCTACGTGGGCATCTAC ATTCTCATTGCTGTGGGAGCTGTGATGATGTTTGTAGGCTTCCTGGGGTGCTATGGGGCCATC CAGGAGTCCCAGTGTCTGCTGGGGACGTTCTTCACCTGCCTTGTGATCCTGTTTGCCTGTGAG GTGGCTGCAGGCATCTGGGGCTTCGTAAACAAAGACCAGATCGCCAAGGATGTGAAGCAGTTC TATGACCAGGCCCTTCAGCAAGCTGTGATGGATGATGATGCCAACAATGCCAAGGCTGTGGTG AAGACTTTCCATGAGACGCTCAACTGTTGTGGCTCCAACGCACTGACCACACTGACTACCACC ATACTGAGGAACAGCCTGTGTCCCTCAGGAGGAGGAGGAAGCAGGGTCATTCCAGTCTCTGGA CCTGCCAGGTGTCTTAGCCAGTCCCGAAACCTGCTGAAGACCACAGATGACATGGTGAAGACG GCCAGAGAAAAACTGAAACATTATTCCTGCACTGCTGAAGACATCGATCATGAAGACATCACA CGGGACCAAACCAGCACATTGAAGACCTGTTTACCACTGGAACTACACAAGAACGAGAGTTGC CTGGCTACTAGAGAGACTTCTTCCACAACAAGAGGGAGCTGCCTGCCCCCACAGAAGACGTCT TTGATGATGACCCTGTGCCTTGGTAGCATCTATGAGGACTTGAAGATGTACCAGACAGAGTTC CAGGCCATCAACGCAGCACTTCAGAATCACAACCATCAGCAGATCATTCTAGACAAGGGCATG CTGGTGGCCATCGATGAGCTGATGCAGTCTCTGAATCATAATGGCGAGACTCTGCGCCAGAAA CCTCCTGTGGGAGAAGCAGACCCTTACAGAGTGAAAATGAAGCTCTGCATCCTGCTTCACGCC TTCAGCACCCGCGTCGTGACCATCAACAGGGTGATGGGCTATCTGAGCTCCGCCGGAGGAGGA GGAAGCGGCGGCAACATACTCACCCCCTTACTGCAGCAAGATTGTCATCAGAAAATCGATGAG CTCTTCTCTGGGAAGCTGTACCTCATTGGAATTGCAGCCATTGTGGTAGCTGTCATTATGATC TTTGAGATGATTCTGAGCATGGTGCTGTGCTGTGGCATCCGGAACAGCTCCGTGTACTGA | 96 |
| | | |
| IL-12*β* | MCPQKLTISWFAIVLLVSPLMAMWELEKDVYVVEVDWTPDAPGETVNLTCDTPEEDDITWTSD QRHGVIGSGKTLTITVKEFLDAGQYTCHKGGETLSHSHLLLHKKENGIWSTEILKNFKNKTFL KCEAPNYSGRFTCSWLVQRNMDLKFNIKSSSSSPDSRAVTCGMASLSAEKVTLDQRDYEKYSV SCQEDVTCPTAEETLPIELALEARQQNKYENYSTSFFIRDIIKPDPPKNLQMKPLKNSQVEVS WEYPDSWSTPHSYFSLKFFVRIQRKKEKMKETEEGCNQKGAFLVEKTSTEVQCKGGNVCVQAQ DRYYNSSCSKWACVPCRVRS | 97 |
| | ATGTGTCCTCAGAAGCTAACCATCTCCTGGTTTGCCATCGTTTTGCTGGTGTCTCCACTCATG GCCATGTGGGAGCTGGAGAAAGACGTTTATGTTGTAGAGGTGGACTGGACTCCCGATGCCCCT GGAGAAACAGTGAACCTCACCTGTGACACGCCTGAAGAAGATGACATCACCTGGACCTCAGAC | 98 |

**[Table 8-3]**

| | | |
|---|---|---|
| | CAGAGACATGGAGTCATAGGCTCTGGAAAGACCCTGACCATCACTGTCAAAGAGTTTCTAGAT GCTGGCCAGTACACCTGCCACAAAGGAGGCGAGACTCTGAGCCACTCACATCTGCTGCTCCAC AAGAAGGAAAATGGAATTTGGTCCACTGAAATTTTAAAAAATTTCAAAAACAAGACTTTCCTG AAGTGTGAAGCACCAAATTACTCCGGACGGTTCACGTGCTCATGGCTGGTGCAAAGAAACATG GACTTGAAGTTCAACATCAAGAGCAGTAGCAGTTCCCCTGACTCTCGGGCAGTGACATGTGGA ATGGCGTCTCTGTCTGCAGAGAAGGTCACACTGGACCAAAGGGACTATGAGAAGTATTCAGTG TCCTGCCAGGAGGATGTCACCTGCCCAACTGCCGAGGAGACCCTGCCCATTGAACTGGCGTTG GAAGCACGGCAGCAGAATAAATATGAGAACTACAGCACCAGCTTCTTCATCAGGGACATCATC AAACCAGACCCGCCCAAGAACTTGCAGATGAAGCCTTTGAAGAACTCACAGGTGGAGGTCAGC TGGGAGTACCCTGACTCCTGGAGCACTCCCCATTCCTACTTCTCCCTCAAGTTCTTTGTTCGA ATCCAGCGCAAGAAAGAAAAGATGAAGGAGACAGAGGAGGGGTGTAACCAGAAAGGTGCGTTC CTCGTAGAGAAGACATCTACCGAAGTCCAATGCAAAGGCGGGAATGTCTGCGTGCAAGCTCAG GATCGCTATTACAATTCCTCATGCAGCAAGTGGGCATGTGTTCCCTGCAGGGTCCGATCCTAG | |

**[Table 9-1]**

| | Sequence | SEQ ID NO: |
|---|---|---|
| IL-6 (from which signal peptide is removed) | FPTSQVRRGDFTEDTTPNRPVYTTSQVGGLITHVLWEIVEMRKELCNGNSDCMNNDDALAENN LKLPEIQRNDGCYQTGYNQEICLLKISSGLLEYHSYLEYMKNNLKDNKKDKARVLQRDTETLI HIFNQEVKDLHKIVLPTPISNALLTDKLESQKEWLRTKTIQFILKSLEEFLKVTLRSTRQT | 99 |
| | TTCCCTACTTCACAAGTCCGGAGAGGAGACTTCACAGAGGATACCACTCCCAACAGACCTGTC TATACCACTTCACAAGTCGGAGGCTTAATTACACATGTTCTCTGGGAAATCGTGGAAATGAGA AAAGAGTTGTGCAATGGCAATTCTGATTGTATGAACAACGATGATGCACTTGCAGAAAACAAT CTGAAACTTCCAGAGATACAAAGAAATGATGGATGCTACCAAACTGGATATAATCAGGAAATT TGCCTATTGAAAATTTCCTCTGGTCTTCTGGAGTACCATAGCTACCTGGAGTACATGAAGAAC AACTTAAAAGATAACAAGAAAGACAAAGCCAGAGTCCTTCAGAGAGATACAGAAACTCTAATT CATATCTTCAACCAAGAGGTAAAAGATTTACATAAAATAGTCCTTCCTACCCCAATTTCCAAT GCTCTCCTAACAGATAAGCTGGAGTCACAGAAGGAGTGGCTAAGGACCAAGACCATCCAATTC ATCTTGAAATCACTTGAAGAATTTCTAAAAGTCACTTTGAGATCTACTCGGCAAACC | 100 |
| CD81 (amino acids 1 to 177) | MGVEGCTKCIKYLLFVFNFVFWLAGGVILGVALWLRHDPQTTSLLYLELGNKPAPNTFYVGIY ILIAVGAVMMFVGFLGCYGAIQESQCLLGTFFTCLVILFACEVAAGIWGFVNKDQIAKDVKQF YDQALQQAVMDDDANNAKAVVKTFHETLNCCGSNALTTLTTTILRNSLCPS | 61 |
| | ATGGGGGTGGAGGGCTGCACCAAATGCATCAAATACCTGCTCTTCGTCTTCAATTTCGTCTTC TGGCTGGCTGGAGGCGTGATCCTAGGTGTAGCTCTGTGGTTGCGTCATGATCCACAGACCACC AGCCTGCTGTACCTGGAACTGGGAAACAAACCGGCACCCAACACCTTCTACGTGGGCATCTAC ATTCTCATTGCTGTGGGAGCTGTGATGATGTTTGTAGGCTTCCTGGGGTGCTATGGGGCCATC CAGGAGTCCCAGTGTCTGCTGGGGACGTTCTTCACCTGCCTTGTGATCCTGTTTGCCTGTGAG GTGGCTGCAGGCATCTGGGGCTTCGTAAACAAAGACCAGATCGCCAAGGATGTGAAGCAGTTC TATGACCAGGCCCTTCAGCAAGCTGTGATGGATGATGATGCCAACAATGCCAAGGCTGTGGTG AAGACTTTCCATGAGACGCTCAACTGTTGTGGCTCCAACGCACTGACCACACTGACTACCACC ATACTGAGGAACAGCCTGTGTCCCTCA | 62 |
| CD81 (amino acids 178 to 236) | GGNILTPLLQQDCHQKIDELFSGKLYLIGIAA1VVAVIMIFEMILSMVLCCGIRNSSVY | 63 |
| | GGCGGCAACATACTCACCCCCTTACTGCAGCAAGATTGTCATCAGAAAATCGATGAGCTCTTC TCTGGGAAGCTGTACCTCATTGGAATTGCAGCCATTGTGGTAGCTGTCATTATGATCTTTGAG ATGATTCTGAGCATGGTGCTGTGCTGTGGCATCCGGAACAGCTCCGTGTACTGA | 64 |
| CD81-IL -6 | MGVEGCTKCIKYLLFVFNFVFWLAGGVILGVALWLRHDPQTTSLLYLELGNKPAPNTFYVGIY ILIAVGAVMMFVGFLGCYGAIQESQCLLGTFFTCLVILFACEVAAGIWGFVNKDQIAKDVKQF YDQALQQAVMDDDANNAKAVVKTFHETLNCCGSNALTTLTTTILRNSLCPSGGGGSFPTSQVR RGDFTEDTTPNRPVYTTSQVGGLITHVLWEIVEMRKELCNGNSDCMNNDDALAENNLKLPEIQ RNDGCYQTGYNQEICLLKISSGLLEYHSYLEYMKNNLKDNKKDKARVLQRDTETLIHIFNQEV | 101 |

**[Table 9-2]**

| | | |
|---|---|---|
| | KDLHKIVLPTPISNALLTDKLESQKEWLRTKTIQFILKSLEEFLKVTLRSTRQTGGGGSGGNI LTPLLQQDCHQKIDELFSGKLYLIGIAAIVVAVIMIFEMILSMVLCCGIRNSSVY | |
| | ATGGGGGTGGAGGGCTGCACCAAATGCATCAAATACCTGCTCTTCGTCTTCAATTTCGTCTTC TGGCTGGCTGGAGGCGTGATCCTAGGTGTAGCTCTGTGGTTGCGTCATGATCCACAGACCACC AGCCTGCTGTACCTGGAACTGGGAAACAAACCGGCACCCAACACCTTCTACGTGGGCATCTAC ATTCTCATTGCTGTGGGAGCTGTGATGATGTTTGTAGGCTTCCTGGGGTGCTATGGGGCCATC CAGGAGTCCCAGTGTCTGCTGGGGACGTTCTTCACCTGCCTTGTGATCCTGTTTGCCTGTGAG GTGGCTGCAGGCATCTGGGGCTTCGTAAACAAAGACCAGATCGCCAAGGATGTGAAGCAGTTC TATGACCAGGCCCTTCAGCAAGCTGTGATGGATGATGATGCCAACAATGCCAAGGCTGTGGTG AAGACTTTCCATGAGACGCTCAACTGTTGTGGCTCCAACGCACTGACCACACTGACTACCACC ATACTGAGGAACAGCCTGTGTCCCTCAGGAGGAGGAGGAAGCTTCCCTACTTCACAAGTCCGG AGAGGAGACTTCACAGAGGATACCACTCCCAACAGACCTGTCTATACCACTTCACAAGTCGGA GGCTTAATTACACATGTTCTCTGGGAAATCGTGGAAATGAGAAAAGAGTTGTGCAATGGCAAT TCTGATTGTATGAACAACGATGATGCACTTGCAGAAAACAATCTGAAACTTCCAGAGATACAA AGAAATGATGGATGCTACCAAACTGGATATAATCAGGAAATTTGCCTATTGAAAATTTCCTCT GGTCTTCTGGAGTACCATAGCTACCTGGAGTACATGAAGAACAACTTAAAAGATAACAAGAAA GACAAAGCCAGAGTCCTTCAGAGAGATACAGAAACTCTAATTCATATCTTCAACCAAGAGGTA AAAGATTTACATAAAATAGTCCTTCCTACCCCAATTTCCAATGCTCTCCTAACAGATAAGCTG GAGTCACAGAAGGAGTGGCTAAGGACCAAGACCATCCAATTCATCTTGAAATCACTTGAAGAA TTTCTAAAAGTCACTTTGAGATCTACTCGGCAAACCGGAGGAGGAGGAAGCGGCGGCAACATA CTCACCCCCTTACTGCAGCAAGATTGTCATCAGAAAATCGATGAGCTCTTCTCTGGGAAGCTG TACCTCATTGGAATTGCAGCCATTGTGGTAGCTGTCATTATGATCTTTGAGATGATTCTGAGC ATGGTGCTGTGCTGTGGCATCCGGAACAGCTCCGTGTACTGA | 102 |

**[Table 10-1]**

| | Sequence | SEQ ID NO: |
|---|---|---|
| hCD80 | MGHTRRQGTSPSKCPYLNFFQLLVLAGLSHFCSGVIHVTKEVKEVATLSCGHNVSVEELAQTR IYWQKEKKMVLTMMSGDMNIWPEYKNRTIFDITNNLSIVILALRPSDEGTYECVVLKYEKDAF KREHLAEVTLSVKADFPTPSISDFEIPTSNIRRIICSTSGGFPEPHLSWLENGEELNAINTTV SQDPETELYAVSSKLDFNMTTNHSFMCLIKYGHLRVNQTFNWNTTKQEHFPDNLLPSWAITLI SVNGIFVICCLTYCFAPRCRERRRNERLRRESVRPV | 103 |
| | ATGGGCCACACACGGAGGCAGGGAACATCACCATCCAAGTGTCCATACCTCAATTTCTTTCAG CTCTTGGTGCTGGCTGGTCTTTCTCACTTCTGTTCAGGTGTTATCCACGTGACCAAGGAAGTG AAAGAAGTGGCAACGCTGTCCTGTGGTCACAATGTTTCTGTTGAAGAGCTGGCACAAACTCGC ATCTACTGGCAAAAGGAGAAGAAAATGGTGCTGACTATGATGTCTGGGGACATGAATATATGG CCCGAGTACAAGAACCGGACCATCTTTGATATCACTAATAACCTCTCCATTGTGATCCTGGCT CTGCGCCCATCTGACGAGGGCACATACGAGTGTGTTGTTCTGAAGTATGAAAAAGACGCTTTC AAGCGGGAACACCTGGCTGAAGTGACGTTATCAGTCAAAGCTGACTTCCCTACACCTAGTATA TCTGACTTTGAAATTCCAACTTCTAATATTAGAAGGATAATTTGCTCAACCTCTGGAGGTTTT CCAGAGCCTCACCTCTCCTGGTTGGAAAATGGAGAAGAATTAAATGCCATCAACACAACAGTT TCCCAAGATCCTGAAACTGAGCTCTATGCTGTTAGCAGCAAACTGGATTTCAATATGACAACC AACCACAGCTTCATGTGTCTCATCAAGTATGGACATTTAAGAGTGAATCAGACCTTCAACTGG AATACAACCAAGCAAGAGCATTTTCCTGATAACCTGCTCCCATCCTGGGCCATTACCTTAATC TCAGTAAATGGAATTTTTGTGATATGCTGCCTGACCTACTGCTTTGCCCCAAGATGCAGAGAG AGAAGGAGGAATGAGAGATTGAGAAGGGAAAGTGTACGCCCTGTA | 104 |
| hCD9 | MPVKGGTKCIKYLLFGFNFIFWLAGIAVLAIGLWLRFDSQTKSIFEQETNNNNNSSFYTGVYIL IGAGALMMLVGFLGCCGAVQESQCMLGLFFGFLLVIFAIEIAAAIWGYSHKDEVIKEVQEFYK DTYNKLKTKDEPQRETLKAIHYALNCCGLAGGVEQFISDICPKKDVLETFTVKSCPDAIKEVF DNKFHIIGAVGIGIAVVMIFGMIFSMILCCAIRRNREMV | 105 |
| | ATGCCGGTCAAAGGAGGCACCAAGTGCATCAAATACCTGCTGTTCGGATTTAACTTCATCTTC TGGCTTGCCGGGATTGCTGTCCTTGCCATTGGACTATGGCTCCGATTCGACTCTCAGACCAAG AGCATCTTCGAGCAAGAAACTAATAATAATAATTCCAGCTTCTACACAGGAGTCTATATTCTG ATCGGAGCCGGCGCCCTCATGATGCTGGTGGGCTTCCTGGGCTGCTGCGGGGCTGTGCAGGAG TCCCAGTGCATGCTGGGACTGTTCTTCGGCTTCCTCTTGGTGATATTCGCCATTGAAATAGCT GCGGCCATCTGGGGATATTCCCACAAGGATGAGGTGATTAAGGAAGTCCAGGAGTTTTACAAG GACACCTACAACAAGCTGAAAACCAAGGATGAGCCCCAGCGGGAAACGCTGAAAGCCATCCAC TATGCGTTGAACTGCTGTGGTTTGGCTGGGGGCGTGGAACAGTTTATCTCAGACATCTGCCCC AAGAAGGACGTACTCGAAACCTTCACCGTGAAGTCCTGTCCTGATGCCATCAAAGAGGTCTTC GACAATAAATTCCACATCATCGGCGCAGTGGGCATCGGCATTGCCGTGGTCATGATATTTGGC | 106 |

**[Table 10-2]**

| | | |
|---|---|---|
| | ATGATCTTCAGTATGATCTTGTGCTGTGCTATCCGCAGGAACCGCGAGATGGTCTAG | |
| hCD80-h CD9 | MGHTRRQGTSPSKCPYLNFFQLLVLAGLSHFCSGVIHVTKEVKEVATLSCGHNVSVEELAQTR IYWQKEKKMVLTMMSGDMNIWPEYKNRTIFDITNNLSIVILALRPSDEGTYECVVLKYEKDAF KREHLAEVTLSVKADFPTPSISDFEIPTSNIRRIICSTSGGFPEPHLSWLENGEELNAINTTV SQDPETELYAVSSKLDFNMTTNHSFMCLIKYGHLRVNQTFNWNTTKQEHFPDNLLPSWAITLI SVNGIFVICCLTYCFAPRCRERRRNERLRRESVRPVMPVKGGTKCIKYLLFGFNFIFWLAGIA VLAIGLWLRFDSQTKSIFEQETNNNNSSFYTGVYILIGAGALMMLVGFLGCCGAVQESQCMLG LFFGFLLVIFAIEIAAAIWGYSHKDEVIKEVQEFYKDTYNKLKTKDEPQRETLKAIHYALNCC GLAGGVEQFISDICPKKDVLETFTVKSCPDAIKEVFDNKFHIIGAVGIGIAVVMIFGMIFSMI LCCAIRRNREMV | 107 |
| | ATGGGCCACACACGGAGGCAGGGAACATCACCATCCAAGTGTCCATACCTCAATTTCTTTCAG CTCTTGGTGCTGGCTGGTCTTTCTCACTTCTGTTCAGGTGTTATCCACGTGACCAAGGAAGTG AAAGAAGTGGCAACGCTGTCCTGTGGTCACAATGTTTCTGTTGAAGAGCTGGCACAAACTCGC ATCTACTGGCAAAAGGAGAAGAAAATGGTGCTGACTATGATGTCTGGGGACATGAATATATGG CCCGAGTACAAGAACCGGACCATCTTTGATATCACTAATAACCTCTCCATTGTGATCCTGGCT CTGCGCCCATCTGACGAGGGCACATACGAGTGTGTTGTTCTGAAGTATGAAAAAGACGCTTTC AAGCGGGAACACCTGGCTGAAGTGACGTTATCAGTCAAAGCTGACTTCCCTACACCTAGTATA TCTGACTTTGAAATTCCAACTTCTAATATTAGAAGGATAATTTGCTCAACCTCTGGAGGTTTT CCAGAGCCTCACCTCTCCTGGTTGGAAAATGGAGAAGAATTAAATGCCATCAACACAACAGTT TCCCAAGATCCTGAAACTGAGCTCTATGCTGTTAGCAGCAAACTGGATTTCAATATGACAACC AACCACAGCTTCATGTGTCTCATCAAGTATGGACATTTAAGAGTGAATCAGACCTTCAACTGG AATACAACCAAGCAAGAGCATTTTCCTGATAACCTGCTCCCATCCTGGGCCATTACCTTAATC TCAGTAAATGGAATTTTTGTGATATGCTGCCTGACCTACTGCTTTGCCCCAAGATGCAGAGAG AGAAGGAGGAATGAGAGATTGAGAAGGGAAAGTGTACGCCCTGTAATGCCGGTCAAAGGAGGC ACCAAGTGCATCAAATACCTGCTGTTCGGATTTAACTTCATCTTCTGGCTTGCCGGGATTGCT GTCCTTGCCATTGGACTATGGCTCCGATTCGACTCTCAGACCAAGAGCATCTTCGAGCAAGAA ACTAATAATAATAATTCCAGCTTCTACACAGGAGTCTATATTCTGATCGGAGCCGGCGCCCTC ATGATGCTGGTGGGCTTCCTGGGCTGCTGCGGGGCTGTGCAGGAGTCCCAGTGCATGCTGGGA CTGTTCTTCGGCTTCCTCTTGGTGATATTCGCCATTGAAATAGCTGCGGCCATCTGGGGATAT TCCCACAAGGATGAGGTGATTAAGGAAGTCCAGGAGTTTTACAAGGACACCTACAACAAGCTG AAAACCAAGGATGAGCCCCAGCGGGAAACGCTGAAAGCCATCCACTATGCGTTGAACTGCTGT GGTTTGGCTGGGGGCGTGGAACAGTTTATCTCAGACATCTGCCCCAAGAAGGACGTACTCGAA ACCTTCACCGTGAAGTCCTGTCCTGATGCCATCAAAGAGGTCTTCGACAATAAATTCCACATC ATCGGCGCAGTGGGCATCGGCATTGCCGTGGTCATGATATTTGGCATGATCTTCAGTATGATC TTGTGCTGTGCTATCCGCAGGAACCGCGAGATGGTCTAG | 108 |

**[Table 11-1]**

| | Sequence | SEQ ID NO: |
|---|---|---|
| hIL-2 (from which signal peptide is removed) | | 109 |
| | GCACCTACTTCAAGTTCTACAAAGAAAACACAGCTACAACTGGAGCATTTACTGCTGGATTTA CAGATGATTTTGAATGGAATTAATAATTACAAGAATCCCAAACTCACCAGGATGCTCACATTT AAGTTTTACATGCCCAAGAAGGCCACAGAACTGAAACATCTTCAGTGTCTAGAAGAAGAACTC AAACCTCTGGAGGAAGTGCTAAATTTAGCTCAAAGCAAAAACTTTCACTTAAGACCCAGGGAC TTAATCAGCAATATCAACGTAATAGTTCTGGAACTAAAGGGATCTGAAACAACATTCATGTGT GAATATGCTGATGAGACAGCAACCATTGTAGAATTTCTGAACAGATGGATTACCTTTTGTCAA AGCATCATCTCAACACTGACT | 110 |
| hCD63 (amino acids 1 to 170) | MAVEGGMKCVKFLLYVLLLAFCACAVGLIAVGVGAQLVLSQTIIQGATPGSLLPVVIIAVGVF LFLVAFVGCCGACKENYCLMITFAIFLSLIMLVEVAAAIAGYVFRDKVMSEFNNNFRQQMENY PKNNHTASILDRMQADFKCCGAANYTDWEKIPSMSKNRVPDSCC | 111 |
| | ATGGCGGTGGAAGGAGGAATGAAATGTGTGAAGTTCTTGCTCTACGTCCTCCTGCTGGCCTTT TGCGCCTGTGCAGTGGGACTGATTGCCGTGGGTGTCGGGGCACAGCTTGTCCTGAGTCAGACC ATAATCCAGGGGGCTACCCCTGGCTCTCTGTTGCCAGTGGTCATCATCGCAGTGGGTGTCTTC CTCTTCCTGGTGGCTTTTGTGGGCTGCTGCGGGGCCTGCAAGGAGAACTATTGTCTTATGATC ACGTTTGCCATCTTTCTGTCTCTTATCATGTTGGTGGAGGTGGCCGCAGCCATTGCTGGCTAT GTGTTTAGAGATAAGGTGATGTCAGAGTTTAATAACAACTTCCGGCAGCAGATGGAGAATTAC CCGAAAAACAACCACACTGCTTCGATCCTGGACAGGATGCAGGCAGATTTTAAGTGCTGTGGG GCTGCTAACTACACAGATTGGGAGAAAATCCCTTCCATGTCGAAGAACCGAGTCCCCGACTCC TGCTGC | 112 |
| h C D 6 3 (amino acids 171 to 238) | INVTVGCGINFNEKAIHKEGCVEKIGGWLRKNVLVVAAAALGIAFVEVLGIVFACCLVKSIRS GYEVM | 113 |
| | ATTAATGTTACTGTGGGCTGTGGGATTAATTTCAACGAGAAGGCGATCCATAAGGAGGGCTGT GTGGAGAAGATTGGGGGCTGGCTGAGGAAAAATGTGCTGGTGGTAGCTGCAGCAGCCCTTGGA ATTGCTTTTGTCGAGGTTTTGGGAATTGTCTTTGCCTGCTGCCTCGTGAAGAGTATCAGAAGT GGCTACGAGGTGATGTAG | 114 |
| bCD63-I L-2 | MAVEGGMKCVKFLLYVLLLAFCACAVGLIAVGVGAQLVLSQTIIQGATPGSLLPVVIIAVGVF LFLVAFVGCCGACKENYCLMITFAIFLSLIMLVEVAAAIAGYVFRDKVMSEFNNNFRQQMENY PKNNHTASILDRMQADFKCCGAANYTDWEKIPSMSKNRVPDSCCGGGGSAPTSSSTKKTQLQL EHLLLDLQMILNGINNYKNPKLTRMLTFKFYMPKKATELKHLQCLEEELKPLEEVLNLAQSKN FHLRPRDLISNINVIVLELKGSETTFMCEYADETATIVEFLNRWITFCQSIISTLTGGGGSIN | 115 |

**[Table 11-2]**

| | | |
|---|---|---|
| | VTVGCGINFNEKAIHKEGCVEKIGGWLRKNVLVVAAAALGIAFVEVLGIVFACCLVKSIRSGY EVM | |
| | ATGGCGGTGGAAGGAGGAATGAAATGTGTGAAGTTCTTGCTCTACGTCCTCCTGCTGGCCTTT TGCGCCTGTGCAGTGGGACTGATTGCCGTGGGTGTCGGGGCACAGCTTGTCCTGAGTCAGACC ATAATCCAGGGGGCTACCCCTGGCTCTCTGTTGCCAGTGGTCATCATCGCAGTGGGTGTCTTC CTCTTCCTGGTGGCTTTTGTGGGCTGCTGCGGGGCCTGCAAGGAGAACTATTGTCTTATGATC ACGTTTGCCATCTTTCTGTCTCTTATCATGTTGGTGGAGGTGGCCGCAGCCATTGCTGGCTAT GTGTTTAGAGATAAGGTGATGTCAGAGTTTAATAACAACTTCCGGCAGCAGATGGAGAATTAC CCGAAAAACAACCACACTGCTTCGATCCTGGACAGGATGCAGGCAGATTTTAAGTGCTGTGGG GCTGCTAACTACACAGATTGGGAGAAAATCCCTTCCATGTCGAAGAACCGAGTCCCCGACTCC TGCTGCGGAGGAGGAGGAAGCGCACCTACTTCAAGTTCTACAAAGAAAACACAGCTACAACTG GAGCATTTACTGCTGGATTTACAGATGATTTTGAATGGAATTAATAATTACAAGAATCCCAAA CTCACCAGGATGCTCACATTTAAGTTTTACATGCCCAAGAAGGCCACAGAACTGAAACATCTT CAGTGTCTAGAAGAAGAACTCAAACCTCTGGAGGAAGTGCTAAATTTAGCTCAAAGCAAAAAC TTTCACTTAAGACCCAGGGACTTAATCAGCAATATCAACGTAATAGTTCTGGAACTAAAGGGA TCTGAAACAACATTCATGTGTGAATATGCTGATGAGACAGCAACCATTGTAGAATTTCTGAAC AGATGGATTACCTTTTGTCAAAGCATCATCTCAACACTGACTGGAGGAGGAGGAAGCATTAAT GTTACTGTGGGCTGTGGGATTAATTTCAACGAGAAGGCGATCCATAAGGAGGGCTGTGTGGAG AAGATTGGGGGCTGGCTGAGGAAAAATGTGCTGGTGGTAGCTGCAGCAGCCCTTGGAATTGCT TTTGTCGAGGTTTTGGGAATTGTCTTTGCCTGCTGCCTCGTGAAGAGTATCAGAAGTGGCTAC GAGGTGATGTAG | 116 |

**[Table 12-1]**

| | Sequence | SEQ ID NO: |
|---|---|---|
| Signal peptide of h *β*₂ microglobulin | MSRSVALAVLALLSLSGLEA | 117 |
| | ATGTCTCGCTCCGTGGCCTTAGCTGTGCTCGCGCTACTCTCTCTTTCTGGCCTGGAGGCT | 118 |
| WT1 peptide 1 (for MHC class I molecule) | CYTWNQMNL | 119 |
| | TGCTACACCTGGAACCAGATGAACCTG | 120 |
| Peptide linker 1 | GGGASGGGGSGGGGS | 5 |
| | GGCGGAGGTGCCTCTGGCGGTGGGGGCAGCGGTGGAGGGGGCAGT | 6 |
| h *β*₂ Microglobulin (from which signal peptide is removed) | | 121 |
| | ATCCAGCGTACTCCAAAGATTCAGGTTTACTCACGTCATCCAGCAGAGAATGGAAAGTCAAAT TTCCTGAATTGCTATGTGTCTGGGTTTCATCCATCCGACATTGAAGTTGACTTACTGAAGAAT GGAGAGAGAATTGAAAAAGTGGAGCATTCAGACTTGTCTTTCAGCAAGGACTGGTCTTTCTAT CTCTTGTACTACACTGAATTCACCCCCACTGAAAAAGATGAGTATGCCTGCCGTGTGAACCAT GTGACTTTGTCACAGCCCAAGATAGTTAAGTGGGATCGAGACATG | 122 |
| hMHC class Iα chain (from which signal peptide is removed) | GSHSMRYFSTSVSRPGRGEPRFIAVGYVDDTQFVRFDSDAASQRMEPRAPWIEQEGPEYWDEE TGKVKAHSQTDRENLRIALRYYNQSEAGSHTLQMMFGCDVGSDGRFLRGYHQYAYDGKDYIAL KEDLRSWTAADMAAQITKRKWEAAHVAEQQRAYLEGTCVDGLRRYLENGKETLQRTDPPKTHM THHPISDHEATLRCWALGFYPAEITLTWQRDGEDQTQDTELVETRPAGDGTFQKWAAVVVPSG EEQRYTCHVQHEGLPKPLTLRWEPSSQPTVPIVGIIAGLVLLGAVITGAVVAAVMWRRNSSDR KGGSYSQAASSDSAQGSDVSLTACKV | 123 |
| | GGCTCCCACTCCATGAGGTATTTCTCCACATCCGTGTCCCGGCCCGGCCGCGGGGAGCCCCGC TTCATCGCCGTGGGCTACGTGGACGACACGCAGTTCGTGCGGTTCGACAGCGACGCCGCGAGC CAGAGGATGGAGCCGCGGGCGCCGTGGATAGAGCAGGAGGGGCCGGAGTATTGGGACGAGGAG ACAGGGAAAGTGAAGGCCCACTCACAGACTGACCGAGAGAACCTGCGGATCGCGCTCCGCTAC TACAACCAGAGCGAGGCCGGTTCTCACACCCTCCAGATGATGTTTGGCTGCGACGTGGGGTCG GACGGGCGCTTCCTCCGCGGGTACCACCAGTACGCCTACGACGGCAAGGATTACATCGCCCTG AAAGAGGACCTGCGCTCTTGGACCGCGGCGGACATGGCGGCTCAGATCACCAAGCGCAAGTGG GAGGCGGCCCATGTGGCGGAGCAGCAGAGAGCCTACCTGGAGGGCACGTGCGTGGACGGGCTC CGCAGATACCTGGAGAACGGGAAGGAGACGCTGCAGCGCACGGACCCCCCCAAGACACATATG ACCCACCACCCCATCTCTGACCATGAGGCCACTCTGAGATGCTGGGCCCTGGGCTTCTACCCT GCGGAGATCACACTGACCTGGCAGCGGGATGGGGAGGACCAGACCCAGGACACGGAGCTTGTG GAGACCAGGCCTGCAGGGGATGGAACCTTCCAGAAGTGGGCAGCTGTGGTGGTACCTTCTGGA GAGGAGCAGAGATACACCTGCCATGTGCAGCATGAGGGTCTGCCCAAGCCCCTCACCCTGAGA TGGGAGCCATCTTCCCAGCCCACCGTCCCCATCGTGGGCATCATTGCTGGCCTGGTTCTCCTT | 124 |

**[Table 12-2]**

| | | |
|---|---|---|
| | GGAGCTGTGATCACTGGAGCTGTGGTCGCTGCTGTGATGTGGAGGAGGAACAGCTCAGATAGA AAAGGAGGGAGCTACTCTCAGGCTGCAAGCAGTGACAGTGCCCAGGGCTCTGATGTGTCTCTC ACAGCTTGTAAAGTG | |
| Peptide linker 2 | GGGGSGGGGSGGGGSGGGGS | 11 |
| | GGGGGGGGAGGCTCCGGTGGAGGGGGGTCTGGAGGGGGGGGGTCTGGTGGAGGCGGAAGT | 12 |
| h Single chain MHC class I molecule (*β*₂ microglobulin (from which signal peptide is removed) + peptide linker 2 + MHC class Iα chain (from which signal peptide is removed)) | IQRTPKIQVYSRHPAENGKSNFLNCYVSGFHPSDIEVDLLKNGERIEKVEHSDLSFSKDWSFY LLYYTEFTPTEKDEYACRVNHVTLSQPKIVKWDRDMGGGGSGGGGSGGGGSGGGGSGSHSMRY FSTSVSRPGRGEPRFIAVGYVDDTQFVRFDSDAASQRMEPRAPWIEQEGPEYWDEETGKVKAH SQTDRENLRIALRYYNQSEAGSHTLQMMFGCDVGSDGRFLRGYHQYAYDGKDYIALKEDLRSW TAADMAAQITKRKWEAAHVAEQQRAYLEGTCVDGLRRYLENGKETLQRTDPPKTHMTHHPISD HEATLRCWALGFYPAEITLTWQRDGEDQTQDTELVETRPAGDGTFQKWAAVVVPSGEEQRYTC HVQHEGLPKPLTLRWEPSSQPTVPIVGIIAGLVLLGAVITGAVVAAVMWRRNSSDRKGGSYSQ AASSDSAQGSDVSLTACKV | 125 |
| | ATCCAGCGTACTCCAAAGATTCAGGTTTACTCACGTCATCCAGCAGAGAATGGAAAGTCAAAT TTCCTGAATTGCTATGTGTCTGGGTTTCATCCATCCGACATTGAAGTTGACTTACTGAAGAAT GGAGAGAGAATTGAAAAAGTGGAGCATTCAGACTTGTCTTTCAGCAAGGACTGGTCTTTCTAT CTCTTGTACTACACTGAATTCACCCCCACTGAAAAAGATGAGTATGCCTGCCGTGTGAACCAT GTGACTTTGTCACAGCCCAAGATAGTTAAGTGGGATCGAGACATGGGGGGGGGAGGCTCCGGT GGAGGGGGGTCTGGAGGGGGGGGGTCTGGTGGAGGCGGAAGTGGCTCCCACTCCATGAGGTAT TTCTCCACATCCGTGTCCCGGCCCGGCCGCGGGGAGCCCCGCTTCATCGCCGTGGGCTACGTG GACGACACGCAGTTCGTGCGGTTCGACAGCGACGCCGCGAGCCAGAGGATGGAGCCGCGGGCG CCGTGGATAGAGCAGGAGGGGCCGGAGTATTGGGACGAGGAGACAGGGAAAGTGAAGGCCCAC TCACAGACTGACCGAGAGAACCTGCGGATCGCCCTCCGCTACTACAACCAGAGCGAGGCCGGT TCTCACACCCTCCAGATGATGTTTGGCTGCGACGTGGGGTCGGACGGGCGCTTCCTCCGCGGG TACCACCAGTACGCCTACGACGGCAAGGATTACATCGCCCTGAAAGAGGACCTGCGCTCTTGG ACCGCGGCGGACATGGCGGCTCAGATCACCAAGCGCAAGTGGGAGGCGGCCCATGTGGCGGAG CAGCAGAGAGCCTACCTGGAGGGCACGTGCGTGGACGGGCTCCGCAGATACCTGGAGAACGGG AAGGAGACGCTGCAGCGCACGGACCCCCCCAAGACACATATGACCCACCACCCCATCTCTGAC CATGAGGCCACTCTGAGATGCTGGGCCCTGGGCTTCTACCCTGCGGAGATCACACTGACCTGG CAGCGGGATGGGGAGGACCAGACCCAGGACACGGAGCTTGTGGAGACCAGGCCTGCAGGGGAT GGAACCTTCCAGAAGTGGGCAGCTGTGGTGGTACCTTCTGGAGAGGAGCAGAGATACACCTGC CATGTGCAGCATGAGGGTCTGCCCAAGCCCCTCACCCTGAGATGGGAGCCATCTTCCCAGCCC ACCGTCCCCATCGTGGGCATCATTGCTGGCCTGGTTCTCCTTGGAGCTGTGATCACTGGAGCT GTGGTCGCTGCTGTGATGTGGAGGAGGAACAGCTCAGATAGAAAAGGAGGGAGCTACTCTCAG GCTGCAAGCAGTGACAGTGCCCAGGGCTCTGATGTGTCTCTCACAGCTTGTAAAGTG | 126 |

**[Table 12-3]**

| | | |
|---|---|---|
| | MSRSVALAVLALLSLSGLEACYTWNQMNLGGGASGGGGSGGGGSIQRTPKIQVYSRHPAENGK SNFLNCYVSGFHPSDIEVDLLKNGERIEKVEHSDLSFSKDWSFYLLYYTEFTPTEKDEYACRV NHVTLSQPKIVKWDRDMGGGGSGGGGSGGGGSGGGGSGSHSMRYFSTSVSRPGRGEPRFIAVG YVDDTQFVRFDSDAASQRMEPRAPWIEQEGPEYWDEETGKVKAHSQTDRENLRIALRYYNQSE AGSHTLQMMFGCDVGSDGRFLRGYHQYAYDGKDYIALKEDLRSWTAADMAAQITKRKWEAAHV AEQQRAYLEGTCVDGLRRYLENGKETLQRTDPPKTHMTHHPISDHEATLRCWALGFYPAEITL TWQRDGEDQTQDTELVETRPAGDGTFQKWAAVVVPSGEEQRYTCHVQHEGLPKPLTLRWEPSS QPTVPIVGIIAGLVLLGAVITGAVVAAVMWRRNSSDRKGGSYSQAASSDSAQGSDVSLTACKV | 127 |
| hsc-Tri mer (WT1 peptide 1 + peptide linker 1 + single chain MHC class I molecule) | ATGTCTCGCTCCGTGGCCTTAGCTGTGCTCGCGCTACTCTCTCTTTCTGGCCTGGAGGCTTGC TACACCTGGAACCAGATGAACCTGGGCGGAGGTGCCTCTGGCGGTGGGGGCAGCGGTGGAGGG GGCAGTATCCAGCGTACTCCAAAGATTCAGGTTTACTCACGTCATCCAGCAGAGAATGGAAAG TCAAATTTCCTGAATTGCTATGTGTCTGGGTTTCATCCATCCGACATTGAAGTTGACTTACTG AAGAATGGAGAGAGAATTGAAAAAGTGGAGCATTCAGACTTGTCTTTCAGCAAGGACTGGTCT TTCTATCTCTTGTACTACACTGAATTCACCCCCACTGAAAAAGATGAGTATGCCTGCCGTGTG AACCATGTGACTTTGTCACAGCCCAAGATAGTTAAGTGGGATCGAGACATGGGGGGGGGAGGC TCCGGTGGAGGGGGGTCTGGAGGGGGGGGGTCTGGTGGAGGCGGAAGTGGCTCCCACTCCATG AGGTATTTCTCCACATCCGTGTCCCGGCCCGGCCGCGGGGAGCCCCGCTTCATCGCCGTGGGC TACGTGGACGACACGCAGTTCGTGCGGTTCGACAGCGACGCCGCGAGCCAGAGGATGGAGCCG CGGGCGCCGTGGATAGAGCAGGAGGGGCCGGAGTATTGGGACGAGGAGACAGGGAAAGTGAAG GCCCACTCACAGACTGACCGAGAGAACCTGCGGATCGCGCTCCGCTACTACAACCAGAGCGAG GCCGGTTCTCACACCCTCCAGATGATGTTTGGCTGCGACGTGGGGTCGGACGGGCGCTTCCTC CGCGGGTACCACCAGTACGCCTACGACGGCAAGGATTACATCGCCCTGAAAGAGGACCTGCGC TCTTGGACCGCGGCGGACATGGCGGCTCAGATCACCAAGCGCAAGTGGGAGGCGGCCCATGTG GCGGAGCAGCAGAGAGCCTACCTGGAGGGCACGTGCGTGGACGGGCTCCGCAGATACCTGGAG AACGGGAAGGAGACGCTGCAGCGCACGGACCCCCCCAAGACACATATGACCCACCACCCCATC TCTGACCATGAGGCCACTCTGAGATGCTGGGCCCTGGGCTTCTACCCTGCGGAGATCACACTG ACCTGGCAGCGGGATGGGGAGGACCAGACCCAGGACACGGAGCTTGTGGAGACCAGGCCTGCA GGGGATGGAACCTTCCAGAAGTGGGCAGCTGTGGTGGTACCTTCTGGAGAGGAGCAGAGATAC ACCTGCCATGTGCAGCATGAGGGTCTGCCCAAGCCCCTCACCCTGAGATGGGAGCCATCTTCC CAGCCCACCGTCCCCATCGTGGGCATCATTGCTGGCCTGGTTCTCCTTGGAGCTGTGATCACT GGAGCTGTGGTCGCTGCTGTGATGTGGAGGAGGAACAGCTCAGATAGAAAAGGAGGGAGCTAC TCTCAGGCTGCAAGCAGTGACAGTGCCCAGGGCTCTGATGTGTCTCTCACAGCTTGTAAAGTG | 128 |
| h CD 8 1 | MGVEGCTKCIKYLLFVFNFVFWLAGGVILGVALWLRHDPQTTNLLYLELGDKPAPNTFYVGIY ILIAVGAVMMFVGFLGCYGAIQESQCLLGTFFTCLVILFACEVAAGIWGFVNKDQIAKDVKQF YDQALQQAVVDDDANNAKAVVKTFHETLDCCGSSTLTALTTSVLKNNLCPSGSNIISNLFKED | 129 |

**[Table 12-4]**

| | | |
|---|---|---|
| | CHQKIDDLFSGKLYLIGIAAIVVAVIMIFEMILSMVLCCGIRNSSVY | |
| | ATGGGAGTGGAGGGCTGCACCAAGTGCATCAAGTACCTGCTCTTCGTCTTCAATTTCGTCTTC TGGCTGGCTGGAGGCGTGATCCTGGGTGTGGCCCTGTGGCTCCGCCATGACCCGCAGACCACC AACCTCCTGTATCTGGAGCTGGGAGACAAGCCCGCGCCCAACACCTTCTATGTAGGCATCTAC ATCCTCATCGCTGTGGGCGCTGTCATGATGTTCGTTGGCTTCCTGGGCTGCTACGGGGCCATC CAGGAATCCCAGTGCCTGCTGGGGACGTTCTTCACCTGCCTGGTCATCCTGTTTGCCTGTGAG GTGGCCGCCGGCATCTGGGGCTTTGTCAACAAGGACCAGATCGCCAAGGATGTGAAGCAGTTC TATGACCAGGCCCTACAGCAGGCCGTGGTGGATGATGACGCCAACAACGCCAAGGCTGTGGTG AAGACCTTCCACGAGACGCTTGACTGCTGTGGCTCCAGCACACTGACTGCTTTGACCACCTCA GTGCTCAAGAACAATTTGTGTCCCTCGGGCAGCAACATCATCAGCAACCTCTTCAAGGAGGAC TGCCACCAGAAGATCGATGACCTCTTCTCCGGGAAGCTGTACCTCATCGGCATTGCTGCCATC GTGGTCGCTGTGATCATGATCTTCGAGATGATCCTGAGCATGGTGCTGTGCTGTGGCATCCGG AACAGCTCCGTGTACTGA | 130 |
| hsc-Tri mer-CD8 1 (sc-Tri mer+CD8 1) | MSRSVALAVLALLSLSGLEACYTWNQMNLGGGASGGGGSGGGGSIQRTPKIQVYSRHPAENGK SNFLNCYVSGFHPSDIEVDLLKNGERIEKVEHSDLSFSKDWSFYLLYYTEFTPTEKDEYACRV NHVTLSQPKIVKWDRDMGGGGSGGGGSGGGGSGGGGSGSHSMRYFSTSVSRPGRGEPRFIAVG YVDDTQFVRFDSDAASQRMEPRAPWIEQEGPEYWDEETGKVKAHSQTDRENLRIALRYYNQSE AGSHTLQMMFGCDVGSDGRFLRGYHQYAYDGKDYIALKEDLRSWTAADMAAQITKRKWEAAHV AEQQRAYLEGTCVDGLRRYLENGKETLQRTDPPKTHMTHHPISDHEATLRCWALGFYPAEITL TWQRDGEDQTQDTELVETRPAGDGTFQKWAAVVVPSGEEQRYTCHVQHEGLPKPLTLRWEPSS QPTVPIVGIIAGLVLLGAVITGAVVAAVMWRRNSSDRKGGSYSQAASSDSAQGSDVSLTACKV MGVEGCTKCIKYLLFVFNFVFWLAGGVILGVALWLRHDPQTTNLLYLELGDKPAPNTFYVGIY ILIAVGAVMMFVGFLGCYGAIQESQCLLGTFFTCLVILFACEVAAGIWGFVNKDQIAKDVKQF YDQALQQAVVDDDANNAKAVVKTFHETLDCCGSSTLTALTTSVLKNNLCPSGSNIISNLFKED CHQKIDDLFSGKLYLIGIAAIVVAVIMIFEMILSMVLCCGIRNSSVY | 131 |
| | ATGTCTCGCTCCGTGGCCTTAGCTGTGCTCGCGCTACTCTCTCTTTCTGGCCTGGAGGCTTGC TACACCTGGAACCAGATGAACCTGGGCGGAGGTGCCTCTGGCGGTGGGGGCAGCGGTGGAGGG GGCAGTATCCAGCGTACTCCAAAGATTCAGGTTTACTCACGTCATCCAGCAGAGAATGGAAAG TCAAATTTCCTGAATTGCTATGTGTCTGGGTTTCATCCATCCGACATTGAAGTTGACTTACTG AAGAATGGAGAGAGAATTGAAAAAGTGGAGCATTCAGACTTGTCTTTCAGCAAGGACTGGTCT TTCTATCTCTTGTACTACACTGAATTCACCCCCACTGAAAAAGATGAGTATGCCTGCCGTGTG AACCATGTGACTTTGTCACAGCCCAAGATAGTTAAGTGGGATCGAGACATGGGGGGGGGAGGC TCCGGTGGAGGGGGGTCTGGAGGGGGGGGGTCTGGTGGAGGCGGAAGTGGCTCCCACTCCATG AGGTATTTCTCCACATCCGTGTCCCGGCCCGGCCGCGGGGAGCCCCGCTTCATCGCCGTGGGC TACGTGGACGACACGCAGTTCGTGCGGTTCGACAGCGACGCCGCGAGCCAGAGGATGGAGCCG | 132 |

**[Table 12-5]**

| | | |
|---|---|---|
| | CGGGCGCCGTGGATAGAGCAGGAGGGGCCGGAGTATTGGGACGAGGAGACAGGGAAAGTGAAG GCCCACTCACAGACTGACCGAGAGAACCTGCGGATCGCGCTCCGCTACTACAACCAGAGCGAG GCCGGTTCTCACACCCTCCAGATGATGTTTGGCTGCGACGTGGGGTCGGACGGGCGCTTCCTC CGCGGGTACCACCAGTACGCCTACGACGGCAAGGATTACATCGCCCTGAAAGAGGACCTGCGC TCTTGGACCGCGGCGGACATGGCGGCTCAGATCACCAAGCGCAAGTGGGAGGCGGCCCATGTG GCGGAGCAGCAGAGAGCCTACCTGGAGGGCACGTGCGTGGACGGGCTCCGCAGATACCTGGAG AACGGGAAGGAGACGCTGCAGCGCACGGACCCCCCCAAGACACATATGACCCACCACCCCATC TCTGACCATGAGGCCACTCTGAGATGCTGGGCCCTGGGCTTCTACCCTGCGGAGATCACACTG ACCTGGCAGCGGGATGGGGAGGACCAGACCCAGGACACGGAGCTTGTGGAGACCAGGCCTGCA GGGGATGGAACCTTCCAGAAGTGGGCAGCTGTGGTGGTACCTTCTGGAGAGGAGCAGAGATAC ACCTGCCATGTGCAGCATGAGGGTCTGCCCAAGCCCCTCACCCTGAGATGGGAGCCATCTTCC CAGCCCACCGTCCCCATCGTGGGCATCATTGCTGGCCTGGTTCTCCTTGGAGCTGTGATCACT GGAGCTGTGGTCGCTGCTGTGATGTGGAGGAGGAACAGCTCAGATAGAAAAGGAGGGAGCTAC TCTCAGGCTGCAAGCAGTGACAGTGCCCAGGGCTCTGATGTGTCTCTCACAGCTTGTAAAGTG ATGGGAGTGGAGGGCTGCACCAAGTGCATCAAGTACCTGCTCTTCGTCTTCAATTTCGTCTTC TGGCTGGCTGGAGGCGTGATCCTGGGTGTGGCCCTGTGGCTCCGCCATGACCCGCAGACCACC AACCTCCTGTATCTGGAGCTGGGAGACAAGCCCGCGCCCAACACCTTCTATGTAGGCATCTAC ATCCTCATCGCTGTGGGCGCTGTCATGATGTTCGTTGGCTTCCTGGGCTGCTACGGGGCCATC CAGGAATCCCAGTGCCTGCTGGGGACGTTCTTCACCTGCCTGGTCATCCTGTTTGCCTGTGAG GTGGCCGCCGGCATCTGGGGCTTTGTCAACAAGGACCAGATCGCCAAGGATGTGAAGCAGTTC TATGACCAGGCCCTACAGCAGGCCGTGGTGGATGATGACGCCAACAACGCCAAGGCTGTGGTG AAGACCTTCCACGAGACGCTTGACTGCTGTGGCTCCAGCACACTGACTGCTTTGACCACCTCA GTGCTCAAGAACAATTTGTGTCCCTCGGGCAGCAACATCATCAGCAACCTCTTCAAGGAGGAC TGCCACCAGAAGATCGATGACCTCTTCTCCGGGAAGCTGTACCTCATCGGCATTGCTGCCATC GTGGTCGCTGTGATCATGATCTTCGAGATGATCCTGAGCATGGTGCTGTGCTGTGGCATCCGG AACAGCTCCGTGTACTGA | |

**[Table 13-1]**

| | Sequence | SEQ ID NO: |
|---|---|---|
| Signal peptide of *β*₂ microglobulin | MARSVTLVFLVLVSLTGLYA | 1 |
| | ATGGCTCGCTCGGTGACCCTGGTCTTTCTGGTGCTTGTCTCACTGACCGGCCTGTATGCT | 2 |
| OVA peptide 1 (for MHC class I molecule) | SIINFEKL | 3 |
| | TCCATTATAAATTTTGAAAAGTTG | 4 |
| Peptide linker 1 | GGGASGGGGSGGGGS | 5 |
| | GGCGGAGGTGCCTCTGGCGGTGGGGGCAGCGGTGGAGGGGGCAGT | 6 |
| *β*₂ Microglobulin (from which signal peptide is removed) | | 7 |
| | ATCCAGAAAACCCCTCAAATTCAAGTATACTCACGCCACCCACCGGAGAATGGGAAGCCGAAC ATACTGAACTGCTACGTAACACAGTTCCACCCGCCTCACATTGAAATCCAAATGCTGAAGAAC GGGAAAAAAATTCCTAAAGTAGAGATGTCAGATATGTCCTTCAGCAAGGACTGGTCTTTCTAT ATCCTGGCTCACACTGAATTCACCCCCACTGAGACTGATACATACGCCTGCAGAGTTAAGCAT GCCAGTATGGCCGAGCCCAAGACCGTCTACTGGGATCGAGACATG | 8 |
| MHC class Iα chain (from which signal peptide is removed) | GPHSLRYFVTAVSRPGLGEPRYMEVGYVDDTEFVRFDSDAENPRYEPRARWMEQEGPEYWERE TQKAKGNEQSFRVDLRTLLGYYNQSKGGSHTIQVISGCEVGSDGRLLRGYQQYAYDGCDYIAL NEDLKTWTAADMAALITKHKWEQAGEAERLRAYLEGTCVEWLRRYLKNGNATLLRTDSPKAHV THHSRPEDKVTLRCWALGFYPADITLTWQLNGEELIQDMELVETRPAGDGTFQKWASVVVPLG KEQYYTCHVYHQGLPEPLTLRWEPPPSTVSNMATVAVLVVLGAAIVTGAVVAFVMKMRRRNTG GKGGDYALAPGSQTSDLSLPDCKVMVHDPHSLA | 9 |
| | GGCCCACACTCGCTGAGGTATTTCGTCACCGCCGTGTCCCGGCCCGGCCTCGGGGAGCCCCGG TACATGGAAGTCGGCTACGTGGACGACACGGAGTTCGTGCGCTTCGACAGCGACGCGGAGAAT CCGAGATATGAGCCGCGGGCGCGGTGGATGGAGCAGGAGGGGCCCGAGTATTGGGAGCGGGAG ACACAGAAAGCCAAGGGCAATGAGCAGAGTTTCCGAGTGGACCTGAGGACCCTGCTCGGCTAC TACAACCAGAGCAAGGGCGGCTCTCACACTATTCAGGTGATCTCTGGCTGTGAAGTGGGGTCC GACGGGCGACTCCTCCGCGGGTACCAGCAGTACGCCTACGACGGCTGCGATTACATCGCCCTG AACGAAGACCTGAAAACGTGGACGGCGGCGGACATGGCGGCGCTGATCACCAAACACAAGTGG GAGCAGGCTGGTGAAGCAGAGAGACTCAGGGCCTACCTGGAGGGCACGTGCGTGGAGTGGCTC CGCAGATACCTGAAGAACGGGAACGCGACGCTGCTGCGCACAGATTCCCCAAAGGCCCATGTG ACCCATCACAGCAGACCTGAAGATAAAGTCACCCTGAGGTGCTGGGCCCTGGGCTTCTACCCT GCTGACATCACCCTGACCTGGCAGTTGAATGGGGAGGAGCTGATCCAGGACATGGAGCTTGTG GAGACCAGGCCTGCAGGGGATGGAACCTTCCAGAAGTGGGCATCTGTGGTGGTGCCTCTTGGG AAGGAGCAGTATTACACATGCCATGTGTACCATCAGGGGCTGCCTGAGCCCCTCACCCTGAGA | 10 |

**[Table 13-2]**

| | | |
|---|---|---|
| | TGGGAGCCTCCTCCATCCACTGTCTCCAACATGGCGACCGTTGCTGTTCTGGTTGTCCTTGGA GCTGCAATAGTCACTGGAGCTGTGGTGGCTTTTGTGATGAAGATGAGAAGGAGAAACACAGGT GGAAAAGGAGGGGACTATGCTCTGGCTCCAGGCTCCCAGACCTCTGATCTGTCTCTCCCAGAT TGTAAAGTGATGGTTCATGACCCTCATTCTCTAGCG | |
| Peptide linker 2 | GGGGSGGGGSGGGGSGGGGS | 11 |
| | GGGGGGGGAGGCTCCGGTGGAGGGGGGTCTGGAGGGGGGGGGTCTGGTGGAGGCGGAAGT | 12 |
| Single chain MHC class I molecule (β₂ microglobulin (from which signal peptide is removed) + peptide linker 2 + MHC class Iα chain (from which signal peptide is removed)) | IQKTPQIQVYSRHPPENGKPNILNCYVTQFHPPHIEIQMLKNGKKIPKVEMSDMSFSKDWSFY ILAHTEFTPTETDTYACRVKHASMAEPKTVYWDRDMGGGGSGGGGSGGGGSGGGGSGPHSLRY FVTAVSRPGLGEPRYMEVGYVDDTEFVRFDSDAENPRYEPRARWMEQEGPEYWERETQKAKGN EQSFRVDLRTLLGYYNQSKGGSHTIQVISGCEVGSDGRLLRGYQQYAYDGCDYIALNEDLKTW TAADMAALITKHKWEQAGEAERLRAYLEGTCVEWLRRYLKNGNATLLRTDSPKAHVTHHSRPE DKVTLRCWALGFYPADITLTWQLNGEELIQDMELVETRPAGDGTFQKWASVVVPLGKEQYYTC HVYHQGLPEPLTLRWEPPPSTVSNMATVAVLVVLGAAIVTGAVVAFVMKMRRRNTGGKGGDYA LAPGSQTSDLSLPDCKVMVHDPHSLA | 65 |
| | ATCCAGAAAACCCCTCAAATTCAAGTATACTCACGCCACCCACCGGAGAATGGGAAGCCGAAC ATACTGAACTGCTACGTAACACAGTTCCACCCGCCTCACATTGAAATCCAAATGCTGAAGAAC GGGAAAAAAATTCCTAAAGTAGAGATGTCAGATATGTCCTTCAGCAAGGACTGGTCTTTCTAT ATCCTGGCTCACACTGAATTCACCCCCACTGAGACTGATACATACGCCTGCAGAGTTAAGCAT GCCAGTATGGCCGAGCCCAAGACCGTCTACTGGGATCGAGACATGGGGGGGGGAGGCTCCGGT GGAGGGGGGTCTGGAGGGGGGGGGTCTGGTGGAGGCGGAAGTGGCCCACACTCGCTGAGGTAT TTCGTCACCGCCGTGTCCCGGCCCGGCCTCGGGGAGCCCCGGTACATGGAAGTCGGCTACGTG GACGACACGGAGTTCGTGCGCTTCGACAGCGACGCGGAGAATCCGAGATATGAGCCGCGGGCG CGGTGGATGGAGCAGGAGGGGCCCGAGTATTGGGAGCGGGAGACACAGAAAGCCAAGGGCAAT GAGCAGAGTTTCCGAGTGGACCTGAGGACCCTGCTCGGCTACTACAACCAGAGCAAGGGCGGC TCTCACACTATTCAGGTGATCTCTGGCTGTGAAGTGGGGTCCGACGGGCGACTCCTCCGCGGG TACCAGCAGTACGCCTACGACGGCTGCGATTACATCGCCCTGAACGAAGACCTGAAAACGTGG ACGGCGGCGGACATGGCGGCGCTGATCACCAAACACAAGTGGGAGCAGGCTGGTGAAGCAGAG AGACTCAGGGCCTACCTGGAGGGCACGTGCGTGGAGTGGCTCCGCAGATACCTGAAGAACGGG AACGCGACGCTGCTGCGCACAGATTCCCCAAAGGCCCATGTGACCCATCACAGCAGACCTGAA GATAAAGTCACCCTGAGGTGCTGGGCCCTGGGCTTCTACCCTGCTGACATCACCCTGACCTGG CAGTTGAATGGGGAGGAGCTGATCCAGGACATGGAGCTTGTGGAGACCAGGCCTGCAGGGGAT GGAACCTTCCAGAAGTGGGCATCTGTGGTGGTGCCTCTTGGGAAGGAGCAGTATTACACATGC CATGTGTACCATCAGGGGCTGCCTGAGCCCCTCACCCTGAGATGGGAGCCTCCTCCATCCACT GTCTCCAACATGGCGACCGTTGCTGTTCTGGTTGTCCTTGGAGCTGCAATAGTCACTGGAGCT GTGGTGGCTTTTGTGATGAAGATGAGAAGGAGAAACACAGGTGGAAAAGGAGGGGACTATGCT | 66 |

**[Table 13-3]**

| | | |
|---|---|---|
| | CTGGCTCCAGGCTCCCAGACCTCTGATCTGTCTCTCCCAGATTGTAAAGTGATGGTTCATGAC CCTCATTCTCTAGCG | |
| sc-Trim cr (OVA peptide 1 + peptide linker 1 + single chain MHC class I molecule) | MARSVTLVFLVLVSLTGLYASIINFEKLGGGASGGGGSGGGGSIQKTPQIQVYSRHPPENGKP NILNCYVTQFHPPHIEIQMLKNGKKIPKVEMSDMSFSKDWSFYILAHTEFTPTETDTYACRVK HASMAEPKTVYWDRDMGGGGSGGGGSGGGGSGGGGSGPHSLRYFVTAVSRPGLGEPRYMEVGY VDDTEFVRFDSDAENPRYEPRARWMEQEGPEYWERETQKAKGNEQSFRVDLRTLLGYYNQSKG GSHTIQVISGCEVGSDGRLLRGYQQYAYDGCDYIALNEDLKTWTAADMAALITKHKWEQAGEA ERLRAYLEGTCVEWLRRYLKNGNATLLRTDSPKAHVTHHSRPEDKVTLRCWALGFYPADITLT WQLNGEELIQDMELVETRPAGDGTFQKWASVVVPLGKEQYYTCHVYHQGLPEPLTLRWEPPPS TVSNMATVAVLVVLGAAIVTGAVVAFVMKMRRRNTGGKGGDYALAPGSQTSDLSLPDCKVMVH DPHSLA | 13 |
| | ATGGCTCGCTCGGTGACCCTGGTCTTTCTGGTGCTTGTCTCACTGACCGGCCTGTATGCTTCC ATTATAAATTTTGAAAAGTTGGGCGGAGGTGCCTCTGGCGGTGGGGGCAGCGGTGGAGGGGGC AGTATCCAGAAAACCCCTCAAATTCAAGTATACTCACGCCACCCACCGGAGAATGGGAAGCCG AACATACTGAACTGCTACGTAACACAGTTCCACCCGCCTCACATTGAAATCCAAATGCTGAAG AACGGGAAAAAAATTCCTAAAGTAGAGATGTCAGATATGTCCTTCAGCAAGGACTGGTCTTTC TATATCCTGGCTCACACTGAATTCACCCCCACTGAGACTGATACATACGCCTGCAGAGTTAAG CATGCCAGTATGGCCGAGCCCAAGACCGTCTACTGGGATCGAGACATGGGGGGGGGAGGCTCC GGTGGAGGGGGGTCTGGAGGGGGGGGGTCTGGTGGAGGCGGAAGTGGCCCACACTCGCTGAGG TATTTCGTCACCGCCGTGTCCCGGCCCGGCCTCGGGGAGCCCCGGTACATGGAAGTCGGCTAC GTGGACGACACGGAGTTCGTGCGCTTCGACAGCGACGCGGAGAATCCGAGATATGAGCCGCGG GCGCGGTGGATGGAGCAGGAGGGGCCCGAGTATTGGGAGCGGGAGACACAGAAAGCCAAGGGC AATGAGCAGAGTTTCCGAGTGGACCTGAGGACCCTGCTCGGCTACTACAACCAGAGCAAGGGC GGCTCTCACACTATTCAGGTGATCTCTGGCTGTGAAGTGGGGTCCGACGGGCGACTCCTCCGC GGGTACCAGCAGTACGCCTACGACGGCTGCGATTACATCGCCCTGAACGAAGACCTGAAAACG TGGACGGCGGCGGACATGGCGGCGCTGATCACCAAACACAAGTGGGAGCAGGCTGGTGAAGCA GAGAGACTCAGGGCCTACCTGGAGGGCACGTGCGTGGAGTGGCTCCGCAGATACCTGAAGAAC GGGAACGCGACGCTGCTGCGCACAGATTCCCCAAAGGCCCATGTGACCCATCACAGCAGACCT GAAGATAAAGTCACCCTGAGGTGCTGGGCCCTGGGCTTCTACCCTGCTGACATCACCCTGACC TGGCAGTTGAATGGGGAGGAGCTGATCCAGGACATGGAGCTTGTGGAGACCAGGCCTGCAGGG GATGGAACCTTCCAGAAGTGGGCATCTGTGGTGGTGCCTCTTGGGAAGGAGCAGTATTACACA TGCCATGTGTACCATCAGGGGCTGCCTGAGCCCCTCACCCTGAGATGGGAGCCTCCTCCATCC ACTGTCTCCAACATGGCGACCGTTGCTGTTCTGGTTGTCCTTGGAGCTGCAATAGTCACTGGA GCTGTGGTGGCTTTTGTGATGAAGATGAGAAGGAGAAACACAGGTGGAAAAGGAGGGGACTAT GCTCTGGCTCCAGGCTCCCAGACCTCTGATCTGTCTCTCCCAGATTGTAAAGTGATGGTTCAT | 14 |

**[Table 13-4]**

| | | |
|---|---|---|
| | GACCCTCATTCTCTAGCG | |
| IL-2 (from which signal peptide is removed) | APTSSSTSSSTAEAQQQQQQQQQQQHLEQLLMDLQELLSRMENYRNLKLPRMLTFKFYLPKQ ATELKDLQCLEDELGPLRHVLDLTQSKSFQLEDAENFISNIRVTVVKLKGSDNTFECQFDDES ATVVDFLRRWIAFCQSIISTSPQ | 25 |
| | GCACCCACTTCAAGCTCCACTTCAAGCTCTACAGCGGAAGCACAGCAGCAGCAGCAGCAGCAG CAGCAGCAGCAGCAGCACCTGGAGCAGCTGTTGATGGACCTACAGGAGCTCCTGAGCAGGATG GAGAATTACAGGAACCTGAAACTCCCCAGGATGCTCACCTTCAAATTTTACTTGCCCAAGCAG GCCACAGAATTGAAAGATCTTCAGTGCCTAGAAGATGAACTTGGACCTCTGCGGCATGTTCTG GATTTGACTCAAAGCAAAAGCTTTCAATTGGAAGATGCTGAGAATTTCATCAGCAATATCAGA GTAACTGTTGTAAAACTAAAGGGCTCTGACAACACATTTGAGTGCCAATTCGATGATGAGTCA GCAACTGTGGTGGACTTTCTGAGGAGATGGATAGCCTTCTGTCAAAGCATCATCTCAACAAGC CCTCAA | 26 |
| CD81 (amino acids 1 to 177) | MGVEGCTKCIKYLLFVFNFVFWLAGGVILGVALWLRHDPQTTSLLYLELGNKPAPNTFYVGIY ILIAVGAVMMFVGFLGCYGAIQESQCLLGTFFTCLVILFACEVAAGIWGFVNKDQIAKDVKQF YDQALQQAVMDDDANNAKAVVKTFHETLNCCGSNALTTLTTTILRNSLCPS | 61 |
| | ATGGGGGTGGAGGGCTGCACCAAATGCATCAAATACCTGCTCTTCGTCTTCAATTTCGTCTTC TGGCTGGCTGGAGGCGTGATCCTAGGTGTAGCTCTGTGGTTGCGTCATGATCCACAGACCACC AGCCTGCTGTACCTGGAACTGGGAAACAAACCGGCACCCAACACCTTCTACGTGGGCATCTAC ATTCTCATTGCTGTGGGAGCTGTGATGATGTTTGTAGGCTTCCTGGGGTGCTATGGGGCCATC CAGGAGTCCCAGTGTCTGCTGGGGACGTTCTTCACCTGCCTTGTGATCCTGTTTGCCTGTGAG GTGGCTGCAGGCATCTGGGGCTTCGTAAACAAAGACCAGATCGCCAAGGATGTGAAGCAGTTC TATGACCAGGCCCTTCAGCAAGCTGTGATGGATGATGATGCCAACAATGCCAAGGCTGTGGTG AAGACTTTCCATGAGACGCTCAACTGTTGTGGCTCCAACGCACTGACCACACTGACTACCACC ATACTGAGGAACAGCCTGTGTCCCTCA | 62 |
| CD81 (amino acids 178 to 236) | GGNILTPLLQQDCHQKIDELFSGKLYLIGIAAIVVAVIMIFEMILSMVLCCGIRNSSVY | 63 |
| | GGCGGCAACATACTCACCCCCTTACTGCAGCAAGATTGTCATCAGAAAATCGATGAGCTCTTC TCTGGGAAGCTGTACCTCATTGGAATTGCAGCCATTGTGGTAGCTGTCATTATGATCTTTGAG ATGATTCTGAGCATGGTGCTGTGCTGTGGCATCCGGAACAGCTCCGTGTACTGA | 64 |
| CD81-IL -2 | | 133 |
| | ATGGGGGTGGAGGGCTGCACCAAATGCATCAAATACCTGCTCTTCGTCTTCAATTTCGTCTTC TGGCTGGCTGGAGGCGTGATCCTAGGTGTAGCTCTGTGGTTGCGTCATGATCCACAGACCACC AGCCTGCTGTACCTGGAACTGGGAAACAAACCGGCACCCAACACCTTCTACGTGGGCATCTAC ATTCTCATTGCTGTGGGAGCTGTGATGATGTTTGTAGGCTTCCTGGGGTGCTATGGGGCCATC | 134 |

**[Table 13-5]**

| | | |
|---|---|---|
| | CAGGAGTCCCAGTGTCTGCTGGGGACGTTCTTCACCTGCCTTGTGATCCTGTTTGCCTGTGAG GTGGCTGCAGGCATCTGGGGCTTCGTAAACAAAGACCAGATCGCCAAGGATGTGAAGCAGTTC TATGACCAGGCCCTTCAGCAAGCTGTGATGGATGATGATGCCAACAATGCCAAGGCTGTGGTG AAGACTTTCCATGAGACGCTCAACTGTTGTGGCTCCAACGCACTGACCACACTGACTACCACC ATACTGAGGAACAGCCTGTGTCCCTCAGGAGGAGGAGGAAGCGCACCCACTTCAAGCTCCACT TCAAGCTCTACAGCGGAAGCACAGCAGCAGCAGCAGCAGCAGCAGCAGCAGCAGCAGCACCTG GAGCAGCTGTTGATGGACCTACAGGAGCTCCTGAGCAGGATGGAGAATTACAGGAACCTGAAA CTCCCCAGGATGCTCACCTTCAAATTTTACTTGCCCAAGCAGGCCACAGAATTGAAAGATCTT CAGTGCCTAGAAGATGAACTTGGACCTCTGCGGCATGTTCTGGATTTGACTCAAAGCAAAAGC TTTCAATTGGAAGATGCTGAGAATTTCATCAGCAATATCAGAGTAACTGTTGTAAAACTAAAG GGCTCTGACAACACATTTGAGTGCCAATTCGATGATGAGTCAGCAACTGTGGTGGACTTTCTG AGGAGATGGATAGCCTTCTGTCAAAGCATCATCTCAACAAGCCCTCAAGGAGGAGGAGGAAGC GGCGGCAACATACTCACCCCCTTACTGCAGCAAGATTGTCATCAGAAAATCGATGAGCTCTTC TCTGGGAAGCTGTACCTCATTGGAATTGCAGCCATTGTGGTAGCTGTCATTATGATCTTTGAG ATGATTCTGAGCATGGTGCTGTGCTGTGGCATCCGGAACAGCTCCGTGTACTGA | |
| sc-Trim er-CD81 -IL-2 (sc-Tri mer+CD8 1) | MARSVTLVFLVLVSLTGLYASIINFEKLGGGASGGGGSGGGGSIQKTPQIQVYSRHPPENGKP NILNCYVTQFHPPHIEIQMLKNGKKIPKVEMSDMSFSKDWSFYILAHTEFTPTETDTYACRVK HASMAEPKTVYWDRDMGGGGSGGGGSGGGGSGGGGSGPHSLRYFVTAVSRPGLGEPRYMEVGY VDDTEFVRFDSDAENPRYEPRARWMEQEGPEYWERETQKAKGNEQSFRVDLRTLLGYYNQSKG GSHTIQVISGCEVGSDGRLLRGYQQYAYDGCDYIALNEDLKTWTAADMAALITKHKWEQAGEA ERLRAYLEGTCVEWLRRYLKNGNATLLRTDSPKAHVTHHSRPEDKVTLRCWALGFYPADITLT WQLNGEELIQDMELVETRPAGDGTFQKWASVVVPLGKEQYYTCHVYHQGLPEPLTLRWEPPPS TVSNMATVAVLVVLGAAIVTGAVVAFVMKMRRRNTGGKGGDYALAPGSQTSDLSLPDCKVMVH DPHSLAMGVEGCTKCIKYLLFVFNFVFWLAGGVILGVALWLRHDPQTTSLLYLELGNKPAPNT FYVGIYILIAVGAVMMFVGFLGCYGAIQESQCLLGTFFTCLVILFACEVAAGIWGFVNKDQIA KDVKQFYDQALQQAVMDDDANNAKAVVKTFHETLNCCGSNALTTLTTTILRNSLCPSGGGGSA PTSSSTSSSTAEAQQQQQQQQQQQQHLEQLLMDLQELLSRMENYRNLKLPRMLTFKFYLPKQA TELKDLQCLEDELGPLRHVLDLTQSKSFQLEDAENFISNIRVTVVKLKGSDNTFECQFDDESA TVVDFLRRWIAFCQSIISTSPQGGGGSGGNILTPLLQQDCHQKIDELFSGKLYLIGIAAIVVA VIMIFEMILSMVLCCGIRNSSVY | 135 |
| | ATGGCTCGCTCGGTGACCCTGGTCTTTCTGGTGCTTGTCTCACTGACCGGCCTGTATGCTTCC ATTATAAATTTTGAAAAGTTGGGCGGAGGTGCCTCTGGCGGTGGGGGCAGCGGTGGAGGGGGC AGTATCCAGAAAACCCCTCAAATTCAAGTATACTCACGCCACCCACCGGAGAATGGGAAGCCG AACATACTGAACTGCTACGTAACACAGTTCCACCCGCCTCACATTGAAATCCAAATGCTGAAG AACGGGAAAAAAATTCCTAAAGTAGAGATGTCAGATATGTCCTTCAGCAAGGACTGGTCTTTC TATATCCTGGCTCACACTGAATTCACCCCCACTGAGACTGATACATACGCCTGCAGAGTTAAG CATGCCAGTATGGCCGAGCCCAAGACCGTCTACTGGGATCGAGACATGGGGGGGGGAGGCTCC GGTGGAGGGGGGTCTGGAGGGGGGGGGTCTGGTGGAGGCGGAAGTGGCCCACACTCGCTGAGG TATTTCGTCACCGCCGTGTCCCGGCCCGGCCTCGGGGAGCCCCGGTACATGGAAGTCGGCTAC GTGGACGACACGGAGTTCGTGCGCTTCGACAGCGACGCGGAGAATCCGAGATATGAGCCGCGG GCGCGGTGGATGGAGCAGGAGGGGCCCGAGTATTGGGAGCGGGAGACACAGAAAGCCAAGGGC AATGAGCAGAGTTTCCGAGTGGACCTGAGGACCCTGCTCGGCTACTACAACCAGAGCAAGGGC GGCTCTCACACTATTCAGGTGATCTCTGGCTGTGAAGTGGGGTCCGACGGGCGACTCCTCCGC GGGTACCAGCAGTACGCCTACGACGGCTGCGATTACATCGCCCTGAACGAAGACCTGAAAACG TGGACGGCGGCGGACATGGCGGCGCTGATCACCAAACACAAGTGGGAGCAGGCTGGTGAAGCA GAGAGACTCAGGGCCTACCTGGAGGGCACGTGCGTGGAGTGGCTCCGCAGATACCTGAAGAAC GGGAACGCGACGCTGCTGCGCACAGATTCCCCAAAGGCCCATGTGACCCATCACAGCAGACCT GAAGATAAAGTCACCCTGAGGTGCTGGGCCCTGGGCTTCTACCCTGCTGACATCACCCTGACC TGGCAGTTGAATGGGGAGGAGCTGATCCAGGACATGGAGCTTGTGGAGACCAGGCCTGCAGGG GATGGAACCTTCCAGAAGTGGGCATCTGTGGTGGTGCCTCTTGGGAAGGAGCAGTATTACACA | 136 |

**[Table 13-6]**

| | | |
|---|---|---|
| | TGCCATGTGTACCATCAGGGGCTGCCTGAGCCCCTCACCCTGAGATGGGAGCCTCCTCCATCC ACTGTCTCCAACATGGCGACCGTTGCTGTTCTGGTTGTCCTTGGAGCTGCAATAGTCACTGGA GCTGTGGTGGCTTTTGTGATGAAGATGAGAAGGAGAAACACAGGTGGAAAAGGAGGGGACTAT GCTCTGGCTCCAGGCTCCCAGACCTCTGATCTGTCTCTCCCAGATTGTAAAGTGATGGTTCAT GACCCTCATTCTCTAGCGATGGGGGTGGAGGGCTGCACCAAATGCATCAAATACCTGCTCTTC GTCTTCAATTTCGTCTTCTGGCTGGCTGGAGGCGTGATCCTAGGTGTAGCTCTGTGGTTGCGT CATGATCCACAGACCACCAGCCTGCTGTACCTGGAACTGGGAAACAAACCGGCACCCAACACC TTCTACGTGGGCATCTACATTCTCATTGCTGTGGGAGCTGTGATGATGTTTGTAGGCTTCCTG GGGTGCTATGGGGCCATCCAGGAGTCCCAGTGTCTGCTGGGGACGTTCTTCACCTGCCTTGTG ATCCTGTTTGCCTGTGAGGTGGCTGCAGGCATCTGGGGCTTCGTAAACAAAGACCAGATCGCC AAGGATGTGAAGCAGTTCTATGACCAGGCCCTTCAGCAAGCTGTGATGGATGATGATGCCAAC AATGCCAAGGCTGTGGTGAAGACTTTCCATGAGACGCTCAACTGTTGTGGCTCCAACGCACTG ACCACACTGACTACCACCATACTGAGGAACAGCCTGTGTCCCTCAGGAGGAGGAGGAAGCGCA CCCACTTCAAGCTCCACTTCAAGCTCTACAGCGGAAGCACAGCAGCAGCAGCAGCAGCAGCAG CAGCAGCAGCAGCACCTGGAGCAGCTGTTGATGGACCTACAGGAGCTCCTGAGCAGGATGGAG AATTACAGGAACCTGAAACTCCCCAGGATGCTCACCTTCAAATTTTACTTGCCCAAGCAGGCC ACAGAATTGAAAGATCTTCAGTGCCTAGAAGATGAACTTGGACCTCTGCGGCATGTTCTGGAT TTGACTCAAAGCAAAAGCTTTCAATTGGAAGATGCTGAGAATTTCATCAGCAATATCAGAGTA ACTGTTGTAAAACTAAAGGGCTCTGACAACACATTTGAGTGCCAATTCGATGATGAGTCAGCA ACTGTGGTGGACTTTCTGAGGAGATGGATAGCCTTCTGTCAAAGCATCATCTCAACAAGCCCT CAAGGAGGAGGAGGAAGCGGCGGCAACATACTCACCCCCTTACTGCAGCAAGATTGTCATCAG AAAATCGATGAGCTCTTCTCTGGGAAGCTGTACCTCATTGGAATTGCAGCCATTGTGGTAGCT GTCATTATGATCTTTGAGATGATTCTGAGCATGGTGCTGTGCTGTGGCATCCGGAACAGCTCC GTGTACTGA | |
| Aka-Luc | MEDAKNIKKGPAPFYPLEDGTAGEQLHKAMKRYALVPGAIAFTDAHIQVDVTYAEYFEMSVRL AEAMRRYGLNTNHRIVVCSENSSQFFMPVLGALFIGVAVAPANDIYNERELLNSMGISQPTVV FVSKKGLRKVLNVQKKLPIIRKIIIMDSKTDYQGFQSMYTFVTSHLPPSFNEYDFVPESFDRD KTIALIMNSSGSTGLPKGVALPHRTACVRFSHARDPIFGYQNIPDTAILSVVPFHHGFGMFTT LGYLICGFRVVLMYRFEEELFLRSLQDYKIQSALLVPTLFSCLAKSTLIDKYDLSSLREIASG GAPLSKEVGEAVAKRFRLPGIRQGYGLTETTNAVMITPEGDRKPGSVGKVVPFFEAKVVDLVT GKTLGVNQRGELCVRGPMIMSGYVNNPEATNALIDKDGWLHSGDIAYWDEDEHFFIVDRLKSL IKYKGYQVAPAELEGILLQHPYIFDAGVAGLPDDDAGELPAAVVVLEHGKTMTEKEIVDYVAS QVTTAKKLRGGVVFVDEVPRGSTGKLDARKIREILTKAKKDGKIAV | 137 |
| | ATGGAAGATGCCAAAAACATTAAGAAGGGCCCAGCGCCGTTCTACCCACTCGAAGACGGGACC GCCGGCGAGCAGCTGCACAAAGCCATGAAGCGCTACGCCCTGGTGCCCGGCGCCATCGCCTTT ACCGACGCACATATTCAGGTGGACGTTACCTACGCCGAGTACTTCGAGATGAGCGTTCGGCTG GCAGAAGCTATGAGGCGCTATGGGCTGAATACAAACCATCGGATCGTGGTGTGCAGCGAGAAT AGCTCGCAGTTCTTCATGCCCGTGTTGGGTGCCCTGTTCATCGGTGTGGCTGTGGCCCCAGCT AACGACATCTACAACGAGCGCGAGCTGCTGAACAGCATGGGCATCAGCCAGCCCACCGTCGTA TTCGTGAGCAAGAAAGGGCTGCAAAGGTCCTCAACGTGCAAAAGAAGCTACCGATCATACGA AAGATCATCATCATGGATAGCAAGACCGACTACCAGGGCTTCCAAAGCATGTACACCTTCGTG ACTTCCCATTTGCCACCCAGCTTCAACGAGTACGACTTCGTGCCCGAGAGCTTCGACCGGGAC AAAACCATCGCCCTGATCATGAACAGTAGTGGTAGTACAGGATTACCCAAGGGCGTAGCCCTA CCGCACCGCACCGCTTGTGTCCGATTCAGTCATGCCCGCGACCCCATCTTCGGCTACCAGAAC ATCCCCGACACCGCTATCCTCAGCGTGGTGCCATTTCACCACGGCTTCGGCATGTTCACCACG CTGGGCTACTTGATCTGCGGCTTTCGGGTCGTGCTCATGTACCGCTTCGAGGAGGAGCTATTC TTGCGCAGCTTGCAAGACTATAAGATTCAATCTGCCCTGCTGGTGCCCACACTATTTAGCTGC CTCGCTAAGAGCACTCTCATCGACAAGTACGACCTAAGCAGCTTGCGCGAGATCGCCAGCGGC GGGGCGCCGCTCAGCAAGGAGGTAGGTGAGGCCGTGGCCAAACGCTTCCGCCTACCAGGCATC CGCCAGGGCTATGGCCTGACAGAAACAACCAACGCCGTCATGATCACCCCCGAGGGGGACCGT AAGCCTGGCTCAGTAGGCAAGGTGGTGCCCTTCTTCGAGGCTAAGGTGGTAGACTTGGTCACC GGTAAGACACTGGGTGTGAACCAGCGCGGTGAGCTGTGCGTCCGTGGCCCCATGATCATGAGC | 138 |

**[Table 13-7]**

| | | |
|---|---|---|
| | GGCTACGTTAACAACCCCGAGGCTACGAACGCTCTCATCGACAAGGACGGCTGGCTGCACAGC GGCGACATCGCCTACTGGGACGAGGACGAGCACTTCTTCATCGTGGACCGGCTGAAGAGCCTG ATCAAATACAAGGGCTACCAGGTAGCCCCAGCCGAACTGGAGGGCATCCTGCTGCAACACCCC TACATCTTCGACGCCGGAGTCGCCGGCCTGCCCGACGACGATGCCGGCGAGCTGCCCGCCGCA GTCGTCGTGTTGGAACACGGTAAAACCATGACCGAGAAAGAGATCGTGGACTATGTGGCCAGC CAGGTTACAACCGCCAAGAAGCTGCGCGGTGGTGTTGTGTTTGTGGATGAAGTCCCTAGAGGA TCGACCGGCAAGTTAGACGCCCGCAAGATCCGCGAGATTCTCACTAAGGCCAAGAAGGACGGC AAGATCGCCGTG | |
| C D 6 3 | MAVEGGMKCVKFLLYVLLLAFCACAVGLIAIGVAVQVVLKQAITHETTAGSLLPVVIIAVGAF LFLVAFVGCCGACKENYCLMITFAIFLSLIMLVEVAVAIAGYVFRDQVKSEFNKSFQQQMQNY LKDNKTATILDKLQKENNCCGASNYTDWENIPGMAKDRVPDSCCINITVGCGNDFKESTIHTQ GCVETIAIWLRKNILLVAAAALGIAFVEVLGIIFSCCLVKSIRSGYEVM | 27 |
| | ATGGCGGTGGAAGGAGGAATGAAGTGTGTCAAGTTTTTGCTCTACGTTCTCCTGCTGGCCTTC TGCGCCTGTGCAGTGGGATTGATCGCCATTGGTGTAGCGGTTCAGGTTGTCTTGAAGCAGGCC ATTACCCATGAGACTACTGCTGGCTCGCTGTTGCCTGTGGTCATCATTGCAGTGGGTGCCTTC CTCTTCCTGGTGGCCTTTGTGGGCTGCTGTGGGGCCTGCAAGGAGAACTACTGTCTCATGATT ACATTTGCCATCTTCCTGTCTCTTATCATGCTTGTGGAGGTGGCTGTGGCCATTGCTGGCTAT GTGTTTAGAGACCAGGTGAAGTCAGAGTTTAATAAAAGCTTCCAGCAGCAGATGCAGAATTAC CTTAAAGACAACAAAACAGCCACTATTTTGGACAAATTGCAGAAAGAAAATAACTGCTGTGGA GCTTCTAACTACACAGACTGGGAAAACATCCCCGGCATGGCCAAGGACAGAGTCCCCGATTCT TGCTGCATCAACATAACTGTGGGCTGTGGGAATGATTTCAAGGAATCCACTATCCATACCCAG GGCTGCGTGGAGACTATAGCAATATGGCTAAGGAAGAACATACTGCTGGTGGCTGCAGCGGCC CTGGGCATTGCTTTTGTGGAGGTCTTGGGAATTATCTTCTCCTGCTGTCTGGTGAAGAGTATT CGAAGTGGCTATGAAGTAATGTAG | 28 |
| C D 6 3 (amino acids 1 to 170) | MAVEGGMKCVKFLLYVLLLAFCACAVGLIAIGVAVQVVLKQAITHETTAGSLLPVVIIAVGAF LFLVAFVGCCGACKENYCLMITFAIFLSLIMLVEVAVAIAGYVFRDQVKSEFNKSFQQQMQNY LKDNKTATILDKLQKENNCCGASNYTDWENIPGMAKDRVPDSCC | 57 |
| | ATGGCGGTGGAAGGAGGAATGAAGTGTGTCAAGTTTTTGCTCTACGTTCTCCTGCTGGCCTTC TGCGCCTGTGCAGTGGGATTGATCGCCATTGGTGTAGCGGTTCAGGTTGTCTTGAAGCAGGCC ATTACCCATGAGACTACTGCTGGCTCGCTGTTGCCTGTGGTCATCATTGCAGTGGGTGCCTTC CTCTTCCTGGTGGCCTTTGTGGGCTGCTGTGGGGCCTGCAAGGAGAACTACTGTCTCATGATT ACATTTGCCATCTTCCTGTCTCTTATCATGCTTGTGGAGGTGGCTGTGGCCATTGCTGGCTAT GTGTTTAGAGACCAGGTGAAGTCAGAGTTTAATAAAAGCTTCCAGCAGCAGATGCAGAATTAC CTTAAAGACAACAAAACAGCCACTATTTTGGACAAATTGCAGAAAGAAAATAACTGCTGTGGA GCTTCTAACTACACAGACTGGGAAAACATCCCCGGCATGGCCAAGGACAGAGTCCCCGATTCT TGCTGC | 58 |
| C D 6 3 (amino acids 171 to 238) | INITVGCGNDFKESTIHTQGCVETIAIWLRKNILLVAAAALGIAFVEVLGIIFSCCLVKSIRS GYEVM | 59 |

**[Table 13-8]**

| | | |
|---|---|---|
| | ATCAACATAACTGTGGGCTGTGGGAATGATTTCAAGGAATCCACTATCCATACCCAGGGCTGC GTGGAGACTATAGCAATATGGCTAAGGAAGAACATACTGCTGGTGGCTGCAGCGGCCCTGGGC ATTGCTTTTGTGGAGGTCTTGGGAATTATCTTCTCCTGCTGTCTGGTGAAGAGTATTCGAAGT GGCTATGAAGTAATGTAG | 60 |
| Peptide linker 3 | GGGGS | 29 |
| | GGAGGAGGAGGAAGC | 30 |
| C D 6 3 -Aka-Luc | MAVEGGMKCVKFLLYVLLLAFCACAVGLIAIGVAVQVVLKQAITHETTAGSLLPVVIIAVGAF LFLVAFVGCCGACKENYCLMITFAIFLSLIMLVEVAVAIAGYVFRDQVKSEFNKSFQQQMQNY LKDNKTATILDKLQKENNCCGASNYTDWENIPGMAKDRVPDSCCGGGGSMEDAKNIKKGPAPF YPLEDGTAGEQLHKAMKRYALVPGAIAFTDAHIQVDVTYAEYFEMSVRLAEAMRRYGLNTNHR IVVCSENSSQFFMPVLGALFIGVAVAPANDIYNERELLNSMGISQPTVVFVSKKGLRKVLNVQ KKLPIIRKIIIMDSKTDYQGFQSMYTFVTSHLPPSFNEYDFVPESFDRDKTIALIMNSSGSTG LPKGVALPHRTACVRFSHARDPIFGYQNIPDTAILSVVPFHHGFGMFTTLGYLICGFRVVLMY RFEEELFLRSLQDYKIQSALLVPTLFSCLAKSTLIDKYDLSSLREIASGGAPLSKEVGEAVAK RFRLPGIRQGYGLTETTNAVMITPEGDRKPGSVGKVVPFFEAKVVDLVTGKTLGVNQRGELCV RGPMIMSGYVNNPEATNALIDKDGWLHSGDIAYWDEDEHFFIVDRLKSLIKYKGYQVAPAELE GILLQHPYIFDAGVAGLPDDDAGELPAAVVVLEHGKTMTEKEIVDYVASQVTTAKKLRGGVVF VDEVPRGSTGKLDARKIREILTKAKKDGKIAVGGGGSINITVGCGNDFKESTIHTQGCVETIA IWLRKNILLVAAAALGIAFVEVLGIIFSCCLVKSIRSGYEVM | 139 |
| | ATGGCGGTGGAAGGAGGAATGAAGTGTGTCAAGTTTTTGCTCTACGTTCTCCTGCTGGCCTTC TGCGCCTGTGCAGTGGGATTGATCGCCATTGGTGTAGCGGTTCAGGTTGTCTTGAAGCAGGCC ATTACCCATGAGACTACTGCTGGCTCGCTGTTGCCTGTGGTCATCATTGCAGTGGGTGCCTTC CTCTTCCTGGTGGCCTTTGTGGGCTGCTGTGGGGCCTGCAAGGAGAACTACTGTCTCATGATT ACATTTGCCATCTTCCTGTCTCTTATCATGCTTGTGGAGGTGGCTGTGGCCATTGCTGGCTAT GTGTTTAGAGACCAGGTGAAGTCAGAGTTTAATAAAAGCTTCCAGCAGCAGATGCAGAATTAC CTTAAAGACAACAAAACAGCCACTATTTTGGACAAATTGCAGAAAGAAAATAACTGCTGTGGA GCTTCTAACTACACAGACTGGGAAAACATCCCCGGCATGGCCAAGGACAGAGTCCCCGATTCT TGCTGCGGTGGTGGTGGTTCTATGGAAGATGCCAAAAACATTAAGAAGGGCCCAGCGCCGTTC TACCCACTCGAAGACGGGACCGCCGGCGAGCAGCTGCACAAAGCCATGAAGCGCTACGCCCTG GTGCCCGGCGCCATCGCCTTTACCGACGCACATATTCAGGTGGACGTTACCTACGCCGAGTAC TTCGAGATGAGCGTTCGGCTGGCAGAAGCTATGAGGCGCTATGGGCTGAATACAAACCATCGG ATCGTGGTGTGCAGCGAGAATAGCTCGCAGTTCTTCATGCCCGTGTTGGGTGCCCTGTTCATC GGTGTGGCTGTGGCCCCAGCTAACGACATCTACAACGAGCGCGAGCTGCTGAACAGCATGGGC ATCAGCCAGCCCACCGTCGTATTCGTGAGCAAGAAAGGGCTGCAAAAGGTCCTCAACGTGCAA AAGAAGCTACCGATCATACGAAAGATCATCATCATGGATAGCAAGACCGACTACCAGGGCTTC CAAAGCATGTACACCTTCGTGACTTCCCATTTGCCACCCAGCTTCAACGAGTACGACTTCGTG CCCGAGAGCTTCGACCGGGACAAAACCATCGCCCTGATCATGAACAGTAGTGGTAGTACAGGA TTACCCAAGGGCGTAGCCCTACCGCACCGCACCGCTTGTGTCCGATTCAGTCATGCCCGCGAC CCCATCTTCGGCTACCAGAACATCCCCGACACCGCTATCCTCAGCGTGGTGCCATTTCACCAC GGCTTCGGCATGTTCACCACGCTGGGCTACTTGATCTGCGGCTTTCGGGTCGTGCTCATGTAC CGCTTCGAGGAGGAGCTATTCTTGCGCAGCTTGCAAGACTATAAGATTCAATCTGCCCTGCTG GTGCCCACACTATTTAGCTGCCTCGCTAAGAGCACTCTCATCGACAAGTACGACCTAAGCAGC TTGCGCGAGATCGCCAGCGGCGGGGCGCCGCTCAGCAAGGAGGTAGGTGAGGCCGTGGCCAAA CGCTTCCGCCTACCAGGCATCCGCCAGGGCTATGGCCTGACAGAAACAACCAACGCCGTCATG ATCACCCCCGAGGGGGACCGTAAGCCTGGCTCAGTAGGCAAGGTGGTGCCCTTCTTCGAGGCT AAGGTGGTAGACTTGGTCACCGGTAAGACACTGGGTGTGAACCAGCGCGGTGAGCTGTGCGTC CGTGGCCCCATGATCATGAGCGGCTACGTTAACAACCCCGAGGCTACGAACGCTCTCATCGAC AAGGACGGCTGGCTGCACAGCGGCGACATCGCCTACTGGGACGAGGACGAGCACTTCTTCATC GTGGACCGGCTGAAGAGCCTGATCAAATACAAGGGCTACCAGGTAGCCCCAGCCGAACTGGAG GGCATCCTGCTGCAACACCCCTACATCTTCGACGCCGGAGTCGCCGGCCTGCCCGACGACGAT | 140 |

**[Table 13-9]**

| | | |
|---|---|---|
| | GCCGGCGAGCTGCCCGCCGCAGTCGTCGTGTTGGAACACGGTAAAACCATGACCGAGAAAGAG ATCGTGGACTATGTGGCCAGCCAGGTTACAACCGCCAAGAAGCTGCGCGGTGGTGTTGTGTTT GTGGATGAAGTCCCTAGAGGATCGACCGGCAAGTTAGACGCCCGCAAGATCCGCGAGATTCTC ACTAAGGCCAAGAAGGACGGCAAGATCGCCGTGGGTGGTGGTGGTTCTATCAACATAACTGTG GGCTGTGGGAATGATTTCAAGGAATCCACTATCCATACCCAGGGCTGCGTGGAGACTATAGCA ATATGGCTAAGGAAGAACATACTGCTGGTGGCTGCAGCGGCCCTGGGCATTGCTTTTGTGGAG GTCTTGGGAATTATCTTCTCCTGCTGTCTGGTGAAGAGTATTCGAAGTGGCTATGAAGTAATG TAG | |

**[Table 14-1]**

| | Sequence | SEQ ID NO: |
|---|---|---|
| Signal peptide of h *β* ₂ microglobulin | MSRSVALAVLALLSLSGLEA | 121 |
| | ATGTCTCGCTCCGTGGCCTTAGCTGTGCTCGCGCTACTCTCTCTTTCTGGCCTGGAGGCT | 122 |
| SARS-CoV2 peptide 1 (for MHC class I molecule) | KLWAQCVQL | 141 |
| | AAACTGTGGGCCCAGTGTGTGCAGCTG | 142 |
| Peptide linker 1 | GGGASGGGGSGGGGS | 5 |
| | GGCGGAGGTGCCTCTGGCGGTGGGGGCAGCGGTGGAGGGGGCAGT | 6 |
| h *β* ₂ Microglobulin (from which signal peptide is removed) | | 121 |
| | ATCCAGCGTACTCCAAAGATTCAGGTTTACTCACGTCATCCAGCAGAGAATGGAAAGTCAAAT TTCCTGAATTGCTATGTGTCTGGGTTTCATCCATCCGACATTGAAGTTGACTTACTGAAGAAT GGAGAGAGAATTGAAAAAGTGGAGCATTCAGACTTGTCTTTCAGCAAGGACTGGTCTTTCTAT CTCTTGTACTACACTGAATTCACCCCCACTGAAAAAGATGAGTATGCCTGCCGTGTGAACCAT GTGACTTTGTCACAGCCCAAGATAGTTAAGTGGGATCGAGACATG | 122 |
| hMHC class I (HLA-A0201) *α* chain (from which signal peptide is removed) | GSHSMRYFFTSVSRPGRGEPRFIAVGYVDDTQFVRFDSDAASQRMEPRAPWIEQEGPEYWDGE TRKVKAHSQTHRVDLGTLRGYYNQSEAGSHTVQRMYGCDVGSDWRFLRGYHQYAYDGKDYIAL KEDLRSWTAADMAAQTTKHKWEAAHVAEQLRAYLEGTCVEWLRRYLENGKETLQRTDAPKTHM THHAVSDHEATLRCWALSFYPAEITLTWQRDGEDQTQDTELVETRPAGDGTFQKWAAVVVPSG QEQRYTCHVQHEGLPKPLTLRWEPSSQPTIPIVGIIAGLVLFGAVITGAVVAAVMWRRKSSDR KGGSYSQAASSDSAQGSDVSLTACKV | 143 |
| | GGCTCTCACTCCATGAGGTATTTCTTCACATCCGTGTCCCGGCCCGGCCGCGGGGAGCCCCGC TTCATCGCAGTGGGCTACGTGGACGACACGCAGTTCGTGCGGTTCGACAGCGACGCCGCGAGC CAGAGGATGGAGCCGCGGGCGCCGTGGATAGAGCAGGAGGGTCCGGAGTATTGGGACGGGGAG ACACGGAAAGTGAAGGCCCACTCACAGACTCACCGAGTGGACCTGGGGACCCTGCGCGGCTAC TACAACCAGAGCGAGGCCGGTTCTCACACCGTCCAGAGGATGTATGGCTGCGACGTGGGGTCG GACTGGCGCTTCCTCCGCGGGTACCACCAGTACGCCTACGACGGCAAGGATTACATCGCCCTG AAAGAGGACCTGCGCTCTTGGACCGCGGCGGACATGGCAGCTCAGACCACCAAGCACAAGTGG GAGGCGGCCCATGTGGCGGAGCAGTTGAGAGCCTACCTGGAGGGCACGTGCGTGGAGTGGCTC CGCAGATACCTGGAGAACGGGAAGGAGACGCTGCAGCGCACGGACGCCCCCAAAACGCATATG ACTCACCACGCTGTCTCTGACCATGAAGCCACCCTGAGGTGCTGGGCCCTGAGCTTCTACCCT GCGGAGATCACACTGACCTGGCAGCGGGATGGGGAGGACCAGACCCAGGACACGGAGCTCGTG GAGACCAGGCCTGCAGGGGATGGAACCTTCCAGAAGTGGGCGGCTGTGGTGGTGCCTTCTGGA CAGGAGCAGAGATACACCTGCCATGTGCAGCATGAGGGTTTGCCCAAGCCCCTCACCCTGAGA TGGGAGCCGTCTTCCCAGCCCACCATCCCCATCGTGGGCATCATTGCTGGCCTGGTTCTCTTT GGAGCTGTGATCACTGGAGCTGTGGTCGCTGCTGTGATGTGGAGGAGGAAGAGCTCAGATAGA AAAGGAGGGAGCTACTCTCAGGCTGCAAGCAGTGACAGTGCCCAGGGCTCTGATGTGTCTCTC ACAGCTTGTAAAGTG | 144 |
| Peptide linker 2 | GGGGSGGGGSGGGGSGGGGS | 11 |
| | GGGGGGGGAGGCTCCGGTGGAGGGGGGTCTGGAGGGGGGGGGTCTGGTGGAGGCGGAAGT | 12 |
| | IQRTPKIQVYSRHPAENGKSNFLNCYVSGFHPSDIEVDLLKNGERIEKVEHSDLSFSKDWSFY | 145 |

**[Table 14-2]**

| | | |
|---|---|---|
| h single chain MH C class I molecule (*β* ₂ microglobulin (from which signal peptide is removed) + peptide linker 2 + MHC class I (HLA-A0201) *α* chain (from which signal peptide is removed)) | LLYYTEFTPTEKDEYACRVNHVTLSQPKIVKWDRDMGGGGSGGGGSGGGGSGGGGSGSHSMRY FFTSVSRPGRGEPRFIAVGYVDDTQFVRFDSDAASQRMEPRAPWIEQEGPEYWDGETRKVKAH SQTHRVDLGTLRGYYNQSEAGSHTVQRMYGCDVGSDWRFLRGYHQYAYDGK DYIALKEDLRSWTAADMAAQTTKHKWEAAHVAEQLRAYLEGTCVEWLRRYLENGKETLQRTDA PKTHMTHHAVSDHEATLRCWALSFYPAEITLTWQRDGEDQTQDTELVETRPAGDGTFQKWAAV VVPSGQEQRYTCHVQHEGLPKPLTLRWEPSSQPTIPIVGIIAGLVLFGAVITGAVVAAVMWRR KSSDRKGGSYSQAASSDSAQGSDVSLTACKV | |
| | ATCCAGCGTACTCCAAAGATTCAGGTTTACTCACGTCATCCAGCAGAGAATGGAAAGTCAAAT TTCCTGAATTGCTATGTGTCTGGGTTTCATCCATCCGACATTGAAGTTGACTTACTGAAGAAT GGAGAGAGAATTGAAAAAGTGGAGCATTCAGACTTGTCTTTCAGCAAGGACTGGTCTTTCTAT CTCTTGTACTACACTGAATTCACCCCCACTGAAAAAGATGAGTATGCCTGCCGTGTGAACCAT GTGACTTTGTCACAGCCCAAGATAGTTAAGTGGGATCGAGACATGGGAGGCGGCGGGTCTGGC GGCGGCGGCAGTGGCGGTGGCGGTAGCGGCGGAGGTGGATCTGGCTCTCACTCCATGAGGTAT TTCTTCACATCCGTGTCCCGGCCCGGCCGCGGGGAGCCCCGCTTCATCGCAGTGGGCTACGTG GACGACACGCAGTTCGTGCGGTTCGACAGCGACGCCGCGAGCCAGAGGATGGAGCCGCGGGCG CCGTGGATAGAGCAGGAGGGTCCGGAGTATTGGGACGGGGAGACACGGAAAGTGAAGGCCCAC TCACAGACTCACCGAGTGGACCTGGGGACCCTGCGCGGCTACTACAACCAGAGCGAGGCCGGT TCTCACACCGTCCAGAGGATGTATGGCTGCGACGTGGGGTCGGACTGGCGCTTCCTCCGCGGG TACCACCAGTACGCCTACGACGGCAAGGATTACATCGCCCTGAAAGAGGACCTGCGCTCTTGG ACCGCGGCGGACATGGCAGCTCAGACCACCAAGCACAAGTGGGAGGCGGCCCATGTGGCGGAG CAGTTGAGAGCCTACCTGGAGGGCACGTGCGTGGAGTGGCTCCGCAGATACCTGGAGAACGGG AAGGAGACGCTGCAGCGCACGGACGCCCCCAAAACGCATATGACTCACCACGCTGTCTCTGAC CATGAAGCCACCCTGAGGTGCTGGGCCCTGAGCTTCTACCCTGCGGAGATCACACTGACCTGG CAGCGGGATGGGGAGGACCAGACCCAGGACACGGAGCTCGTGGAGACCAGGCCTGCAGGGGAT GGAACCTTCCAGAAGTGGGCGGCTGTGGTGGTGCCTTCTGGACAGGAGCAGAGATACACCTGC CATGTGCAGCATGAGGGTTTGCCCAAGCCCCTCACCCTGAGATGGGAGCCGTCTTCCCAGCCC ACCATCCCCATCGTGGGCATCATTGCTGGCCTGGTTCTCTTTGGAGCTGTGATCACTGGAGCT GTGGTCGCTGCTGTGATGTGGAGGAGGAAGAGCTCAGATAGAAAAGGAGGGAGCTACTCTCAG GCTGCAAGCAGTGACAGTGCCCAGGGCTCTGATGTGTCTCTCACAGCTTGTAAAGTG | 146 |
| h s c - T r i m e r (SARS-CoV2 peptide 1 + peptide linker 1 + single chain MHC class I (HLA-A0201) molecule) | MSRSVALAVLALLSLSGLEAKLWAQCVQLGGGASGGGGSGGGGSIQRTPKIQVYSRHPAENGK SNFLNCYVSGFHPSDIEVDLLKNGERIEKVEHSDLSFSKDWSFYLLYYTEFTPTEKDEYACRV NHVTLSQPKIVKWDRDMGGGGSGGGGSGGGGSGGGGSGSHSMRYFFTSVSRPGRGEPRFIAVG YVDDTQFVRFDSDAASQRMEPRAPWIEQEGPEYWDGETRKVKAHSQTHRVDLGTLRGYYNQSE AGSHTVQRMYGCDVGSDWRFLRGYHQYAYDGKDYIALKEDLRSWTAADMAAQTTKHKWEAAHV AEQLRAYLEGTCVEWLRRYLENGKETLQRTDAPKTHMTHHAVSDHEATLRCWALSFYPAEITL TWQRDGEDQTQDTELVETRPAGDGTFQKWAAVVVPSGQEQRYTCHVQHEGLPKPLTLRWEPSS QPTIPIVGIIAGLVLFGAVITGAVVAAVMWRRKSSDRKGGSYSQAASSDSAQGSDVSLTACKV | 147 |
| | ATGTCTCGCTCCGTGGCCTTAGCTGTGCTCGCGCTACTCTCTCTTTCTGGCCTGGAGGCTAAA CTGTGGGCCCAGTGTGTGCAGCTGGGCGGAGGGGCATCAGGCGGCGGTGGGTCAGGTGGAGGT GGGAGTATCCAGCGTACTCCAAAGATTCAGGTTTACTCACGTCATCCAGCAGAGAATGGAAAG TCAAATTTCCTGAATTGCTATGTGTCTGGGTTTCATCCATCCGACATTGAAGTTGACTTACTG AAGAATGGAGAGAGAATTGAAAAAGTGGAGCATTCAGACTTGTCTTTCAGCAAGGACTGGTCT TTCTATCTCTTGTACTACACTGAATTCACCCCCACTGAAAAAGATGAGTATGCCTGCCGTGTG AACCATGTGACTTTGTCACAGCCCAAGATAGTTAAGTGGGATCGAGACATGGGAGGCGGCGGG TCTGGCGGCGGCGGCAGTGGCGGTGGCGGTAGCGGCGGAGGTGGATCTGGCTCTCACTCCATG AGGTATTTCTTCACATCCGTGTCCCGGCCCGGCCGCGGGGAGCCCCGCTTCATCGCAGTGGGC TACGTGGACGACACGCAGTTCGTGCGGTTCGACAGCGACGCCGCGAGCCAGAGGATGGAGCCG CGGGCGCCGTGGATAGAGCAGGAGGGTCCGGAGTATTGGGACGGGGAGACACGGAAAGTGAAG GCCCACTCACAGACTCACCGAGTGGACCTGGGGACCCTGCGCGGCTACTACAACCAGAGCGAG GCCGGTTCTCACACCGTCCAGAGGATGTATGGCTGCGACGTGGGGTCGGACTGGCGCTTCCTC CGCGGGTACCACCAGTACGCCTACGACGGCAAGGATTACATCGCCCTGAAAGAGGACCTGCGC TCTTGGACCGCGGCGGACATGGCAGCTCAGACCACCAAGCACAAGTGGGAGGCGGCCCATGTG GCGGAGCAGTTGAGAGCCTACCTGGAGGGCACGTGCGTGGAGTGGCTCCGCAGATACCTGGAG | 148 |

**[Table 14-3]**

| | | |
|---|---|---|
| | AACGGGAAGGAGACGCTGCAGCGCACGGACGCCCCCAAAACGCATATGACTCACCACGCTGTC TCTGACCATGAAGCCACCCTGAGGTGCTGGGCCCTGAGCTTCTACCCTGCGGAGATCACACTG ACCTGGCAGCGGGATGGGGAGGACCAGACCCAGGACACGGAGCTCGTGGAGACCAGGCCTGCA GGGGATGGAACCTTCCAGAAGTGGGCGGCTGTGGTGGTGCCTTCTGGACAGGAGCAGAGATAC ACCTGCCATGTGCAGCATGAGGGTTTGCCCAAGCCCCTCACCCTGAGATGGGAGCCGTCTTCC CAGCCCACCATCCCCATCGTGGGCATCATTGCTGGCCTGGTTCTCTTTGGAGCTGTGATCACT GGAGCTGTGGTCGCTGCTGTGATGTGGAGGAGGAAGAGCTCAGATAGAAAAGGAGGGAGCTAC TCTCAGGCTGCAAGCAGTGACAGTGCCCAGGGCTCTGATGTGTCTCTCACAGCTTGTAAAGTG | |
| h C D 8 1 | MGVEGCTKCIKYLLFVFNFVFWLAGGVILGVALWLRHDPQTTNLLYLELGDKPAPNTFYVGIY ILIAVGAVMMFVGFLGCYGAIQESQCLLGTFFTCLVILFACEVAAGIWGFVNKDQIAKDVKQF YDQALQQAVVDDDANNAKAVVKTFHETLDCCGSSTLTALTTSVLKNNLCPSGSNIISNLFKED CHQKIDDLFSGKLYLIGIAAIVVAVIMIFEMILSMVLCCGIRNSSVY | 129 |
| | ATGGGAGTGGAGGGCTGCACCAAGTGCATCAAGTACCTGCTCTTCGTCTTCAATTTCGTCTTC TGGCTGGCTGGAGGCGTGATCCTGGGTGTGGCCCTGTGGCTCCGCCATGACCCGCAGACCACC AACCTCCTGTATCTGGAGCTGGGAGACAAGCCCGCGCCCAACACCTTCTATGTAGGCATCTAC ATCCTCATCGCTGTGGGCGCTGTCATGATGTTCGTTGGCTTCCTGGGCTGCTACGGGGCCATC CAGGAATCCCAGTGCCTGCTGGGGACGTTCTTCACCTGCCTGGTCATCCTGTTTGCCTGTGAG GTGGCCGCCGGCATCTGGGGCTTTGTCAACAAGGACCAGATCGCCAAGGATGTGAAGCAGTTC TATGACCAGGCCCTACAGCAGGCCGTGGTGGATGATGACGCCAACAACGCCAAGGCTGTGGTG AAGACCTTCCACGAGACGCTTGACTGCTGTGGCTCCAGCACACTGACTGCTTTGACCACCTCA GTGCTCAAGAACAATTTGTGTCCCTCGGGCAGCAACATCATCAGCAACCTCTTCAAGGAGGAC TGCCACCAGAAGATCGATGACCTCTTCTCCGGGAAGCTGTACCTCATCGGCATTGCTGCCATC GTGGTCGCTGTGATCATGATCTTCGAGATGATCCTGAGCATGGTGCTGTGCTGTGGCATCCGG AACAGCTCCGTGTACTGA | 130 |
| h s c - T r i m e r - C D 8 1 (SARS-CoV2 s c - T r i m e r + C D 8 1) | MSRSVALAVLALLSLSGLEAKLWAQCVQLGGGASGGGGSGGGGSIQRTPKIQVYSRHPAENGK SNFLNCYVSGFHPSDIEVDLLKNGERIEKVEHSDLSFSKDWSFYLLYYTEFTPTEKDEYACRV NHVTLSQPKIVKWDRDMGGGGSGGGGSGGGGSGGGGSGSHSMRYFFTSVSRPGRGEPRFIAVG YVDDTQFVRFDSDAASQRMEPRAPWIEQEGPEYWDGETRKVKAHSQTHRVDLGTLRGYYNQSE AGSHTVQRMYGCDVGSDWRFLRGYHQYAYDGKDYIALKEDLRSWTAADMAAQTTKHKWEAAHV AEQLRAYLEGTCVEWLRRYLENGKETLQRTDAPKTHMTHHAVSDHEATLRCWALSFYPAEITL TWQRDGEDQTQDTELVETRPAGDGTFQKWAAVVVPSGQEQRYTCHVQHEGLPKPLTLRWEPSS QPTIPIVGIIAGLVLFGAVITGAVVAAVMWRRKSSDRKGGSYSQAASSDSAQGSDVSLTACKV MGVEGCTKCIKYLLFVFNFVFWLAGGVILGVALWLRHDPQTTNLLYLELGDKPAPNTFYVGIY ILIAVGAVMMFVGFLGCYGAIQESQCLLGTFFTCLVILFACEVAAGIWGFVNKDQIAKDVKQF YDQALQQAVVDDDANNAKAVVKTFHETLDCCGSSTLTALTTSVLKNNLCPSGSNIISNLFKED CHQKIDDLFSGKLYLIGIAAIVVAVIMIFEMILSMVLCCGIRNSSVY | 149 |
| | ATGTCTCGCTCCGTGGCCTTAGCTGTGCTCGCGCTACTCTCTCTTTCTGGCCTGGAGGCTAAA CTGTGGGCCCAGTGTGTGCAGCTGGGCGGAGGGGCATCAGGCGGCGGTGGGTCAGGTGGAGGT GGGAGTATCCAGCGTACTCCAAAGATTCAGGTTTACTCACGTCATCCAGCAGAGAATGGAAAG TCAAATTTCCTGAATTGCTATGTGTCTGGGTTTCATCCATCCGACATTGAAGTTGACTTACTG AAGAATGGAGAGAGAATTGAAAAAGTGGAGCATTCAGACTTGTCTTTCAGCAAGGACTGGTCT TTCTATCTCTTGTACTACACTGAATTCACCCCCACTGAAAAAGATGAGTATGCCTGCCGTGTG AACCATGTGACTTTGTCACAGCCCAAGATAGTTAAGTGGGATCGAGACATGGGAGGCGGCGGG TCTGGCGGCGGCGGCAGTGGCGGTGGCGGTAGCGGCGGAGGTGGATCTGGCTCTCACTCCATG AGGTATTTCTTCACATCCGTGTCCCGGCCCGGCCGCGGGGAGCCCCGCTTCATCGCAGTGGGC | 150 |

**[Table 14-4]**

| | | |
|---|---|---|
| | TACGTGGACGACACGCAGTTCGTGCGGTTCGACAGCGACGCCGCGAGCCAGAGGATGGAGCCG CGGGCGCCGTGGATAGAGCAGGAGGGTCCGGAGTATTGGGACGGGGAGACACGGAAAGTGAAG GCCCACTCACAGACTCACCGAGTGGACCTGGGGACCCTGCGCGGCTACTACAACCAGAGCGAG GCCGGTTCTCACACCGTCCAGAGGATGTATGGCTGCGACGTGGGGTCGGACTGGCGCTTCCTC CGCGGGTACCACCAGTACGCCTACGACGGCAAGGATTACATCGCCCTGAAAGAGGACCTGCGC TCTTGGACCGCGGCGGACATGGCAGCTCAGACCACCAAGCACAAGTGGGAGGCGGCCCATGTG GCGGAGCAGTTGAGAGCCTACCTGGAGGGCACGTGCGTGGAGTGGCTCCGCAGATACCTGGAG AACGGGAAGGAGACGCTGCAGCGCACGGACGCCCCCAAAACGCATATGACTCACCACGCTGTC TCTGACCATGAAGCCACCCTGAGGTGCTGGGCCCTGAGCTTCTACCCTGCGGAGATCACACTG ACCTGGCAGCGGGATGGGGAGGACCAGACCCAGGACACGGAGCTCGTGGAGACCAGGCCTGCA GGGGATGGAACCTTCCAGAAGTGGGCGGCTGTGGTGGTGCCTTCTGGACAGGAGCAGAGATAC ACCTGCCATGTGCAGCATGAGGGTTTGCCCAAGCCCCTCACCCTGAGATGGGAGCCGTCTTCC CAGCCCACCATCCCCATCGTGGGCATCATTGCTGGCCTGGTTCTCTTTGGAGCTGTGATCACT GGAGCTGTGGTCGCTGCTGTGATGTGGAGGAGGAAGAGCTCAGATAGAAAAGGAGGGAGCTAC TCTCAGGCTGCAAGCAGTGACAGTGCCCAGGGCTCTGATGTGTCTCTCACAGCTTGTAAAGTG ATGGGAGTGGAGGGCTGCACCAAGTGCATCAAGTACCTGCTCTTCGTCTTCAATTTCGTCTTC TGGCTGGCTGGAGGCGTGATCCTGGGTGTGGCCCTGTGGCTCCGCCATGACCCGCAGACCACC AACCTCCTGTATCTGGAGCTGGGAGACAAGCCCGCGCCCAACACCTTCTATGTAGGCATCTAC ATCCTCATCGCTGTGGGCGCTGTCATGATGTTCGTTGGCTTCCTGGGCTGCTACGGGGCCATC CAGGAATCCCAGTGCCTGCTGGGGACGTTCTTCACCTGCCTGGTCATCCTGTTTGCCTGTGAG GTGGCCGCCGGCATCTGGGGCTTTGTCAACAAGGACCAGATCGCCAAGGATGTGAAGCAGTTC TATGACCAGGCCCTACAGCAGGCCGTGGTGGATGATGACGCCAACAACGCCAAGGCTGTGGTG AAGACCTTCCACGAGACGCTTGACTGCTGTGGCTCCAGCACACTGACTGCTTTGACCACCTCA GTGCTCAAGAACAATTTGTGTCCCTCGGGCAGCAACATCATCAGCAACCTCTTCAAGGAGGAC TGCCACCAGAAGATCGATGACCTCTTCTCCGGGAAGCTGTACCTCATCGGCATTGCTGCCATC GTGGTCGCTGTGATCATGATCTTCGAGATGATCCTGAGCATGGTGCTGTGCTGTGGCATCCGG AACAGCTCCGTGTACTGA | |

### Preparation of Fetal Bovine Serum from which Exosomes Are Removed

After 10 mL of inactivated FBS and 2 mL of a 50% poly(ethylene glycol) 10,000 solution (manufactured by Sigma-Aldrich, #81280) were stirred at 4°C for 2 hours, exosomes were precipitated under centrifugation conditions of 1,500 × g, 4°C, and 30 minutes, and supernatant was collected to obtain fetal bovine serum from which the exosomes were removed.

### [Example 1] Antigen-Presenting Extracellular Vesicles Containing MHC Class I Molecules and T-Cell Stimulatory Cytokines in Membrane

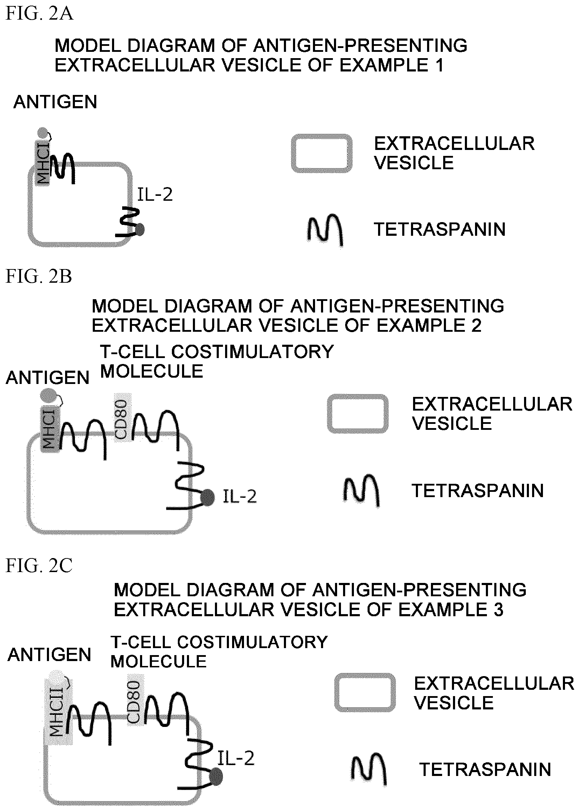

HEK293T cells were seeded in a cell culture dish and cultured in a Dulbecco's modified Eagle medium to which 2% fetal bovine serum and penicillin/streptomycin were added. Cells at about 50% confluence were transfected with two plasmids (pCAG vectors encoding sc-Trimer-CD81 and CD63-IL-2, respectively) at the same time using polyethylenimine "Max" (manufactured by Polysciences Inc.) according to the manufacturer's instructions. The medium was replaced 3 hours after the transfection, and 24 hours after the transfection, the medium was replaced with a Dulbecco's modified Eagle medium to which 2% fetal bovine exosomes-removed serum and penicillin/streptomycin were added. 72 hours after the transfection, supernatant was collected, and then the supernatant was centrifuged at 300 g for 5 minutes after being passed through a 0.22 µm filter. Supernatant was collected, and the supernatant was centrifuged at 2,000 g for 20 minutes. A supernatant was collected, and the supernatant was centrifuged at 10,000 g for 30 minutes. Then the supernatant was removed, and pellets were washed with PBS. After PBS was added to the pellets and the pellets were centrifuged at 100,000 g for 2 hours, supernatant was removed, and the pellets suspended in 100 µL of PBS were used as antigen-presenting extracellular vesicles of Example 1 (Fig. 2A). The concentration of the extracellular vesicles was measured according to the manufacturer's instructions using a BCA protein assay kit (manufactured by Thermo Fisher Scientific Inc.).

### [Example 2] Antigen-Presenting Extracellular Vesicles Containing MHC Class I Molecules, T-Cell Costimulatory Molecules, and T-Cell Stimulatory Cytokines in Membrane

HEK293T cells were seeded in a cell culture dish and cultured in a Dulbecco's modified Eagle medium to which 2% fetal bovine serum and penicillin/streptomycin were added. Cells at about 50% confluence were transfected with the three plasmids (pCAG vectors encoding sc-Trimer-CD81, CD80-CD9, and CD63-IL-2, respectively) prepared above at the same time using polyethylenimine "Max" (manufactured by Polysciences Inc.) according to the manufacturer's instructions. The medium was replaced 3 hours after the transfection, and 24 hours after the transfection, the medium was replaced with a Dulbecco's modified Eagle medium to which 2% fetal bovine exosomes-removed serum and penicillin/streptomycin were added. 72 hours after the transfection, a supernatant was collected, and then the supernatant was centrifuged at 300 g for 5 minutes after being passed through a 0.22 µm filter. A supernatant was collected, and the supernatant was centrifuged at 2,000 g for 20 minutes. A supernatant was collected, and the supernatant was centrifuged at 10,000 g for 30 minutes. After supernatant was collected and the supernatant was centrifuged at 100,000 g for 2 hours, the supernatant was removed, and pellets were washed with PBS. After PBS was added to the pellets and the pellets were centrifuged at 100,000 g for 2 hours, supernatant was removed, and the pellets suspended in 100 µL of PBS were used as antigen-presenting extracellular vesicles of Example 2 (Fig. 2B). The concentration of the antigen-presenting extracellular vesicles was measured according to the manufacturer's instructions using a BCA protein assay kit (manufactured by Thermo Fisher Scientific Inc.).

### [Example 3] Antigen-Presenting Extracellular Vesicles 1 Containing MHC Class II Molecules, T-Cell Costimulatory Molecules, and T-Cell Stimulatory Cytokines in Membrane

HEK293T cells were seeded in a cell culture dish and cultured in a Dulbecco's modified Eagle medium to which 2% fetal bovine serum and penicillin/streptomycin were added. Cells at about 50% confluence were transfected with the four plasmids (pCAG vectors encoding sc-Dimer-CD81, an MHC class IIα chain, CD80-CD9, and CD63-IL-2, respectively) prepared above at the same time using polyethylenimine "Max" (manufactured by Polysciences Inc.) according to the manufacturer's instructions. The medium was replaced 3 hours after the transfection, and 24 hours after the transfection, the medium was replaced with a Dulbecco's modified Eagle medium to which 2% fetal bovine serum from which exosomes were removed and penicillin/streptomycin were added. 72 hours after the transfection, a supernatant was collected, and then the supernatant was centrifuged at 300 g for 5 minutes after being passed through a 0.22 µm filter. Supernatant was collected, and the supernatant was centrifuged at 2,000 g for 20 minutes. Supernatant was collected, and the supernatant was centrifuged at 10,000 g for 30 minutes. Then, supernatant was removed, and pellets were washed with PBS. After PBS was added to the pellets and the pellets were centrifuged at 100,000 g for 2 hours, supernatant was removed, and the pellets suspended in 100 µL of PBS were used as antigen-presenting extracellular vesicles of Example 3 (Fig. 2C). The concentration of the antigen-presenting extracellular vesicles was measured according to the manufacturer's instructions using a BCA protein assay kit (manufactured by Thermo Fisher Scientific Inc.).

### [Example 4] Antigen-Presenting Extracellular Vesicles 2 Containing MHC Class II Molecules, T-Cell Costimulatory Molecules, and T-Cell Stimulatory Cytokines in Membrane

HEK293T cells were seeded in a cell culture dish and cultured in a Dulbecco's modified Eagle medium to which 2% fetal bovine serum and penicillin/streptomycin were added. Cells at about 50% confluence were transfected with five plasmids (pCAG vectors encoding sc-Dimer-CD81, an MHC class IIα chain, CD80-CD9, TGF-β-MFGE8, and CD63-IL-2, respectively) at the same time using polyethylenimine "Max" (manufactured by Polysciences Inc.) according to the manufacturer's instructions. The medium was replaced 3 hours after the transfection, and 24 hours after the transfection, the medium was replaced with a Dulbecco's modified Eagle medium to which 2% fetal bovine exosomes-removed serum and penicillin/streptomycin were added. 72 hours after the transfection, supernatant was collected, and then the supernatant was centrifuged at 300 g for 5 minutes after being passed through a 0.22 µm filter. Supernatant was collected, and the supernatant was centrifuged at 2,000 g for 20 minutes. Supernatant was collected, and the supernatant was centrifuged at 10,000 g for 30 minutes. After a supernatant was collected and the supernatant was centrifuged at 100,000 g for 2 hours, the supernatant was removed, and pellets were washed with PBS. After PBS was added to the pellets and the pellets were centrifuged at 100,000 g for 2 hours, supernatant was removed, and the pellets suspended in 100 µL of PBS were used as antigen-presenting extracellular vesicles of Example 4 (Fig. 2D). The concentration of the antigen-presenting extracellular vesicles was measured according to the manufacturer's instructions using a BCA protein assay kit (manufactured by Thermo Fisher Scientific Inc.).

### [Example 5] Antigen-Presenting Extracellular Vesicles 3 Containing MHC Class II Molecules, T-Cell Costimulatory Molecules, and T-Cell Stimulatory Cytokines in Membrane

HEK293T cells were seeded in a cell culture dish and cultured in a Dulbecco's modified Eagle medium to which 2% fetal bovine serum and penicillin/streptomycin were added. Cells at about 50% confluence were transfected with the four plasmids (pCAG vectors encoding sc-Dimer-CD81, an MHC class IIα chain, CD80-CD9, and CD81-IL-4, respectively) prepared above at the same time using polyethylenimine "Max" (manufactured by Polysciences Inc.) according to the manufacturer's instructions. The medium was replaced 3 hours after the transfection, and 24 hours after the transfection, the medium was replaced with a Dulbecco's modified Eagle medium to which 2% fetal bovine exosomes-removed serum and penicillin/streptomycin were added. 72 hours after the transfection, supernatant was collected, and then the supernatant was centrifuged at 300 g for 5 minutes after being passed through a 0.22 µm filter. Supernatant was collected, and the supernatant was centrifuged at 2,000 g for 20 minutes. Supernatant was collected, and the supernatant was centrifuged at 10,000 g for 30 minutes. After supernatant was collected and the supernatant was centrifuged at 100,000 g for 2 hours, the supernatant was removed, and pellets were washed with PBS. After PBS was added to the pellets and the pellets were centrifuged at 100,000 g for 2 hours, a supernatant was removed, and the pellets suspended in 100 µL of PBS were used as antigen-presenting extracellular vesicles of Example 5 (Fig. 2E). The concentration of the antigen-presenting extracellular vesicles was measured according to the manufacturer's instructions using a BCA protein assay kit (manufactured by Thermo Fisher Scientific Inc.).

### [Reference Example 1] Control Extracellular Vesicles

HEK293T cells were seeded in a cell culture dish and cultured in a Dulbecco's modified Eagle medium to which 2% fetal bovine serum and penicillin/streptomycin were added. The medium was replaced with cells at about 50% confluence, and after 24 hours, the medium was replaced with a Dulbecco's modified Eagle medium to which 2% fetal bovine exosomes-removed serum and penicillin/streptomycin were added. 48 hours after the replacement with the medium from which exosomes were removed, supernatant was collected, and then the supernatant was centrifuged at 300 g for 5 minutes after being passed through a 0.22 µm filter. Supernatant was collected, and the supernatant was centrifuged at 2,000 g for 20 minutes. Supernatant was collected, and the supernatant was centrifuged at 10,000 g for 30 minutes. After a supernatant was collected and the supernatant was centrifuged at 100,000 g for 2 hours, the supernatant was removed, and pellets were washed with PBS. After PBS was added to the pellets and the pellets were centrifuged at 100,000 g for 2 hours, a supernatant was removed, and the pellets suspended in 100 µL of PBS were used as extracellular vesicles of Reference Example 1. The concentration of the antigen-presenting extracellular vesicles was measured according to the manufacturer's instructions using a BCA protein assay kit (manufactured by Thermo Fisher Scientific Inc.).

### [Reference Example 2] Extracellular Vesicles Containing MHC Class I Molecules in Membrane

HEK293T cells were seeded in a cell culture dish and cultured in a Dulbecco's modified Eagle medium to which 2% fetal bovine serum and penicillin/streptomycin were added. Cells at about 50% confluence were transfected with a plasmid (a pCAG vector encoding sc-Trimer-CD81) using polyethylenimine "Max" (manufactured by Polysciences Inc.) according to manufacturer's instructions. The medium was replaced 3 hours after the transfection, and 24 hours after the transfection, the medium was replaced with a Dulbecco's modified Eagle medium to which 2% fetal bovine exosomes-removed serum and penicillin/streptomycin were added. 72 hours after the transfection, supernatant was collected, and then the supernatant was centrifuged at 300 g for 5 minutes after being passed through a 0.22 µm filter. Supernatant was collected, and the supernatant was centrifuged at 2,000 g for 20 minutes. Supernatant was collected, and the supernatant was centrifuged at 10,000 g for 30 minutes. After a supernatant was collected and the supernatant was centrifuged at 100,000 g for 2 hours, the supernatant was removed, and pellets were washed with PBS. After PBS was added to the pellets and the pellets were centrifuged at 100,000 g for 2 hours, supernatant was removed, and the pellets suspended in 100 µL of PBS were used as extracellular vesicles of Reference Example 2. The concentration of the extracellular vesicles was measured according to the manufacturer's instructions using a BCA protein assay kit (manufactured by Thermo Fisher Scientific Inc.).

### [Reference Example 3] Extracellular Vesicles Containing T-Cell Costimulatory Molecules in Membrane

HEK293T cells were seeded in a cell culture dish and cultured in a Dulbecco's modified Eagle medium to which 2% fetal bovine serum and penicillin/streptomycin were added. Cells at about 50% confluence were transfected with a plasmid (a pCAG vector encoding CD80-CD9) using polyethylenimine "Max" (manufactured by Polysciences Inc.) according to manufacturer's instructions. The medium was replaced 3 hours after the transfection, and 24 hours after the transfection, the medium was replaced with a Dulbecco's modified Eagle medium to which 2% fetal bovine exosomes-removed serum and penicillin/streptomycin were added. 72 hours after the transfection, supernatant was collected, and then the supernatant was centrifuged at 300 g for 5 minutes after being passed through a 0.22 µm filter. Supernatant was collected, and the supernatant was centrifuged at 2,000 g for 20 minutes. Supernatant was collected, and the supernatant was centrifuged at 10,000 g for 30 minutes. After supernatant was collected and the supernatant was centrifuged at 100,000 g for 2 hours, the supernatant was removed, and pellets were washed with PBS. After PBS was added to the pellets and the pellets were centrifuged at 100,000 g for 2 hours, supernatant was removed, and the pellets suspended in 100 µL of PBS were used as extracellular vesicles of Reference Example 3. The concentration of the extracellular vesicles was measured according to the manufacturer's instructions using a BCA protein assay kit (manufactured by Thermo Fisher Scientific Inc.).

### [Reference Example 4] Extracellular Vesicles Containing T-Cell Stimulatory Cytokines in Membrane

HEK293T cells were seeded in a cell culture dish and cultured in a Dulbecco's modified Eagle medium to which 2% fetal bovine serum and penicillin/streptomycin were added. Cells at about 50% confluence were transfected with a plasmid (a pCAG vector encoding CD63-IL-2) using polyethylenimine "Max" (Polysciences Inc.) according to manufacturer's instructions. The medium was replaced 3 hours after the transfection, and 24 hours after the transfection, the medium was replaced with a Dulbecco's modified Eagle medium to which 2% fetal bovine exosomes-removed serum and penicillin/streptomycin were added. 72 hours after the transfection, supernatant was collected, and then the supernatant was centrifuged at 300 g for 5 minutes after being passed through a 0.22 µm filter. Supernatant was collected, and the supernatant was centrifuged at 2,000 g for 20 minutes. Supernatant was collected, and the supernatant was centrifuged at 10,000 g for 30 minutes. After supernatant was collected and the supernatant was centrifuged at 100,000 g for 2 hours, the supernatant was removed, and pellets were washed with PBS. After PBS was added to the pellets and the pellets were centrifuged at 100,000 g for 2 hours, supernatant was removed, and the pellets suspended in 100 µL of PBS were used as extracellular vesicles of Reference Example 4. The concentration of the extracellular vesicles was measured according to the manufacturer's instructions using a BCA protein assay kit (manufactured by Thermo Fisher Scientific Inc.).

### [Reference Example 5] Extracellular Vesicles Containing MHC Class I Molecules and T-Cell Costimulatory Molecules in Membrane

HEK293T cells were seeded in a cell culture dish and cultured in a Dulbecco's modified Eagle medium to which 2% fetal bovine serum and penicillin/streptomycin were added. Cells at about 50% confluence were transfected with two plasmids (pCAG vectors encoding sc-Trimer-CD81 and CD80-CD9, respectively) at the same time using polyethylenimine "Max" (manufactured by Polysciences Inc.) according to the manufacturer's instructions. The medium was replaced 3 hours after the transfection, and 24 hours after the transfection, the medium was replaced with a Dulbecco's modified Eagle medium to which 2% fetal bovine exosomes-removed serum and penicillin/streptomycin were added. 72 hours after the transfection, supernatant was collected, and then the supernatant was centrifuged at 300 g for 5 minutes after being passed through a 0.22 µm filter. Supernatant was collected, and the supernatant was centrifuged at 2,000 g for 20 minutes. Supernatant was collected, and the supernatant was centrifuged at 10,000 g for 30 minutes. After a supernatant was collected and the supernatant was centrifuged at 100,000 g for 2 hours, the supernatant was removed, and pellets were washed with PBS. After PBS was added to the pellets and the pellets were centrifuged at 100,000 g for 2 hours, supernatant was removed, and the pellets suspended in 100 µL of PBS were used as extracellular vesicles of Reference Example 5. The concentration of the extracellular vesicles was measured according to the manufacturer's instructions using a BCA protein assay kit (manufactured by Thermo Fisher Scientific Inc.).

### Test Example 1-1: Flow Cytometry Analysis of Fusion Protein Contained in Membrane of Extracellular Vesicle

The antigen-presenting extracellular vesicles of Example 2 were immunostained by a PS Capture (trademark) exosome flow cytometry kit (manufactured by FUJIFILM Wako Pure Chemical Corporation) according to the manufacturer's instruction. Antibodies used for staining are as follows (staining time: 15 minutes, temperature: 4°C). After the staining, expression of each fusion protein was detected with a flow cytometer FACSCantoII (manufactured by BD Biosciences).
- APC-conjugated anti-mouse H-2KbOVA complex antibody (25-D1.16, manufactured by Biolegend, Inc.)
- PE-conjugated anti-mouse CD80 antibody (16-10A1, manufactured by Biolegend, Inc.)
- Brilliant Violet421-conjugated anti-mouse IL-2 antibody (JES6-5H4, manufactured by Biolegend, Inc.)

The results are illustrated in Fig. 3A.

### [Results]

From the results of Test Example 1-1, it could be seen that MHC class I molecules presenting OVA antigens, CD80, and IL-2 were contained in the membrane of the antigen-presenting extracellular vesicle of Example 2 (Fig. 3A).

### Test Example 1-2: Flow Cytometry Analysis of Fusion Protein Contained in Membrane of Extracellular Vesicle

The antigen-presenting extracellular vesicles of Example 3 were immunostained by a PS Capture (trademark) exosome flow cytometry kit (manufactured by FUJIFILM Wako Pure Chemical Corporation) according to the manufacturer's instruction. The antibodies used for the staining are as follows. After the staining, expression of each fusion protein was detected with a flow cytometer FACSCantoII (manufactured by BD Biosciences).
- APC-conjugated anti-mouse IL-2 antibody (JES6-5H4, manufactured by Biolegend, Inc.)
- PE-conjugated anti-mouse CD80 antibody (16-10A1, manufactured by Biolegend, Inc.)
- APC-Cy7-conjugated anti-mouse I-A/I-E antibody (M5/114.15.2, manufactured by Biolegend, Inc.)

The results are illustrated in Fig. 3B.

### [Results]

From the results of Test Example 1-2, it could be seen that MHC class II molecules presenting OVA antigens, CD80, and IL-2 were contained in the membrane of the antigen-presenting extracellular vesicle of Example 3 (Fig. 3B).

### Test Example 1-3: Flow Cytometry Analysis of Fusion Protein Contained in Membrane of Extracellular Vesicle

The antigen-presenting extracellular vesicles of Example 4 were immunostained by a PS Capture (trademark) exosome flow cytometry kit (manufactured by FUJIFILM Wako Pure Chemical Corporation) according to the manufacturer's instruction. The antibodies used for the staining are as follows. After the staining, expression of each fusion protein was detected with a flow cytometer FACSCantoII (manufactured by BD Biosciences).
- APC-conjugated anti-mouse IL-2 antibody (JES6-5H4, manufactured by Biolegend, Inc.)
- PE-conjugated anti-mouse CD80 antibody (16-10A1, manufactured by Biolegend, Inc.)
- APC-Cy7-conjugated anti-mouse I-A/I-E antibody (M5/114.15.2, manufactured by Biolegend, Inc.)
- APC-conjugated anti-mouse LAP (TGF-β1) antibody (TW7-16B4, manufactured by Biolegend, Inc.)

The results are illustrated in Fig. 3C.

### [Results]

From the results of Test Example 1-3, it could be seen that MHC class II molecules presenting OVA antigens, CD80, IL-2, and TGF-β1 were contained in the membrane of the antigen-presenting extracellular vesicle of Example 4 (Fig. 3C).

### Test Example 1-4: Flow Cytometry Analysis of Fusion Protein Contained in Membrane of Extracellular Vesicle

The antigen-presenting extracellular vesicles of Example 5 were immunostained by a PS Capture (trademark) exosome flow cytometry kit (manufactured by FUJIFILM Wako Pure Chemical Corporation) according to the manufacturer's instruction. The antibodies used for the staining are as follows. After the staining, expression of each fusion protein was detected with a flow cytometer FACSCantoII (manufactured by BD Biosciences).
- Alexa Fluor 488-conjugated anti-mouse IL-4 antibody (11B11, manufactured by Biolegend, Inc.)
- PE-conjugated anti-mouse CD80 antibody (16-10A1, manufactured by Biolegend, Inc.)
- APC-Cy7-conjugated anti-mouse I-A/I-E antibody (M5/114.15.2, manufactured by Biolegend, Inc.)

The results are illustrated in Fig. 3D.

### [Results]

From the results of Test Example 1-4, it could be seen that MHC class II molecules presenting OVA antigens, CD80, and IL-4 were contained in the membrane of the antigen-presenting extracellular vesicle of Example 5 (Fig. 3D).

### Test Example 2: Experiment on Activation of OVA-Specific CD8-Positive T Cells (OT-1 T Cells) In Vitro by Antigen-Presenting Extracellular Vesicles

The following test was conducted in vitro to determine whether the antigen-presenting extracellular vesicles activate antigen-specific CD8-positive T cells.

Lymph nodes extracted from an OT-1 mouse, which was an OVA-reactive TCR transgenic mouse, were disrupted on a 100 µm filter to obtain a lymph node cell suspension. The cell suspension was stained using CellTrace Violet (manufactured by Thermo Fisher Scientific Inc.) as a cell proliferation assay reagent according to the manufacturer's instructions. 2 × 10⁵ stained lymph node cells were suspended in 200 µL of an RPMI1640 medium to which 10% fetal bovine serum, 50 µM 2-mercaptoethanol, and penicillin/streptomycin were added, the antigen-presenting extracellular vesicles of Example 1 or 2 (final concentration: 3 µg/mL), a mixture of three types of the extracellular vesicles of Reference Examples 2 to 4 (final concentration of each of the three types of the extracellular vesicles: 3 µg/mL), or the extracellular vesicles of Reference Examples 1, 2, or 5 (final concentration: 3 µg/mL) were added, culture was performed in a 96 well round bottom plate for 3 days, and then, immunostaining was performed. Antibodies used for staining are as follows (staining time: 15 minutes, temperature: 4°C). After the staining, a luminescence intensity of CellTrace Violet as a cell proliferation assay reagent in the OT-1 T cells was detected with a flow cytometer FACSCantoII (manufactured by BD Biosciences).
- APC-conjugated anti-mouse CD8 antibody (53-6.7, manufactured by Biolegend, Inc.)
- PE-conjugated anti-mouse TCR Vb5.1,5.2 antibody (MR9-4, manufactured by Biolegend, Inc.)

The results are illustrated in Fig. 4.

### [Results]

From the results of Test Example 2, it was confirmed that the antigen-presenting extracellular vesicles of Examples 1 and 2 remarkably differentiated and/or proliferated antigen-specific CD8-positive T cells in comparison with the mixture of the three types of the extracellular vesicles of Reference Examples 2 to 4 or the extracellular vesicles of Reference Examples 1, 2, and 5 (Fig. 4).

### Test Example 3: Experiment on Activation of OVA-Specific CD8-Positive T Cells (OT-1 T Cells) In Vivo by Antigen-Presenting Extracellular Vesicles

The following test was conducted in vivo to determine whether the antigen-presenting extracellular vesicles activate antigen-specific CD8-positive T cells.

Lymph nodes were extracted from an OT-1 mouse, which was OVA-reactive TCR transgenic mouse, and the same lymphocyte suspension as that of Test Example 2 was prepared. Lymph nodes were similarly extracted from a CD45.1 congenic mouse, and a lymphocyte suspension was prepared. The respective lymphocyte suspensions were mixed at a ratio of 1:1, and the mixture was stained using CellTrace Violet as a cell proliferation assay reagent. 1 × 10⁷ CellTrace Violet-stained mixed lymphocyte suspension suspended in PBS was transferred from the tail vein of the CD45.1/CD45.2 congenic mouse. The next day, a mixture (IL-2/anti-IL-2 antibody complex) of 50 µg of the antigen-presenting extracellular vesicles of Example 2 or the extracellular vesicles of Reference Example 1, or 1.5 µg of IL-2 (manufactured by Biolegend, Inc.) and 50 µg of anti-mouse IL-2 antibodies (S4B6-1, manufactured by Bio X Cell) was transferred to from the tail vein into a CD45.1/CD45.2 congenic mouse. 4 days after cell transfer, lymph nodes were extracted from the recipient mouse, and a lymphocyte suspension was prepared and immunostained. Antibodies used for staining are as follows (staining time: 15 minutes, temperature: 4°C). After the staining, a luminescence intensity of CellTrace Violet as a cell proliferation assay reagent in the transferred OT-1 T cells and wild-type CD8T cells was detected with a flow cytometer FACSCantoII (manufactured by BD Biosciences).
- PE-Cy7-conjugated anti-mouse CD8 antibody (53-6.7, manufactured by Biolegend, Inc.)
- PE-conjugated anti-mouse TCR Vb5.1,5.2 antibody (MR9-4, manufactured by Biolegend, Inc.)
- FITC-conjugated anti-mouse CD45.1 antibody (A20, manufactured by Biolegend, Inc.)
- APC-conjugated anti-mouse CD45.2 antibody (104, manufactured by Biolegend, Inc.)

The results are illustrated in Fig. 5.

### [Results]

From the results of Test Example 3, it could be seen that the antigen-presenting extracellular vesicles of Example 2 hardly activated other CD8-positive T cells (antigen-non-specific CD8-positive T cells) and remarkably differentiated and/or proliferated antigen-specific CD8-positive T cells in vivo in comparison with the extracellular vesicles of Reference Example 1 (Fig. 5). In addition, it is possible that serious side effects such as cytokine storm are low because the antigen-presenting extracellular vesicles of Example 2 hardly activate other CD8-positive T cells (antigen-non-specific CD8-positive T cells) in comparison with the IL-2/anti-IL-2 antibody complex (Fig. 5).

### Test Example 4: Experiment on Activation of OVA-Specific CD4-Positive T Cells (OT-2 T Cells) In Vitro by Antigen-Presenting Extracellular Vesicles

The following test was conducted in vitro to determine whether the antigen-presenting extracellular vesicles activate antigen-specific CD4-positive T cells.

Lymph nodes extracted from an OT-2 mouse, which was an OVA-reactive CD4TCR transgenic mouse, were disrupted on a 100 µm filter to obtain a lymph node cell suspension. The cell suspension was stained using CellTrace Violet (manufactured by Thermo Fisher Scientific Inc.) as a cell proliferation assay reagent according to the manufacturer's instructions. 2 × 10⁵ stained lymph node cells were suspended in 200 µL of an RPMI1640 medium to which 10% fetal bovine serum, 50 µM 2-mercaptoethanol, and penicillin/streptomycin were added, the antigen-presenting extracellular vesicles of Example 3 or the extracellular vesicles of Reference Example 1 were added so that the final concentration was 10 µg/mL, and culture was performed in a 96 well round bottom plate for 4 days. After 4 days, the cells were recovered and immunostained. Antibodies used for staining are as follows (staining time: 15 minutes, temperature: 4°C). After the staining, a luminescence intensity of CellTrace Violet as a cell proliferation assay reagent in the OT-2 T cells was detected with a flow cytometer FACSCantoII (manufactured by BD Biosciences).
- PE-Cy7-conjugated anti-mouse CD4 antibody (RM4-5, manufactured by Biolegend, Inc.)
- APC-conjugated anti-mouse TCR Vb5.1,5.2 antibody (MR9-4, manufactured by Biolegend, Inc.)

The results are illustrated in Fig. 6.

### [Results]

From the results of Test Example 4, it was confirmed that the antigen-presenting extracellular vesicles of Example 3 remarkably differentiated and/or proliferated antigen-specific CD4 T cells in comparison with the extracellular vesicles of Reference Example 1 (Fig. 6).

### Test Example 5: Experiment on Differentiation Induction of OVA-Specific CD4-Positive T Cells (OT-2 T Cells) In Vitro into Regulatory T Cells by Antigen-Presenting Extracellular Vesicles

The following test was conducted in vitro to determine whether the antigen-presenting extracellular vesicles induce antigen-specific CD4-positive T cells into regulatory T cells (Treg).

Lymph nodes extracted from an OT-2 mouse, which was an OVA-reactive CD4TCR transgenic mouse, were disrupted on a 100 µm filter to obtain a lymph node cell suspension. The cell suspension was stained using CellTrace Violet (manufactured by Thermo Fisher Scientific Inc.) as a cell proliferation assay reagent according to the manufacturer's instructions. 2 × 10⁵ stained lymph node cells were suspended in 200 µL of an RPMI1640 medium to which 10% fetal bovine serum, 50 µM 2-mercaptoethanol, and penicillin/streptomycin were added, the antigen-presenting extracellular vesicles of Example 4 or the extracellular vesicles of Reference Example 1 were added so that the final concentration was 10 µg/mL, and culture was performed in a 96 well round bottom plate for 4 days. After 4 days, the cells were recovered, and extracellular immunostaining was performed. Antibodies used for staining are as follows (staining time: 15 minutes, temperature: 4°C). After the extracellular staining, intracellular immunostaining was performed using True-Nuclear Transcription Factor Buffer Set (manufactured by Biolegend, Inc.) and anti-mouse FOXP3 antibodies according to the manufacturer's instructions. After the intracellular staining, expression of CD25 molecules and FOXP3 molecules as markers of regulatory T cells on the OT-2 T cells was detected with a flow cytometer FACSCantoII (manufactured by BD Biosciences).
- PE-conjugated anti-mouse CD25 antibody (PC61, manufactured by Biolegend, Inc.)
- PE-Cy7-conjugated anti-mouse CD4 antibody (RM4-5, manufactured by Biolegend, Inc.)
- APC-conjugated anti-mouse TCR Vb5.1,5.2 antibody (MR9-4, manufactured by Biolegend, Inc.)
- Alexa Fluor 488-conjugated anti-mouse FOXP3 antibody (MF-14, manufactured by Biolegend, Inc.)

The results are illustrated in Fig. 7.

### [Results]

From the results of Test Example 5, it could be seen that the antigen-presenting extracellular vesicles of Example 4 induced differentiation of the antigen-specific CD4-positive T cells into regulatory T cells (preferably, regulatory T cells expressing Foxp3) in comparison with the extracellular vesicles of Reference Example 1 (Fig. 7).

### Test Example 6: Experiment on Differentiation Induction of OVA-Specific CD4-Positive T Cells (OT-2 T Cells) In Vitro into Th2T Cells by Antigen-Presenting Extracellular Vesicles

The following test was conducted in vitro to determine whether antigen-presenting extracellular vesicles induce differentiation of antigen-specific CD4-positive T cells into Th2T cells.

Lymph nodes extracted from an OT-2 mouse, which was an OVA-reactive CD4TCR transgenic mouse, were disrupted on a 100 µm filter to obtain a lymph node cell suspension. The cell suspension was stained using CellTrace Violet (manufactured by Thermo Fisher Scientific Inc.) as a cell proliferation assay reagent according to the manufacturer's instructions. 2 × 10⁵ stained lymph node cells were suspended in 200 µL of an RPMI1640 medium to which 10% fetal bovine serum, 50 µM 2-mercaptoethanol, and penicillin/streptomycin were added, the antigen-presenting extracellular vesicles of Example 3 or 5 or the extracellular vesicles of Reference Example 1 were added so that the final concentration was 10 µg/mL, and culture was performed in a 96 well round bottom plate for 4 days. After 4 days, the cells were recovered, and extracellular immunostaining was performed. Antibodies used for staining are as follows (staining time: 15 minutes, temperature: 4°C). After the extracellular staining, intracellular immunostaining was performed using True-Nuclear Transcription Factor Buffer Set (manufactured by Biolegend, Inc.) and anti-GATA3 antibodies according to the manufacturer's instructions. After the intracellular staining, a luminescence intensity of CellTrace Violet as a cell proliferation assay reagent in the OT-2 T cells and expression of GATA3 as a marker of Th2T cells were detected with a flow cytometer FACSCantoII (manufactured by BD Biosciences).
- PE-conjugated anti-mouse TCR Vb5.1,5.2 antibody (MR9-4, manufactured by Biolegend, Inc.)
- PE-Cy7-conjugated anti-mouse CD4 antibody (RM4-5, manufactured by Biolegend, Inc.)
- APC-conjugated anti-GATA3 antibody (16E10A23, manufactured by Biolegend, Inc.)

The results are illustrated in Fig. 8.

### [Results]

From the results of Test Example 6, it could be seen that the antigen-presenting extracellular vesicles of Examples 3 and 5 induced differentiation of antigen-specific CD4-positive T cells into Th2 cells in vitro in comparison with the extracellular vesicles of Reference Example 1 (Fig. 8). The Th2 cells secrete cytokines such as IL-4 or IL-5, activate differentiation of naive B cells that recognize the same antigen, and promote induction of antigen-specific IgE production (that is, activation of humoral immunity).

### [Example 6] Antigen-Presenting Extracellular Vesicles 4 Containing MHC Class II Molecules, T-Cell Costimulatory Molecules, and T-Cell Stimulatory Cytokines in Membrane

HEK293T cells were seeded in a cell culture dish and cultured in a Dulbecco's modified Eagle medium to which 2% fetal bovine serum and penicillin/streptomycin were added. Cells at about 50% confluence were transfected with the four plasmids (pCAG vectors encoding sc-Dimer-CD81-IL-12p40, an MHC class IIα chain, CD80-CD9, and IL-12p35, respectively) prepared above at the same time using polyethylenimine "Max" (manufactured by Polysciences Inc.) according to the manufacturer's instructions. 3 to 12 hours after the transfection, the medium was replaced, and 24 hours after the transfection, the medium was replaced with a Dulbecco's modified Eagle medium to which 2% fetal bovine exosomes-removed serum and penicillin/streptomycin were added. 72 hours after the transfection, a supernatant was collected, and then the supernatant was centrifuged at 300 g for 5 minutes after being passed through a 0.22 µm filter. Supernatant was collected, and the supernatant was centrifuged at 2,000 g for 20 minutes. Supernatant was collected, and the supernatant was centrifuged at 10,000 g for 30 minutes. After supernatant was collected and the supernatant was centrifuged at 100,000 g for 2 hours, the supernatant was removed, and pellets were washed with PBS. After PBS was added to the pellets and the pellets were centrifuged at 100,000 g for 2 hours, supernatant was removed, and the pellets suspended in 100 µL of PBS were used as antigen-presenting extracellular vesicles of Example 6. The concentration of the antigen-presenting extracellular vesicles was measured according to the manufacturer's instructions using a BCA protein assay kit (manufactured by Thermo Fisher Scientific Inc.).

### Test Example 1-5: Flow Cytometry Analysis of Fusion Protein Contained in Membrane of Extracellular Vesicle

The antigen-presenting extracellular vesicles of Example 6 were immunostained by a PS Capture (trademark) exosome flow cytometry kit (manufactured by FUJIFILM Wako Pure Chemical Corporation) according to the manufacturer's instruction. The antibodies used for the staining are as follows. After the staining, expression of each fusion protein was detected with a flow cytometer FACSCantoII (manufactured by BD Biosciences).
- Alexa Fluor 488-conjugated anti-mouse I-A/I-E antibody M5/114.15.2, manufactured by Biolegend, Inc.)
- PE-conjugated anti-mouse IL-12 antibody (C15.6, manufactured by Biolegend, Inc.)
- The results of APC-conjugated anti-mouse CD80 antibody (16-10A1, manufactured by Biolegend, Inc.) are illustrated in Fig. 3E.

### [Results]

From the results of Test Example 1-5, it could be confirmed that MHC class II molecules presenting OVA antigens, CD80, and functional IL-12 were contained in the membrane of the antigen-presenting extracellular vesicle of Example 6 (Fig. 3E).

### [Example 7] Antigen-Presenting Extracellular Vesicles 5 Containing MHC Class II Molecules, T-Cell Costimulatory Molecules, and T-Cell Stimulatory Cytokines in Membrane

HEK293T cells were seeded in a cell culture dish and cultured in a Dulbecco's modified Eagle medium to which 2% fetal bovine serum and penicillin/streptomycin were added. Cells at about 50% confluence were transfected with the five plasmids (pCAG vectors encoding sc-Dimer-CD81, an MHC class IIα chain, CD80-CD9, CD81-IL-6, and TGF-β-MFGE8, respectively) prepared above at the same time using polyethylenimine "Max" (manufactured by Polysciences Inc.) according to the manufacturer's instructions. 3 to 12 hours after the transfection, the medium was replaced, and 24 hours after the transfection, the medium was replaced with a Dulbecco's modified Eagle medium to which 2% fetal bovine exosomes-removed serum and penicillin/streptomycin were added. 72 hours after the transfection, a supernatant was collected, and then the supernatant was centrifuged at 300 g for 5 minutes after being passed through a 0.22 µm filter. Supernatant was collected, and the supernatant was centrifuged at 2,000 g for 20 minutes. Supernatant was collected, and the supernatant was centrifuged at 10,000 g for 30 minutes. After supernatant was collected and the supernatant was centrifuged at 100,000 g for 2 hours, the supernatant was removed, and pellets were washed with PBS. After PBS was added to the pellets and the pellets were centrifuged at 100,000 g for 2 hours, supernatant was removed, and the pellets suspended in 100 µL of PBS were used as antigen-presenting extracellular vesicles of Example 7. The concentration of the antigen-presenting extracellular vesicles was measured according to the manufacturer's instructions using a BCA protein assay kit (manufactured by Thermo Fisher Scientific Inc.).

### Test Example 1-6: Flow Cytometry Analysis of Fusion Protein Contained in Membrane of Extracellular Vesicle

The antigen-presenting extracellular vesicles of Example 7 were immunostained by a PS Capture (trademark) exosome flow cytometry kit (manufactured by FUJIFILM Wako Pure Chemical Corporation) according to the manufacturer's instruction. The antibodies used for the staining are as follows. After the staining, expression of each fusion protein was detected with a flow cytometer FACSCantoII (manufactured by BD Biosciences).
- FITC-conjugated anti-mouse CD80 antibody (16-10A1, manufactured by Biolegend, Inc.)
- PE-conjugated anti-mouse IL-6 antibody (MP5-20F3, manufactured by Biolegend, Inc.)
- APC-Cy7-conjugated anti-mouse I-A/I-E antibody (M5/114.15.2, manufactured by Biolegend, Inc.)
- APC-conjugated anti-mouse LAP (TGF-β1) antibody (TW7-16B4, manufactured by Biolegend, Inc.)

The results are illustrated in Fig. 3F.

### [Results]

From the results of Test Example 1-6, it could be seen that MHC class II molecules presenting OVA antigens, CD80, IL-6, and TGFb were contained in the membrane of the antigen-presenting extracellular vesicle of Example 7 (Fig. 3F).

### [Example 8] Establishment of Cell Strain Stably Expressing MHC Class I Molecules, T-Cell Costimulatory Molecules, and T-Cell Stimulatory Cytokines and Preparation of Antigen-Presenting Extracellular Vesicles

PLAT-A cells were seeded in a cell culture dish and cultured in a Dulbecco's modified Eagle medium to which 2% fetal bovine serum and penicillin/streptomycin were added. Cells at about 50% confluence were transfected with a pMX vector encoding CD80-CD9 or sc-Trimer-CD81-IL-2 using polyethylenimine "Max" (manufactured by Polysciences Inc.) according to manufacturer's instructions. 12 hours after the transfection, the medium was replaced, and 60 hours after the transfection, a supernatant was collected and centrifuged at 300 g for 5 minutes. The collected supernatant was used as virus particles. HEK293 cells were seeded in a cell culture dish and cultured in a Dulbecco's modified Eagle medium to which 2% fetal bovine serum and penicillin/streptomycin were added. A DOTAP transfection reagent (Roche) was added to viral particles in which the CD80-CD9 adjusted above was incorporated into the cells at about 50% confluence according to the manufacturer's instructions, and the mixture was added to HEK293 cells. The cells to which the viral particles were added were centrifuged at 2,500 rpm for 3 minutes. 24 hours after the transfection, the medium was replaced, and 1 week after the transfection, CD80-positive cells were sorted with FACSMelody (manufactured by BD Biosciences). The sorted CD80-positive cells were cultured for 1 week, and the cultured cells were seeded in a dish and cultured in a Dulbecco's modified Eagle medium to which 2% fetal bovine serum and penicillin/streptomycin were added. A DOTAP transfection reagent was added to viral particles in which the sc-Trimer-CD81-IL-2 prepared above was incorporated into the cells at about 50% confluence according to the manufacturer's instructions, and the mixture was added to CD80-positive HEK293 cells. The cells to which the viral particles were added were centrifuged at 2,500 rpm for 3 minutes. 24 hours after the transfection, the medium was replaced, and 1 week after the transfection, CD80-positive and MHCI-positive cells were sorted with FACSMelody (manufactured by BD Biosciences). The sorted cells were used as stable expression cells. The stable expression cells were seeded in a dish and cultured in a Dulbecco's modified Eagle medium to which 2% fetal bovine serum and penicillin/streptomycin were added. The supernatant of the cells at about 50% confluence was replaced with a Dulbecco's modified Eagle medium to which 2% fetal bovine exosomes-removed and penicillin/streptomycin were added. 72 hours after the medium replacement, supernatant was collected, and then the supernatant was centrifuged at 300 g for 5 minutes after being passed through a 0.22 µm filter. Supernatant was collected, and the supernatant was centrifuged at 2,000 g for 20 minutes. Supernatant was collected, and the supernatant was centrifuged at 10,000 g for 30 minutes. After supernatant was collected and the supernatant was centrifuged at 100,000 g for 2 hours, the supernatant was removed, and pellets were washed with PBS. After PBS was added to the pellets and the pellets were centrifuged at 100,000 g for 2 hours, supernatant was removed, and the pellets suspended in 100 µL of PBS were used as antigen-presenting extracellular vesicles of Example 8.

### Test Example 1-7: Flow Cytometry Analysis of Fusion Protein Contained in Membrane of Extracellular Vesicle

The antigen-presenting extracellular vesicles of Example 8 were immunostained by a PS Capture (trademark) exosome flow cytometry kit (manufactured by FUJIFILM Wako Pure Chemical Corporation) according to the manufacturer's instruction. Antibodies used for staining are as follows (staining time: 15 minutes, temperature: 4°C). After the staining, expression of each fusion protein was detected with a flow cytometer FACSCantoII (manufactured by BD Biosciences).
- PE-conjugated anti-mouse H-2KbOVA complex antibody (25-D1.16, manufactured by Biolegend, Inc.)
- FITC-conjugated anti-mouse CD80 antibody (16-10A1, manufactured by Biolegend, Inc.)
- APC-conjugated anti-mouse IL-2 antibody (JES6-5H4, manufactured by Biolegend, Inc.)

The results are illustrated in Fig. 3G.

### [Results]

From the results of Test Example 1-7, it could be confirmed that MHC class I molecules presenting OVA antigens, CD80, and IL-2 were contained in the membrane of the antigen-presenting extracellular vesicle of Example 8 (Fig. 3G).

### [Example 9] Antigen-Presenting Extracellular Vesicles Containing HLA Class I Molecules, Human T-Cell Costimulatory Molecules, and Human T-Cell Stimulatory Cytokines in Membrane

HEK293T cells in which B2m was deleted were seeded in a cell culture dish and cultured in a Dulbecco's modified Eagle medium to which 2% fetal bovine serum and penicillin/streptomycin were added. Cells at about 50% confluence were transfected with the two plasmids (pCAG vectors encoding HLAsc-Trimer-human CD81, human CD80-human CD9, and CD63-IL2, respectively) prepared above at the same time using polyethylenimine "Max" (manufactured by Polysciences Inc.) according to the manufacturer's instructions. 3 to 12 hours after the transfection, the medium was replaced, and 24 hours after the transfection, the medium was replaced with a Dulbecco's modified Eagle medium to which 2% fetal bovine exosomes-removed serum and penicillin/streptomycin were added. 72 hours after the transfection, supernatant was collected, and then the supernatant was centrifuged at 300 g for 5 minutes after being passed through a 0.22 µm filter. Supernatant was collected, and the supernatant was centrifuged at 2,000 g for 20 minutes. Supernatant was collected, and the supernatant was centrifuged at 10,000 g for 30 minutes. After supernatant was collected and the supernatant was centrifuged at 100,000 g for 2 hours, the supernatant was removed, and pellets were washed with PBS. After PBS was added to the pellets and the pellets were centrifuged at 100,000 g for 2 hours, supernatant was removed, and the pellets suspended in 100 µL of PBS were used as human antigen-presenting extracellular vesicles of Example 9. The concentration of the antigen-presenting extracellular vesicles was measured according to the manufacturer's instructions using a BCA protein assay kit (manufactured by Thermo Fisher Scientific Inc.).

By using SARS-CoV2sc-Trimer-hCD81 instead of hsc-Trimer-hCD81, it is possible to prepare human antigen-presenting extracellular vesicles presenting antigen-presenting MHC molecules presenting SARS-CoV2 peptides as antigens, hCD80, and hIL-2 on a surface thereof.

### Test Example 1-8: Flow Cytometry Analysis of Fusion Protein Contained in Membrane of Extracellular Vesicle

The antigen-presenting extracellular vesicles of Example 9 were immunostained by a PS Capture (trademark) exosome flow cytometry kit (manufactured by FUJIFILM Wako Pure Chemical Corporation) according to the manufacturer's instruction. Antibodies used for staining are as follows (staining time: 15 minutes, temperature: 4°C). After the staining, expression of each fusion protein was detected with a flow cytometer FACSCantoII (manufactured by BD Biosciences).
- APC-conjugated anti-human IL-2 antibody (MQ1-17H12, manufactured by Biolegend, Inc.)
- PE-conjugated anti-human CD80 antibody (2D10, manufactured by Biolegend, Inc.)
- APC-conjugated anti-human β2m antibody (2M2, manufactured by Biolegend, Inc.)

The results are illustrated in Fig. 3H.

### [Results]

From the results of Test Example 1-8, it could be confirmed that MHC class I molecules presenting WT1 antigens, hCD80, and hIL-2 were contained in the membrane of the antigen-presenting extracellular vesicle of Example 9 (Fig. 3H).

### Test Example 7: Experiment on Differentiation Induction of OVA-Specific CD4-Positive T Cells (OT-2 T Cells) In Vitro into Th1T Cells by Antigen-Presenting Extracellular Vesicles

The following test was conducted in vitro to determine whether the antigen-presenting extracellular vesicles induce differentiation of antigen-specific CD4-positive T cells into Th1T cells.

Lymph nodes extracted from an OT-2 mouse, which was an OVA-reactive CD4TCR transgenic mouse, were disrupted on a 100 µm filter to obtain a lymph node cell suspension. The cell suspension was stained using CellTrace Violet (manufactured by Thermo Fisher Scientific Inc.) as a cell proliferation assay reagent according to the manufacturer's instructions. 2 × 10⁵ stained lymph node cells were suspended in 200 µL of an RPMI1640 medium to which 10% fetal bovine serum, 50 µM 2-mercaptoethanol, and penicillin/streptomycin were added, the antigen-presenting extracellular vesicles of Example 3 or 6 or the extracellular vesicles of Reference Example 1 were added so that the final concentration was 10 µg/mL, and culture was performed in a 96 well round bottom plate for 4 days. After 4 days, the cells were recovered, and extracellular immunostaining was performed. Antibodies used for staining are as follows (staining time: 15 minutes, temperature: 4°C). After the extracellular staining, intracellular immunostaining was performed using True-Nuclear Transcription Factor Buffer Set (manufactured by Biolegend, Inc.) and anti-T-bet antibodies according to the manufacturer's instructions. After the intracellular staining, a luminescence intensity of CellTrace Violet as a cell proliferation assay reagent in the OT-2 T cells and expression of T-bet as a marker of Th1T cells were detected with a flow cytometer FACSCantoII (manufactured by BD Biosciences).
- PerCP/Cy5.5-conjugated anti-mouse TCRVa2 antibody (B20.1, manufactured by Biolegend, Inc.)
- APC-Cy7-conjugated anti-mouse CD4 antibody (RM4-5, manufactured by Biolegend, Inc.)
- PE-conjugated anti-T-bet antibody (4B10, manufactured by Biolegend, Inc.) The results are illustrated in Fig. 9.

### [Results]

From the results of Test Example 7, it was confirmed that the antigen-presenting extracellular vesicles of Example 6 induced differentiation of antigen-specific CD4-positive T cells into Th1 cells in vitro in comparison with the extracellular vesicles of Reference Example 1 (Fig. 9). The Th1 cells produce IFN-γ, IL-2, or the like, and promote activation of macrophages and cytotoxic T cells that destroy pathogen cells, virus-infected cells, cancer cells, and the like (that is, activation of cellular immunity).

### Test Example 8: Experiment on Differentiation Induction of OVA-Specific CD4-Positive T Cells (OT-2 T Cells) In Vitro into Thl7T Cells by Antigen-Presenting Extracellular Vesicles

The following test was conducted in vitro to determine whether the antigen-presenting extracellular vesicles induce differentiation of antigen-specific CD4-positive T cells into Th17T cells.

Lymph nodes extracted from a mouse obtained by mating an OVA-reactive CD4TCR transgenic mouse and an RORrt-GFP mouse were disrupted on a 100 µm filter to obtain a lymph node cell suspension. The cell suspension was stained using CellTrace Violet (manufactured by Thermo Fisher Scientific Inc.) as a cell proliferation assay reagent according to the manufacturer's instructions. 2 × 10⁵ stained lymph node cells were suspended in 200 µL of an RPMI1640 medium to which 10% fetal bovine serum, 50 µM 2-mercaptoethanol, and penicillin/streptomycin were added, the antigen-presenting vesicles of Example 7 or the extracellular vesicles of Reference Example 1 were added so that the final concentration was 10 µg/mL, and culture was performed in a 96 well round bottom plate for 4 days. After 4 days, the cells were recovered, and extracellular immunostaining was performed. Antibodies used for staining are as follows (staining time: 15 minutes, temperature: 4°C). After the intracellular staining, a luminescence intensity of CellTrace Violet as a cell proliferation assay reagent in the OT-2 T cells and expression of RORrt as a marker of Th17T cells were detected with a flow cytometer FACSCantoII (manufactured by BD Biosciences).
- APC-conjugated anti-mouse TCRVa2 antibody (B20.1, manufactured by Biolegend, Inc.)
- PE-Cy7-conjugated anti-mouse CD4 antibody (RM4-5, manufactured by Biolegend, Inc.)
- PE-conjugated anti-mouse TCR Vb5.1,5.2 antibody (MR9-4, manufactured by Biolegend, Inc.)

The results are illustrated in Fig. 10.

### [Results]

From the results of Test Example 8, it could be seen that the antigen-presenting extracellular vesicles of Example 7 induced differentiation of antigen-specific CD4-positive T cells into Th17 cells in vitro in comparison with the extracellular vesicles of Reference Example 1 (Fig. 10). Unlike the Th1 or Th2 cells, the Th17 cells produce inflammatory cytokines such as IL-17, IL-21, IL-22, and TNF-α to induce inflammation, promote recruitment and proliferation of neutrophils and monocytes, and contribute to infection defense of fungi (including pathogenic fungi such as candida, *Staphylococcus aureus,* and *Streptococcus pyogenes*).

### Test Example 9: Experiment on Activation of OVA-Specific CD8-Positive T Cells (OT-1 T Cells) In Vitro by Antigen-Presenting Extracellular Vesicles Obtained by Purification of Stable Cell Strain

The following test was conducted in vitro to determine whether the antigen-presenting extracellular vesicles obtained by purification of a stable cell line activate antigen-specific CD8-positive T cells.

Lymph nodes extracted from an OT-1 mouse, which was an OVA-reactive TCR transgenic mouse, were disrupted on a 100 µm filter to obtain a lymph node cell suspension. The cell suspension was stained using CellTrace Violet (manufactured by Thermo Fisher Scientific Inc.) as a cell proliferation assay reagent according to the manufacturer's instructions. 2 × 10⁵ stained lymph node cells were suspended in 200 µL of an RPMI1640 medium to which 10% fetal bovine serum, 50 µM 2-mercaptoethanol, and penicillin/streptomycin were added, the antigen-presenting extracellular vesicles of Example 1 or 8 or the extracellular vesicles of Reference Example 1 were added so that the final concentration was 9 µg/mL, and culture was performed in a 96 well round bottom plate for 4 days. After the culture in the 96 well round bottom plate for 3 days, immunostaining was performed. Antibodies used for staining are as follows (staining time: 15 minutes, temperature: 4°C). After the staining, a luminescence intensity of CellTrace Violet as a cell proliferation assay reagent in the OT-1 T cells was detected with a flow cytometer FACSCantoII (manufactured by BD Biosciences).
- APC-conjugated anti-mouse CD8 antibody (53-6.7, manufactured by Biolegend, Inc.)
- PE-conjugated anti-mouse TCR Vb5.1,5.2 antibody (MR9-4, manufactured by Biolegend, Inc.)

The results are illustrated in Fig. 11.

### [Results]

From the results of Test Example 9, it was confirmed that the antigen-presenting extracellular vesicles of Examples 1 and 8 remarkably proliferated antigen-specific CD8-positive T cells in comparison with the extracellular vesicles of Reference Example 1 (Fig. 11). This indicates that not only the extracellular vesicles in which the constitutional requirement (A) exemplified by sc-Trimer-CD81 and the constitutional requirement (B) exemplified by CD81-IL-2 are present as different proteins, but also the extracellular vesicles in which a fusion protein having both functions, which is exemplified by sc-Trimer-CD81-IL-2, is present, exhibit equivalent effects on T cells.

### Test Example 10: Experiment for Evaluating Anti-Tumor Effect by Antigen-Presenting Extracellular Vesicles

In order to determine whether the antigen-presenting extracellular vesicles obtained by purification of a stable cell strain have an anti-tumor effect, 1 × 10⁵ B16 melanoma cells expressing OVA were subcutaneously ingested in a CD45.1/CD45.2 congenic mouse, and 1 × 10⁵ OT-1T cells were transferred after 3 days. After 1 day, 4 days, and 7 days after the OT-1T cell transfer, 50 µg of the antigen-presenting extracellular vesicles of Example 8 or the extracellular vesicles of Reference Example 1 were transferred from the tail vein of the recipient mouse, and the size of the B16 melanoma cells was observed.

### [Results]

From the results of Test Example 10, it was confirmed that the antigen-presenting extracellular vesicles of Example 8 remarkably suppressed proliferation of B16 melanoma cells in comparison with the extracellular vesicles of Reference Example 1 (Fig. 12). This indicates that not only the extracellular vesicles in which the constitutional requirement (A) exemplified by sc-Trimer-CD81 and the constitutional requirement (B) exemplified by CD81-IL-2 are present as different proteins, but also the extracellular vesicles in which a fusion protein having both functions, which is exemplified by sc-Trimer-CD81-IL-2, is present, exhibit equivalent medicinal effects.

### [Example 10] Preparation of mRNA Expressing sc-Trimer-CD81-IL-2 Fusion Protein

A pET-15b vector encoding sc-Trimer-CD81-IL-2 was linearized using EagI, the linearized vector was purified using a FastGene Gel/PCR extraction kit (NIPPON Genetics Co., Ltd.), and transcription, capping, and polyA addition were performed on the purified vector in vitro using T7 mScript Standard mRNA Production System (manufactured by CELLSCRIPT, LLC) according to the manufacturer's instructions. The synthesized mRNA was used as RNA producing antigen-presenting cells and antigen-presenting extracellular vesicles of Example 10 (Fig. IN).

### Reference Example 6: Preparation of mRNA Expressing CD63-AkaLuc Fusion Protein

A pET-15b vector encoding CD63-AkaLuc was linearized using EcoRI, the linearized vector was purified using a FastGene Gel/PCR extraction kit (NIPPON Genetics Co., Ltd.), and transcription, capping, and polyA addition were performed on the purified vector in vitro using T7 mScript Standard mRNA Production System (manufactured by CELLSCRIPT, LLC) according to the manufacturer's instructions. The synthesized mRNA was used as a control RNA of Reference Example 6 (Fig. 10).

### Test Example 11: Experiment on Activation of OVA-Specific CD8-Positive T Cells (OT-1 T Cells) In Vivo by mRNA Expressing sc-Trimer-CD81-IL-2 Fusion Protein

The following test was conducted in vivo to determine whether mRNA expressing a sc-Trimer-CD81-IL-2 fusion protein activated antigen-specific CD8-positive T cells.

Lymph nodes extracted from an OT-1 mouse, which was an OVA-reactive TCR transgenic mouse, were disrupted on a 100 µm filter to obtain a lymph node cell suspension. Lymph nodes were similarly extracted from a CD45.1 congenic mouse, and a lymphocyte suspension was prepared. The respective lymphocyte suspensions were mixed at a ratio of 1:1, and the mixture was stained using CellTrace Violet as a cell proliferation assay reagent. 1 × 10⁷ CellTrace Violet-stained mixed lymphocyte suspension suspended in PBS was transferred from the tail vein of the CD45.1/CD45.2 congenic mouse. The next day, 10 µg of mRNA of Example 10 or Reference Example 6 was mixed with an in vivo-jetRNA transfection reagent (Polyplus-transfection SA) according to the manufacturer's instructions, and the mixture was transferred from a tail vein to a CD45.1/CD45.2 congenic mouse. 4 days after cell transfer, the spleen was extracted from the recipient mouse, and a lymphocyte suspension was prepared and immunostained. Antibodies used for staining are as follows (staining time: 15 minutes, temperature: 4°C). After the staining, a luminescence intensity of CellTrace Violet as a cell proliferation assay reagent in the transferred OT-1 T cells and wild-type CD8T cells was detected with a flow cytometer FACSCantoII (manufactured by BD Biosciences).
- PE-Cy7-conjugated anti-mouse CD8 antibody (53-6.7, manufactured by Biolegend, Inc.)
- PE-conjugated anti-mouse TCR Vb5.1,5.2 antibody (MR9-4, manufactured by Biolegend, Inc.)
- FITC-conjugated anti-mouse CD45.1 antibody (A20, manufactured by Biolegend, Inc.)
- APC-conjugated anti-mouse CD45.2 antibody (104, manufactured by Biolegend, Inc.)

The results are illustrated in Fig. 13.

### [Results]

From the results of Test Example 11, it was confirmed that mRNA of Example 10 hardly activated other CD8-positive T cells (antigen-non-specific CD8-positive T cells) and remarkably differentiated and/or proliferated antigen-specific CD8-positive T cells in vivo in comparison with mRNA of Reference Example 6 (Fig. 13). Although not particularly limited to the principle, it is understood that the polynucleotide according to the present invention is introduced into any cell in the CD45.1/CD45.2 congenic mouse, a sc-Trimer-CD81-IL-2 fusion protein is expressed on the membrane surface of the cell and/or the membrane surface of the extracellular vesicle secreted from the cell to produce antigen-presenting cells and/or antigen-presenting extracellular vesicles, and the produced antigen-presenting cells and/or antigen-presenting extracellular vesicles are brought into contact with OT-1 T cells to proliferate OVA-reactive CD8T cells. This indicates that an effect equivalent to the effect of the T cell activation of the antigen-presenting extracellular vesicles shown in Test Examples 1 to 10 can also be obtained in vivo by the polynucleotide for producing antigen-presenting extracellular vesicles.

### Test Example 12: Experiment on Activation of Intrinsic OVA-Reactive T Cells by mRNA Expressing sc-Trimer-CD81-IL-2 Fusion Protein

10 µg of mRNA of Example 10 or Reference Example 6 was mixed with an in vivo-jetRNA transfection reagent (Polyplus-transfection SA) according to the manufacturer's instructions, and the mixture was transferred from a tail vein of a C57BL/6 mouse. 4 days after transfer, the spleen was extracted from the recipient mouse, a lymphocyte suspension was prepared, and OVA-reactive T cells were immunostained with a tetramer according to the manufacturer's instructions. The antibodies used for the staining are as follows. After the staining, tetramer-positive cells were detected with a flow cytometer FACSCantoII (manufactured by BD Biosciences).
- APC-conjugated H-2Kb OVA Tetramer (Tetramer Shop ApS)
- PE-conjugated H-2Kb OVA Tetramer (Tetramer Shop ApS)
- Brilliant Violet421-conjugated anti-mouse CD8 antibody (53-6.7, manufactured by Biolegend, Inc.)

### [Results]

From the results of Test Example 12, it was confirmed that mRNA of Example 10 remarkably proliferated the OVA-reactive CD8T cells that were intrinsically present in comparison with mRNA of Reference Example 6 (Fig. 14). Although not particularly limited to the principle, it is understood that the polynucleotide according to the present invention is introduced into any cell in the C57BL/6 congenic mouse, a sc-Trimer-CD81-IL-2 fusion protein is expressed on the membrane surface of the cell and/or the membrane surface of the extracellular vesicle secreted from the cell to produce antigen-presenting cells and/or antigen-presenting extracellular vesicles, and the produced antigen-presenting cells and/or antigen-presenting extracellular vesicles contact with intrinsic T cells to proliferate OVA-reactive CD8T cells. This indicates that an effect equivalent to the effect of the T cell activation of the antigen-presenting extracellular vesicles as a pharmaceutical shown in Test Examples 1 to 10 can also be obtained in vivo by the polynucleotide for producing antigen-presenting extracellular vesicles.

Hereinabove, as described in the examples, the antigen-presenting cells and the polynucleotide for producing antigen-presenting extracellular vesicles described in the present specification can satisfactorily activate, proliferate, and/or differentiate antigen-specific T cells (for example, antigen-specific CD8-positive T cells, antigen-specific CD4-positive cells, and the like).

Hereinafter, a summary of the sequences included in the sequence listing will be described.

**[Table 15]**

| | SEQ ID NO: | |
|---|---|---|
| | Amino acid sequence | Polynucleotide |
| Signal peptide of *β* ₂ microglobulin | 1 | 2 |
| *β* ₂ Microglobulin (from which signal peptide is removed) | 7 | 8 |
| M H C class I *α* chain (from which signal peptide is removed) | 9 | 10 |
| Signal peptide of MH C class II *β* chain | 33 | 34 |
| MHC class II *β* chain (from which signal peptide is removed) | 37 | 38 |
| MHC class II *α* chain (full length) | 45 | 46 |
| MHC class II *α* chain (from which signal peptide is removed) | 71 | 72 |

**[Table 16]**

| | SEQ ID NO: | |
|---|---|---|
| | Amino acid sequence | Polynucleotide |
| O V A peptide 1 (for MHC class I molecule) | 3 | 4 |
| O V A peptide 2 (for MHC class II molecule) | 35 | 36 |

**[Table 17]**

| | SEQ ID NO: | |
|---|---|---|
| | Amino acid sequence | Polynucleotide |
| CD9 (full length) | 21 | 22 |
| CD63 (full length) | 27 | 28 |
| CD63 (partial sequence containing TM1 to TM3 ) | 57 | 58 |
| CD63 (partial sequence containing TM4 ) | 59 | 60 |
| CD81 (full length) | 15 | 16 |
| CD81 (partial sequence containing TM1 to TM3 ) | 61 | 62 |
| C D81 (partial sequence containing TM4 ) | 63 | 64 |
| MFG - E8 (from which signal peptide is removed) | 49 | 50 |

**[Table 18]**

| | SEQ ID NO: | |
|---|---|---|
| | Amino acid sequence | Polynucleotide |
| IL - 2 (from which signal peptide is removed) | 25 | 26 |
| IL - 4 (from which signal peptide is removed) | 53 | 54 |
| TGF - *β* 1 (full length) | 47 | 48 |
| TGF - *β* 1 (from which signal peptide is removed) | 73 | 74 |

**[Table 19]**

| | SEQ ID NO: | |
|---|---|---|
| | Amino acid sequence | Polynucleotide |
| CD80 (full length) | 19 | 20 |
| CD80 (from which signal peptide is removed) | 67 | 68 |

**[Table 20]**

| | SEQ ID NO: | |
|---|---|---|
| | Amino acid sequence | Polynucleotide |
| Peptide linker 1 | 5 | 6 |
| Peptide linker 2 | 1 | 12 |
| Peptide linker 3 | 29 | 30 |
| Peptide linker 4 | 39 | 40 |
| Peptide linker 5 | 77 | 78 |

**[Table 21]**

| | SEQ ID NO: | |
|---|---|---|
| | Amino acid sequence | Polynucleotide |
| Single chain MH C class I molecule ( *β* ₂ microglobulin + MHC class I *α* chain) | 65 | 66 |
| sc - Trimer (O V A peptide 1 + single chain MHC class I molecule) | 13 | 14 |
| sc - Trimer + CD81 (full length) | 17 | 18 |
| sc - Dimer (OVA peptide 2 + MHC class II *β* chain) | 41 | 42 |
| sc -Dimer + C D 8 1 (full length) | 43 | 44 |
| CD63 - IL - 2 | 31 | 32 |
| CD81 - IL - 4 | 55 | 56 |
| TGF - *β* 1 (full length) + MGF - E8 (from which signal peptide is removed) | 51 | 52 |
| TGF - *β* 1 (from which signal peptide is removed) + MG F - 8E (from which signal peptide is removed) | 75 | 76 |
| CD80 (full length) + CD9 (full length) | 23 | 24 |
| CD80 (from which signal peptide is removed) + CD9 (full length) | 69 | 70 |

**[Table 22]**

| | SEQ ID N0: | |
|---|---|---|
| | Amino acid sequence | Polynucleotide |
| PNE tag | 79 | 80 |
| Anti- PNE tag nanobody (full length) | 81 | 82 |
| Anti- PNE tag nanobody (from which signal peptide is removed) | 83 | 84 |
| CD8a | 85 | 86 |
| Anti- PNE tag nanobody (full length) + CD8a + CD81 (full length) | 87 | 88 |
| Anti- PNE tag nanobody (from which signal peptide is removed) + CD8a + CD81 (full length) | 89 | 90 |

**[Table 23-1]**

| | SEQ ID NO: | |
|---|---|---|
| | Amino acid sequence | Polynucleotide |
| sc-Dimer-CD81-IL-12α | 91 | 92 |
| IL-12α (from which signal peptide is removed) | 93 | 94 |
| CD81-IL-12α | 95 | 96 |
| IL-12β | 97 | 98 |
| IL-6 (from which signal peptide is removed) | 99 | 100 |
| CD81-IL-6 | 101 | 102 |
| hCD80 | 103 | 104 |
| hCD9 | 105 | 106 |
| hCD80-hCD9 | 107 | 108 |
| hIL-2 (from which signal peptide is removed) | 109 | 110 |
| hCD63 (amino acids 1 to 170) | 111 | 112 |
| hCD63 (amino acids 171 to 238) | 113 | 114 |
| hCD63 - IL - 2 | 115 | 116 |
| Signal peptide of h β2 microglobulin | 117 | 118 |
| WT 1 PEPTIDE 1 (for MHC class I molecule) | 119 | 120 |
| hβ2 Microglobulin (from which signal peptide is removed) | 121 | 122 |
| hMHC class I a chain (from which signal peptide is removed) | 123 | 124 |
| h single chain M H C class I molecule (β2 microglobulin (from which signal peptide is removed) + peptide linker 2 + MHC class I α chain (from which signal peptide is removed)) | 125 | 126 |
| hsc-Trimer (WT 1 peptide 1 + peptide linker 1 + single chain MHC class I molecule) | 127 | 128 |

**[Table 23-2]**

| | | |
|---|---|---|
| hCD81 | 129 | 130 |
| hsc-Trimer-CD81 (sc-Trimer+CD81) | 131 | 132 |
| CD81-IL-2 | 133 | 134 |
| sc-Trimer-CD81-IL-2 (sc-Trimer+CD81) | 135 | 136 |
| Aka-Luc | 137 | 138 |
| CD63 -Aka-Luc | 139 | 140 |
| SARS-CoV2 peptide 1 | 141 | 142 |
| hMHC class I (HLA-A0201) α chain (from which signal peptide is removed) | 143 | 144 |
| h single chain MHC class I molecule (β2 microglobulin (from which signal peptide is removed) + peptide linker 2 + MHC class I (HLA-A0201) α chain (from which signal peptide is removed)) | 145 | 146 |
| hsc-Trimer (SARS-CoV2 peptide 1 + peptide linker 1 + single chain MHC class I (HLA-A0201) molecule) | 147 | 148 |
| hsc-Trimer-CD81 (SARS-CoV2sc-Trimer + CD81 ) | 149 | 150 |

## Claims

1. A polynucleotide comprising at least one sequence selected from the group consisting of:
(a) a sequence encoding a fusion protein (A) which comprises an antigen-presenting MHC molecule and is capable of presenting the antigen-presenting MHC molecule outside membrane of an extracellular vesicle;
(b) a sequence encoding a fusion protein (B) which comprises at least one T-cell stimulatory cytokine or subunit thereof and is capable of presenting the T-cell stimulatory cytokine outside membrane of an extracellular vesicle;
(c) a sequence encoding a fusion protein (C) which comprises a T-cell costimulatory molecule and is capable of presenting the T-cell costimulatory molecule outside membrane of an extracellular vesicle;
(d) a sequence encoding a fusion protein (D) which comprises an antigen-presenting MHC molecule and at least one T-cell stimulatory cytokine or subunit thereof and is capable of presenting the antigen and the T-cell stimulatory cytokine outside membrane of an extracellular vesicle; and
(e) a sequence encoding a fusion protein (E) which comprises an antigen-presenting MHC molecule, at least one T-cell stimulatory cytokine or subunit thereof, and a T-cell costimulatory molecule, and is capable of presenting the antigen, the T-cell stimulatory cytokine, and the T-cell costimulatory molecule outside membrane of an extracellular vesicle.

2. The polynucleotide according to claim 1, wherein the fusion protein defined as (A) contains an antigen-presenting MHC molecule, and a membrane protein capable of being expressed in membrane of an extracellular vesicle or a transmembrane domain thereof or a protein capable of binding to membrane of an extracellular vesicle or a domain thereof.

3. The polynucleotide according to claim 1, wherein the fusion protein defined as (B) contains at least one T-cell stimulatory cytokine or subunit thereof, and membrane protein capable of being expressed in membrane of an extracellular vesicle or a transmembrane domain thereof or a protein capable of binding to membrane of an extracellular vesicle or a domain thereof.

4. The polynucleotide according to claim 1, wherein the fusion protein defined as (A) contains an antigen-presenting MHC molecule, and a tetraspanin or a transmembrane domain thereof or MFG-E8 or a domain thereof.

5. The polynucleotide according to claim 1, wherein the fusion protein defined as (B) comprises at least one T-cell stimulatory cytokine or subunit thereof and a partial sequence of a tetraspanin, the partial sequence of the tetraspanin contains at least two transmembrane domains, and the at least one T-cell stimulatory cytokine is disposed between the two transmembrane domains.

6. The polynucleotide according to claim 1, wherein the fusion protein defined as (B) comprises at least one T-cell stimulatory cytokine or subunit thereof and MFG-E8 or a domain thereof.

7. The polynucleotide according to claim 1, wherein the fusion protein defined as (A) comprises an amino acid sequence consisting of, from an N-terminal side thereof,
(A-1) an MHC molecule-restricted antigen peptide,
(A-2) a spacer sequence which may be optionally present,
(A-3) a single chain MHC molecule,
(A-4) a spacer sequence which may be optionally present, and
(A-5) a tetraspanin.

8. The polynucleotide according to claim 1, wherein the fusion protein defined as (A) comprises an amino acid sequence consisting of, from an N-terminal side thereof,
(A-1) an MHC molecule-restricted antigen peptide,
(A-2) a spacer sequence which may be optionally present,
(A-3) an MHC class Iα chain, β₂ microglobulin, an MHC class IIα chain, or an MHC class IIβ chain,
(A-4) a spacer sequence which may be optionally present, and
(A-5) a tetraspanin.

9. The polynucleotide according to claim 8, wherein an amino acid sequence of β₂ microglobulin, an MHC class Iα chain, an MHC class IIβ chain, or an MHC class IIα chain is further comprised in the fusion protein.

10. The polynucleotide according to claim 1, wherein the fusion protein defined as (B) comprises an amino acid sequence consisting of, from an N-terminal side thereof,
(B-1) a partial sequence of a tetraspanin containing, from an N-terminal side thereof, a transmembrane domain 1, a small extracellular loop, a transmembrane domain 2, a small intracellular loop, and a transmembrane domain 3,
(B-2) a spacer sequence which may be optionally present,
(B-3) a first T-cell stimulatory cytokine,
(B-4) a spacer sequence which may be optionally present, and
(B-5) a partial sequence of a tetraspanin containing a transmembrane domain 4.

11. The polynucleotide according to claim 1, wherein the fusion protein defined as (B) comprises an amino acid sequence consisting of, from an N-terminal side thereof,
(B-3) a first T-cell stimulatory cytokine,
(B-4) a spacer sequence which may be optionally present, and
(B-5) MFG-E8.

12. The polynucleotide according to claim 1, wherein the fusion protein defined as (A) comprises an amino acid sequence consisting of, from an N-terminal side thereof,
(A-1) an MHC class I molecule-restricted antigen peptide,
(A-2) a spacer sequence which may be optionally present,
(A-3) a single chain MHC class I molecule,
(A-4) a spacer sequence which may be optionally present, and
(A-5) a tetraspanin.

13. The polynucleotide according to claim 1, wherein the fusion protein defined as (A) comprises an amino acid sequence consisting of, from an N-terminal side thereof,
(A-1) an MHC class II molecule-restricted antigen peptide,
(A-2) a spacer sequence which may be optionally present,
(A-3) an MHC class IIβ chain,
(A-4) a spacer sequence which may be optionally present, and
(A-5) a tetraspanin.

14. The polynucleotide according to claim 13, wherein (A-6) an amino acid sequence of an MHC class IIα chain is further comprised.

15. The polynucleotide according to claim 1, wherein the T-cell stimulatory cytokine is IL-2, IL-4, IL-6, IL-12, a subunit of IL-12, or TGF-β.

16. The polynucleotide according to claim 1, wherein the fusion protein defined as (C) comprises a T-cell costimulatory molecule, and membrane protein capable of being expressed in membrane of an extracellular vesicle or a transmembrane domain thereof or a protein capable of binding to membrane of an extracellular vesicle or a domain thereof.

17. The polynucleotide according to claim 1, wherein the fusion protein defined as (C) comprises a T-cell costimulatory molecule, and a tetraspanin or a transmembrane domain thereof or MFG-E8 or a domain thereof.

18. The polynucleotide according to claim 1, wherein the fusion protein defined as (C) comprises an amino acid sequence consisting of, from an N-terminal side thereof,
(C-1) a T-cell costimulatory molecule,
(C-2) a spacer sequence which may be optionally present, and
(C-3) a tetraspanin.

19. The polynucleotide according to claim 1, wherein the fusion protein defined as (D) comprises the antigen-presenting MHC molecule, the at least one T-cell stimulatory cytokine or subunit thereof, and membrane protein capable of being localized to membrane of an extracellular vesicle or a transmembrane domain thereof or a protein capable of binding to membrane of an extracellular vesicle or a membrane-binding domain thereof.

20. The polynucleotide according to claim 19, wherein the membrane protein capable of being localized onto membrane of an extracellular vesicle or the protein capable of binding to membrane of an extracellular vesicle is a tetraspanin or MFG-E8.

21. The polynucleotide according to claim 1, wherein the fusion protein defined as (D) comprises an amino acid sequence encoding, from an N-terminal side thereof,
(D-1) an MHC molecule-restricted antigen peptide,
(D-2) a spacer sequence which may be optionally present,
(D-3) a single chain MHC molecule,
(D-4) a spacer sequence which may be optionally present, and
(D-5) a fusion peptide comprising a tetraspanin or a transmembrane domain thereof or MFG-E8 or a transmembrane domain thereof, and the at least one T-cell stimulatory cytokine or subunit thereof, in this order.

22. The polynucleotide according to claim 1, wherein the fusion protein defined as (D) comprises an amino acid sequence encoding, from an N-terminal side thereof,
(D-1) a fusion peptide comprising a tetraspanin or a transmembrane domain thereof or MFG-E8 or a transmembrane domain thereof, and the at least one T-cell stimulatory cytokine or subunit thereof,
(D-2) a spacer sequence which may be optionally present,
(D-3) a single chain MHC molecule,
(D-4) a spacer sequence which may be optionally present, and
(D-5) an MHC molecule-restricted antigen peptide, in this order.

23. The polynucleotide according to claim 21 or 22, wherein the fusion peptide comprising a tetraspanin or a transmembrane domain thereof or MFG-E8 or a transmembrane domain thereof, and the at least one T-cell stimulatory cytokine or subunit thereof comprises an amino acid sequence encoding, from an N-terminal side thereof,
(1) a partial sequence of a tetraspanin containing a transmembrane domain 1, a small extracellular loop, a transmembrane domain 2, a small intracellular loop, and a transmembrane domain 3,
(2) a spacer sequence which may be optionally present,
(3) the at least one T-cell stimulatory cytokine or subunit thereof,
(4) a spacer sequence which may be optionally present, and
(5) a partial sequence of a tetraspanin containing a transmembrane domain 4, in this order.

24. The polynucleotide according to claim 21 or 22, wherein the fusion peptide comprising a tetraspanin or a transmembrane domain thereof or MFG-E8 or a transmembrane domain thereof, and the at least one T-cell stimulatory cytokine or subunit thereof contains an amino acid sequence encoding, from an N-terminal side thereof,
(1) the at least one T-cell stimulatory cytokine or subunit thereof,
(2) a spacer sequence which may be present, and
(3) MFG-E8, in this order.

25. The polynucleotide according to claim 21 or 22, wherein the MHC molecule-restricted antigen peptide is an MHC class I molecule-restricted antigen peptide, and the single chain MHC molecule contains an extracellular domain of an MHC class Iα chain.

26. The polynucleotide according to claim 21 or 22, wherein the MHC molecule-restricted antigen peptide is an MHC class II molecule-restricted antigen peptide, and the single chain MHC molecule comprises an extracellular domain of an MHC class IIα chain and/or an extracellular domain of an MHC class IIβ chain.

27. The polynucleotide according to claim 1, comprising the sequence defined as (a) and the sequence defined as (b).

28. The polynucleotide according to claim 27, further comprising the sequence defined as (c).

29. The polynucleotide according to claim 1, comprising the sequence defined as (d).

30. The polynucleotide according to claim 29, further comprising the sequence defined as (c).

31. The polynucleotide according to claim 1, comprising the sequence defined as (e).

32. A vector comprising the polynucleotide according to any one of claims 1 to 31.

33. A pharmaceutical composition comprising the polynucleotide according to any one of claims 1 to 31 or the vector according to claim 32, and a pharmacologically acceptable carrier.

34. A pharmaceutical composition for treating and preventing an infectious disease, comprising the polynucleotide according to any one of claims 1 to 31 or the vector according to claim 32, and a pharmacologically acceptable carrier.

35. A pharmaceutical composition for treating or preventing cancer, comprising the polynucleotide according to any one of claims 1 to 31 or the vector according to claim 32.

36. A pharmaceutical composition for treating or preventing an autoimmune disease, comprising the polynucleotide according to any one of claims 1 to 31 or the vector according to claim 32.

37. A pharmaceutical composition for treating or preventing an allergic disease, comprising the polynucleotide according to any one of claims 1 to 31 or the vector according to claim 32.

38. A method for activating and/or proliferating T cells against a specific antigen, the method comprising:
introducing the polynucleotide according to any one of claims 1 to 31 or the vector according to claim 32 to cells in vitro or ex vivo to produce antigen-presenting cells and/or antigen-presenting extracellular vesicles; and
contacting the produced antigen-presenting cells and/or antigen-presenting extracellular vesicles with T cells in vitro or ex vivo.
